# EUROPEAN PATENT SPECIFICATION

(11) **EP 2 939 022 B1**
(45) Date of publication and mention of the grant of the patent: **09.09.2020**
(21) Application number: 13867314.0
(22) Date of filing: 26.12.2013
(51) Int. Cl.: G01N 33/50, G01N 33/68, G01N 33/566

(54) **BIOMARKERS OF PRETERM BIRTH**
BIOMARKER EINER FRÜHGEBURT
BIOMARQUEURS DE LA NAISSANCE PRÉMATURÉE

(30) Priority: 28.12.2012 US 201261747150 P; 12.03.2013 US 201313797933
(43) Date of publication of application: 04.11.2015
(73) Proprietor: NX PRENATAL INC., Louisville, KY 40207 (US)
(72) Inventor: EZRIN, Alan, M., Miami, FL 33133 (US); BROHMAN, Brian, D., Prospect, KY 40059 (US)
(74) Representative: Cooley (UK) LLP
(86) International application number: PCT/US2013/077868
(87) International publication number: WO 2014/105985

(56) References cited:
- WO-A1-2008/056114
- WO-A1-2008/098734
- WO-A1-2009/031721
- WO-A2-2008/063928
- WO-A2-2008/063928
- WO-A2-2012/174282
- US-A1- 2011 236 953
- US-A1- 2012 021 442
- US-A1- 2012 021 442
- TITA A T N ET AL: "Progesterone for preterm birth prevention: an evolving intervention", AMERICAN JOURNAL OF OBSTETRICS & GYNECOLOGY, MOSBY, ST LOUIS, MO, US, vol. 200, no. 3, 1 March 2009 (2009-03-01) , pages 219-224, XP026069819, ISSN: 0002-9378, DOI: 10.1016/J.AJOG.2008.12.035 [retrieved on 2009-02-28]
- SINGH, PP ET AL.: 'Exosomes Isolated From Mycobacteria-Infected Mice Or Cultured Macrophages Can Recruit And Activate Immune Cells In Vitro And In Vivo.' JOURNAL OF IMMUNOLOGY vol. 189, no. 2, 20 June 2012, pages 777 - 785, XP055259142
- PEREIRA LEONARDO ET AL: "Identification of novel protein biomarkers of preterm birth in human cervical-vaginal fluid", JOURNAL OF PROTEOME RESEARCH,, vol. 6, no. 4, 1 January 2007 (2007-01-01) , pages 1269-1276, XP002485466, ISSN: 1535-3893, DOI: 10.1021/PR0605421 [retrieved on 2007-03-21]

### SEQUENCE LISTING

<110> NX Pharmagen
   Ezrin, Alan
   Brohman, Brian
<120> Biomarkers of Preterm Birth
<130> 674982000840
<150> US 13/797,933
   <151> 2013-03-12
<150> US 61/747,150
   <151> 2012-12-28
<160> 100
<170> FastSEQ for Windows Version 4.0
<210> 1
   <211> 1474
   <212> PRT
   <213> Homo sapiens
<400> 1
   Met Gly Lys Asn Lys Leu Leu His Pro Ser Leu Val Leu Leu Leu Leu
    1               5                  10                  15
   Val Leu Leu Pro Thr Asp Ala Ser Val Ser Gly Lys Pro Gln Tyr Met
               20                  25                  30
   Val Leu Val Pro Ser Leu Leu His Thr Glu Thr Thr Glu Lys Gly Cys
           35                  40                  45
   Val Leu Leu Ser Tyr Leu Asn Glu Thr Val Thr Val Ser Ala Ser Leu
       50                  55                  60
   Glu Ser Val Arg Gly Asn Arg Ser Leu Phe Thr Asp Leu Glu Ala Glu
   65                  70                  75                  80
   Asn Asp Val Leu His Cys Val Ala Phe Ala Val Pro Lys Ser Ser Ser
                   85                  90                  95
   Asn Glu Glu Val Met Phe Leu Thr Val Gln Val Lys Gly Pro Thr Gln
               100                 105                 110
   Glu Phe Lys Lys Arg Thr Thr Val Met Val Lys Asn Glu Asp Ser Leu
           115                 120                 125
   Val Phe Val Gln Thr Asp Lys Ser Ile Tyr Lys Pro Gly Gln Thr Val
       130                 135                 140
   Lys Phe Arg Val Val Ser Met Asp Glu Asn Phe His Pro Leu Asn Glu
   145                 150                 155                 160
   Leu Ile Pro Leu Val Tyr Ile Gln Asp Pro Lys Gly Asn Arg Ile Ala
                   165                 170                 175
   Gln Trp Gln Ser Phe Gln Leu Glu Gly Gly Leu Lys Gln Phe Ser Phe
               180                 185                 190
   Pro Leu Ser Ser Glu Pro Phe Gln Gly Ser Tyr Lys Val Val Val Gln
           195                 200                 205
   Lys Lys Ser Gly Gly Arg Thr Glu His Pro Phe Thr Val Glu Glu Phe
       210                 215                 220
   Val Leu Pro Lys Phe Glu Val Gln Val Thr Val Pro Lys Ile Ile Thr
   225                 230                 235                 240
   Ile Leu Glu Glu Glu Met Asn Val Ser Val Cys Gly Leu Tyr Thr Tyr
                   245                 250                 255
   Gly Lys Pro Val Pro Gly His Val Thr Val Ser Ile Cys Arg Lys Tyr
               260                 265                 270
   Ser Asp Ala Ser Asp Cys His Gly Glu Asp Ser Gln Ala Phe Cys Glu
           275                 280                 285
   Lys Phe Ser Gly Gln Leu Asn Ser His Gly Cys Phe Tyr Gln Gln Val
       290                 295                 300
   Lys Thr Lys Val Phe Gln Leu Lys Arg Lys Glu Tyr Glu Met Lys Leu
   305                 310                 315                 320
   His Thr Glu Ala Gln Ile Gln Glu Glu Gly Thr Val Val Glu Leu Thr
                   325                 330                 335
   Gly Arg Gln Ser Ser Glu Ile Thr Arg Thr Ile Thr Lys Leu Ser Phe
               340                 345                 350
   Val Lys Val Asp Ser His Phe Arg Gln Gly Ile Pro Phe Phe Gly Gln
           355                 360                 365
   Val Arg Leu Val Asp Gly Lys Gly Val Pro Ile Pro Asn Lys Val Ile
       370                 375                 380
   Phe Ile Arg Gly Asn Glu Ala Asn Tyr Tyr Ser Asn Ala Thr Thr Asp
   385                 390                 395                 400
   Glu His Gly Leu Val Gln Phe Ser Ile Asn Thr Thr Asn Val Met Gly
                   405                 410                 415
   Thr Ser Leu Thr Val Arg Val Asn Tyr Lys Asp Arg Ser Pro Cys Tyr
               420                 425                 430
   Gly Tyr Gln Trp Val Ser Glu Glu His Glu Glu Ala His His Thr Ala
           435                 440                 445
   Tyr Leu Val Phe Ser Pro Ser Lys Ser Phe Val His Leu Glu Pro Met
       450                 455                 460
   Ser His Glu Leu Pro Cys Gly His Thr Gln Thr Val Gln Ala His Tyr
   465                 470                 475                 480
   Ile Leu Asn Gly Gly Thr Leu Leu Gly Leu Lys Lys Leu Ser Phe Tyr
                   485                 490                 495
   Tyr Leu Ile Met Ala Lys Gly Gly Ile Val Arg Thr Gly Thr His Gly
               500                 505                 510
   Leu Leu Val Lys Gln Glu Asp Met Lys Gly His Phe Ser Ile Ser Ile
           515                 520                 525
   Pro Val Lys Ser Asp Ile Ala Pro Val Ala Arg Leu Leu Ile Tyr Ala
       530                 535                 540
   Val Leu Pro Thr Gly Asp Val Ile Gly Asp Ser Ala Lys Tyr Asp Val
   545                 550                 555                 560
   Glu Asn Cys Leu Ala Asn Lys Val Asp Leu Ser Phe Ser Pro Ser Gln
                   565                 570                 575
   Ser Leu Pro Ala Ser His Ala His Leu Arg Val Thr Ala Ala Pro Gln
               580                 585                 590
   Ser Val Cys Ala Leu Arg Ala Val Asp Gln Ser Val Leu Leu Met Lys
           595                 600                 605
   Pro Asp Ala Glu Leu Ser Ala Ser Ser Val Tyr Asn Leu Leu Pro Glu
       610                 615                 620
   Lys Asp Leu Thr Gly Phe Pro Gly Pro Leu Asn Asp Gln Asp Asn Glu
   625                 630                 635                 640
   Asp Cys Ile Asn Arg His Asn Val Tyr Ile Asn Gly Ile Thr Tyr Thr
                   645                 650                 655
   Pro Val Ser Ser Thr Asn Glu Lys Asp Met Tyr Ser Phe Leu Glu Asp
               660                 665                 670
   Met Gly Leu Lys Ala Phe Thr Asn Ser Lys Ile Arg Lys Pro Lys Met
           675                 680                 685
   Cys Pro Gln Leu Gln Gln Tyr Glu Met His Gly Pro Glu Gly Leu Arg
       690                 695                 700
   Val Gly Phe Tyr Glu Ser Asp Val Met Gly Arg Gly His Ala Arg Leu
   705                 710                 715                 720
   Val His Val Glu Glu Pro His Thr Glu Thr Val Arg Lys Tyr Phe Pro
                   725                 730                 735
   Glu Thr Trp Ile Trp Asp Leu Val Val Val Asn Ser Ala Gly Val Ala
               740                 745                 750
   Glu Val Gly Val Thr Val Pro Asp Thr Ile Thr Glu Trp Lys Ala Gly
           755                 760                 765
   Ala Phe Cys Leu Ser Glu Asp Ala Gly Leu Gly Ile Ser Ser Thr Ala
       770                 775                 780
   Ser Leu Arg Ala Phe Gln Pro Phe Phe Val Glu Leu Thr Met Pro Tyr
   785                 790                 795                 800
   Ser Val Ile Arg Gly Glu Ala Phe Thr Leu Lys Ala Thr Val Leu Asn
                   805                 810                 815
   Tyr Leu Pro Lys Cys Ile Arg Val Ser Val Gln Leu Glu Ala Ser Pro
               820                 825                 830
   Ala Phe Leu Ala Val Pro Val Glu Lys Glu Gln Ala Pro His Cys Ile
           835                 840                 845
   Cys Ala Asn Gly Arg Gln Thr Val Ser Trp Ala Val Thr Pro Lys Ser
       850                 855                 860
   Leu Gly Asn Val Asn Phe Thr Val Ser Ala Glu Ala Leu Glu Ser Gln
   865                 870                 875                 880
   Glu Leu Cys Gly Thr Glu Val Pro Ser Val Pro Glu His Gly Arg Lys
                   885                 890                 895
   Asp Thr Val Ile Lys Pro Leu Leu Val Glu Pro Glu Gly Leu Glu Lys
               900                 905                 910
   Glu Thr Thr Phe Asn Ser Leu Leu Cys Pro Ser Gly Gly Glu Val Ser
           915                 920                 925
   Glu Glu Leu Ser Leu Lys Leu Pro Pro Asn Val Val Glu Glu Ser Ala
       930                 935                 940
   Arg Ala Ser Val Ser Val Leu Gly Asp Ile Leu Gly Ser Ala Met Gln
   945                 950                 955                 960
   Asn Thr Gln Asn Leu Leu Gln Met Pro Tyr Gly Cys Gly Glu Gln Asn
                   965                 970                 975
   Met Val Leu Phe Ala Pro Asn Ile Tyr Val Leu Asp Tyr Leu Asn Glu
               980                 985                 990
   Thr Gln Gln Leu Thr Pro Glu Ile Lys Ser Lys Ala Ile Gly Tyr Leu
           995                 1000                1005
   Asn Thr Gly Tyr Gln Arg Gln Leu Asn Tyr Lys His Tyr Asp Gly Ser
       1010                1015                1020
   Tyr Ser Thr Phe Gly Glu Arg Tyr Gly Arg Asn Gln Gly Asn Thr Trp
   1025                1030                1035                1040
   Leu Thr Ala Phe Val Leu Lys Thr Phe Ala Gln Ala Arg Ala Tyr Ile
                   1045                1050                1055
   Phe Ile Asp Glu Ala His Ile Thr Gln Ala Leu Ile Trp Leu Ser Gln
               1060                1065                1070
   Arg Gln Lys Asp Asn Gly Cys Phe Arg Ser Ser Gly Ser Leu Leu Asn
           1075                1080                1085
   Asn Ala Ile Lys Gly Gly Val Glu Asp Glu Val Thr Leu Ser Ala Tyr
       1090                1095                1100
   Ile Thr Ile Ala Leu Leu Glu Ile Pro Leu Thr Val Thr His Pro Val
   1105                1110                1115                1120
   Val Arg Asn Ala Leu Phe Cys Leu Glu Ser Ala Trp Lys Thr Ala Gln
                   1125                1130                1135
   Glu Gly Asp His Gly Ser His Val Tyr Thr Lys Ala Leu Leu Ala Tyr
               1140                1145                1150
   Ala Phe Ala Leu Ala Gly Asn Gln Asp Lys Arg Lys Glu Val Leu Lys
           1155                1160                1165
   Ser Leu Asn Glu Glu Ala Val Lys Lys Asp Asn Ser Val His Trp Glu
       1170                1175                1180
   Arg Pro Gln Lys Pro Lys Ala Pro Val Gly His Phe Tyr Glu Pro Gln
   1185                1190                1195                1200
   Ala Pro Ser Ala Glu Val Glu Met Thr Ser Tyr Val Leu Leu Ala Tyr
                   1205                1210                1215
   Leu Thr Ala Gln Pro Ala Pro Thr Ser Glu Asp Leu Thr Ser Ala Thr
               1220                1225                1230
   Asn Ile Val Lys Trp Ile Thr Lys Gln Gln Asn Ala Gln Gly Gly Phe
           1235                1240                1245
   Ser Ser Thr Gln Asp Thr Val Val Ala Leu His Ala Leu Ser Lys Tyr
       1250                1255                1260
   Gly Ala Ala Thr Phe Thr Arg Thr Gly Lys Ala Ala Gln Val Thr Ile
   1265                1270                1275                1280
   Gln Ser Ser Gly Thr Phe Ser Ser Lys Phe Gln Val Asp Asn Asn Asn
                   1285                1290                1295
   Arg Leu Leu Leu Gln Gln Val Ser Leu Pro Glu Leu Pro Gly Glu Tyr
               1300                1305                1310
   Ser Met Lys Val Thr Gly Glu Gly Cys Val Tyr Leu Gln Thr Ser Leu
           1315                1320                1325
   Lys Tyr Asn Ile Leu Pro Glu Lys Glu Glu Phe Pro Phe Ala Leu Gly
       1330                1335                1340
   Val Gln Thr Leu Pro Gln Thr Cys Asp Glu Pro Lys Ala His Thr Ser
   1345                1350                1355                1360
   Phe Gln Ile Ser Leu Ser Val Ser Tyr Thr Gly Ser Arg Ser Ala Ser
                   1365                1370                1375
   Asn Met Ala Ile Val Asp Val Lys Met Val Ser Gly Phe Ile Pro Leu
               1380                1385                1390
   Lys Pro Thr Val Lys Met Leu Glu Arg Ser Asn His Val Ser Arg Thr
           1395                1400                1405
   Glu Val Ser Ser Asn His Val Leu Ile Tyr Leu Asp Lys Val Ser Asn
       1410                1415                1420
   Gln Thr Leu Ser Leu Phe Phe Thr Val Leu Gln Asp Val Pro Val Arg
   1425                1430                1435                1440
   Asp Leu Lys Pro Ala Ile Val Lys Val Tyr Asp Tyr Tyr Glu Thr Asp
                   1445                1450                1455
   Glu Phe Ala Ile Ala Glu Tyr Asn Ala Pro Cys Ser Lys Asp Leu Gly
               1460                1465                1470
   Asn Ala
<210> 2
   <211> 4563
   <212> PRT
   <213> Homo sapiens
<400> 2
   Met Asp Pro Pro Arg Pro Ala Leu Leu Ala Leu Leu Ala Leu Pro Ala
    1               5                  10                  15
   Leu Leu Leu Leu Leu Leu Ala Gly Ala Arg Ala Glu Glu Glu Met Leu
               20                  25                  30
   Glu Asn Val Ser Leu Val Cys Pro Lys Asp Ala Thr Arg Phe Lys His
           35                  40                  45
   Leu Arg Lys Tyr Thr Tyr Asn Tyr Glu Ala Glu Ser Ser Ser Gly Val
       50                  55                  60
   Pro Gly Thr Ala Asp Ser Arg Ser Ala Thr Arg Ile Asn Cys Lys Val
   65                  70                  75                  80
   Glu Leu Glu Val Pro Gln Leu Cys Ser Phe Ile Leu Lys Thr Ser Gln
                   85                  90                  95
   Cys Thr Leu Lys Glu Val Tyr Gly Phe Asn Pro Glu Gly Lys Ala Leu
               100                 105                 110
   Leu Lys Lys Thr Lys Asn Ser Glu Glu Phe Ala Ala Ala Met Ser Arg
           115                 120                 125
   Tyr Glu Leu Lys Leu Ala Ile Pro Glu Gly Lys Gln Val Phe Leu Tyr
       130                 135                 140
   Pro Glu Lys Asp Glu Pro Thr Tyr Ile Leu Asn Ile Lys Arg Gly Ile
   145                 150                 155                 160
   Ile Ser Ala Leu Leu Val Pro Pro Glu Thr Glu Glu Ala Lys Gln Val
                   165                 170                 175
   Leu Phe Leu Asp Thr Val Tyr Gly Asn Cys Ser Thr His Phe Thr Val
               180                 185                 190
   Lys Thr Arg Lys Gly Asn Val Ala Thr Glu Ile Ser Thr Glu Arg Asp
           195                 200                 205
   Leu Gly Gln Cys Asp Arg Phe Lys Pro Ile Arg Thr Gly Ile Ser Pro
       210                 215                 220
   Leu Ala Leu Ile Lys Gly Met Thr Arg Pro Leu Ser Thr Leu Ile Ser
   225                 230                 235                 240
   Ser Ser Gln Ser Cys Gln Tyr Thr Leu Asp Ala Lys Arg Lys His Val
                   245                 250                 255
   Ala Glu Ala Ile Cys Lys Glu Gln His Leu Phe Leu Pro Phe Ser Tyr
               260                 265                 270
   Lys Asn Lys Tyr Gly Met Val Ala Gln Val Thr Gln Thr Leu Lys Leu
           275                 280                 285
   Glu Asp Thr Pro Lys Ile Asn Ser Arg Phe Phe Gly Glu Gly Thr Lys
       290                 295                 300
   Lys Met Gly Leu Ala Phe Glu Ser Thr Lys Ser Thr Ser Pro Pro Lys
   305                 310                 315                 320
   Gln Ala Glu Ala Val Leu Lys Thr Leu Gln Glu Leu Lys Lys Leu Thr
                   325                 330                 335
   Ile Ser Glu Gln Asn Ile Gln Arg Ala Asn Leu Phe Asn Lys Leu Val
               340                 345                 350
   Thr Glu Leu Arg Gly Leu Ser Asp Glu Ala Val Thr Ser Leu Leu Pro
           355                 360                 365
   Gln Leu Ile Glu Val Ser Ser Pro Ile Thr Leu Gln Ala Leu Val Gln
       370                 375                 380
   Cys Gly Gln Pro Gln Cys Ser Thr His Ile Leu Gln Trp Leu Lys Arg
   385                 390                 395                 400
   Val His Ala Asn Pro Leu Leu Ile Asp Val Val Thr Tyr Leu Val Ala
                   405                 410                 415
   Leu Ile Pro Glu Pro Ser Ala Gln Gln Leu Arg Glu Ile Phe Asn Met
               420                 425                 430
   Ala Arg Asp Gln Arg Ser Arg Ala Thr Leu Tyr Ala Leu Ser His Ala
           435                 440                 445
   Val Asn Asn Tyr His Lys Thr Asn Pro Thr Gly Thr Gln Glu Leu Leu
       450                 455                 460
   Asp Ile Ala Asn Tyr Leu Met Glu Gln Ile Gln Asp Asp Cys Thr Gly
   465                 470                 475                 480
   Asp Glu Asp Tyr Thr Tyr Leu Ile Leu Arg Val Ile Gly Asn Met Gly
                   485                 490                 495
   Gln Thr Met Glu Gln Leu Thr Pro Glu Leu Lys Ser Ser Ile Leu Lys
               500                 505                 510
   Cys Val Gln Ser Thr Lys Pro Ser Leu Met Ile Gln Lys Ala Ala Ile
           515                 520                 525
   Gln Ala Leu Arg Lys Met Glu Pro Lys Asp Lys Asp Gln Glu Val Leu
       530                 535                 540
   Leu Gln Thr Phe Leu Asp Asp Ala Ser Pro Gly Asp Lys Arg Leu Ala
   545                 550                 555                 560
   Ala Tyr Leu Met Leu Met Arg Ser Pro Ser Gln Ala Asp Ile Asn Lys
                   565                 570                 575
   Ile Val Gln Ile Leu Pro Trp Glu Gln Asn Glu Gln Val Lys Asn Phe
               580                 585                 590
   Val Ala Ser His Ile Ala Asn Ile Leu Asn Ser Glu Glu Leu Asp Ile
           595                 600                 605
   Gln Asp Leu Lys Lys Leu Val Lys Glu Ala Leu Lys Glu Ser Gln Leu
       610                 615                 620
   Pro Thr Val Met Asp Phe Arg Lys Phe Ser Arg Asn Tyr Gln Leu Tyr
   625                 630                 635                 640
   Lys Ser Val Ser Leu Pro Ser Leu Asp Pro Ala Ser Ala Lys Ile Glu
                   645                 650                 655
   Gly Asn Leu Ile Phe Asp Pro Asn Asn Tyr Leu Pro Lys Glu Ser Met
               660                 665                 670
   Leu Lys Thr Thr Leu Thr Ala Phe Gly Phe Ala Ser Ala Asp Leu Ile
           675                 680                 685
   Glu Ile Gly Leu Glu Gly Lys Gly Phe Glu Pro Thr Leu Glu Ala Leu
       690                 695                 700
   Phe Gly Lys Gln Gly Phe Phe Pro Asp Ser Val Asn Lys Ala Leu Tyr
   705                 710                 715                 720
   Trp Val Asn Gly Gln Val Pro Asp Gly Val Ser Lys Val Leu Val Asp
                   725                 730                 735
   His Phe Gly Tyr Thr Lys Asp Asp Lys His Glu Gln Asp Met Val Asn
               740                 745                 750
   Gly Ile Met Leu Ser Val Glu Lys Leu Ile Lys Asp Leu Lys Ser Lys
           755                 760                 765
   Glu Val Pro Glu Ala Arg Ala Tyr Leu Arg Ile Leu Gly Glu Glu Leu
   770                 775                 780
   Gly Phe Ala Ser Leu His Asp Leu Gln Leu Leu Gly Lys Leu Leu Leu
   785                 790                 795                 800
   Met Gly Ala Arg Thr Leu Gln Gly Ile Pro Gln Met Ile Gly Glu Val
                   805                 810                 815
   Ile Arg Lys Gly Ser Lys Asn Asp Phe Phe Leu His Tyr Ile Phe Met
               820                 825                 830
   Glu Asn Ala Phe Glu Leu Pro Thr Gly Ala Gly Leu Gln Leu Gln Ile
           835                 840                 845
   Ser Ser Ser Gly Val Ile Ala Pro Gly Ala Lys Ala Gly Val Lys Leu
       850                 855                 860
   Glu Val Ala Asn Met Gln Ala Glu Leu Val Ala Lys Pro Ser Val Ser
   865                 870                 875                 880
   Val Glu Phe Val Thr Asn Met Gly Ile Ile Ile Pro Asp Phe Ala Arg
                   885                 890                 895
   Ser Gly Val Gln Met Asn Thr Asn Phe Phe His Glu Ser Gly Leu Glu
               900                 905                 910
   Ala His Val Ala Leu Lys Ala Gly Lys Leu Lys Phe Ile Ile Pro Ser
           915                 920                 925
   Pro Lys Arg Pro Val Lys Leu Leu Ser Gly Gly Asn Thr Leu His Leu
       930                 935                 940
   Val Ser Thr Thr Lys Thr Glu Val Ile Pro Pro Leu Ile Glu Asn Arg
   945                 950                 955                 960
   Gln Ser Trp Ser Val Cys Lys Gln Val Phe Pro Gly Leu Asn Tyr Cys
                   965                 970                 975
   Thr Ser Gly Ala Tyr Ser Asn Ala Ser Ser Thr Asp Ser Ala Ser Tyr
               980                 985                 990
   Tyr Pro Leu Thr Gly Asp Thr Arg Leu Glu Leu Glu Leu Arg Pro Thr
           995                 1000                1005
   Gly Glu Ile Glu Gln Tyr Ser Val Ser Ala Thr Tyr Glu Leu Gln Arg
       1010                1015                1020
   Glu Asp Arg Ala Leu Val Asp Thr Leu Lys Phe Val Thr Gln Ala Glu
   1025                1030                1035                1040
   Gly Ala Lys Gln Thr Glu Ala Thr Met Thr Phe Lys Tyr Asn Arg Gln
                   1045                1050                1055
   Ser Met Thr Leu Ser Ser Glu Val Gln Ile Pro Asp Phe Asp Val Asp
               1060                1065                1070
   Leu Gly Thr Ile Leu Arg Val Asn Asp Glu Ser Thr Glu Gly Lys Thr
           1075                1080                1085
   Ser Tyr Arg Leu Thr Leu Asp Ile Gln Asn Lys Lys Ile Thr Glu Val
       1090                1095                1100
   Ala Leu Met Gly His Leu Ser Cys Asp Thr Lys Glu Glu Arg Lys Ile
   1105                1110                1115                1120
   Lys Gly Val Ile Ser Ile Pro Arg Leu Gln Ala Glu Ala Arg Ser Glu
                   1125                1130                1135
   Ile Leu Ala His Trp Ser Pro Ala Lys Leu Leu Leu Gln Met Asp Ser
               1140                1145                1150
   Ser Ala Thr Ala Tyr Gly Ser Thr Val Ser Lys Arg Val Ala Trp His
           1155                1160                1165
   Tyr Asp Glu Glu Lys Ile Glu Phe Glu Trp Asn Thr Gly Thr Asn Val
       1170                1175                1180
   Asp Thr Lys Lys Met Thr Ser Asn Phe Pro Val Asp Leu Ser Asp Tyr
   1185                1190                1195                1200
   Pro Lys Ser Leu His Met Tyr Ala Asn Arg Leu Leu Asp His Arg Val
                   1205                1210                1215
   Pro Gln Thr Asp Met Thr Phe Arg His Val Gly Ser Lys Leu Ile Val
               1220                1225                1230
   Ala Met Ser Ser Trp Leu Gln Lys Ala Ser Gly Ser Leu Pro Tyr Thr
           1235                1240                1245
   Gln Thr Leu Gln Asp His Leu Asn Ser Leu Lys Glu Phe Asn Leu Gln
       1250                1255                1260
   Asn Met Gly Leu Pro Asp Phe His Ile Pro Glu Asn Leu Phe Leu Lys
   1265                1270                1275                1280
   Ser Asp Gly Arg Val Lys Tyr Thr Leu Asn Lys Asn Ser Leu Lys Ile
                   1285                1290                1295
   Glu Ile Pro Leu Pro Phe Gly Gly Lys Ser Ser Arg Asp Leu Lys Met
               1300                1305                1310
   Leu Glu Thr Val Arg Thr Pro Ala Leu His Phe Lys Ser Val Gly Phe
           1315                1320                1325
   His Leu Pro Ser Arg Glu Phe Gln Val Pro Thr Phe Thr Ile Pro Lys
       1330                1335                1340
   Leu Tyr Gln Leu Gln Val Pro Leu Leu Gly Val Leu Asp Leu Ser Thr
   1345                1350                1355                1360
   Asn Val Tyr Ser Asn Leu Tyr Asn Trp Ser Ala Ser Tyr Ser Gly Gly
                   1365                1370                1375
   Asn Thr Ser Thr Asp His Phe Ser Leu Arg Ala Arg Tyr His Met Lys
               1380                1385                1390
   Ala Asp Ser Val Val Asp Leu Leu Ser Tyr Asn Val Gln Gly Ser Gly
           1395                1400                1405
   Glu Thr Thr Tyr Asp His Lys Asn Thr Phe Thr Leu Ser Tyr Asp Gly
       1410                1415                1420
   Ser Leu Arg His Lys Phe Leu Asp Ser Asn Ile Lys Phe Ser His Val
   1425                1430                1435                1440
   Glu Lys Leu Gly Asn Asn Pro Val Ser Lys Gly Leu Leu Ile Phe Asp
                   1445                1450                1455
   Ala Ser Ser Ser Trp Gly Pro Gln Met Ser Ala Ser Val His Leu Asp
               1460                1465                1470
   Ser Lys Lys Lys Gln His Leu Phe Val Lys Glu Val Lys Ile Asp Gly
           1475                1480                1485
   Gln Phe Arg Val Ser Ser Phe Tyr Ala Lys Gly Thr Tyr Gly Leu Ser
       1490                1495                1500
   Cys Gln Arg Asp Pro Asn Thr Gly Arg Leu Asn Gly Glu Ser Asn Leu
   1505                1510                1515                1520
   Arg Phe Asn Ser Ser Tyr Leu Gln Gly Thr Asn Gln Ile Thr Gly Arg
                   1525                1530                1535
   Tyr Glu Asp Gly Thr Leu Ser Leu Thr Ser Thr Ser Asp Leu Gln Ser
               1540                1545                1550
   Gly Ile Ile Lys Asn Thr Ala Ser Leu Lys Tyr Glu Asn Tyr Glu Leu
           1555                1560                1565
   Thr Leu Lys Ser Asp Thr Asn Gly Lys Tyr Lys Asn Phe Ala Thr Ser
       1570                1575                1580
   Asn Lys Met Asp Met Thr Phe Ser Lys Gln Asn Ala Leu Leu Arg Ser
   1585                1590                1595                1600
   Glu Tyr Gln Ala Asp Tyr Glu Ser Leu Arg Phe Phe Ser Leu Leu Ser
                   1605                1610                1615
   Gly Ser Leu Asn Ser His Gly Leu Glu Leu Asn Ala Asp Ile Leu Gly
               1620                1625                1630
   Thr Asp Lys Ile Asn Ser Gly Ala His Lys Ala Thr Leu Arg Ile Gly
           1635                1640                1645
   Gln Asp Gly Ile Ser Thr Ser Ala Thr Thr Asn Leu Lys Cys Ser Leu
       1650                1655                1660
   Leu Val Leu Glu Asn Glu Leu Asn Ala Glu Leu Gly Leu Ser Gly Ala
   1665                1670                1675                1680
   Ser Met Lys Leu Thr Thr Asn Gly Arg Phe Arg Glu His Asn Ala Lys
                   1685                1690                1695
   Phe Ser Leu Asp Gly Lys Ala Ala Leu Thr Glu Leu Ser Leu Gly Ser
               1700                1705                1710
   Ala Tyr Gln Ala Met Ile Leu Gly Val Asp Ser Lys Asn Ile Phe Asn
           1715                1720                1725
   Phe Lys Val Ser Gln Glu Gly Leu Lys Leu Ser Asn Asp Met Met Gly
       1730                1735                1740
   Ser Tyr Ala Glu Met Lys Phe Asp His Thr Asn Ser Leu Asn Ile Ala
   1745 1750 1755 1760
   Gly Leu Ser Leu Asp Phe Ser Ser Lys Leu Asp Asn Ile Tyr Ser Ser
   1765 1770 1775
   Asp Lys Phe Tyr Lys Gln Thr Val Asn Leu Gln Leu Gln Pro Tyr Ser
   1780 1785 1790
   Leu Val Thr Thr Leu Asn Ser Asp Leu Lys Tyr Asn Ala Leu Asp Leu
   1795 1800 1805
   Thr Asn Asn Gly Lys Leu Arg Leu Glu Pro Leu Lys Leu His Val Ala
   1810 1815 1820
   Gly Asn Leu Lys Gly Ala Tyr Gln Asn Asn Glu Ile Lys His Ile Tyr
   1825 1830 1835 1840
   Ala Ile Ser Ser Ala Ala Leu Ser Ala Ser Tyr Lys Ala Asp Thr Val
   1845 1850 1855
   Ala Lys Val Gln Gly Val Glu Phe Ser His Arg Leu Asn Thr Asp Ile
   1860 1865 1870
   Ala Gly Leu Ala Ser Ala Ile Asp Met Ser Thr Asn Tyr Asn Ser Asp
   1875 1880 1885
   Ser Leu His Phe Ser Asn Val Phe Arg Ser Val Met Ala Pro Phe Thr
   1890 1895 1900
   Met Thr Ile Asp Ala His Thr Asn Gly Asn Gly Lys Leu Ala Leu Trp
   1905 1910 1915 1920
   Gly Glu His Thr Gly Gln Leu Tyr Ser Lys Phe Leu Leu Lys Ala Glu
   1925 1930 1935
   Pro Leu Ala Phe Thr Phe Ser His Asp Tyr Lys Gly Ser Thr Ser His
   1940 1945 1950
   His Leu Val Ser Arg Lys Ser Ile Ser Ala Ala Leu Glu His Lys Val
   1955 1960 1965
   Ser Ala Leu Leu Thr Pro Ala Glu Gln Thr Gly Thr Trp Lys Leu Lys
   1970 1975 1980
   Thr Gln Phe Asn Asn Asn Glu Tyr Ser Gln Asp Leu Asp Ala Tyr Asn
   1985 1990 1995 2000
   Thr Lys Asp Lys Ile Gly Val Glu Leu Thr Gly Arg Thr Leu Ala Asp
   2005 2010 2015
   Leu Thr Leu Leu Asp Ser Pro Ile Lys Val Pro Leu Leu Leu Ser Glu
   2020 2025 2030
   Pro Ile Asn Ile Ile Asp Ala Leu Glu Met Arg Asp Ala Val Glu Lys
   2035 2040 2045
   Pro Gln Glu Phe Thr Ile Val Ala Phe Val Lys Tyr Asp Lys Asn Gln
   2050 2055 2060
   Asp Val His Ser Ile Asn Leu Pro Phe Phe Glu Thr Leu Gln Glu Tyr
   2065 2070 2075 2080
   Phe Glu Arg Asn Arg Gln Thr Ile Ile Val Val Leu Glu Asn Val Gln
   2085 2090 2095
   Arg Asn Leu Lys His Ile Asn Ile Asp Gln Phe Val Arg Lys Tyr Arg
   2100 2105 2110
   Ala Ala Leu Gly Lys Leu Pro Gln Gln Ala Asn Asp Tyr Leu Asn Ser
   2115 2120 2125
   Phe Asn Trp Glu Arg Gln Val Ser His Ala Lys Glu Lys Leu Thr Ala
   2130 2135 2140
   Leu Thr Lys Lys Tyr Arg Ile Thr Glu Asn Asp Ile Gln Ile Ala Leu
   2145 2150 2155 2160
   Asp Asp Ala Lys Ile Asn Phe Asn Glu Lys Leu Ser Gln Leu Gln Thr
   2165 2170 2175
   Tyr Met Ile Gln Phe Asp Gln Tyr Ile Lys Asp Ser Tyr Asp Leu His
   2180 2185 2190
   Asp Leu Lys Ile Ala Ile Ala Asn Ile Ile Asp Glu Ile Ile Glu Lys
   2195 2200 2205
   Leu Lys Ser Leu Asp Glu His Tyr His Ile Arg Val Asn Leu Val Lys
   2210 2215 2220
   Thr Ile His Asp Leu His Leu Phe Ile Glu Asn Ile Asp Phe Asn Lys
   2225 2230 2235 2240
   Ser Gly Ser Ser Thr Ala Ser Trp Ile Gln Asn Val Asp Thr Lys Tyr
                   2245                2250                2255
   Gln Ile Arg Ile Gln Ile Gln Glu Lys Leu Gln Gln Leu Lys Arg His
               2260                2265                2270
   Ile Gln Asn Ile Asp Ile Gln His Leu Ala Gly Lys Leu Lys Gln His
           2275                2280                2285
   Ile Glu Ala Ile Asp Val Arg Val Leu Leu Asp Gln Leu Gly Thr Thr
       2290                2295                2300
   Ile Ser Phe Glu Arg Ile Asn Asp Ile Leu Glu His Val Lys His Phe
   2305                2310                2315                2320
   Val Ile Asn Leu Ile Gly Asp Phe Glu Val Ala Glu Lys Ile Asn Ala
                   2325                2330                2335
   Phe Arg Ala Lys Val His Glu Leu Ile Glu Arg Tyr Glu Val Asp Gln
               2340                2345                2350
   Gln Ile Gln Val Leu Met Asp Lys Leu Val Glu Leu Ala His Gln Tyr
           2355                2360                2365
   Lys Leu Lys Glu Thr Ile Gln Lys Leu Ser Asn Val Leu Gln Gln Val
       2370                2375                2380
   Lys Ile Lys Asp Tyr Phe Glu Lys Leu Val Gly Phe Ile Asp Asp Ala
   2385                2390                2395                2400
   Val Lys Lys Leu Asn Glu Leu Ser Phe Lys Thr Phe Ile Glu Asp Val
                   2405                2410                2415
   Asn Lys Phe Leu Asp Met Leu Ile Lys Lys Leu Lys Ser Phe Asp Tyr
               2420                2425                2430
   His Gln Phe Val Asp Glu Thr Asn Asp Lys Ile Arg Glu Val Thr Gln
           2435                2440                2445
   Arg Leu Asn Gly Glu Ile Gln Ala Leu Glu Leu Pro Gln Lys Ala Glu
       2450                2455                2460
   Ala Leu Lys Leu Phe Leu Glu Glu Thr Lys Ala Thr Val Ala Val Tyr
   2465                2470                2475                2480
   Leu Glu Ser Leu Gln Asp Thr Lys Ile Thr Leu Ile Ile Asn Trp Leu
                   2485                2490                2495
   Gln Glu Ala Leu Ser Ser Ala Ser Leu Ala His Met Lys Ala Lys Phe
               2500                2505                2510
   Arg Glu Thr Leu Glu Asp Thr Arg Asp Arg Met Tyr Gln Met Asp Ile
           2515                2520                2525
   Gln Gln Glu Leu Gln Arg Tyr Leu Ser Leu Val Gly Gln Val Tyr Ser
       2530                2535                2540
   Thr Leu Val Thr Tyr Ile Ser Asp Trp Trp Thr Leu Ala Ala Lys Asn
   2545                2550                2555                2560
   Leu Thr Asp Phe Ala Glu Gln Tyr Ser Ile Gln Asp Trp Ala Lys Arg
                   2565                2570                2575
   Met Lys Ala Leu Val Glu Gln Gly Phe Thr Val Pro Glu Ile Lys Thr
               2580                2585                2590
   Ile Leu Gly Thr Met Pro Ala Phe Glu Val Ser Leu Gln Ala Leu Gln
           2595                2600                2605
   Lys Ala Thr Phe Gln Thr Pro Asp Phe Ile Val Pro Leu Thr Asp Leu
       2610                2615                2620
   Arg Ile Pro Ser Val Gln Ile Asn Phe Lys Asp Leu Lys Asn Ile Lys
   2625                2630                2635                2640
   Ile Pro Ser Arg Phe Ser Thr Pro Glu Phe Thr Ile Leu Asn Thr Phe
                   2645                2650                2655
   His Ile Pro Ser Phe Thr Ile Asp Phe Val Glu Met Lys Val Lys Ile
               2660                2665                2670
   Ile Arg Thr Ile Asp Gln Met Leu Asn Ser Glu Leu Gln Trp Pro Val
           2675                2680                2685
   Pro Asp Ile Tyr Leu Arg Asp Leu Lys Val Glu Asp Ile Pro Leu Ala
       2690                2695                2700
   Arg Ile Thr Leu Pro Asp Phe Arg Leu Pro Glu Ile Ala Ile Pro Glu
   2705                2710                2715                2720
   Phe Ile Ile Pro Thr Leu Asn Leu Asn Asp Phe Gln Val Pro Asp Leu
                   2725                2730                2735
   His Ile Pro Glu Phe Gln Leu Pro His Ile Ser His Thr Ile Glu Val
               2740                2745                2750
   Pro Thr Phe Gly Lys Leu Tyr Ser Ile Leu Lys Ile Gln Ser Pro Leu
   2755 2760 2765
   Phe Thr Leu Asp Ala Asn Ala Asp Ile Gly Asn Gly Thr Thr Ser Ala
   2770 2775 2780
   Asn Glu Ala Gly Ile Ala Ala Ser Ile Thr Ala Lys Gly Glu Ser Lys
   2785 2790 2795 2800
   Leu Glu Val Leu Asn Phe Asp Phe Gln Ala Asn Ala Gln Leu Ser Asn
   2805 2810 2815
   Pro Lys Ile Asn Pro Leu Ala Leu Lys Glu Ser Val Lys Phe Ser Ser
   2820 2825 2830
   Lys Tyr Leu Arg Thr Glu His Gly Ser Glu Met Leu Phe Phe Gly Asn
   2835 2840 2845
   Ala Ile Glu Gly Lys Ser Asn Thr Val Ala Ser Leu His Thr Glu Lys
   2850 2855 2860
   Asn Thr Leu Glu Leu Ser Asn Gly Val Ile Val Lys Ile Asn Asn Gln
   2865 2870 2875 2880
   Leu Thr Leu Asp Ser Asn Thr Lys Tyr Phe His Lys Leu Asn Ile Pro
   2885 2890 2895
   Lys Leu Asp Phe Ser Ser Gln Ala Asp Leu Arg Asn Glu Ile Lys Thr
   2900 2905 2910
   Leu Leu Lys Ala Gly His Ile Ala Trp Thr Ser Ser Gly Lys Gly Ser
   2915 2920 2925
   Trp Lys Trp Ala Cys Pro Arg Phe Ser Asp Glu Gly Thr His Glu Ser
   2930 2935 2940
   Gln Ile Ser Phe Thr Ile Glu Gly Pro Leu Thr Ser Phe Gly Leu Ser
   2945 2950 2955 2960
   Asn Lys Ile Asn Ser Lys His Leu Arg Val Asn Gln Asn Leu Val Tyr
   2965 2970 2975
   Glu Ser Gly Ser Leu Asn Phe Ser Lys Leu Glu Ile Gln Ser Gln Val
   2980 2985 2990
   Asp Ser Gln His Val Gly His Ser Val Leu Thr Ala Lys Gly Met Ala
   2995 3000 3005
   Leu Phe Gly Glu Gly Lys Ala Glu Phe Thr Gly Arg His Asp Ala His
   3010 3015 3020
   Leu Asn Gly Lys Val Ile Gly Thr Leu Lys Asn Ser Leu Phe Phe Ser
   3025 3030 3035 3040
   Ala Gln Pro Phe Glu Ile Thr Ala Ser Thr Asn Asn Glu Gly Asn Leu
   3045 3050 3055
   Lys Val Arg Phe Pro Leu Arg Leu Thr Gly Lys Ile Asp Phe Leu Asn
   3060 3065 3070
   Asn Tyr Ala Leu Phe Leu Ser Pro Ser Ala Gln Gln Ala Ser Trp Gln
   3075 3080 3085
   Val Ser Ala Arg Phe Asn Gln Tyr Lys Tyr Asn Gln Asn Phe Ser Ala
   3090 3095 3100
   Gly Asn Asn Glu Asn Ile Met Glu Ala His Val Gly Ile Asn Gly Glu
   3105 3110 3115 3120
   Ala Asn Leu Asp Phe Leu Asn Ile Pro Leu Thr Ile Pro Glu Met Arg
   3125 3130 3135
   Leu Pro Tyr Thr Ile Ile Thr Thr Pro Pro Leu Lys Asp Phe Ser Leu
   3140 3145 3150
   Trp Glu Lys Thr Gly Leu Lys Glu Phe Leu Lys Thr Thr Lys Gln Ser
   3155 3160 3165
   Phe Asp Leu Ser Val Lys Ala Gln Tyr Lys Lys Asn Lys His Arg His
   3170 3175 3180
   Ser Ile Thr Asn Pro Leu Ala Val Leu Cys Glu Phe Ile Ser Gln Ser
   3185 3190 3195 3200
   Ile Lys Ser Phe Asp Arg His Phe Glu Lys Asn Arg Asn Asn Ala Leu
   3205 3210 3215
   Asp Phe Val Thr Lys Ser Tyr Asn Glu Thr Lys Ile Lys Phe Asp Lys
   3220 3225 3230
   Tyr Lys Ala Glu Lys Ser His Asp Glu Leu Pro Arg Thr Phe Gln Ile
   3235 3240 3245
   Pro Gly Tyr Thr Val Pro Val Val Asn Val Glu Val Ser Pro Phe Thr
       3250                3255                3260
   Ile Glu Met Ser Ala Phe Gly Tyr Val Phe Pro Lys Ala Val Ser Met
   3265                3270                3275                3280
   Pro Ser Phe Ser Ile Leu Gly Ser Asp Val Arg Val Pro Ser Tyr Thr
                   3285                3290                3295
   Leu Ile Leu Pro Ser Leu Glu Leu Pro Val Leu His Val Pro Arg Asn
               3300                3305                3310
   Leu Lys Leu Ser Leu Pro Asp Phe Lys Glu Leu Cys Thr Ile Ser His
           3315                3320                3325
   Ile Phe Ile Pro Ala Met Gly Asn Ile Thr Tyr Asp Phe Ser Phe Lys
       3330                3335                3340
   Ser Ser Val Ile Thr Leu Asn Thr Asn Ala Glu Leu Phe Asn Gln Ser
   3345                3350                3355                3360
   Asp Ile Val Ala His Leu Leu Ser Ser Ser Ser Ser Val Ile Asp Ala
                   3365                3370                3375
   Leu Gln Tyr Lys Leu Glu Gly Thr Thr Arg Leu Thr Arg Lys Arg Gly
               3380                3385                3390
   Leu Lys Leu Ala Thr Ala Leu Ser Leu Ser Asn Lys Phe Val Glu Gly
           3395                3400                3405
   Ser His Asn Ser Thr Val Ser Leu Thr Thr Lys Asn Met Glu Val Ser
       3410                3415                3420
   Val Ala Thr Thr Thr Lys Ala Gln Ile Pro Ile Leu Arg Met Asn Phe
   3425                3430                3435                3440
   Lys Gln Glu Leu Asn Gly Asn Thr Lys Ser Lys Pro Thr Val Ser Ser
                   3445                3450                3455
   Ser Met Glu Phe Lys Tyr Asp Phe Asn Ser Ser Met Leu Tyr Ser Thr
               3460                3465                3470
   Ala Lys Gly Ala Val Asp His Lys Leu Ser Leu Glu Ser Leu Thr Ser
           3475                3480                3485
   Tyr Phe Ser Ile Glu Ser Ser Thr Lys Gly Asp Val Lys Gly Ser Val
       3490                3495                3500
   Leu Ser Arg Glu Tyr Ser Gly Thr Ile Ala Ser Glu Ala Asn Thr Tyr
   3505                3510                3515                3520
   Leu Asn Ser Lys Ser Thr Arg Ser Ser Val Lys Leu Gln Gly Thr Ser
                   3525                3530                3535
   Lys Ile Asp Asp Ile Trp Asn Leu Glu Val Lys Glu Asn Phe Ala Gly
               3540                3545                3550
   Glu Ala Thr Leu Gln Arg Ile Tyr Ser Leu Trp Glu His Ser Thr Lys
           3555                3560                3565
   Asn His Leu Gln Leu Glu Gly Leu Phe Phe Thr Asn Gly Glu His Thr
       3570                3575                3580
   Ser Lys Ala Thr Leu Glu Leu Ser Pro Trp Gln Met Ser Ala Leu Val
   3585                3590                3595                3600
   Gln Val His Ala Ser Gln Pro Ser Ser Phe His Asp Phe Pro Asp Leu
                   3605                3610                3615
   Gly Gln Glu Val Ala Leu Asn Ala Asn Thr Lys Asn Gln Lys Ile Arg
               3620                3625                3630
   Trp Lys Asn Glu Val Arg Ile His Ser Gly Ser Phe Gln Ser Gln Val
           3635                3640                3645
   Glu Leu Ser Asn Asp Gln Glu Lys Ala His Leu Asp Ile Ala Gly Ser
       3650                3655                3660
   Leu Glu Gly His Leu Arg Phe Leu Lys Asn Ile Ile Leu Pro Val Tyr
   3665                3670                3675                3680
   Asp Lys Ser Leu Trp Asp Phe Leu Lys Leu Asp Val Thr Thr Ser Ile
                   3685                3690                3695
   Gly Arg Arg Gln His Leu Arg Val Ser Thr Ala Phe Val Tyr Thr Lys
               3700                3705                3710
   Asn Pro Asn Gly Tyr Ser Phe Ser Ile Pro Val Lys Val Leu Ala Asp
           3715                3720                3725
   Lys Phe Ile Ile Pro Gly Leu Lys Leu Asn Asp Leu Asn Ser Val Leu
       3730                3735                3740
   Val Met Pro Thr Phe His Val Pro Phe Thr Asp Leu Gln Val Pro Ser
   3745                3750                3755                3760
   Cys Lys Leu Asp Phe Arg Glu Ile Gln Ile Tyr Lys Lys Leu Arg Thr
   3765 3770 3775
   Ser Ser Phe Ala Leu Asn Leu Pro Thr Leu Pro Glu Val Lys Phe Pro
   3780 3785 3790
   Glu Val Asp Val Leu Thr Lys Tyr Ser Gln Pro Glu Asp Ser Leu Ile
   3795 3800 3805
   Pro Phe Phe Glu Ile Thr Val Pro Glu Ser Gln Leu Thr Val Ser Gln
   3810 3815 3820
   Phe Thr Leu Pro Lys Ser Val Ser Asp Gly Ile Ala Ala Leu Asp Leu
   3825 3830 3835 3840
   Asn Ala Val Ala Asn Lys Ile Ala Asp Phe Glu Leu Pro Thr Ile Ile
   3845 3850 3855
   Val Pro Glu Gln Thr Ile Glu Ile Pro Ser Ile Lys Phe Ser Val Pro
   3860 3865 3870
   Ala Gly Ile Val Ile Pro Ser Phe Gln Ala Leu Thr Ala Arg Phe Glu
   3875 3880 3885
   Val Asp Ser Pro Val Tyr Asn Ala Thr Trp Ser Ala Ser Leu Lys Asn
   3890 3895 3900
   Lys Ala Asp Tyr Val Glu Thr Val Leu Asp Ser Thr Cys Ser Ser Thr
   3905 3910 3915 3920
   Val Gln Phe Leu Glu Tyr Glu Leu Asn Val Leu Gly Thr His Lys Ile
   3925 3930 3935
   Glu Asp Gly Thr Leu Ala Ser Lys Thr Lys Gly Thr Phe Ala His Arg
   3940 3945 3950
   Asp Phe Ser Ala Glu Tyr Glu Glu Asp Gly Lys Tyr Glu Gly Leu Gln
   3955 3960 3965
   Glu Trp Glu Gly Lys Ala His Leu Asn Ile Lys Ser Pro Ala Phe Thr
   3970 3975 3980
   Asp Leu His Leu Arg Tyr Gln Lys Asp Lys Lys Gly Ile Ser Thr Ser
   3985 3990 3995 4000
   Ala Ala Ser Pro Ala Val Gly Thr Val Gly Met Asp Met Asp Glu Asp
   4005 4010 4015
   Asp Asp Phe Ser Lys Trp Asn Phe Tyr Tyr Ser Pro Gln Ser Ser Pro
   4020 4025 4030
   Asp Lys Lys Leu Thr Ile Phe Lys Thr Glu Leu Arg Val Arg Glu Ser
   4035 4040 4045
   Asp Glu Glu Thr Gln Ile Lys Val Asn Trp Glu Glu Glu Ala Ala Ser
   4050 4055 4060
   Gly Leu Leu Thr Ser Leu Lys Asp Asn Val Pro Lys Ala Thr Gly Val
   4065 4070 4075 4080
   Leu Tyr Asp Tyr Val Asn Lys Tyr His Trp Glu His Thr Gly Leu Thr
   4085 4090 4095
   Leu Arg Glu Val Ser Ser Lys Leu Arg Arg Asn Leu Gln Asn Asn Ala
   4100 4105 4110
   Glu Trp Val Tyr Gln Gly Ala Ile Arg Gln Ile Asp Asp Ile Asp Val
   4115 4120 4125
   Arg Phe Gln Lys Ala Ala Ser Gly Thr Thr Gly Thr Tyr Gln Glu Trp
   4130 4135 4140
   Lys Asp Lys Ala Gln Asn Leu Tyr Gln Glu Leu Leu Thr Gln Glu Gly
   4145 4150 4155 4160
   Gln Ala Ser Phe Gln Gly Leu Lys Asp Asn Val Phe Asp Gly Leu Val
   4165 4170 4175
   Arg Val Thr Gln Glu Phe His Met Lys Val Lys His Leu Ile Asp Ser
   4180 4185 4190
   Leu Ile Asp Phe Leu Asn Phe Pro Arg Phe Gln Phe Pro Gly Lys Pro
   4195 4200 4205
   Gly Ile Tyr Thr Arg Glu Glu Leu Cys Thr Met Phe Ile Arg Glu Val
   4210 4215 4220
   Gly Thr Val Leu Ser Gln Val Tyr Ser Lys Val His Asn Gly Ser Glu
   4225 4230 4235 4240
   Ile Leu Phe Ser Tyr Phe Gln Asp Leu Val Ile Thr Leu Pro Phe Glu
   4245 4250 4255
   Leu Arg Lys His Lys Leu Ile Asp Val Ile Ser Met Tyr Arg Glu Leu
   4260 4265 4270
   Leu Lys Asp Leu Ser Lys Glu Ala Gln Glu Val Phe Lys Ala Ile Gln
   4275 4280 4285
   Ser Leu Lys Thr Thr Glu Val Leu Arg Asn Leu Gln Asp Leu Leu Gln
   4290 4295 4300
   Phe Ile Phe Gln Leu Ile Glu Asp Asn Ile Lys Gln Leu Lys Glu Met
   4305 4310 4315 4320
   Lys Phe Thr Tyr Leu Ile Asn Tyr Ile Gln Asp Glu Ile Asn Thr Ile
   4325 4330 4335
   Phe Ser Asp Tyr Ile Pro Tyr Val Phe Lys Leu Leu Lys Glu Asn Leu
   4340 4345 4350
   Cys Leu Asn Leu His Lys Phe Asn Glu Phe Ile Gln Asn Glu Leu Gln
   4355 4360 4365
   Glu Ala Ser Gln Glu Leu Gln Gln Ile His Gln Tyr Ile Met Ala Leu
   4370 4375 4380
   Arg Glu Glu Tyr Phe Asp Pro Ser Ile Val Gly Trp Thr Val Lys Tyr
   4385 4390 4395 4400
   Tyr Glu Leu Glu Glu Lys Ile Val Ser Leu Ile Lys Asn Leu Leu Val
   4405 4410 4415
   Ala Leu Lys Asp Phe His Ser Glu Tyr Ile Val Ser Ala Ser Asn Phe
   4420 4425 4430
   Thr Ser Gln Leu Ser Ser Gln Val Glu Gln Phe Leu His Arg Asn Ile
   4435 4440 4445
   Gln Glu Tyr Leu Ser Ile Leu Thr Asp Pro Asp Gly Lys Gly Lys Glu
   4450 4455 4460
   Lys Ile Ala Glu Leu Ser Ala Thr Ala Gln Glu Ile Ile Lys Ser Gln
   4465 4470 4475 4480
   Ala Ile Ala Thr Lys Lys Ile Ile Ser Asp Tyr His Gln Gln Phe Arg
   4485 4490 4495
   Tyr Lys Leu Gln Asp Phe Ser Asp Gln Leu Ser Asp Tyr Tyr Glu Lys
   4500 4505 4510
   Phe Ile Ala Glu Ser Lys Arg Leu Ile Asp Leu Ser Ile Gln Asn Tyr
   4515 4520 4525
   His Thr Phe Leu Ile Tyr Ile Thr Glu Leu Leu Lys Lys Leu Gln Ser
   4530 4535 4540
   Thr Thr Val Met Asn Pro Tyr Met Lys Leu Ala Pro Gly Glu Leu Thr
   4545 4550 4555 4560
   Ile Ile Leu
<210> 3
   <211> 345
   <212> PRT
   <213> Homo sapiens
<400> 3
   Met Ile Ser Pro Val Leu Ile Leu Phe Ser Ser Phe Leu Cys His Val
    1               5                  10                  15
   Ala Ile Ala Gly Arg Thr Cys Pro Lys Pro Asp Asp Leu Pro Phe Ser
               20                  25                  30
   Thr Val Val Pro Leu Lys Thr Phe Tyr Glu Pro Gly Glu Glu Ile Thr
           35                  40                  45
   Tyr Ser Cys Lys Pro Gly Tyr Val Ser Arg Gly Gly Met Arg Lys Phe
       50                  55                  60
   Ile Cys Pro Leu Thr Gly Leu Trp Pro Ile Asn Thr Leu Lys Cys Thr
   65                  70                  75                  80
   Pro Arg Val Cys Pro Phe Ala Gly Ile Leu Glu Asn Gly Ala Val Arg
                   85                  90                  95
   Tyr Thr Thr Phe Glu Tyr Pro Asn Thr Ile Ser Phe Ser Cys Asn Thr
               100                 105                 110
   Gly Phe Tyr Leu Asn Gly Ala Asp Ser Ala Lys Cys Thr Glu Glu Gly
           115                 120                 125
   Lys Trp Ser Pro Glu Leu Pro Val Cys Ala Pro Ile Ile Cys Pro Pro
       130                 135                 140
   Pro Ser Ile Pro Thr Phe Ala Thr Leu Arg Val Tyr Lys Pro Ser Ala
   145                 150                 155                 160
   Gly Asn Asn Ser Leu Tyr Arg Asp Thr Ala Val Phe Glu Cys Leu Pro
                   165                 170                 175
   Gln His Ala Met Phe Gly Asn Asp Thr Ile Thr Cys Thr Thr His Gly
               180                 185                 190
   Asn Trp Thr Lys Leu Pro Glu Cys Arg Glu Val Lys Cys Pro Phe Pro
           195                 200                 205
   Ser Arg Pro Asp Asn Gly Phe Val Asn Tyr Pro Ala Lys Pro Thr Leu
       210                 215                 220
   Tyr Tyr Lys Asp Lys Ala Thr Phe Gly Cys His Asp Gly Tyr Ser Leu
   225                 230                 235                 240
   Asp Gly Pro Glu Glu Ile Glu Cys Thr Lys Leu Gly Asn Trp Ser Ala
                   245                 250                 255
   Met Pro Ser Cys Lys Ala Ser Cys Lys Val Pro Val Lys Lys Ala Thr
               260                 265                 270
   Val Val Tyr Gln Gly Glu Arg Val Lys Ile Gln Glu Lys Phe Lys Asn
           275                 280                 285
   Gly Met Leu His Gly Asp Lys Val Ser Phe Phe Cys Lys Asn Lys Glu
       290                 295                 300
   Lys Lys Cys Ser Tyr Thr Glu Asp Ala Gln Cys Ile Asp Gly Thr Ile
   305                 310                 315                 320
   Glu Val Pro Lys Cys Phe Lys Glu His Ser Ser Leu Ala Phe Trp Lys
                   325                 330                 335
   Thr Asp Ala Ser Asp Val Lys Pro Cys
               340                 345
<210> 4
   <211> 705
   <212> PRT
   <213> Homo sapiens
<400> 4
   Met Trp Leu Leu Tyr Leu Leu Val Pro Ala Leu Phe Cys Arg Ala Gly
    1               5                  10                  15
   Gly Ser Ile Pro Ile Pro Gln Lys Leu Phe Gly Glu Val Thr Ser Pro
               20                  25                  30
   Leu Phe Pro Lys Pro Tyr Pro Asn Asn Phe Glu Thr Thr Thr Val Ile
           35                  40                  45
   Thr Val Pro Thr Gly Tyr Arg Val Lys Leu Val Phe Gln Gln Phe Asp
       50                  55                  60
   Leu Glu Pro Ser Glu Gly Cys Phe Tyr Asp Tyr Val Lys Ile Ser Ala
   65                  70                  75                  80
   Asp Lys Lys Ser Leu Gly Arg Phe Cys Gly Gln Leu Gly Ser Pro Leu
                   85                  90                  95
   Gly Asn Pro Pro Gly Lys Lys Glu Phe Met Ser Gln Gly Asn Lys Met
               100                 105                 110
   Leu Leu Thr Phe His Thr Asp Phe Ser Asn Glu Glu Asn Gly Thr Ile
           115                 120                 125
   Met Phe Tyr Lys Gly Phe Leu Ala Tyr Tyr Gln Ala Val Asp Leu Asp
       130                 135                 140
   Glu Cys Ala Ser Arg Ser Lys Ser Gly Glu Glu Asp Pro Gln Pro Gln
   145                 150                 155                 160
   Cys Gln His Leu Cys His Asn Tyr Val Gly Gly Tyr Phe Cys Ser Cys
                   165                 170                 175
   Arg Pro Gly Tyr Glu Leu Gln Glu Asp Thr His Ser Cys Gln Ala Glu
               180                 185                 190
   Cys Ser Ser Glu Leu Tyr Thr Glu Ala Ser Gly Tyr Ile Ser Ser Leu
           195                 200                 205
   Glu Tyr Pro Arg Ser Tyr Pro Pro Asp Leu Arg Cys Asn Tyr Ser Ile
       210                 215                 220
   Arg Val Glu Arg Gly Leu Thr Leu His Leu Lys Phe Leu Glu Pro Phe
   225                 230                 235                 240
   Asp Ile Asp Asp His Gln Gln Val His Cys Pro Tyr Asp Gln Leu Gln
                   245                 250                 255
   Ile Tyr Ala Asn Gly Lys Asn Ile Gly Glu Phe Cys Gly Lys Gln Arg
               260                 265                 270
   Pro Pro Asp Leu Asp Thr Ser Ser Asn Ala Val Asp Leu Leu Phe Phe
           275                 280                 285
   Thr Asp Glu Ser Gly Asp Ser Arg Gly Trp Lys Leu Arg Tyr Thr Thr
       290                 295                 300
   Glu Ile Ile Lys Cys Pro Gln Pro Lys Thr Leu Asp Glu Phe Thr Ile
   305                 310                 315                 320
   Ile Gln Asn Leu Gln Pro Gln Tyr Gln Phe Arg Asp Tyr Phe Ile Ala
                   325                 330                 335
   Thr Cys Lys Gln Gly Tyr Gln Leu Ile Glu Gly Asn Gln Val Leu His
               340                 345                 350
   Ser Phe Thr Ala Val Cys Gln Asp Asp Gly Thr Trp His Arg Ala Met
           355                 360                 365
   Pro Arg Cys Lys Ile Lys Asp Cys Gly Gln Pro Arg Asn Leu Pro Asn
       370                 375                 380
   Gly Asp Phe Arg Tyr Thr Thr Thr Met Gly Val Asn Thr Tyr Lys Ala
   385                 390                 395                 400
   Arg Ile Gln Tyr Tyr Cys His Glu Pro Tyr Tyr Lys Met Gln Thr Arg
                   405                 410                 415
   Ala Gly Ser Arg Glu Ser Glu Gln Gly Val Tyr Thr Cys Thr Ala Gln
               420                 425                 430
   Gly Ile Trp Lys Asn Glu Gln Lys Gly Glu Lys Ile Pro Arg Cys Leu
           435                 440                 445
   Pro Val Cys Gly Lys Pro Val Asn Pro Val Glu Gln Arg Gln Arg Ile
       450                 455                 460
   Ile Gly Gly Gln Lys Ala Lys Met Gly Asn Phe Pro Trp Gln Val Phe
   465                 470                 475                 480
   Thr Asn Ile His Gly Arg Gly Gly Gly Ala Leu Leu Gly Asp Arg Trp
                   485                 490                 495
   Ile Leu Thr Ala Ala His Thr Leu Tyr Pro Lys Glu His Glu Ala Gln
               500                 505                 510
   Ser Asn Ala Ser Leu Asp Val Phe Leu Gly His Thr Asn Val Glu Glu
           515                 520                 525
   Leu Met Lys Leu Gly Asn His Pro Ile Arg Arg Val Ser Val His Pro
       530                 535                 540
   Asp Tyr Arg Gln Asp Glu Ser Tyr Asn Phe Glu Gly Asp Ile Ala Leu
   545                 550                 555                 560
   Leu Glu Leu Glu Asn Ser Val Thr Leu Gly Pro Asn Leu Leu Pro Ile
                   565                 570                 575
   Cys Leu Pro Asp Asn Asp Thr Phe Tyr Asp Leu Gly Leu Met Gly Tyr
               580                 585                 590
   Val Ser Gly Phe Gly Val Met Glu Glu Lys Ile Ala His Asp Leu Arg
           595                 600                 605
   Phe Val Arg Leu Pro Val Ala Asn Pro Gln Ala Cys Glu Asn Trp Leu
       610                 615                 620
   Arg Gly Lys Asn Arg Met Asp Val Phe Ser Gln Asn Met Phe Cys Ala
   625                 630                 635                 640
   Gly His Pro Ser Leu Lys Gln Asp Ala Cys Gln Gly Asp Ser Gly Gly
                   645                 650                 655
   Val Phe Ala Val Arg Asp Pro Asn Thr Asp Arg Trp Val Ala Thr Gly
               660                 665                 670
   Ile Val Ser Trp Gly Ile Gly Cys Ser Arg Gly Tyr Gly Phe Tyr Thr
           675                 680                 685
   Lys Val Leu Asn Tyr Val Asp Trp Ile Lys Lys Glu Met Glu Glu Glu
       690                 695                 700
   Asp
   705
<210> 5
   <211> 688
   <212> PRT
   <213> Homo sapiens
<400> 5
<210> 6
   <211> 1663
   <212> PRT
   <213> Homo sapiens
<400> 6
<210> 7
   <211> 1231
   <212> PRT
   <213> Homo sapiens
<400> 7
   Met Arg Leu Leu Ala Lys Ile Ile Cys Leu Met Leu Trp Ala Ile Cys
    1               5                  10                  15
   Val Ala Glu Asp Cys Asn Glu Leu Pro Pro Arg Arg Asn Thr Glu Ile
               20                  25                  30
   Leu Thr Gly Ser Trp Ser Asp Gln Thr Tyr Pro Glu Gly Thr Gln Ala
           35                  40                  45
   Ile Tyr Lys Cys Arg Pro Gly Tyr Arg Ser Leu Gly Asn Val Ile Met
       50                  55                  60
   Val Cys Arg Lys Gly Glu Trp Val Ala Leu Asn Pro Leu Arg Lys Cys
   65                  70                  75                  80
   Gln Lys Arg Pro Cys Gly His Pro Gly Asp Thr Pro Phe Gly Thr Phe
                   85                  90                  95
   Thr Leu Thr Gly Gly Asn Val Phe Glu Tyr Gly Val Lys Ala Val Tyr
               100                 105                 110
   Thr Cys Asn Glu Gly Tyr Gln Leu Leu Gly Glu Ile Asn Tyr Arg Glu
           115                 120                 125
   Cys Asp Thr Asp Gly Trp Thr Asn Asp Ile Pro Ile Cys Glu Val Val
       130                 135                 140
   Lys Cys Leu Pro Val Thr Ala Pro Glu Asn Gly Lys Ile Val Ser Ser
   145                 150                 155                 160
   Ala Met Glu Pro Asp Arg Glu Tyr His Phe Gly Gln Ala Val Arg Phe
                   165                 170                 175
   Val Cys Asn Ser Gly Tyr Lys Ile Glu Gly Asp Glu Glu Met His Cys
               180                 185                 190
   Ser Asp Asp Gly Phe Trp Ser Lys Glu Lys Pro Lys Cys Val Glu Ile
           195                 200                 205
   Ser Cys Lys Ser Pro Asp Val Ile Asn Gly Ser Pro Ile Ser Gln Lys
       210                 215                 220
   Ile Ile Tyr Lys Glu Asn Glu Arg Phe Gln Tyr Lys Cys Asn Met Gly
   225                 230                 235                 240
   Tyr Glu Tyr Ser Glu Arg Gly Asp Ala Val Cys Thr Glu Ser Gly Trp
                   245                 250                 255
   Arg Pro Leu Pro Ser Cys Glu Glu Lys Ser Cys Asp Asn Pro Tyr Ile
               260                 265                 270
   Pro Asn Gly Asp Tyr Ser Pro Leu Arg Ile Lys His Arg Thr Gly Asp
           275                 280                 285
   Glu Ile Thr Tyr Gln Cys Arg Asn Gly Phe Tyr Pro Ala Thr Arg Gly
       290                 295                 300
   Asn Thr Ala Lys Cys Thr Ser Thr Gly Trp Ile Pro Ala Pro Arg Cys
   305                 310                 315                 320
   Thr Leu Lys Pro Cys Asp Tyr Pro Asp Ile Lys His Gly Gly Leu Tyr
                   325                 330                 335
   His Glu Asn Met Arg Arg Pro Tyr Phe Pro Val Ala Val Gly Lys Tyr
               340                 345                 350
   Tyr Ser Tyr Tyr Cys Asp Glu His Phe Glu Thr Pro Ser Gly Ser Tyr
           355                 360                 365
   Trp Asp His Ile His Cys Thr Gln Asp Gly Trp Ser Pro Ala Val Pro
       370                 375                 380
   Cys Leu Arg Lys Cys Tyr Phe Pro Tyr Leu Glu Asn Gly Tyr Asn Gln
   385                 390                 395                 400
   Asn Tyr Gly Arg Lys Phe Val Gln Gly Lys Ser Ile Asp Val Ala Cys
                   405                 410                 415
   His Pro Gly Tyr Ala Leu Pro Lys Ala Gln Thr Thr Val Thr Cys Met
               420                 425                 430
   Glu Asn Gly Trp Ser Pro Thr Pro Arg Cys Ile Arg Val Lys Thr Cys
           435                 440                 445
   Ser Lys Ser Ser Ile Asp Ile Glu Asn Gly Phe Ile Ser Glu Ser Gln
       450                 455                 460
   Tyr Thr Tyr Ala Leu Lys Glu Lys Ala Lys Tyr Gln Cys Lys Leu Gly
   465                 470                 475                 480
   Tyr Val Thr Ala Asp Gly Glu Thr Ser Gly Ser Ile Thr Cys Gly Lys
                   485                 490                 495
   Asp Gly Trp Ser Ala Gln Pro Thr Cys Ile Lys Ser Cys Asp Ile Pro
               500                 505                 510
   Val Phe Met Asn Ala Arg Thr Lys Asn Asp Phe Thr Trp Phe Lys Leu
           515                 520                 525
   Asn Asp Thr Leu Asp Tyr Glu Cys His Asp Gly Tyr Glu Ser Asn Thr
       530                 535                 540
   Gly Ser Thr Thr Gly Ser Ile Val Cys Gly Tyr Asn Gly Trp Ser Asp
   545                 550                 555                 560
   Leu Pro Ile Cys Tyr Glu Arg Glu Cys Glu Leu Pro Lys Ile Asp Val
                   565                 570                 575
   His Leu Val Pro Asp Arg Lys Lys Asp Gln Tyr Lys Val Gly Glu Val
               580                 585                 590
   Leu Lys Phe Ser Cys Lys Pro Gly Phe Thr Ile Val Gly Pro Asn Ser
           595                 600                 605
   Val Gln Cys Tyr His Phe Gly Leu Ser Pro Asp Leu Pro Ile Cys Lys
       610                 615                 620
   Glu Gln Val Gln Ser Cys Gly Pro Pro Pro Glu Leu Leu Asn Gly Asn
   625                 630                 635                 640
   Val Lys Glu Lys Thr Lys Glu Glu Tyr Gly His Ser Glu Val Val Glu
                   645                 650                 655
   Tyr Tyr Cys Asn Pro Arg Phe Leu Met Lys Gly Pro Asn Lys Ile Gln
               660                 665                 670
   Cys Val Asp Gly Glu Trp Thr Thr Leu Pro Val Cys Ile Val Glu Glu
           675                 680                 685
   Ser Thr Cys Gly Asp Ile Pro Glu Leu Glu His Gly Trp Ala Gln Leu
       690                 695                 700
   Ser Ser Pro Pro Tyr Tyr Tyr Gly Asp Ser Val Glu Phe Asn Cys Ser
   705                 710                 715                 720
   Glu Ser Phe Thr Met Ile Gly His Arg Ser Ile Thr Cys Ile His Gly
                   725                 730                 735
   Val Trp Thr Gln Leu Pro Gln Cys Val Ala Ile Asp Lys Leu Lys Lys
               740                 745                 750
   Cys Lys Ser Ser Asn Leu Ile Ile Leu Glu Glu His Leu Lys Asn Lys
           755                 760                 765
   Lys Glu Phe Asp His Asn Ser Asn Ile Arg Tyr Arg Cys Arg Gly Lys
       770                 775                 780
   Glu Gly Trp Ile His Thr Val Cys Ile Asn Gly Arg Trp Asp Pro Glu
   785                 790                 795                 800
   Val Asn Cys Ser Met Ala Gln Ile Gln Leu Cys Pro Pro Pro Pro Gln
                   805                 810                 815
   Ile Pro Asn Ser His Asn Met Thr Thr Thr Leu Asn Tyr Arg Asp Gly
               820                 825                 830
   Glu Lys Val Ser Val Leu Cys Gln Glu Asn Tyr Leu Ile Gln Glu Gly
           835                 840                 845
   Glu Glu Ile Thr Cys Lys Asp Gly Arg Trp Gln Ser Ile Pro Leu Cys
       850                 855                 860
   Val Glu Lys Ile Pro Cys Ser Gln Pro Pro Gln Ile Glu His Gly Thr
   865                 870                 875                 880
   Ile Asn Ser Ser Arg Ser Ser Gln Glu Ser Tyr Ala His Gly Thr Lys
                   885                 890                 895
   Leu Ser Tyr Thr Cys Glu Gly Gly Phe Arg Ile Ser Glu Glu Asn Glu
               900                 905                 910
   Thr Thr Cys Tyr Met Gly Lys Trp Ser Ser Pro Pro Gln Cys Glu Gly
           915                 920                 925
   Leu Pro Cys Lys Ser Pro Pro Glu Ile Ser His Gly Val Val Ala His
       930                 935                 940
   Met Ser Asp Ser Tyr Gln Tyr Gly Glu Glu Val Thr Tyr Lys Cys Phe
   945                 950                 955                 960
   Glu Gly Phe Gly Ile Asp Gly Pro Ala Ile Ala Lys Cys Leu Gly Glu
                   965                 970                 975
   Lys Trp Ser His Pro Pro Ser Cys Ile Lys Thr Asp Cys Leu Ser Leu
                   980                 985                 990
   Pro Ser Phe Glu Asn Ala Ile Pro Met Gly Glu Lys Lys Asp Val Tyr
               995                 1000                1005
   Lys Ala Gly Glu Gln Val Thr Tyr Thr Cys Ala Thr Tyr Tyr Lys Met
       1010                1015                1020
   Asp Gly Ala Ser Asn Val Thr Cys Ile Asn Ser Arg Trp Thr Gly Arg
   1025                1030                1035                1040
   Pro Thr Cys Arg Asp Thr Ser Cys Val Asn Pro Pro Thr Val Gln Asn
                   1045                1050                1055
   Ala Tyr Ile Val Ser Arg Gln Met Ser Lys Tyr Pro Ser Gly Glu Arg
               1060                1065                1070
   Val Arg Tyr Gln Cys Arg Ser Pro Tyr Glu Met Phe Gly Asp Glu Glu
           1075                1080                1085
   Val Met Cys Leu Asn Gly Asn Trp Thr Glu Pro Pro Gln Cys Lys Asp
       1090                1095                1100
   Ser Thr Gly Lys Cys Gly Pro Pro Pro Pro Ile Asp Asn Gly Asp Ile
   1105                1110                1115                1120
   Thr Ser Phe Pro Leu Ser Val Tyr Ala Pro Ala Ser Ser Val Glu Tyr
                   1125                1130                1135
   Gln Cys Gln Asn Leu Tyr Gln Leu Glu Gly Asn Lys Arg Ile Thr Cys
               1140                1145                1150
   Arg Asn Gly Gln Trp Ser Glu Pro Pro Lys Cys Leu His Pro Cys Val
           1155                1160                1165
   Ile Ser Arg Glu Ile Met Glu Asn Tyr Asn Ile Ala Leu Arg Trp Thr
       1170                1175                1180
   Ala Lys Gln Lys Leu Tyr Ser Arg Thr Gly Glu Ser Val Glu Phe Val
   1185                1190                1195                1200
   Cys Lys Arg Gly Tyr Arg Leu Ser Ser Arg Ser His Thr Leu Arg Thr
                   1205                1210                1215
   Thr Cys Trp Asp Gly Lys Leu Glu Tyr Pro Thr Cys Ala Lys Arg
               1220                1225                1230
<210> 8
   <211> 518
   <212> PRT
   <213> Homo sapiens
<400> 8
<210> 9
   <211> 482
   <212> PRT
   <213> Homo sapiens
<400> 9
<210> 10
   <211> 644
   <212> PRT
   <213> Homo sapiens
<400> 10
<210> 11
   <211> 584
   <212> **PRT**
   <213> Homo sapiens
<400> 11
<210> 12
   <211> 585
   <212> PRT
   <213> Homo sapiens
<400> 12
   Met Thr Pro Pro Arg Leu Phe Trp Val Trp Leu Leu Val Ala Gly Thr
    1               5                  10                  15
   Gln Gly Val Asn Asp Gly Asp Met Arg Leu Ala Asp Gly Gly Ala Thr
               20                  25                  30
   Asn Gln Gly Arg Val Glu Ile Phe Tyr Arg Gly Gln Trp Gly Thr Val
           35                  40                  45
   Cys Asp Asn Leu Trp Asp Leu Thr Asp Ala Ser Val Val Cys Arg Ala
       50                  55                  60
   Leu Gly Phe Glu Asn Ala Thr Gln Ala Leu Gly Arg Ala Ala Phe Gly
   65                  70                  75                  80
   Gln Gly Ser Gly Pro Ile Met Leu Asp Glu Val Gln Cys Thr Gly Thr
                   85                  90                  95
   Glu Ala Ser Leu Ala Asp Cys Lys Ser Leu Gly Trp Leu Lys Ser Asn
               100                 105                 110
   Cys Arg His Glu Arg Asp Ala Gly Val Val Cys Thr Asn Glu Thr Arg
           115                 120                 125
   Ser Thr His Thr Leu Asp Leu Ser Arg Glu Leu Ser Glu Ala Leu Gly
       130                 135                 140
   Gln Ile Phe Asp Ser Gln Arg Gly Cys Asp Leu Ser Ile Ser Val Asn
   145                 150                 155                 160
   Val Gln Gly Glu Asp Ala Leu Gly Phe Cys Gly His Thr Val Ile Leu
                   165                 170                 175
   Thr Ala Asn Leu Glu Ala Gln Ala Leu Trp Lys Glu Pro Gly Ser Asn
                   180                 185                 190
   Val Thr Met Ser Val Asp Ala Glu Cys Val Pro Met Val Arg Asp Leu
               195                 200                 205
   Leu Arg Tyr Phe Tyr Ser Arg Arg Ile Asp Ile Thr Leu Ser Ser Val
       210                 215                 220
   Lys Cys Phe His Lys Leu Ala Ser Ala Tyr Gly Ala Arg Gln Leu Gln
   225                 230                 235                 240
   Gly Tyr Cys Ala Ser Leu Phe Ala Ile Leu Leu Pro Gln Asp Pro Ser
                   245                 250                 255
   Phe Gln Met Pro Leu Asp Leu Tyr Ala Tyr Ala Val Ala Thr Gly Asp
               260                 265                 270
   Ala Leu Leu Glu Lys Leu Cys Leu Gln Phe Leu Ala Trp Asn Phe Glu
           275                 280                 285
   Ala Leu Thr Gln Ala Glu Ala Trp Pro Ser Val Pro Thr Asp Leu Leu
       290                 295                 300
   Gln Leu Leu Leu Pro Arg Ser Asp Leu Ala Val Pro Ser Glu Leu Ala
   305                 310                 315                 320
   Leu Leu Lys Ala Val Asp Thr Trp Ser Trp Gly Glu Arg Ala Ser His
                   325                 330                 335
   Glu Glu Val Glu Gly Leu Val Glu Lys Ile Arg Phe Pro Met Met Leu
               340                 345                 350
   Pro Glu Glu Leu Phe Glu Leu Gln Phe Asn Leu Ser Leu Tyr Trp Ser
           355                 360                 365
   His Glu Ala Leu Phe Gln Lys Lys Thr Leu Gln Ala Leu Glu Phe His
       370                 375                 380
   Thr Val Pro Phe Gln Leu Leu Ala Arg Tyr Lys Gly Leu Asn Leu Thr
   385                 390                 395                 400
   Glu Asp Thr Tyr Lys Pro Arg Ile Tyr Thr Ser Pro Thr Trp Ser Ala
                   405                 410                 415
   Phe Val Thr Asp Ser Ser Trp Ser Ala Arg Lys Ser Gln Leu Val Tyr
               420                 425                 430
   Gln Ser Arg Arg Gly Pro Leu Val Lys Tyr Ser Ser Asp Tyr Phe Gln
           435                 440                 445
   Ala Pro Ser Asp Tyr Arg Tyr Tyr Pro Tyr Gln Ser Phe Gln Thr Pro
       450                 455                 460
   Gln His Pro Ser Phe Leu Phe Gln Asp Lys Arg Val Ser Trp Ser Leu
   465                 470                 475                 480
   Val Tyr Leu Pro Thr Ile Gln Ser Cys Trp Asn Tyr Gly Phe Ser Cys
                   485                 490                 495
   Ser Ser Asp Glu Leu Pro Val Leu Gly Leu Thr Lys Ser Gly Gly Ser
               500                 505                 510
   Asp Arg Thr Ile Ala Tyr Glu Asn Lys Ala Leu Met Leu Cys Glu Gly
           515                 520                 525
   Leu Phe Val Ala Asp Val Thr Asp Phe Glu Gly Trp Lys Ala Ala Ile
       530                 535                 540
   Pro Ser Ala Leu Asp Thr Asn Ser Ser Lys Ser Thr Ser Ser Phe Pro
   545                 550                 555                 560
   Cys Pro Ala Gly His Phe Asn Gly Phe Arg Thr Val Ile Arg Pro Phe
                   565                 570                 575
   Tyr Leu Thr Asn Ser Ser Gly Val Asp
               580                 585
<210> 13
   <211> 114
   <212> PRT
   <213> Homo sapiens
<400> 13
   Met Thr Cys Lys Met Ser Gln Leu Glu Arg Asn Ile Glu Thr Ile Ile
    1               5                  10                  15
   Asn Thr Phe His Gln Tyr Ser Val Lys Leu Gly His Pro Asp Thr Leu
               20                  25                  30
   Asn Gln Gly Glu Phe Lys Glu Leu Val Arg Lys Asp Leu Gln Asn Phe
           35                  40                  45
   Leu Lys Lys Glu Asn Lys Asn Glu Lys Val Ile Glu His Ile Met Glu
       50                  55                  60
   Asp Leu Asp Thr Asn Ala Asp Lys Gln Leu Ser Phe Glu Glu Phe Ile
   65                  70                  75                  80
   Met Leu Met Ala Arg Leu Thr Trp Ala Ser His Glu Lys Met His Glu
                   85                  90                  95
   Gly Asp Glu Gly Pro Gly His His His Lys Pro Gly Leu Gly Glu Gly
               100                 105                 110
   Thr Pro
<210> 14
   <211> 418
   <212> PRT
   <213> Homo sapiens
<400> 14
<210> 15
   <211> 495
   <212> PRT
   <213> Homo sapiens
<400> 15
   Met Ser Met Leu Val Val Phe Leu Leu Leu Trp Gly Val Thr Trp Gly
    1               5                  10                  15
   Pro Val Thr Glu Ala Ala Ile Phe Tyr Glu Thr Gln Pro Ser Leu Trp
               20                  25                  30
   Ala Glu Ser Glu Ser Leu Leu Lys Pro Leu Ala Asn Val Thr Leu Thr
           35                  40                  45
   Cys Gln Ala His Leu Glu Thr Pro Asp Phe Gln Leu Phe Lys Asn Gly
       50                  55                  60
   Val Ala Gln Glu Pro Val His Leu Asp Ser Pro Ala Ile Lys His Gln
   65                  70                  75                  80
   Phe Leu Leu Thr Gly Asp Thr Gln Gly Arg Tyr Arg Cys Arg Ser Gly
                   85                  90                  95
   Leu Ser Thr Gly Trp Thr Gln Leu Ser Lys Leu Leu Glu Leu Thr Gly
               100                 105                 110
   Pro Lys Ser Leu Pro Ala Pro Trp Leu Ser Met Ala Pro Val Ser Trp
           115                 120                 125
   Ile Thr Pro Gly Leu Lys Thr Thr Ala Val Cys Arg Gly Val Leu Arg
       130                 135                 140
   Gly Val Thr Phe Leu Leu Arg Arg Glu Gly Asp His Glu Phe Leu Glu
   145                 150                 155                 160
   Val Pro Glu Ala Gln Glu Asp Val Glu Ala Thr Phe Pro Val His Gln
                   165                 170                 175
   Pro Gly Asn Tyr Ser Cys Ser Tyr Arg Thr Asp Gly Glu Gly Ala Leu
               180                 185                 190
   Ser Glu Pro Ser Ala Thr Val Thr Ile Glu Glu Leu Ala Ala Pro Pro
           195                 200                 205
   Pro Pro Val Leu Met His His Gly Glu Ser Ser Gln Val Leu His Pro
       210                 215                 220
   Gly Asn Lys Val Thr Leu Thr Cys Val Ala Pro Leu Ser Gly Val Asp
   225                 230                 235                 240
   Phe Gln Leu Arg Arg Gly Glu Lys Glu Leu Leu Val Pro Arg Ser Ser
                   245                 250                 255
   Thr Ser Pro Asp Arg Ile Phe Phe His Leu Asn Ala Val Ala Leu Gly
               260                 265                 270
   Asp Gly Gly His Tyr Thr Cys Arg Tyr Arg Leu His Asp Asn Gln Asn
           275                 280                 285
   Gly Trp Ser Gly Asp Ser Ala Pro Val Glu Leu Ile Leu Ser Asp Glu
       290                 295                 300
   Thr Leu Pro Ala Pro Glu Phe Ser Pro Glu Pro Glu Ser Gly Arg Ala
   305                 310                 315                 320
   Leu Arg Leu Arg Cys Leu Ala Pro Leu Glu Gly Ala Arg Phe Ala Leu
                   325                 330                 335
   Val Arg Glu Asp Arg Gly Gly Arg Arg Val His Arg Phe Gln Ser Pro
               340                 345                 350
   Ala Gly Thr Glu Ala Leu Phe Glu Leu His Asn Ile Ser Val Ala Asp
           355                 360                 365
   Ser Ala Asn Tyr Ser Cys Val Tyr Val Asp Leu Lys Pro Pro Phe Gly
       370                 375                 380
   Gly Ser Ala Pro Ser Glu Arg Leu Glu Leu His Val Asp Gly Pro Pro
   385                 390                 395                 400
   Pro Arg Pro Gln Leu Arg Ala Thr Trp Ser Gly Ala Val Leu Ala Gly
                   405                 410                 415
   Arg Asp Ala Val Leu Arg Cys Glu Gly Pro Ile Pro Asp Val Thr Phe
               420                 425                 430
   Glu Leu Leu Arg Glu Gly Glu Thr Lys Ala Val Lys Thr Val Arg Thr
           435                 440                 445
   Pro Gly Ala Ala Ala Asn Leu Glu Leu Ile Phe Val Gly Pro Gln His
       450                 455                 460
   Ala Gly Asn Tyr Arg Cys Arg Tyr Arg Ser Trp Val Pro His Thr Phe
   465                 470                 475                 480
   Glu Ser Glu Leu Ser Asp Pro Val Glu Leu Leu Val Ala Glu Ser
                   485                 490                 495
<210> 16
   <211> 609
   <212> PRT
   <213> Homo sapiens
<400> 16
<210> 17
   <211> 398
   <212> PRT
   <213> Homo sapiens
<400> 17
   Met Glu Gly Ala Ala Leu Leu Arg Val Ser Val Leu Cys Ile Trp Met
    1               5                  10                  15
   Ser Ala Leu Phe Leu Gly Val Gly Val Arg Ala Glu Glu Ala Gly Ala
               20                  25                  30
   Arg Val Gln Gln Asn Val Pro Ser Gly Thr Asp Thr Gly Asp Pro Gln
           35                  40                  45
   Ser Lys Pro Leu Gly Asp Trp Ala Ala Gly Thr Met Asp Pro Glu Ser
       50                  55                  60
   Ser Ile Phe Ile Glu Asp Ala Ile Lys Tyr Phe Lys Glu Lys Val Ser
   65                  70                  75                  80
   Thr Gln Asn Leu Leu Leu Leu Leu Thr Asp Asn Glu Ala Trp Asn Gly
                   85                  90                  95
   Phe Val Ala Ala Ala Glu Leu Pro Arg Asn Glu Ala Asp Glu Leu Arg
               100                 105                 110
   Lys Ala Leu Asp Asn Leu Ala Arg Gln Met Ile Met Lys Asp Lys Asn
           115                 120                 125
   Trp His Asp Lys Gly Gln Gln Tyr Arg Asn Trp Phe Leu Lys Glu Phe
       130                 135                 140
   Pro Arg Leu Lys Ser Glu Leu Glu Asp Asn Ile Arg Arg Leu Arg Ala
   145                 150                 155                 160
   Leu Ala Asp Gly Val Gln Lys Val His Lys Gly Thr Thr Ile Ala Asn
                   165                 170                 175
   Val Val Ser Gly Ser Leu Ser Ile Ser Ser Gly Ile Leu Thr Leu Val
               180                 185                 190
   Gly Met Gly Leu Ala Pro Phe Thr Glu Gly Gly Ser Leu Val Leu Leu
           195                 200                 205
   Glu Pro Gly Met Glu Leu Gly Ile Thr Ala Ala Leu Thr Gly Ile Thr
       210                 215                 220
   Ser Ser Thr Met Asp Tyr Gly Lys Lys Trp Trp Thr Gln Ala Gln Ala
   225                 230                 235                 240
   His Asp Leu Val Ile Lys Ser Leu Asp Lys Leu Lys Glu Val Arg Glu
                   245                 250                 255
   Phe Leu Gly Glu Asn Ile Ser Asn Phe Leu Ser Leu Ala Gly Asn Thr
               260                 265                 270
   Tyr Gln Leu Thr Arg Gly Ile Gly Lys Asp Ile Arg Ala Leu Arg Arg
           275                 280                 285
   Ala Arg Ala Asn Leu Gln Ser Val Pro His Ala Ser Ala Ser Arg Pro
       290                 295                 300
   Arg Val Thr Glu Pro Ile Ser Ala Glu Ser Gly Glu Gln Val Glu Arg
   305                 310                 315                 320
   Val Asn Glu Pro Ser Ile Leu Glu Met Ser Arg Gly Val Lys Leu Thr
                   325                 330                 335
   Asp Val Ala Pro Val Ser Phe Phe Leu Val Leu Asp Val Val Tyr Leu
               340                 345                 350
   Val Tyr Glu Ser Lys His Leu His Glu Gly Ala Lys Ser Glu Thr Ala
           355                 360                 365
   Glu Glu Leu Lys Lys Val Ala Gln Glu Leu Glu Glu Lys Leu Asn Ile
       370                 375                 380
   Leu Asn Asn Asn Tyr Lys Ile Leu Gln Ala Asp Gln Glu Leu
   385                 390                 395
<210> 18
   <211> 1744
   <212> PRT
   <213> Homo sapiens
<400> 18
   Met Arg Leu Leu Trp Gly Leu Ile Trp Ala Ser Ser Phe Phe Thr Leu
    1               5                  10                  15
   Ser Leu Gln Lys Pro Arg Leu Leu Leu Phe Ser Pro Ser Val Val His
               20                  25                  30
   Leu Gly Val Pro Leu Ser Val Gly Val Gln Leu Gln Asp Val Pro Arg
           35                  40                  45
   Gly Gln Val Val Lys Gly Ser Val Phe Leu Arg Asn Pro Ser Arg Asn
       50                  55                  60
   Asn Val Pro Cys Ser Pro Lys Val Asp Phe Thr Leu Ser Ser Glu Arg
   65                  70                  75                  80
   Asp Phe Ala Leu Leu Ser Leu Gln Val Pro Leu Lys Asp Ala Lys Ser
                   85                  90                  95
   Cys Gly Leu His Gln Leu Leu Arg Gly Pro Glu Val Gln Leu Val Ala
               100                 105                 110
   His Ser Pro Trp Leu Lys Asp Ser Leu Ser Arg Thr Thr Asn Ile Gln
           115                 120                 125
   Gly Ile Asn Leu Leu Phe Ser Ser Arg Arg Gly His Leu Phe Leu Gln
       130                 135                 140
   Thr Asp Gln Pro Ile Tyr Asn Pro Gly Gln Arg Val Arg Tyr Arg Val
   145                 150                 155                 160
   Phe Ala Leu Asp Gln Lys Met Arg Pro Ser Thr Asp Thr Ile Thr Val
                   165                 170                 175
   Met Val Glu Asn Ser His Gly Leu Arg Val Arg Lys Lys Glu Val Tyr
               180                 185                 190
   Met Pro Ser Ser Ile Phe Gln Asp Asp Phe Val Ile Pro Asp Ile Ser
           195                 200                 205
   Glu Pro Gly Thr Trp Lys Ile Ser Ala Arg Phe Ser Asp Gly Leu Glu
       210                 215                 220
   Ser Asn Ser Ser Thr Gln Phe Glu Val Lys Lys Tyr Val Leu Pro Asn
   225                 230                 235                 240
   Phe Glu Val Lys Ile Thr Pro Gly Lys Pro Tyr Ile Leu Thr Val Pro
                   245                 250                 255
   Gly His Leu Asp Glu Met Gln Leu Asp Ile Gln Ala Arg Tyr Ile Tyr
               260                 265                 270
   Gly Lys Pro Val Gln Gly Val Ala Tyr Val Arg Phe Gly Leu Leu Asp
           275                 280                 285
   Glu Asp Gly Lys Lys Thr Phe Phe Arg Gly Leu Glu Ser Gln Thr Lys
       290                 295                 300
   Leu Val Asn Gly Gln Ser His Ile Ser Leu Ser Lys Ala Glu Phe Gln
   305                 310                 315                 320
   Asp Ala Leu Glu Lys Leu Asn Met Gly Ile Thr Asp Leu Gln Gly Leu
                   325                 330                 335
   Arg Leu Tyr Val Ala Ala Ala Ile Ile Glu Ser Pro Gly Gly Glu Met
               340                 345                 350
   Glu Glu Ala Glu Leu Thr Ser Trp Tyr Phe Val Ser Ser Pro Phe Ser
           355                 360                 365
   Leu Asp Leu Ser Lys Thr Lys Arg His Leu Val Pro Gly Ala Pro Phe
       370                 375                 380
   Leu Leu Gln Ala Leu Val Arg Glu Met Ser Gly Ser Pro Ala Ser Gly
   385                 390                 395                 400
   Ile Pro Val Lys Val Ser Ala Thr Val Ser Ser Pro Gly Ser Val Pro
                   405                 410                 415
   Glu Val Gln Asp Ile Gln Gln Asn Thr Asp Gly Ser Gly Gln Val Ser
               420                 425                 430
   Ile Pro Ile Ile Ile Pro Gln Thr Ile Ser Glu Leu Gln Leu Ser Val
           435                 440                 445
   Ser Ala Gly Ser Pro His Pro Ala Ile Ala Arg Leu Thr Val Ala Ala
       450                 455                 460
   Pro Pro Ser Gly Gly Pro Gly Phe Leu Ser Ile Glu Arg Pro Asp Ser
   465                 470                 475                 480
   Arg Pro Pro Arg Val Gly Asp Thr Leu Asn Leu Asn Leu Arg Ala Val
                   485                 490                 495
   Gly Ser Gly Ala Thr Phe Ser His Tyr Tyr Tyr Met Ile Leu Ser Arg
               500                 505                 510
   Gly Gln Ile Val Phe Met Asn Arg Glu Pro Lys Arg Thr Leu Thr Ser
           515                 520                 525
   Val Ser Val Phe Val Asp His His Leu Ala Pro Ser Phe Tyr Phe Val
       530                 535                 540
   Ala Phe Tyr Tyr His Gly Asp His Pro Val Ala Asn Ser Leu Arg Val
   545                 550                 555                 560
   Asp Val Gln Ala Gly Ala Cys Glu Gly Lys Leu Glu Leu Ser Val Asp
                   565                 570                 575
   Gly Ala Lys Gln Tyr Arg Asn Gly Glu Ser Val Lys Leu His Leu Glu
               580                 585                 590
   Thr Asp Ser Leu Ala Leu Val Ala Leu Gly Ala Leu Asp Thr Ala Leu
           595                 600                 605
   Tyr Ala Ala Gly Ser Lys Ser His Lys Pro Leu Asn Met Gly Lys Val
       610                 615                 620
   Phe Glu Ala Met Asn Ser Tyr Asp Leu Gly Cys Gly Pro Gly Gly Gly
   625                 630                 635                 640
   Asp Ser Ala Leu Gln Val Phe Gln Ala Ala Gly Leu Ala Phe Ser Asp
                   645                 650                 655
   Gly Asp Gln Trp Thr Leu Ser Arg Lys Arg Leu Ser Cys Pro Lys Glu
               660                 665                 670
   Lys Thr Thr Arg Lys Lys Arg Asn Val Asn Phe Gln Lys Ala Ile Asn
           675                 680                 685
   Glu Lys Leu Gly Gln Tyr Ala Ser Pro Thr Ala Lys Arg Cys Cys Gln
       690                 695                 700
   Asp Gly Val Thr Arg Leu Pro Met Met Arg Ser Cys Glu Gln Arg Ala
   705                 710                 715                 720
   Ala Arg Val Gln Gln Pro Asp Cys Arg Glu Pro Phe Leu Ser Cys Cys
                   725                 730                 735
   Gln Phe Ala Glu Ser Leu Arg Lys Lys Ser Arg Asp Lys Gly Gln Ala
               740                 745                 750
   Gly Leu Gln Arg Ala Leu Glu Ile Leu Gln Glu Glu Asp Leu Ile Asp
           755                 760                 765
   Glu Asp Asp Ile Pro Val Arg Ser Phe Phe Pro Glu Asn Trp Leu Trp
       770                 775                 780
   Arg Val Glu Thr Val Asp Arg Phe Gln Ile Leu Thr Leu Trp Leu Pro
   785                 790                 795                 800
   Asp Ser Leu Thr Thr Trp Glu Ile His Gly Leu Ser Leu Ser Lys Thr
                   805                 810                 815
   Lys Gly Leu Cys Val Ala Thr Pro Val Gln Leu Arg Val Phe Arg Glu
               820                 825                 830
   Phe His Leu His Leu Arg Leu Pro Met Ser Val Arg Arg Phe Glu Gln
           835                 840                 845
   Leu Glu Leu Arg Pro Val Leu Tyr Asn Tyr Leu Asp Lys Asn Leu Thr
       850                 855                 860
   Val Ser Val His Val Ser Pro Val Glu Gly Leu Cys Leu Ala Gly Gly
   865                 870                 875                 880
   Gly Gly Leu Ala Gln Gln Val Leu Val Pro Ala Gly Ser Ala Arg Pro
                   885                 890                 895
   Val Ala Phe Ser Val Val Pro Thr Ala Ala Ala Ala Val Ser Leu Lys
               900                 905                 910
   Val Val Ala Arg Gly Ser Phe Glu Phe Pro Val Gly Asp Ala Val Ser
           915                 920                 925
   Lys Val Leu Gln Ile Glu Lys Glu Gly Ala Ile His Arg Glu Glu Leu
       930                 935                 940
   Val Tyr Glu Leu Asn Pro Leu Asp His Arg Gly Arg Thr Leu Glu Ile
   945                 950                 955                 960
   Pro Gly Asn Ser Asp Pro Asn Met Ile Pro Asp Gly Asp Phe Asn Ser
                   965                 970                 975
   Tyr Val Arg Val Thr Ala Ser Asp Pro Leu Asp Thr Leu Gly Ser Glu
               980                 985                 990
   Gly Ala Leu Ser Pro Gly Gly Val Ala Ser Leu Leu Arg Leu Pro Arg
           995                 1000                1005
   Gly Cys Gly Glu Gln Thr Met Ile Tyr Leu Ala Pro Thr Leu Ala Ala
       1010                1015                1020
   Ser Arg Tyr Leu Asp Lys Thr Glu Gln Trp Ser Thr Leu Pro Pro Glu
   1025                1030                1035                1040
   Thr Lys Asp His Ala Val Asp Leu Ile Gln Lys Gly Tyr Met Arg Ile
                   1045                1050                1055
   Gln Gln Phe Arg Lys Ala Asp Gly Ser Tyr Ala Ala Trp Leu Ser Arg
               1060                1065                1070
   Asp Ser Ser Thr Trp Leu Thr Ala Phe Val Leu Lys Val Leu Ser Leu
           1075                1080                1085
   Ala Gln Glu Gln Val Gly Gly Ser Pro Glu Lys Leu Gln Glu Thr Ser
       1090                1095                1100
   Asn Trp Leu Leu Ser Gln Gln Gln Ala Asp Gly Ser Phe Gln Asp Leu
   1105                1110                1115                1120
   Ser Pro Val Ile His Arg Ser Met Gln Gly Gly Leu Val Gly Asn Asp
                   1125                1130                1135
   Glu Thr Val Ala Leu Thr Ala Phe Val Thr Ile Ala Leu His His Gly
               1140                1145                1150
   Leu Ala Val Phe Gln Asp Glu Gly Ala Glu Pro Leu Lys Gln Arg Val
   1155 1160 1165
   Glu Ala Ser Ile Ser Lys Ala Asn Ser Phe Leu Gly Glu Lys Ala Ser
   1170 1175 1180
   Ala Gly Leu Leu Gly Ala His Ala Ala Ala Ile Thr Ala Tyr Ala Leu
   1185 1190 1195 1200
   Ser Leu Thr Lys Ala Pro Val Asp Leu Leu Gly Val Ala His Asn Asn
   1205 1210 1215
   Leu Met Ala Met Ala Gln Glu Thr Gly Asp Asn Leu Tyr Trp Gly Ser
   1220 1225 1230
   Val Thr Gly Ser Gln Ser Asn Ala Val Ser Pro Thr Pro Ala Pro Arg
   1235 1240 1245
   Asn Pro Ser Asp Pro Met Pro Gln Ala Pro Ala Leu Trp Ile Glu Thr
   1250 1255 1260
   Thr Ala Tyr Ala Leu Leu His Leu Leu Leu His Glu Gly Lys Ala Glu
   1265 1270 1275 1280
   Met Ala Asp Gln Ala Ser Ala Trp Leu Thr Arg Gln Gly Ser Phe Gln
   1285 1290 1295
   Gly Gly Phe Arg Ser Thr Gln Asp Thr Val Ile Ala Leu Asp Ala Leu
   1300 1305 1310
   Ser Ala Tyr Trp Ile Ala Ser His Thr Thr Glu Glu Arg Gly Leu Asn
   1315 1320 1325
   Val Thr Leu Ser Ser Thr Gly Arg Asn Gly Phe Lys Ser His Ala Leu
   1330 1335 1340
   Gln Leu Asn Asn Arg Gln Ile Arg Gly Leu Glu Glu Glu Leu Gln Phe
   1345 1350 1355 1360
   Ser Leu Gly Ser Lys Ile Asn Val Lys Val Gly Gly Asn Ser Lys Gly
   1365 1370 1375
   Thr Leu Lys Val Leu Arg Thr Tyr Asn Val Leu Asp Met Lys Asn Thr
   1380 1385 1390
   Thr Cys Gln Asp Leu Gln Ile Glu Val Thr Val Lys Gly His Val Glu
   1395 1400 1405
   Tyr Thr Met Glu Ala Asn Glu Asp Tyr Glu Asp Tyr Glu Tyr Asp Glu
   1410 1415 1420
   Leu Pro Ala Lys Asp Asp Pro Asp Ala Pro Leu Gln Pro Val Thr Pro
   1425 1430 1435 1440
   Leu Gln Leu Phe Glu Gly Arg Arg Asn Arg Arg Arg Arg Glu Ala Pro
   1445 1450 1455
   Lys Val Val Glu Glu Gln Glu Ser Arg Val His Tyr Thr Val Cys Ile
   1460 1465 1470
   Trp Arg Asn Gly Lys Val Gly Leu Ser Gly Met Ala Ile Ala Asp Val
   1475 1480 1485
   Thr Leu Leu Ser Gly Phe His Ala Leu Arg Ala Asp Leu Glu Lys Leu
   1490 1495 1500
   Thr Ser Leu Ser Asp Arg Tyr Val Ser His Phe Glu Thr Glu Gly Pro
   1505 1510 1515 1520
   His Val Leu Leu Tyr Phe Asp Ser Val Pro Thr Ser Arg Glu Cys Val
   1525 1530 1535
   Gly Phe Glu Ala Val Gln Glu Val Pro Val Gly Leu Val Gln Pro Ala
   1540 1545 1550
   Ser Ala Thr Leu Tyr Asp Tyr Tyr Asn Pro Glu Arg Arg Cys Ser Val
   1555 1560 1565
   Phe Tyr Gly Ala Pro Ser Lys Ser Arg Leu Leu Ala Thr Leu Cys Ser
   1570 1575 1580
   Ala Glu Val Cys Gln Cys Ala Glu Gly Lys Cys Pro Arg Gln Arg Arg
   1585 1590 1595 1600
   Ala Leu Glu Arg Gly Leu Gln Asp Glu Asp Gly Tyr Arg Met Lys Phe
   1605 1610 1615
   Ala Cys Tyr Tyr Pro Arg Val Glu Tyr Gly Phe Gln Val Lys Val Leu
   1620 1625 1630
   Arg Glu Asp Ser Arg Ala Ala Phe Arg Leu Phe Glu Thr Lys Ile Thr
   1635 1640 1645
   Gln Val Leu His Phe Thr Lys Asp Val Lys Ala Ala Ala Asn Gln Met
   1650 1655 1660
   Arg Asn Phe Leu Val Arg Ala Ser Cys Arg Leu Arg Leu Glu Pro Gly
   1665                1670                1675                1680
   Lys Glu Tyr Leu Ile Met Gly Leu Asp Gly Ala Thr Tyr Asp Leu Glu
                   1685                1690                1695
   Gly His Pro Gln Tyr Leu Leu Asp Ser Asn Ser Trp Ile Glu Glu Met
               1700                1705                1710
   Pro Ser Glu Arg Leu Cys Arg Ser Thr Arg Gln Arg Ala Ala Cys Ala
           1715                1720                1725
   Gln Leu Asn Asp Phe Leu Gln Glu Tyr Gly Thr Gln Gly Cys Gln Val
       1730                1735                1740
<210> 19
   <211> 559
   <212> PRT
   <213> Homo sapiens
<400> 19
   Met Ser Ala Cys Arg Ser Phe Ala Val Ala Ile Cys Ile Leu Glu Ile
    1               5                  10                  15
   Ser Ile Leu Thr Ala Gln Tyr Thr Thr Ser Tyr Asp Pro Glu Leu Thr
               20                  25                  30
   Glu Ser Ser Gly Ser Ala Ser His Ile Asp Cys Arg Met Ser Pro Trp
           35                  40                  45
   Ser Glu Trp Ser Gln Cys Asp Pro Cys Leu Arg Gln Met Phe Arg Ser
       50                  55                  60
   Arg Ser Ile Glu Val Phe Gly Gln Phe Asn Gly Lys Arg Cys Thr Asp
   65                  70                  75                  80
   Ala Val Gly Asp Arg Arg Gln Cys Val Pro Thr Glu Pro Cys Glu Asp
                   85                  90                  95
   Ala Glu Asp Asp Cys Gly Asn Asp Phe Gln Cys Ser Thr Gly Arg Cys
               100                 105                 110
   Ile Lys Met Arg Leu Arg Cys Asn Gly Asp Asn Asp Cys Gly Asp Phe
           115                 120                 125
   Ser Asp Glu Asp Asp Cys Glu Ser Glu Pro Arg Pro Pro Cys Arg Asp
       130                 135                 140
   Arg Val Val Glu Glu Ser Glu Leu Ala Arg Thr Ala Gly Tyr Gly Ile
   145                 150                 155                 160
   Asn Ile Leu Gly Met Asp Pro Leu Ser Thr Pro Phe Asp Asn Glu Phe
                   165                 170                 175
   Tyr Asn Gly Leu Cys Asn Arg Asp Arg Asp Gly Asn Thr Leu Thr Tyr
               180                 185                 190
   Tyr Arg Arg Pro Trp Asn Val Ala Ser Leu Ile Tyr Glu Thr Lys Gly
           195                 200                 205
   Glu Lys Asn Phe Arg Thr Glu His Tyr Glu Glu Gln Ile Glu Ala Phe
       210                 215                 220
   Lys Ser Ile Ile Gln Glu Lys Thr Ser Asn Phe Asn Ala Ala Ile Ser
   225                 230                 235                 240
   Leu Lys Phe Thr Pro Thr Glu Thr Asn Lys Ala Glu Gln Cys Cys Glu
                   245                 250                 255
   Glu Thr Ala Ser Ser Ile Ser Leu His Gly Lys Gly Ser Phe Arg Phe
               260                 265                 270
   Ser Tyr Ser Lys Asn Glu Thr Tyr Gln Leu Phe Leu Ser Tyr Ser Ser
           275                 280                 285
   Lys Lys Glu Lys Met Phe Leu His Val Lys Gly Glu Ile His Leu Gly
       290                 295                 300
   Arg Phe Val Met Arg Asn Arg Asp Val Val Leu Thr Thr Thr Phe Val
   305                 310                 315                 320
   Asp Asp Ile Lys Ala Leu Pro Thr Thr Tyr Glu Lys Gly Glu Tyr Phe
                   325                 330                 335
   Ala Phe Leu Glu Thr Tyr Gly Thr His Tyr Ser Ser Ser Gly Ser Leu
               340                 345                 350
   Gly Gly Leu Tyr Glu Leu Ile Tyr Val Leu Asp Lys Ala Ser Met Lys
           355                 360                 365
   Arg Lys Gly Val Glu Leu Lys Asp Ile Lys Arg Cys Leu Gly Tyr His
       370                 375                 380
   Leu Asp Val Ser Leu Ala Phe Ser Glu Ile Ser Val Gly Ala Glu Phe
   385                 390                 395                 400
   Asn Lys Asp Asp Cys Val Lys Arg Gly Glu Gly Arg Ala Val Asn Ile
                   405                 410                 415
   Thr Ser Glu Asn Leu Ile Asp Asp Val Val Ser Leu Ile Arg Gly Gly
               420                 425                 430
   Thr Arg Lys Tyr Ala Phe Glu Leu Lys Glu Lys Leu Leu Arg Gly Thr
           435                 440                 445
   Val Ile Asp Val Thr Asp Phe Val Asn Trp Ala Ser Ser Ile Asn Asp
       450                 455                 460
   Ala Pro Val Leu Ile Ser Gln Lys Leu Ser Pro Ile Tyr Asn Leu Val
   465                 470                 475                 480
   Pro Val Lys Met Lys Asn Ala His Leu Lys Lys Gln Asn Leu Glu Arg
                   485                 490                 495
   Ala Ile Glu Asp Tyr Ile Asn Glu Phe Ser Val Arg Lys Cys His Thr
               500                 505                 510
   Cys Gln Asn Gly Gly Thr Val Ile Leu Met Asp Gly Lys Cys Leu Cys
           515                 520                 525
   Ala Cys Pro Phe Lys Phe Glu Gly Ile Ala Cys Glu Ile Ser Lys Gln
       530                 535                 540
   Lys Ile Ser Glu Gly Leu Pro Ala Leu Glu Phe Pro Asn Glu Lys
   545                 550                 555
<210> 20
   <211> 615
   <212> PRT
   <213> Homo sapiens
<400> 20
<210> 21
   <211> 1014
   <212> PRT
   <213> Homo sapiens
<400> 21
<210> 22
   <211> 474
   <212> PRT
   <213> Homo sapiens
<400> 22
<210> 23
   <211> 353
   <212> PRT
   <213> Homo sapiens
<400> 23
   Ala Ser Pro Thr Ser Pro Lys Val Phe Pro Leu Ser Leu Cys Ser Thr
   1 5 10 15
   Gln Pro Asp Gly Asn Val Val Ile Ala Cys Leu Val Gln Gly Phe Phe
   20 25 30
   Pro Gln Glu Pro Leu Ser Val Thr Trp Ser Glu Ser Gly Gln Gly Val
   35 40 45
   Thr Ala Arg Asn Phe Pro Pro Ser Gln Asp Ala Ser Gly Asp Leu Tyr
   50 55 60
   Thr Thr Ser Ser Gln Leu Thr Leu Pro Ala Thr Gln Cys Leu Ala Gly
   65 70 75 80
   Lys Ser Val Thr Cys His Val Lys His Tyr Thr Asn Pro Ser Gln Asp
   85 90 95
   Val Thr Val Pro Cys Pro Val Pro Ser Thr Pro Pro Thr Pro Ser Pro
   100 105 110
   Ser Thr Pro Pro Thr Pro Ser Pro Ser Cys Cys His Pro Arg Leu Ser
   115 120 125
   Leu His Arg Pro Ala Leu Glu Asp Leu Leu Leu Gly Ser Glu Ala Asn
   130 135 140
   Leu Thr Cys Thr Leu Thr Gly Leu Arg Asp Ala Ser Gly Val Thr Phe
   145 150 155 160
   Thr Trp Thr Pro Ser Ser Gly Lys Ser Ala Val Gln Gly Pro Pro Glu
   165 170 175
   Arg Asp Leu Cys Gly Cys Tyr Ser Val Ser Ser Val Leu Pro Gly Cys
   180 185 190
   Ala Glu Pro Trp Asn His Gly Lys Thr Phe Thr Cys Thr Ala Ala Tyr
   195 200 205
   Pro Glu Ser Lys Thr Pro Leu Thr Ala Thr Leu Ser Lys Ser Gly Asn
   210 215 220
   Thr Phe Arg Pro Glu Val His Leu Leu Pro Pro Pro Ser Glu Glu Leu
   225 230 235 240
   Ala Leu Asn Glu Leu Val Thr Leu Thr Cys Leu Ala Arg Gly Phe Ser
   245 250 255
   Pro Lys Asp Val Leu Val Arg Trp Leu Gln Gly Ser Gln Glu Leu Pro
   260 265 270
   Arg Glu Lys Tyr Leu Thr Trp Ala Ser Arg Gln Glu Pro Ser Gln Gly
   275 280 285
   Thr Thr Thr Phe Ala Val Thr Ser Ile Leu Arg Val Ala Ala Glu Asp
   290 295 300
   Trp Lys Lys Gly Asp Thr Phe Ser Cys Met Val Gly His Glu Ala Leu
   305 310 315 320
   Pro Leu Ala Phe Thr Gln Lys Thr Ile Asp Arg Leu Ala Gly Lys Pro
   325 330 335
   Thr His Val Asn Val Ser Val Val Met Ala Glu Val Asp Gly Thr Cys
   340 345 350
   Tyr
<210> 24
   <211> 473
   <212> PRT
   <213> Homo sapiens
<400> 24
   Gly Ser Ala Ser Ala Pro Thr Leu Phe Pro Leu Val Ser Cys Glu Asn
   1               5                   10                  15
   Ser Pro Ser Asp Thr Ser Ser Val Ala Val Gly Cys Leu Ala Gln Asp
               20                  25                  30
   Phe Leu Pro Asp Ser Ile Thr Leu Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro Val Ile Ala Glu Leu Pro Pro Lys
               100                 105                 110
   Val Ser Val Phe Val Pro Pro Arg Asp Gly Phe Phe Gly Asn Pro Arg
           115                 120                 125
   Lys Ser Lys Leu Ile Cys Gln Ala Thr Gly Phe Ser Pro Arg Gln Ile
       130                 135                 140
   Gln Val Ser Trp Leu Arg Glu Gly Lys Gln Val Gly Ser Gly Val Thr
   145                 150                 155                 160
   Thr Asp Gln Val Gln Ala Glu Ala Lys Glu Ser Gly Pro Thr Thr Tyr
                   165                 170                 175
   Lys Val Thr Ser Thr Leu Thr Ile Lys Glu Ser Asp Trp Leu Gly Gln
               180                 185                 190
   Ser Met Phe Thr Cys Arg Val Asp His Arg Gly Leu Thr Phe Gln Gln
           195                 200                 205
   Asn Ala Ser Ser Met Cys Val Pro Asp Gln Asp Thr Ala Ile Arg Val
       210                 215                 220
   Phe Ala Ile Pro Pro Ser Phe Ala Ser Ile Phe Leu Thr Lys Ser Thr
   225                 230                 235                 240
   Lys Leu Thr Cys Leu Val Thr Asp Leu Thr Thr Tyr Asp Ser Val Thr
                   245                 250                 255
   Ile Ser Trp Thr Arg Gln Asn Gly Glu Ala Val Lys Thr His Thr Asn
               260                 265                 270
   Ile Ser Glu Ser His Pro Asn Ala Thr Phe Ser Ala Val Gly Glu Ala
           275                 280                 285
   Ser Ile Cys Glu Asp Asp Trp Asn Ser Gly Glu Arg Phe Thr Cys Thr
       290                 295                 300
   Val Thr His Thr Asp Leu Pro Ser Pro Leu Lys Gln Thr Ile Ser Arg
   305                 310                 315                 320
   Pro Lys Gly Val Ala Leu His Arg Pro Asp Val Tyr Leu Leu Pro Pro
                   325                 330                 335
   Ala Arg Glu Gln Leu Asn Leu Arg Glu Ser Ala Thr Ile Thr Cys Leu
               340                 345                 350
   Val Thr Gly Phe Ser Pro Ala Asp Val Phe Val Gln Trp Met Gln Arg
           355                 360                 365
   Gly Gln Pro Leu Ser Pro Glu Lys Tyr Val Thr Ser Ala Pro Met Pro
       370                 375                 380
   Glu Pro Gln Ala Pro Gly Arg Tyr Phe Ala His Ser Ile Leu Thr Val
   385                 390                 395                 400
   Ser Glu Glu Glu Trp Asn Thr Gly Glu Thr Tyr Thr Cys Val Ala His
                   405                 410                 415
   Glu Ala Leu Pro Asn Arg Val Thr Glu Arg Thr Val Asp Lys Ser Thr
               420                 425                 430
   Glu Gly Glu Val Ser Ala Asp Glu Glu Gly Phe Glu Asn Leu Trp Ala
           435                 440                 445
   Thr Ala Ser Thr Phe Ile Val Leu Phe Leu Leu Ser Leu Phe Tyr Ser
       450                 455                 460
   Thr Thr Val Thr Leu Phe Lys Val Lys
   465                 470
<210> 25
   <211> 106
   <212> PRT
   <213> Homo sapiens
<400> 25
   Thr Val Ala Ala Pro Ser Val Phe Ile Phe Pro Pro Ser Asp Glu Gln
    1               5                  10                  15
   Leu Lys Ser Gly Thr Ala Ser Val Val Cys Leu Leu Asn Asn Phe Tyr
               20                  25                  30
   Pro Arg Glu Ala Lys Val Gln Trp Lys Val Asp Asn Ala Leu Gln Ser
           35                  40                  45
   Gly Asn Ser Gln Glu Ser Val Thr Glu Gln Asp Ser Lys Asp Ser Thr
       50                  55                  60
   Tyr Ser Leu Ser Ser Thr Leu Thr Leu Ser Lys Ala Asp Tyr Glu Lys
   65                  70                  75                  80
   His Lys Val Tyr Ala Cys Glu Val Thr His Gln Gly Leu Ser Ser Pro
                   85                  90                  95
   Val Thr Lys Ser Phe Asn Arg Gly Glu Cys
               100                 105
<210> 26
   <211> 213
   <212> PRT
   <213> Homo sapiens
<400> 26
   Met Arg Pro Gly Thr Gly Gln Gly Gly Leu Glu Ala Pro Gly Glu Pro
    1               5                  10                  15
   Gly Pro Asn Leu Arg Gln Arg Trp Pro Leu Leu Leu Leu Gly Leu Ala
               20                  25                  30
   Val Val Thr His Gly Leu Leu Arg Pro Thr Ala Ala Ser Gln Ser Arg
           35                  40                  45
   Ala Leu Gly Pro Gly Ala Pro Gly Gly Ser Ser Arg Ser Ser Leu Arg
       50                  55                  60
   Ser Arg Trp Gly Arg Phe Leu Leu Gln Arg Gly Ser Trp Thr Gly Pro
   65                  70                  75                  80
   Arg Cys Trp Pro Arg Gly Phe Gln Ser Lys His Asn Ser Val Thr His
                   85                  90                  95
   Val Phe Gly Ser Gly Thr Gln Leu Thr Val Leu Ser Gln Pro Lys Ala
               100                 105                 110
   Thr Pro Ser Val Thr Leu Phe Pro Pro Ser Ser Glu Glu Leu Gln Ala
           115                 120                 125
   Asn Lys Ala Thr Leu Val Cys Leu Met Asn Asp Phe Tyr Pro Gly Ile
       130                 135                 140
   Leu Thr Val Thr Trp Lys Ala Asp Gly Thr Pro Ile Thr Gln Gly Val
   145                 150                 155                 160
   Glu Met Thr Thr Pro Ser Lys Gln Ser Asn Asn Lys Tyr Ala Ala Ser
                   165                 170                 175
   Ser Tyr Leu Ser Leu Thr Pro Glu Gln Trp Arg Ser Arg Arg Ser Tyr
               180                 185                 190
   Ser Cys Gln Val Met His Glu Gly Ser Thr Val Glu Lys Thr Val Ala
           195                 200                 205
   Pro Ala Glu Cys Ser
       210
<210> 27
   <211> 911
   <212> PRT
   <213> Homo sapiens
<400> 27
   Met Asp Gly Ala Met Gly Pro Arg Gly Leu Leu Leu Cys Met Tyr Leu
   1               5                   10                  15
   Val Ser Leu Leu Ile Leu Gln Ala Met Pro Ala Leu Gly Ser Ala Thr
               20                  25                  30
   Gly Arg Ser Lys Ser Ser Glu Lys Arg Gln Ala Val Asp Thr Ala Val
           35                  40                  45
   Asp Gly Val Phe Ile Arg Ser Leu Lys Val Asn Cys Lys Val Thr Ser
       50                  55                  60
   Arg Phe Ala His Tyr Val Val Thr Ser Gln Val Val Asn Thr Ala Asn
   65                  70                  75                  80
   Glu Ala Arg Glu Val Ala Phe Asp Leu Glu Ile Pro Lys Thr Ala Phe
                   85                  90                  95
   Ile Ser Asp Phe Ala Val Thr Ala Asp Gly Asn Ala Phe Ile Gly Asp
               100                 105                 110
   Ile Lys Asp Lys Val Thr Ala Trp Lys Gln Tyr Arg Lys Ala Ala Ile
           115                 120                 125
   Ser Gly Glu Asn Ala Gly Leu Val Arg Ala Ser Gly Arg Thr Met Glu
       130                 135                 140
   Gln Phe Thr Ile His Leu Thr Val Asn Pro Gln Ser Lys Val Thr Phe
   145                 150                 155                 160
   Gln Leu Thr Tyr Glu Glu Val Leu Lys Arg Asn His Met Gln Tyr Glu
                   165                 170                 175
   Ile Val Ile Lys Val Lys Pro Lys Gln Leu Val His His Phe Glu Ile
               180                 185                 190
   Asp Val Asp Ile Phe Glu Pro Gln Gly Ile Ser Lys Leu Asp Ala Gln
           195                 200                 205
   Ala Ser Phe Leu Pro Lys Glu Leu Ala Ala Gln Thr Ile Lys Lys Ser
       210                 215                 220
   Phe Ser Gly Lys Lys Gly His Val Leu Phe Arg Pro Thr Val Ser Gln
   225                 230                 235                 240
   Gln Gln Ser Cys Pro Thr Cys Ser Thr Ser Leu Leu Asn Gly His Phe
                   245                 250                 255
   Lys Val Thr Tyr Asp Val Ser Arg Asp Lys Ile Cys Asp Leu Leu Val
               260                 265                 270
   Ala Asn Asn His Phe Ala His Phe Phe Ala Pro Gln Asn Leu Thr Asn
           275                 280                 285
   Met Asn Lys Asn Val Val Phe Val Ile Asp Ile Ser Gly Ser Met Arg
       290                 295                 300
   Gly Gln Lys Val Lys Gln Thr Lys Glu Ala Leu Leu Lys Ile Leu Gly
   305                 310                 315                 320
   Asp Met Gln Pro Gly Asp Tyr Phe Asp Leu Val Leu Phe Gly Thr Arg
                   325                 330                 335
   Val Gln Ser Trp Lys Gly Ser Leu Val Gln Ala Ser Glu Ala Asn Leu
               340                 345                 350
   Gln Ala Ala Gln Asp Phe Val Arg Gly Phe Ser Leu Asp Glu Ala Thr
           355                 360                 365
   Asn Leu Asn Gly Gly Leu Leu Arg Gly Ile Glu Ile Leu Asn Gln Val
       370                 375                 380
   Gln Glu Ser Leu Pro Glu Leu Ser Asn His Ala Ser Ile Leu Ile Met
   385                 390                 395                 400
   Leu Thr Asp Gly Asp Pro Thr Glu Gly Val Thr Asp Arg Ser Gln Ile
                   405                 410                 415
   Leu Lys Asn Val Arg Asn Ala Ile Arg Gly Arg Phe Pro Leu Tyr Asn
               420                 425                 430
   Leu Gly Phe Gly His Asn Val Asp Phe Asn Phe Leu Glu Val Met Ser
           435                 440                 445
   Met Glu Asn Asn Gly Arg Ala Gln Arg Ile Tyr Glu Asp His Asp Ala
       450                 455                 460
   Thr Gln Gln Leu Gln Gly Phe Tyr Ser Gln Val Ala Lys Pro Leu Leu
   465                 470                 475                 480
   Val Asp Val Asp Leu Gln Tyr Pro Gln Asp Ala Val Leu Ala Leu Thr
                   485                 490                 495
   Gln Asn His His Lys Gln Tyr Tyr Glu Gly Ser Glu Ile Val Val Ala
               500                 505                 510
   Gly Arg Ile Ala Asp Asn Lys Gln Ser Ser Phe Lys Ala Asp Val Gln
   515 520 525
   Ala His Gly Glu Gly Gln Glu Phe Ser Ile Thr Cys Leu Val Asp Glu
   530 535 540
   Glu Glu Met Lys Lys Leu Leu Arg Glu Arg Gly His Met Leu Glu Asn
   545 550 555 560
   His Val Glu Arg Leu Trp Ala Tyr Leu Thr Ile Gln Glu Leu Leu Ala
   565 570 575
   Lys Arg Met Lys Val Asp Arg Glu Glu Arg Ala Asn Leu Ser Ser Gln
   580 585 590
   Ala Leu Gln Met Ser Leu Asp Tyr Gly Phe Val Thr Pro Leu Thr Ser
   595 600 605
   Met Ser Ile Arg Gly Met Ala Asp Gln Asp Gly Leu Lys Pro Thr Ile
   610 615 620
   Asp Lys Pro Ser Glu Asp Ser Pro Pro Leu Glu Met Leu Gly Pro Arg
   625 630 635 640
   Arg Thr Phe Val Leu Ser Ala Leu Gln Pro Ser Pro Thr His Ser Ser
   645 650 655
   Ser Asn Thr Gln Arg Leu Pro Asp Arg Val Thr Gly Val Asp Thr Asp
   660 665 670
   Pro His Phe Ile Ile His Val Pro Gln Lys Glu Asp Thr Leu Cys Phe
   675 680 685
   Asn Ile Asn Glu Glu Pro Gly Val Ile Leu Ser Leu Val Gln Asp Pro
   690 695 700
   Asn Thr Gly Phe Ser Val Asn Gly Gln Leu Ile Gly Asn Lys Ala Arg
   705 710 715 720
   Ser Pro Gly Gln His Asp Gly Thr Tyr Phe Gly Arg Leu Gly Ile Ala
   725 730 735
   Asn Pro Ala Thr Asp Phe Gln Leu Glu Val Thr Pro Gln Asn Ile Thr
   740 745 750
   Leu Asn Pro Gly Phe Gly Gly Pro Val Phe Ser Trp Arg Asp Gln Ala
   755 760 765
   Val Leu Arg Gln Asp Gly Val Val Val Thr Ile Asn Lys Lys Arg Asn
   770 775 780
   Leu Val Val Ser Val Asp Asp Gly Gly Thr Phe Glu Val Val Leu His
   785 790 795 800
   Arg Val Trp Lys Gly Ser Ser Val His Gln Asp Phe Leu Gly Phe Tyr
   805 810 815
   Val Leu Asp Ser His Arg Met Ser Ala Arg Thr His Gly Leu Leu Gly
   820 825 830
   Gln Phe Phe His Pro Ile Gly Phe Glu Val Ser Asp Ile His Pro Gly
   835 840 845
   Ser Asp Pro Thr Lys Pro Asp Ala Thr Met Val Val Arg Asn Arg Arg
   850 855 860
   Leu Thr Val Thr Arg Gly Leu Gln Lys Asp Tyr Ser Lys Asp Pro Trp
   865 870 875 880
   His Gly Ala Glu Val Ser Cys Trp Phe Ile His Asn Asn Gly Ala Gly
   885 890 895
   Leu Ile Asp Gly Ala Tyr Thr Asp Tyr Ile Val Pro Asp Ile Phe
   900 905 910
<210> 28
   <211> 946
   <212> PRT
   <213> Homo sapiens
<400> 28
   Met Lys Arg Leu Thr Cys Phe Phe Ile Cys Phe Phe Leu Ser Glu Val
    1               5                  10                  15
   Ser Gly Phe Glu Ile Pro Ile Asn Gly Leu Ser Glu Phe Val Asp Tyr
               20                  25                  30
   Glu Asp Leu Val Glu Leu Ala Pro Gly Lys Phe Gln Leu Val Ala Glu
               35                  40                  45
   Asn Arg Arg Tyr Gln Arg Ser Leu Pro Gly Glu Ser Glu Glu Met Met
       50                  55                  60
   Glu Glu Val Asp Gln Val Thr Leu Tyr Ser Tyr Lys Val Gln Ser Thr
   65                  70                  75                  80
   Ile Thr Ser Arg Met Ala Thr Thr Met Ile Gln Ser Lys Val Val Asn
                   85                  90                  95
   Asn Ser Pro Gln Pro Gln Asn Val Val Phe Asp Val Gln Ile Pro Lys
               100                 105                 110
   Gly Ala Phe Ile Ser Asn Phe Ser Met Thr Val Asp Gly Lys Thr Phe
           115                 120                 125
   Arg Ser Ser Ile Lys Glu Lys Thr Val Gly Arg Ala Leu Tyr Ala Gln
       130                 135                 140
   Ala Arg Ala Lys Gly Lys Thr Ala Gly Leu Val Arg Ser Ser Ala Leu
   145                 150                 155                 160
   Asp Met Glu Asn Phe Arg Thr Glu Val Asn Val Leu Pro Gly Ala Lys
                   165                 170                 175
   Val Gln Phe Glu Leu His Tyr Gln Glu Val Lys Trp Arg Lys Leu Gly
               180                 185                 190
   Ser Tyr Glu His Arg Ile Tyr Leu Gln Pro Gly Arg Leu Ala Lys His
           195                 200                 205
   Leu Glu Val Asp Val Trp Val Ile Glu Pro Gln Gly Leu Arg Phe Leu
       210                 215                 220
   His Val Pro Asp Thr Phe Glu Gly His Phe Asp Gly Val Pro Val Ile
   225                 230                 235                 240
   Ser Lys Gly Gln Gln Lys Ala His Val Ser Phe Lys Pro Thr Val Ala
                   245                 250                 255
   Gln Gln Arg Ile Cys Pro Asn Cys Arg Glu Thr Ala Val Asp Gly Glu
               260                 265                 270
   Leu Val Val Leu Tyr Asp Val Lys Arg Glu Glu Lys Ala Gly Glu Leu
           275                 280                 285
   Glu Val Phe Asn Gly Tyr Phe Val His Phe Phe Ala Pro Asp Asn Leu
       290                 295                 300
   Asp Pro Ile Pro Lys Asn Ile Leu Phe Val Ile Asp Val Ser Gly Ser
   305                 310                 315                 320
   Met Trp Gly Val Lys Met Lys Gln Thr Val Glu Ala Met Lys Thr Ile
                   325                 330                 335
   Leu Asp Asp Leu Arg Ala Glu Asp His Phe Ser Val Ile Asp Phe Asn
               340                 345                 350
   Gln Asn Ile Arg Thr Trp Arg Asn Asp Leu Ile Ser Ala Thr Lys Thr
           355                 360                 365
   Gln Val Ala Asp Ala Lys Arg Tyr Ile Glu Lys Ile Gln Pro Ser Gly
       370                 375                 380
   Gly Thr Asn Ile Asn Glu Ala Leu Leu Arg Ala Ile Phe Ile Leu Asn
   385                 390                 395                 400
   Glu Ala Asn Asn Leu Gly Leu Leu Asp Pro Asn Ser Val Ser Leu Ile
                   405                 410                 415
   Ile Leu Val Ser Asp Gly Asp Pro Thr Val Gly Glu Leu Lys Leu Ser
               420                 425                 430
   Lys Ile Gln Lys Asn Val Lys Glu Asn Ile Gln Asp Asn Ile Ser Leu
           435                 440                 445
   Phe Ser Leu Gly Met Gly Phe Asp Val Asp Tyr Asp Phe Leu Lys Arg
       450                 455                 460
   Leu Ser Asn Glu Asn His Gly Ile Ala Gln Arg Ile Tyr Gly Asn Gln
   465                 470                 475                 480
   Asp Thr Ser Ser Gln Leu Lys Lys Phe Tyr Asn Gln Val Ser Thr Pro
                   485                 490                 495
   Leu Leu Arg Asn Val Gln Phe Asn Tyr Pro His Thr Ser Val Thr Asp
               500                 505                 510
   Val Thr Gln Asn Asn Phe His Asn Tyr Phe Gly Gly Ser Glu Ile Val
           515                 520                 525
   Val Ala Gly Lys Phe Asp Pro Ala Lys Leu Asp Gln Ile Glu Ser Val
       530                 535                 540
   Ile Thr Ala Thr Ser Ala Asn Thr Gln Leu Val Leu Glu Thr Leu Ala
   545 550 555 560
   Gln Met Asp Asp Leu Gln Asp Phe Leu Ser Lys Asp Lys His Ala Asp
   565 570 575
   Pro Asp Phe Thr Arg Lys Leu Trp Ala Tyr Leu Thr Ile Asn Gln Leu
   580 585 590
   Leu Ala Glu Arg Ser Leu Ala Pro Thr Ala Ala Ala Lys Arg Arg Ile
   595 600 605
   Thr Arg Ser Ile Leu Gln Met Ser Leu Asp His His Ile Val Thr Pro
   610 615 620
   Leu Thr Ser Leu Val Ile Glu Asn Glu Ala Gly Asp Glu Arg Met Leu
   625 630 635 640
   Ala Asp Ala Pro Pro Gln Asp Pro Ser Cys Cys Ser Gly Ala Leu Tyr
   645 650 655
   Tyr Gly Ser Lys Val Val Pro Asp Ser Thr Pro Ser Trp Ala Asn Pro
   660 665 670
   Ser Pro Thr Pro Val Ile Ser Met Leu Ala Gln Gly Ser Gln Val Leu
   675 680 685
   Glu Ser Thr Pro Pro Pro His Val Met Arg Val Glu Asn Asp Pro His
   690 695 700
   Phe Ile Ile Tyr Leu Pro Lys Ser Gln Lys Asn Ile Cys Phe Asn Ile
   705 710 715 720
   Asp Ser Glu Pro Gly Lys Ile Leu Asn Leu Val Ser Asp Pro Glu Ser
   725 730 735
   Gly Ile Val Val Asn Gly Gln Leu Val Gly Ala Lys Lys Pro Asn Asn
   740 745 750
   Gly Lys Leu Ser Thr Tyr Phe Gly Lys Leu Gly Phe Tyr Phe Gln Ser
   755 760 765
   Glu Asp Ile Lys Ile Glu Ile Ser Thr Glu Thr Ile Thr Leu Ser His
   770 775 780
   Gly Ser Ser Thr Phe Ser Leu Ser Trp Ser Asp Thr Ala Gln Val Thr
   785 790 795 800
   Asn Gln Arg Val Gln Ile Ser Val Lys Lys Glu Lys Val Val Thr Ile
   805 810 815
   Thr Leu Asp Lys Glu Met Ser Phe Ser Val Leu Leu His Arg Val Trp
   820 825 830
   Lys Lys His Pro Val Asn Val Asp Phe Leu Gly Ile Tyr Ile Pro Pro
   835 840 845
   Thr Asn Lys Phe Ser Pro Lys Ala His Gly Leu Ile Gly Gln Phe Met
   850 855 860
   Gln Glu Pro Lys Ile His Ile Phe Asn Glu Arg Pro Gly Lys Asp Pro
   865 870 875 880
   Glu Lys Pro Glu Ala Ser Met Glu Val Lys Gly Gln Lys Leu Ile Ile
   885 890 895
   Thr Arg Gly Leu Gln Lys Asp Tyr Arg Thr Asp Leu Val Phe Gly Thr
   900 905 910
   Asp Val Thr Cys Trp Phe Val His Asn Ser Gly Lys Gly Phe Ile Asp
   915 920 925
   Gly His Tyr Lys Asp Tyr Phe Val Pro Gln Leu Tyr Ser Phe Leu Lys
   930 935 940
   Arg Pro
   945
<210> 29
   <211> 930
   <212> PRT
   <213> Homo sapiens
<400> 29
<210> 30
   <211> 564
   <212> PRT
   <213> Homo sapiens
<400> 30
<210> 31
   <211> 128
   <212> PRT
   <213> Homo sapiens
<400> 31
   Met Ser Leu Arg Leu Asp Thr Thr Pro Ser Cys Asn Ser Ala Arg Pro
    1               5                  10                  15
   Leu His Ala Leu Gln Val Leu Leu Leu Leu Ser Leu Leu Leu Thr Ala
               20                  25                  30
   Leu Ala Ser Ser Thr Lys Gly Gln Thr Lys Arg Asn Leu Ala Lys Gly
           35                  40                  45
   Lys Glu Glu Ser Leu Asp Ser Asp Leu Tyr Ala Glu Leu Arg Cys Met
       50                  55                  60
   Cys Ile Lys Thr Thr Ser Gly Ile His Pro Lys Asn Ile Gln Ser Leu
   65                  70                  75                  80
   Glu Val Ile Gly Lys Gly Thr His Cys Asn Gln Val Glu Val Ile Ala
                   85                  90                  95
   Thr Leu Lys Asp Gly Arg Lys Ile Cys Leu Asp Pro Asp Ala Pro Arg
               100                 105                 110
   Ile Lys Lys Ile Val Gln Lys Lys Leu Ala Gly Asp Glu Ser Ala Asp
           115                 120                 125
<210> 32
   <211> 136
   <212> PRT
   <213> Homo sapiens
<400> 32
<210> 33
   <211> 208
   <212> PRT
   <213> Homo sapiens
<400> 33
   Met Lys Leu Leu Thr Gly Leu Val Phe Cys Ser Leu Val Leu Ser Val
    1               5                  10                  15
   Ser Ser Arg Ser Phe Phe Ser Phe Leu Gly Glu Ala Phe Asp Gly Ala
               20                  25                  30
   Arg Asp Met Trp Arg Ala Tyr Ser Asp Met Arg Glu Ala Asn Tyr Ile
           35                  40                  45
   Gly Ser Asp Lys Tyr Phe His Ala Arg Gly Asn Tyr Asp Ala Ala Lys
       50                  55                  60
   Arg Gly Pro Gly Gly Ala Trp Ala Ala Glu Val Ile Ser Thr Met Arg
   65                  70                  75                  80
   Leu Phe Thr Gly Ile Val Phe Cys Ser Leu Val Met Gly Val Thr Ser
                   85                  90                  95
   Glu Ser Trp Arg Ser Phe Phe Lys Glu Ala Leu Gln Gly Val Gly Asp
               100                 105                 110
   Met Gly Arg Ala Tyr Trp Asp Ile Met Ile Ser Asn His Gln Asn Ser
           115                 120                 125
   Asn Arg Tyr Leu Tyr Ala Arg Gly Asn Tyr Asp Ala Ala Gln Arg Gly
       130                 135                 140
   Pro Gly Gly Val Trp Ala Ala Lys Leu Ile Ser Arg Ser Arg Val Tyr
   145                 150                 155                 160
   Leu Gln Gly Leu Ile Asp Cys Tyr Leu Phe Gly Asn Ser Ser Thr Val
                   165                 170                 175
   Leu Glu Asp Ser Lys Ser Asn Glu Lys Ala Glu Glu Trp Gly Arg Ser
               180                 185                 190
   Gly Lys Asp Pro Asp Arg Phe Arg Pro Asp Gly Leu Pro Lys Lys Tyr
           195                 200                 205
<210> 34
   <211> 599
   <212> PRT
   <213> Homo sapiens
<400> 34
   Met Lys Leu Leu Lys Leu Thr Gly Phe Ile Phe Phe Leu Phe Phe Leu
    1               5                  10                  15
   Thr Glu Ser Leu Thr Leu Pro Thr Gln Pro Arg Asp Ile Glu Asn Phe
               20                  25                  30
   Asn Ser Thr Gln Lys Phe Ile Glu Asp Asn Ile Glu Tyr Ile Thr Ile
           35                  40                  45
   Ile Ala Phe Ala Gln Tyr Val Gln Glu Ala Thr Phe Glu Glu Met Glu
       50                  55                  60
   Lys Leu Val Lys Asp Met Val Glu Tyr Lys Asp Arg Cys Met Ala Asp
   65                  70                  75                  80
   Lys Thr Leu Pro Glu Cys Ser Lys Leu Pro Asn Asn Val Leu Gln Glu
                   85                  90                  95
   Lys Ile Cys Ala Met Glu Gly Leu Pro Gln Lys His Asn Phe Ser His
               100                 105                 110
   Cys Cys Ser Lys Val Asp Ala Gln Arg Arg Leu Cys Phe Phe Tyr Asn
           115                 120                 125
   Lys Lys Ser Asp Val Gly Phe Leu Pro Pro Phe Pro Thr Leu Asp Pro
       130                 135                 140
   Glu Glu Lys Cys Gln Ala Tyr Glu Ser Asn Arg Glu Ser Leu Leu Asn
   145                 150                 155                 160
   His Phe Leu Tyr Glu Val Ala Arg Arg Asn Pro Phe Val Phe Ala Pro
                   165                 170                 175
   Thr Leu Leu Thr Val Ala Val His Phe Glu Glu Val Ala Lys Ser Cys
               180                 185                 190
   Cys Glu Glu Gln Asn Lys Val Asn Cys Leu Gln Thr Arg Ala Ile Pro
           195                 200                 205
   Val Thr Gln Tyr Leu Lys Ala Phe Ser Ser Tyr Gln Lys His Val Cys
       210                 215                 220
   Gly Ala Leu Leu Lys Phe Gly Thr Lys Val Val His Phe Ile Tyr Ile
   225                 230                 235                 240
   Ala Ile Leu Ser Gln Lys Phe Pro Lys Ile Glu Phe Lys Glu Leu Ile
                   245                 250                 255
   Ser Leu Val Glu Asp Val Ser Ser Asn Tyr Asp Gly Cys Cys Glu Gly
               260                 265                 270
   Asp Val Val Gln Cys Ile Arg Asp Thr Ser Lys Val Met Asn His Ile
           275                 280                 285
   Cys Ser Lys Gln Asp Ser Ile Ser Ser Lys Ile Lys Glu Cys Cys Glu
       290                 295                 300
   Lys Lys Ile Pro Glu Arg Gly Gln Cys Ile Ile Asn Ser Asn Lys Asp
   305                 310                 315                 320
   Asp Arg Pro Lys Asp Leu Ser Leu Arg Glu Gly Lys Phe Thr Asp Ser
                   325                 330                 335
   Glu Asn Val Cys Gln Glu Arg Asp Ala Asp Pro Asp Thr Phe Phe Ala
               340                 345                 350
   Lys Phe Thr Phe Glu Tyr Ser Arg Arg His Pro Asp Leu Ser Ile Pro
           355                 360                 365
   Glu Leu Leu Arg Ile Val Gln Ile Tyr Lys Asp Leu Leu Arg Asn Cys
       370                 375                 380
   Cys Asn Thr Glu Asn Pro Pro Gly Cys Tyr Arg Tyr Ala Glu Asp Lys
   385                 390                 395                 400
   Phe Asn Glu Thr Thr Glu Lys Ser Leu Lys Met Val Gln Gln Glu Cys
                   405                 410                 415
   Lys His Phe Gln Asn Leu Gly Lys Asp Gly Leu Lys Tyr His Tyr Leu
               420                 425                 430
   Ile Arg Leu Thr Lys Ile Ala Pro Gln Leu Ser Thr Glu Glu Leu Val
           435                 440                 445
   Ser Leu Gly Glu Lys Met Val Thr Ala Phe Thr Thr Cys Cys Thr Leu
       450                 455                 460
   Ser Glu Glu Phe Ala Cys Val Asp Asn Leu Ala Asp Leu Val Phe Gly
   465                 470                 475                 480
   Glu Leu Cys Gly Val Asn Glu Asn Arg Thr Ile Asn Pro Ala Val Asp
                   485                 490                 495
   His Cys Cys Lys Thr Asn Phe Ala Phe Arg Arg Pro Cys Phe Glu Ser
               500                 505                 510
   Leu Lys Ala Asp Lys Thr Tyr Val Pro Pro Pro Phe Ser Gln Asp Leu
           515                 520                 525
   Phe Thr Phe His Ala Asp Met Cys Gln Ser Gln Asn Glu Glu Leu Gln
       530                 535                 540
   Arg Lys Thr Asp Arg Phe Leu Val Asn Leu Val Lys Leu Lys His Glu
   545                 550                 555                 560
   Leu Thr Asp Glu Glu Leu Gln Ser Leu Phe Thr Asn Phe Ala Asn Val
                   565                 570                 575
   Val Asp Lys Cys Cys Lys Ala Glu Ser Pro Glu Val Cys Phe Asn Glu
               580                 585                 590
   Glu Ser Pro Lys Ile Gly Asn
           595
<210> 35
   <211> 433
   <212> PRT
   <213> Homo sapiens
<400> 35
   Met Phe Ala Gly Cys Phe Phe Phe Leu Leu Asn Gln Ser Arg Asn His
    1               5                  10                  15
   Pro Val Ser Leu Cys Asn Ser Ser Gly Arg Leu Gln Gln Ile Asn Lys
               20                  25                  30
   Ala His His Pro Pro Trp Val Tyr Leu Ser Gln Gln Ser Thr Trp Val
           35                  40                  45
   Gly Pro Glu Ala Ser Asn His Leu His Ala Cys Gln Gly Leu Ser Gly
       50                  55                  60
   Ala Ala Met Lys Ser Leu Val Leu Leu Leu Cys Leu Ala Gln Leu Trp
   65                  70                  75                  80
   Gly Cys His Ser Ala Pro His Gly Pro Gly Leu Ile Tyr Arg Gln Pro
                   85                  90                  95
   Asn Cys Asp Asp Pro Glu Thr Glu Glu Ala Ala Leu Val Ala Ile Asp
               100                 105                 110
   Tyr Ile Asn Gln Asn Leu Pro Trp Gly Tyr Lys His Thr Leu Asn Gln
           115                 120                 125
   Ile Asp Glu Val Lys Val Trp Pro Gln Gln Pro Ser Gly Glu Leu Phe
       130                 135                 140
   Glu Ile Glu Ile Asp Thr Leu Glu Thr Thr Cys His Val Leu Asp Pro
   145                 150                 155                 160
   Thr Pro Val Ala Arg Cys Ser Val Arg Gln Leu Lys Glu His Ala Val
                   165                 170                 175
   Glu Gly Asp Cys Asp Phe Gln Leu Leu Lys Leu Asp Gly Lys Phe Ser
               180                 185                 190
   Val Val Tyr Ala Lys Cys Asp Ser Ser Pro Asp Ser Ala Glu Asp Val
           195                 200                 205
   Arg Lys Val Cys Gln Asp Cys Pro Leu Leu Ala Pro Leu Asn Asp Thr
       210                 215                 220
   Arg Val Val His Ala Ala Lys Ala Ala Leu Ala Ala Phe Asn Ala Gln
   225                 230                 235                 240
   Asn Asn Gly Ser Asn Phe Gln Leu Glu Glu Ile Ser Arg Ala Gln Leu
                   245                 250                 255
   Val Pro Leu Pro Pro Ser Thr Tyr Val Glu Phe Thr Val Ser Gly Thr
               260                 265                 270
   Asp Cys Val Ala Lys Glu Ala Thr Glu Ala Ala Lys Cys Asn Leu Leu
           275                 280                 285
   Ala Glu Lys Gln Tyr Gly Phe Cys Lys Ala Thr Leu Ser Glu Lys Leu
       290                 295                 300
   Gly Gly Ala Glu Val Ala Val Thr Cys Met Val Phe Gln Thr Gln Pro
   305                 310                 315                 320
   Val Ser Ser Gln Pro Gln Pro Glu Gly Ala Asn Glu Ala Val Pro Thr
                   325                 330                 335
   Pro Val Val Asp Pro Asp Ala Pro Pro Ser Pro Pro Leu Gly Ala Pro
               340                 345                 350
   Gly Leu Pro Pro Ala Gly Ser Pro Pro Asp Ser His Val Leu Leu Ala
           355                 360                 365
   Ala Pro Pro Gly His Gln Leu His Arg Ala His Tyr Asp Leu Arg His
       370                 375                 380
   Thr Phe Met Gly Val Val Ser Leu Gly Ser Pro Ser Gly Glu Val Ser
   385                 390                 395                 400
   His Pro Arg Lys Thr Arg Thr Val Val Gln Pro Ser Val Gly Ala Ala
                   405                 410                 415
   Ala Gly Pro Val Val Pro Pro Cys Pro Gly Arg Ile Arg His Phe Lys
               420                 425                 430
   Val
<210> 36
   <211> 352
   <212> PRT
   <213> Homo sapiens
<400> 36
   Met Arg Ser Leu Gly Ala Leu Leu Leu Leu Leu Ser Ala Cys Leu Ala
   1 5 10 15
   Val Ser Ala Gly Pro Val Pro Thr Pro Pro Asp Asn Ile Gln Val Gln
   20 25 30
   Glu Asn Phe Asn Ile Ser Arg Ile Tyr Gly Lys Trp Tyr Asn Leu Ala
   35 40 45
   Ile Gly Ser Thr Cys Pro Trp Leu Lys Lys Ile Met Asp Arg Met Thr
   50 55 60
   Val Ser Thr Leu Val Leu Gly Glu Gly Ala Thr Glu Ala Glu Ile Ser
   65 70 75 80
   Met Thr Ser Thr Arg Trp Arg Lys Gly Val Cys Glu Glu Thr Ser Gly
   85 90 95
   Ala Tyr Glu Lys Thr Asp Thr Asp Gly Lys Phe Leu Tyr His Lys Ser
   100 105 110
   Lys Trp Asn Ile Thr Met Glu Ser Tyr Val Val His Thr Asn Tyr Asp
   115 120 125
   Glu Tyr Ala Ile Phe Leu Thr Lys Lys Phe Ser Arg His His Gly Pro
   130 135 140
   Thr Ile Thr Ala Lys Leu Tyr Gly Arg Ala Pro Gln Leu Arg Glu Thr
   145 150 155 160
   Leu Leu Gln Asp Phe Arg Val Val Ala Gln Gly Val Gly Ile Pro Glu
   165 170 175
   Asp Ser Ile Phe Thr Met Ala Asp Arg Gly Glu Cys Val Pro Gly Glu
   180 185 190
   Gln Glu Pro Glu Pro Ile Leu Ile Pro Arg Val Arg Arg Ala Val Leu
   195 200 205
   Pro Gln Glu Glu Glu Gly Ser Gly Gly Gly Gln Leu Val Thr Glu Val
   210 215 220
   Thr Lys Lys Glu Asp Ser Cys Gln Leu Gly Tyr Ser Ala Gly Pro Cys
   225 230 235 240
   Met Gly Met Thr Ser Arg Tyr Phe Tyr Asn Gly Thr Ser Met Ala Cys
   245 250 255
   Glu Thr Phe Gln Tyr Gly Gly Cys Met Gly Asn Gly Asn Asn Phe Val
   260 265 270
   Thr Glu Lys Glu Cys Leu Gln Thr Cys Arg Thr Val Ala Ala Cys Asn
   275 280 285
   Leu Pro Ile Val Arg Gly Pro Cys Arg Ala Phe Ile Gln Leu Trp Ala
   290 295 300
   Phe Asp Ala Val Lys Gly Lys Cys Val Leu Phe Pro Tyr Gly Gly Cys
   305 310 315 320
   Gln Gly Asn Gly Asn Lys Phe Tyr Ser Glu Lys Glu Cys Arg Glu Tyr
   325 330 335
   Cys Gly Val Pro Gly Asp Gly Asp Glu Glu Leu Leu Arg Phe Ser Asn
   340 345 350
<210> 37
   <211> 223
   <212> PRT
   <213> Homo sapiens
<400> 37
   Met Asn Lys Pro Leu Leu Trp Ile Ser Val Leu Thr Ser Leu Leu Glu
    1               5                  10                  15
   Ala Phe Ala His Thr Asp Leu Ser Gly Lys Val Phe Val Phe Pro Arg
               20                  25                  30
   Glu Ser Val Thr Asp His Val Asn Leu Ile Thr Pro Leu Glu Lys Pro
           35                  40                  45
   Leu Gln Asn Phe Thr Leu Cys Phe Arg Ala Tyr Ser Asp Leu Ser Arg
       50                  55                  60
   Ala Tyr Ser Leu Phe Ser Tyr Asn Thr Gln Gly Arg Asp Asn Glu Leu
   65                  70                  75                  80
   Leu Val Tyr Lys Glu Arg Val Gly Glu Tyr Ser Leu Tyr Ile Gly Arg
                   85                  90                  95
   His Lys Val Thr Ser Lys Val Ile Glu Lys Phe Pro Ala Pro Val His
               100                 105                 110
   Ile Cys Val Ser Trp Glu Ser Ser Ser Gly Ile Ala Glu Phe Trp Ile
           115                 120                 125
   Asn Gly Thr Pro Leu Val Lys Lys Gly Leu Arg Gln Gly Tyr Phe Val
       130                 135                 140
   Glu Ala Gln Pro Lys Ile Val Leu Gly Gln Glu Gln Asp Ser Tyr Gly
   145                 150                 155                 160
   Gly Lys Phe Asp Arg Ser Gln Ser Phe Val Gly Glu Ile Gly Asp Leu
                   165                 170                 175
   Tyr Met Trp Asp Ser Val Leu Pro Pro Glu Asn Ile Leu Ser Ala Tyr
               180                 185                 190
   Gln Gly Thr Pro Leu Pro Ala Asn Ile Leu Asp Trp Gln Ala Leu Asn
           195                 200                 205
   Tyr Glu Ile Arg Gly Tyr Val Ile Ile Lys Pro Leu Val Trp Val
       210                 215                 220
<210> 38
   <211> 267
   <212> PRT
   <213> Homo sapiens
<400> 38
<210> 39
   <211> 189
   <212> PRT
   <213> Homo sapiens
<400> 39
   Met Val Met Leu Leu Leu Leu Leu Ser Ala Leu Ala Gly Leu Phe Gly
    1               5                  10                  15
   Ala Ala Glu Gly Gln Ala Phe His Leu Gly Lys Cys Pro Asn Pro Pro
               20                  25                  30
   Val Gln Glu Asn Phe Asp Val Asn Lys Tyr Leu Gly Arg Trp Tyr Glu
           35                  40                  45
   Ile Glu Lys Ile Pro Thr Thr Phe Glu Asn Gly Arg Cys Ile Gln Ala
       50                  55                  60
   Asn Tyr Ser Leu Met Glu Asn Gly Lys Ile Lys Val Leu Asn Gln Glu
   65                  70                  75                  80
   Leu Arg Ala Asp Gly Thr Val Asn Gln Ile Glu Gly Glu Ala Thr Pro
                   85                  90                  95
   Val Asn Leu Thr Glu Pro Ala Lys Leu Glu Val Lys Phe Ser Trp Phe
               100                 105                 110
   Met Pro Ser Ala Pro Tyr Trp Ile Leu Ala Thr Asp Tyr Glu Asn Tyr
           115                 120                 125
   Ala Leu Val Tyr Ser Cys Thr Cys Ile Ile Gln Leu Phe His Val Asp
       130                 135                 140
   Phe Ala Trp Ile Leu Ala Arg Asn Pro Asn Leu Pro Pro Glu Thr Val
   145                 150                 155                 160
   Asp Ser Leu Lys Asn Ile Leu Thr Ser Asn Asn Ile Asp Val Lys Lys
                   165                 170                 175
   Met Thr Val Thr Asp Gln Val Asn Cys Pro Lys Leu Ser
               180                 185
<210> 40
   <211> 1429
   <212> PRT
   <213> Homo sapiens
<400> 40
   Met Val Ala Ala Ala Ala Ala Thr Glu Ala Arg Leu Arg Arg Arg Thr
   1 5 10 15
   Ala Ala Thr Ala Ala Leu Ala Gly Arg Ser Gly Gly Pro His Trp Asp
   20 25 30
   Trp Asp Val Thr Arg Ala Gly Arg Pro Gly Leu Gly Ala Gly Leu Arg
   35 40 45
   Leu Pro Arg Leu Leu Ser Pro Pro Leu Arg Pro Arg Leu Leu Leu Leu
   50 55 60
   Leu Leu Leu Leu Ser Pro Pro Leu Leu Leu Leu Leu Leu Pro Cys Glu
   65 70 75 80
   Ala Glu Ala Ala Ala Ala Ala Ala Ala Val Ser Gly Ser Ala Ala Ala
   85 90 95
   Glu Ala Lys Glu Cys Asp Arg Pro Cys Val Asn Gly Gly Arg Cys Asn
   100 105 110
   Pro Gly Thr Gly Gln Cys Val Cys Pro Ala Gly Trp Val Gly Glu Gln
   115 120 125
   Cys Gln His Cys Gly Gly Arg Phe Arg Leu Thr Gly Ser Ser Gly Phe
   130 135 140
   Val Thr Asp Gly Pro Gly Asn Tyr Lys Tyr Lys Thr Lys Cys Thr Trp
   145 150 155 160
   Leu Ile Glu Gly Gln Pro Asn Arg Ile Met Arg Leu Arg Phe Asn His
   165 170 175
   Phe Ala Thr Glu Cys Ser Trp Asp His Leu Tyr Val Tyr Asp Gly Asp
   180 185 190
   Ser Ile Tyr Ala Pro Leu Val Ala Ala Phe Ser Gly Leu Ile Val Pro
   195 200 205
   Glu Arg Asp Gly Asn Glu Thr Val Pro Glu Val Val Ala Thr Ser Gly
   210 215 220
   Tyr Ala Leu Leu His Phe Phe Ser Asp Ala Ala Tyr Asn Leu Thr Gly
   225 230 235 240
   Phe Asn Ile Thr Tyr Ser Phe Asp Met Cys Pro Asn Asn Cys Ser Gly
   245 250 255
   Arg Gly Glu Cys Lys Ile Ser Asn Ser Ser Asp Thr Val Glu Cys Glu
               260                 265                 270
   Cys Ser Glu Asn Trp Lys Gly Glu Ala Cys Asp Ile Pro His Cys Thr
           275                 280                 285
   Asp Asn Cys Gly Phe Pro His Arg Gly Ile Cys Asn Ser Ser Asp Val
       290                 295                 300
   Arg Gly Cys Ser Cys Phe Ser Asp Trp Gln Gly Pro Gly Cys Ser Val
   305                 310                 315                 320
   Pro Val Pro Ala Asn Gln Ser Phe Trp Thr Arg Glu Glu Tyr Ser Asn
                   325                 330                 335
   Leu Lys Leu Pro Arg Ala Ser His Lys Ala Val Val Asn Gly Asn Ile
               340                 345                 350
   Met Trp Val Val Gly Gly Tyr Met Phe Asn His Ser Asp Tyr Asn Met
           355                 360                 365
   Val Leu Ala Tyr Asp Leu Ala Ser Arg Glu Trp Leu Pro Leu Asn Arg
       370                 375                 380
   Ser Val Asn Asn Val Val Val Arg Tyr Gly His Ser Leu Ala Leu Tyr
   385                 390                 395                 400
   Lys Asp Lys Ile Tyr Met Tyr Gly Gly Lys Ile Asp Ser Thr Gly Asn
                   405                 410                 415
   Val Thr Asn Glu Leu Arg Val Phe His Ile His Asn Glu Ser Trp Val
               420                 425                 430
   Leu Leu Thr Pro Lys Ala Lys Glu Gln Tyr Ala Val Val Gly His Ser
           435                 440                 445
   Ala His Ile Val Thr Leu Lys Asn Gly Arg Val Val Met Leu Val Ile
       450                 455                 460
   Phe Gly His Cys Pro Leu Tyr Gly Tyr Ile Ser Asn Val Gln Glu Tyr
   465                 470                 475                 480
   Asp Leu Asp Lys Asn Thr Trp Ser Ile Leu His Thr Gln Gly Ala Leu
                   485                 490                 495
   Val Gln Gly Gly Tyr Gly His Ser Ser Val Tyr Asp His Arg Thr Arg
               500                 505                 510
   Ala Leu Tyr Val His Gly Gly Tyr Lys Ala Phe Ser Ala Asn Lys Tyr
           515                 520                 525
   Arg Leu Ala Asp Asp Leu Tyr Arg Tyr Asp Val Asp Thr Gln Met Trp
       530                 535                 540
   Thr Ile Leu Lys Asp Ser Arg Phe Phe Arg Tyr Leu His Thr Ala Val
   545                 550                 555                 560
   Ile Val Ser Gly Thr Met Leu Val Phe Gly Gly Asn Thr His Asn Asp
                   565                 570                 575
   Thr Ser Met Ser His Gly Ala Lys Cys Phe Ser Ser Asp Phe Met Ala
               580                 585                 590
   Tyr Asp Ile Ala Cys Asp Arg Trp Ser Val Leu Pro Arg Pro Asp Leu
           595                 600                 605
   His His Asp Val Asn Arg Phe Gly His Ser Ala Val Leu His Asn Ser
       610                 615                 620
   Thr Met Tyr Val Phe Gly Gly Phe Asn Ser Leu Leu Leu Ser Asp Ile
   625                 630                 635                 640
   Leu Val Phe Thr Ser Glu Gln Cys Asp Ala His Arg Ser Glu Ala Ala
                   645                 650                 655
   Cys Leu Ala Ala Gly Pro Gly Ile Arg Cys Val Trp Asn Thr Gly Ser
               660                 665                 670
   Ser Gln Cys Ile Ser Trp Ala Leu Ala Thr Asp Glu Gln Glu Glu Lys
           675                 680                 685
   Leu Lys Ser Glu Cys Phe Ser Lys Arg Thr Leu Asp His Asp Arg Cys
       690                 695                 700
   Asp Gln His Thr Asp Cys Tyr Ser Cys Thr Ala Asn Thr Asn Asp Cys
   705                 710                 715                 720
   His Trp Cys Asn Asp His Cys Val Pro Arg Asn His Ser Cys Ser Glu
                   725                 730                 735
   Gly Gln Ile Ser Ile Phe Arg Tyr Glu Asn Cys Pro Lys Asp Asn Pro
               740                 745                 750
   Met Tyr Tyr Cys Asn Lys Lys Thr Ser Cys Arg Ser Cys Ala Leu Asp
           755                 760                 765
   Gln Asn Cys Gln Trp Glu Pro Arg Asn Gln Glu Cys Ile Ala Leu Pro
       770                 775                 780
   Glu Asn Ile Cys Gly Ile Gly Trp His Leu Val Gly Asn Ser Cys Leu
   785                 790                 795                 800
   Lys Ile Thr Thr Ala Lys Glu Asn Tyr Asp Asn Ala Lys Leu Phe Cys
                   805                 810                 815
   Arg Asn His Asn Ala Leu Leu Ala Ser Leu Thr Thr Gln Lys Lys Val
               820                 825                 830
   Glu Phe Val Leu Lys Gln Leu Arg Ile Met Gln Ser Ser Gln Ser Met
           835                 840                 845
   Ser Lys Leu Thr Leu Thr Pro Trp Val Gly Leu Arg Lys Ile Asn Val
       850                 855                 860
   Ser Tyr Trp Cys Trp Glu Asp Met Ser Pro Phe Thr Asn Ser Leu Leu
   865                 870                 875                 880
   Gln Trp Met Pro Ser Glu Pro Ser Asp Ala Gly Phe Cys Gly Ile Leu
                   885                 890                 895
   Ser Glu Pro Ser Thr Arg Gly Leu Lys Ala Ala Thr Cys Ile Asn Pro
               900                 905                 910
   Leu Asn Gly Ser Val Cys Glu Arg Pro Ala Asn His Ser Ala Lys Gln
           915                 920                 925
   Cys Arg Thr Pro Cys Ala Leu Arg Thr Ala Cys Gly Asp Cys Thr Ser
       930                 935                 940
   Gly Ser Ser Glu Cys Met Trp Cys Ser Asn Met Lys Gln Cys Val Asp
   945                 950                 955                 960
   Ser Asn Ala Tyr Val Ala Ser Phe Pro Phe Gly Gln Cys Met Glu Trp
                   965                 970                 975
   Tyr Thr Met Ser Thr Cys Pro Pro Glu Asn Cys Ser Gly Tyr Cys Thr
               980                 985                 990
   Cys Ser His Cys Leu Glu Gln Pro Gly Cys Gly Trp Cys Thr Asp Pro
           995                 1000                1005
   Ser Asn Thr Gly Lys Gly Lys Cys Ile Glu Gly Ser Tyr Lys Gly Pro
       1010                1015                1020
   Val Lys Met Pro Ser Gln Ala Pro Thr Gly Asn Phe Tyr Pro Gln Pro
   1025                1030                1035                1040
   Leu Leu Asn Ser Ser Met Cys Leu Glu Asp Ser Arg Tyr Asn Trp Ser
                   1045                1050                1055
   Phe Ile His Cys Pro Ala Cys Gln Cys Asn Gly His Ser Lys Cys Ile
               1060                1065                1070
   Asn Gln Ser Ile Cys Glu Lys Cys Glu Asn Leu Thr Thr Gly Lys His
           1075                1080                1085
   Cys Glu Thr Cys Ile Ser Gly Phe Tyr Gly Asp Pro Thr Asn Gly Gly
       1090                1095                1100
   Lys Cys Gln Pro Cys Lys Cys Asn Gly His Ala Ser Leu Cys Asn Thr
   1105                1110                1115                1120
   Asn Thr Gly Lys Cys Phe Cys Thr Thr Lys Gly Val Lys Gly Asp Glu
                   1125                1130                1135
   Cys Gln Leu Cys Glu Val Glu Asn Arg Tyr Gln Gly Asn Pro Leu Arg
               1140                1145                1150
   Gly Thr Cys Tyr Tyr Thr Leu Leu Ile Asp Tyr Gln Phe Thr Phe Ser
           1155                1160                1165
   Leu Ser Gln Glu Asp Asp Arg Tyr Tyr Thr Ala Ile Asn Phe Val Ala
       1170                1175                1180
   Thr Pro Asp Glu Gln Asn Arg Asp Leu Asp Met Phe Ile Asn Ala Ser
   1185                1190                1195                1200
   Lys Asn Phe Asn Leu Asn Ile Thr Trp Ala Ala Ser Phe Ser Ala Gly
                   1205                1210                1215
   Thr Gln Ala Gly Glu Glu Met Pro Val Val Ser Lys Thr Asn Ile Lys
               1220                1225                1230
   Glu Tyr Lys Asp Ser Phe Ser Asn Glu Lys Phe Asp Phe Arg Asn His
           1235                1240                1245
   Pro Asn Ile Thr Phe Phe Val Tyr Val Ser Asn Phe Thr Trp Pro Ile
       1250                1255                1260
   Lys Ile Gln Ile Ala Phe Ser Gln His Ser Asn Phe Met Asp Leu Val
   1265                1270                1275                1280
   Gln Phe Phe Val Thr Phe Phe Ser Cys Phe Leu Ser Leu Leu Leu Val
                   1285                1290                1295
   Ala Ala Val Val Trp Lys Ile Lys Gln Ser Cys Trp Ala Ser Arg Arg
               1300                1305                1310
   Arg Glu Gln Leu Leu Arg Glu Met Gln Gln Met Ala Ser Arg Pro Phe
           1315                1320                1325
   Ala Ser Val Asn Val Ala Leu Glu Thr Asp Glu Glu Pro Pro Asp Leu
       1330                1335                1340
   Ile Gly Gly Ser Ile Lys Thr Val Pro Lys Pro Ile Ala Leu Glu Pro
   1345                1350                1355                1360
   Cys Phe Gly Asn Lys Ala Ala Val Leu Ser Val Phe Val Arg Leu Pro
                   1365                1370                1375
   Arg Gly Leu Gly Gly Ile Pro Pro Pro Gly Gln Ser Gly Leu Ala Val
               1380                1385                1390
   Ala Ser Ala Leu Val Asp Ile Ser Gln Gln Met Pro Ile Val Tyr Lys
           1395                1400                1405
   Glu Lys Ser Gly Ala Val Arg Asn Arg Lys Gln Gln Pro Pro Ala Gln
       1410                1415                1420
   Pro Gly Thr Cys Ile
   1425
<210> 41
   <211> 291
   <212> PRT
   <213> Homo sapiens
<400> 41
   Leu Leu Ser Leu Leu Leu Leu Leu Gly Pro Ala Val Pro Gln Glu Asn
    1               5                  10                  15
   Gln Asp Gly Arg Tyr Ser Leu Thr Tyr Ile Tyr Thr Gly Leu Ser Lys
               20                  25                  30
   His Val Glu Asp Val Pro Ala Phe Gln Ala Leu Gly Ser Leu Asn Asp
           35                  40                  45
   Leu Gln Phe Phe Arg Tyr Asn Ser Lys Asp Arg Lys Ser Gln Pro Met
       50                  55                  60
   Gly Leu Trp Arg Gln Val Glu Gly Met Glu Asp Trp Lys Gln Asp Ser
   65                  70                  75                  80
   Gln Leu Gln Lys Ala Arg Glu Asp Ile Phe Met Glu Thr Leu Lys Asp
                   85                  90                  95
   Ile Val Glu Tyr Tyr Asn Asp Ser Asn Gly Ser His Val Leu Gln Gly
               100                 105                 110
   Arg Phe Gly Cys Glu Ile Glu Asn Asn Arg Ser Ser Gly Ala Phe Trp
           115                 120                 125
   Lys Tyr Tyr Tyr Asp Gly Lys Asp Tyr Ile Glu Phe Asn Lys Glu Ile
       130                 135                 140
   Pro Ala Trp Val Pro Phe Asp Pro Ala Ala Gln Ile Thr Lys Gln Lys
   145                 150                 155                 160
   Trp Glu Ala Glu Pro Val Tyr Val Gln Arg Ala Lys Ala Tyr Leu Glu
                   165                 170                 175
   Glu Glu Cys Pro Ala Thr Leu Arg Lys Tyr Leu Lys Tyr Ser Lys Asn
               180                 185                 190
   Ile Leu Asp Arg Gln Asp Pro Pro Ser Val Val Val Thr Ser His Gln
           195                 200                 205
   Ala Pro Gly Glu Lys Lys Lys Leu Lys Cys Leu Ala Tyr Asp Phe Tyr
       210                 215                 220
   Pro Gly Lys Ile Asp Val His Trp Thr Arg Ala Gly Glu Val Gln Glu
   225                 230                 235                 240
   Pro Glu Leu Arg Gly Asp Val Leu His Asn Gly Asn Gly Thr Tyr Gln
                   245                 250                 255
   Ser Trp Val Val Val Ala Val Pro Pro Gln Asp Thr Ala Pro Tyr Ser
               260                 265                 270
   Cys His Val Gln His Ser Ser Leu Ala Gln Pro Leu Val Val Pro Trp
           275                 280                 285
   Glu Ala Ser
       290
<210> 42
   <211> 245
   <212> PRT
   <213> Homo sapiens
<400> 42
<210> 43
   <211> 1744
   <212> PRT
   <213> Homo sapiens
<400> 43
   Met Arg Leu Leu Trp Gly Leu Ile Trp Ala Ser Ser Phe Phe Thr Leu
    1               5                  10                  15
   Ser Leu Gln Lys Pro Arg Leu Leu Leu Phe Ser Pro Ser Val Val His
               20                  25                  30
   Leu Gly Val Pro Leu Ser Val Gly Val Gln Leu Gln Asp Val Pro Arg
           35                  40                  45
   Gly Gln Val Val Lys Gly Ser Val Phe Leu Arg Asn Pro Ser Arg Asn
       50                  55                  60
   Asn Val Pro Cys Ser Pro Lys Val Asp Phe Thr Leu Ser Ser Glu Arg
   65                  70                  75                  80
   Asp Phe Ala Leu Leu Ser Leu Gln Val Pro Leu Lys Asp Ala Lys Ser
                       85                  90                  95
   Cys Gly Leu His Gln Leu Leu Arg Gly Pro Glu Val Gln Leu Val Ala
               100                 105                 110
   His Ser Pro Trp Leu Lys Asp Ser Leu Ser Arg Thr Thr Asn Ile Gln
           115                 120                 125
   Gly Ile Asn Leu Leu Phe Ser Ser Arg Arg Gly His Leu Phe Leu Gln
       130                 135                 140
   Thr Asp Gln Pro Ile Tyr Asn Pro Gly Gln Arg Val Arg Tyr Arg Val
   145                 150                 155                 160
   Phe Ala Leu Asp Gln Lys Met Arg Pro Ser Thr Asp Thr Ile Thr Val
                   165                 170                 175
   Met Val Glu Asn Ser His Gly Leu Arg Val Arg Lys Lys Glu Val Tyr
               180                 185                 190
   Met Pro Ser Ser Ile Phe Gln Asp Asp Phe Val Ile Pro Asp Ile Ser
           195                 200                 205
   Glu Pro Gly Thr Trp Lys Ile Ser Ala Arg Phe Ser Asp Gly Leu Glu
       210                 215                 220
   Ser Asn Ser Ser Thr Gln Phe Glu Val Lys Lys Tyr Val Leu Pro Asn
   225                 230                 235                 240
   Phe Glu Val Lys Ile Thr Pro Gly Lys Pro Tyr Ile Leu Thr Val Pro
                   245                 250                 255
   Gly His Leu Asp Glu Met Gln Leu Asp Ile Gln Ala Arg Tyr Ile Tyr
               260                 265                 270
   Gly Lys Pro Val Gln Gly Val Ala Tyr Val Arg Phe Gly Leu Leu Asp
           275                 280                 285
   Glu Asp Gly Lys Lys Thr Phe Phe Arg Gly Leu Glu Ser Gln Thr Lys
       290                 295                 300
   Leu Val Asn Gly Gln Ser His Ile Ser Leu Ser Lys Ala Glu Phe Gln
   305                 310                 315                 320
   Asp Ala Leu Glu Lys Leu Asn Met Gly Ile Thr Asp Leu Gln Gly Leu
                   325                 330                 335
   Arg Leu Tyr Val Ala Ala Ala Ile Ile Glu Tyr Pro Gly Gly Glu Met
               340                 345                 350
   Glu Glu Ala Glu Leu Thr Ser Trp Tyr Phe Val Ser Ser Pro Phe Ser
           355                 360                 365
   Leu Asp Leu Ser Lys Thr Lys Arg His Leu Val Pro Gly Ala Pro Phe
       370                 375                 380
   Leu Leu Gln Ala Leu Val Arg Glu Met Ser Gly Ser Pro Ala Ser Gly
   385                 390                 395                 400
   Ile Pro Val Lys Val Ser Ala Thr Val Ser Ser Pro Gly Ser Val Pro
                   405                 410                 415
   Glu Val Gln Asp Ile Gln Gln Asn Thr Asp Gly Ser Gly Gln Val Ser
               420                 425                 430
   Ile Pro Ile Ile Ile Pro Gln Thr Ile Ser Glu Leu Gln Leu Ser Val
           435                 440                 445
   Ser Ala Gly Ser Pro His Pro Ala Ile Ala Arg Leu Thr Val Ala Ala
       450                 455                 460
   Pro Pro Ser Gly Gly Pro Gly Phe Leu Ser Ile Glu Arg Pro Asp Ser
   465                 470                 475                 480
   Arg Pro Pro Arg Val Gly Asp Thr Leu Asn Leu Asn Leu Arg Ala Val
                   485                 490                 495
   Gly Ser Gly Ala Thr Phe Ser His Tyr Tyr Tyr Met Ile Leu Ser Arg
               500                 505                 510
   Gly Gln Ile Val Phe Met Asn Arg Glu Pro Lys Arg Thr Leu Thr Ser
           515                 520                 525
   Val Ser Val Phe Val Asp His His Leu Ala Pro Ser Phe Tyr Phe Val
       530                 535                 540
   Ala Phe Tyr Tyr His Gly Asp His Pro Val Ala Asn Ser Leu Arg Val
   545                 550                 555                 560
   Asp Val Gln Ala Gly Ala Cys Glu Gly Lys Leu Glu Leu Ser Val Asp
                   565                 570                 575
   Gly Ala Lys Gln Tyr Arg Asn Gly Glu Ser Val Lys Leu His Leu Glu
               580                 585                 590
   Thr Asp Ser Leu Ala Leu Val Ala Leu Gly Ala Leu Asp Thr Ala Leu
           595                 600                 605
   Tyr Ala Ala Gly Ser Lys Ser His Lys Pro Leu Asn Met Gly Lys Val
       610                 615                 620
   Phe Glu Ala Met Asn Ser Tyr Asp Leu Gly Cys Gly Pro Gly Gly Gly
   625                 630                 635                 640
   Asp Ser Ala Leu Gln Val Phe Gln Ala Ala Gly Leu Ala Phe Ser Asp
                   645                 650                 655
   Gly Asp Gln Trp Thr Leu Ser Arg Lys Arg Leu Ser Cys Pro Lys Glu
               660                 665                 670
   Lys Thr Thr Arg Lys Lys Arg Asn Val Asn Phe Gln Lys Ala Ile Asn
           675                 680                 685
   Glu Lys Leu Gly Gln Tyr Ala Ser Pro Thr Ala Lys Arg Cys Cys Gln
       690                 695                 700
   Asp Gly Val Thr Arg Leu Pro Met Met Arg Ser Cys Glu Gln Arg Ala
   705                 710                 715                 720
   Ala Arg Val Gln Gln Pro Asp Cys Arg Glu Pro Phe Leu Ser Cys Cys
                   725                 730                 735
   Gln Phe Ala Glu Ser Leu Arg Lys Lys Ser Arg Asp Lys Gly Gln Ala
               740                 745                 750
   Gly Leu Gln Arg Ala Leu Glu Ile Leu Gln Glu Glu Asp Leu Ile Asp
           755                 760                 765
   Glu Asp Asp Ile Pro Val Arg Ser Phe Phe Pro Glu Asn Trp Leu Trp
       770                 775                 780
   Arg Val Glu Thr Val Asp Arg Phe Gln Ile Leu Thr Leu Trp Leu Pro
   785                 790                 795                 800
   Asp Ser Leu Thr Thr Trp Glu Ile His Gly Leu Ser Leu Ser Lys Thr
                   805                 810                 815
   Lys Gly Leu Cys Val Ala Thr Pro Val Gln Leu Arg Val Phe Arg Glu
               820                 825                 830
   Phe His Leu His Leu Arg Leu Pro Met Ser Val Arg Arg Phe Glu Gln
           835                 840                 845
   Leu Glu Leu Arg Pro Val Leu Tyr Asn Tyr Leu Asp Lys Asn Leu Thr
       850                 855                 860
   Val Ser Val His Val Ser Pro Val Glu Gly Leu Cys Leu Ala Gly Gly
   865                 870                 875                 880
   Gly Gly Leu Ala Gln Gln Val Leu Val Pro Ala Gly Ser Ala Arg Pro
                   885                 890                 895
   Val Ala Phe Ser Val Val Pro Thr Ala Ala Ala Ala Val Ser Leu Lys
               900                 905                 910
   Val Val Ala Arg Gly Ser Phe Glu Phe Pro Val Gly Asp Ala Val Ser
           915                 920                 925
   Lys Val Leu Gln Ile Glu Lys Glu Gly Ala Ile His Arg Glu Glu Leu
       930                 935                 940
   Val Tyr Glu Leu Asn Pro Leu Asp His Arg Gly Arg Thr Leu Glu Ile
   945                 950                 955                 960
   Pro Gly Asn Ser Asp Pro Asn Met Ile Pro Asp Gly Asp Phe Asn Ser
                   965                 970                 975
   Tyr Val Arg Val Thr Ala Ser Asp Pro Leu Asp Thr Leu Gly Ser Glu
               980                 985                 990
   Gly Ala Leu Ser Pro Gly Gly Val Ala Ser Leu Leu Arg Leu Pro Arg
           995                 1000                1005
   Gly Cys Gly Glu Gln Thr Met Ile Tyr Leu Ala Pro Thr Leu Ala Ala
       1010                1015                1020
   Ser Arg Tyr Leu Asp Lys Thr Glu Gln Trp Ser Thr Leu Pro Pro Glu
   1025                1030                1035                1040
   Thr Lys Asp His Ala Val Asp Leu Ile Gln Lys Gly Tyr Met Arg Ile
                   1045                1050                1055
   Gln Gln Phe Arg Lys Ala Asp Gly Ser Tyr Ala Ala Trp Leu Ser Arg
               1060                1065                1070
   Asp Ser Ser Thr Trp Leu Thr Ala Phe Val Leu Lys Val Leu Ser Leu
           1075                1080                1085
   Ala Gln Glu Gln Val Gly Gly Ser Pro Glu Lys Leu Gln Glu Thr Ser
       1090                1095                1100
   Asn Trp Leu Leu Ser Gln Gln Gln Ala Asp Gly Ser Phe Gln Asp Pro
   1105                1110                1115                1120
   Cys Pro Val Leu Asp Arg Ser Met Gln Gly Gly Leu Val Gly Asn Asp
                   1125                1130                1135
   Glu Thr Val Ala Leu Thr Ala Phe Val Thr Ile Ala Leu His His Gly
               1140                1145                1150
   Leu Ala Val Phe Gln Asp Glu Gly Ala Glu Pro Leu Lys Gln Arg Val
           1155                1160                1165
   Glu Ala Ser Ile Ser Lys Ala Asn Ser Phe Leu Gly Glu Lys Ala Ser
       1170                1175                1180
   Ala Gly Leu Leu Gly Ala His Ala Ala Ala Ile Thr Ala Tyr Ala Leu
   1185                1190                1195                1200
   Thr Leu Thr Lys Ala Pro Val Asp Leu Leu Gly Val Ala His Asn Asn
                   1205                1210                1215
   Leu Met Ala Met Ala Gln Glu Thr Gly Asp Asn Leu Tyr Trp Gly Ser
               1220                1225                1230
   Val Thr Gly Ser Gln Ser Asn Ala Val Ser Pro Thr Pro Ala Pro Arg
           1235                1240                1245
   Asn Pro Ser Asp Pro Met Pro Gln Ala Pro Ala Leu Trp Ile Glu Thr
       1250                1255                1260
   Thr Ala Tyr Ala Leu Leu His Leu Leu Leu His Glu Gly Lys Ala Glu
   1265                1270                1275                1280
   Met Ala Asp Gln Ala Ala Ala Trp Leu Thr Arg Gln Gly Ser Phe Gln
                   1285                1290                1295
   Gly Gly Phe Arg Ser Thr Gln Asp Thr Val Ile Ala Leu Asp Ala Leu
               1300                1305                1310
   Ser Ala Tyr Trp Ile Ala Ser His Thr Thr Glu Glu Arg Gly Leu Asn
           1315                1320                1325
   Val Thr Leu Ser Ser Thr Gly Arg Asn Gly Phe Lys Ser His Ala Leu
       1330                1335                1340
   Gln Leu Asn Asn Arg Gln Ile Arg Gly Leu Glu Glu Glu Leu Gln Phe
   1345                1350                1355                1360
   Ser Leu Gly Ser Lys Ile Asn Val Lys Val Gly Gly Asn Ser Lys Gly
                   1365                1370                1375
   Thr Leu Lys Val Leu Arg Thr Tyr Asn Val Leu Asp Met Lys Asn Thr
               1380                1385                1390
   Thr Cys Gln Asp Leu Gln Ile Glu Val Thr Val Lys Gly His Val Glu
           1395                1400                1405
   Tyr Thr Met Glu Ala Asn Glu Asp Tyr Glu Asp Tyr Glu Tyr Asp Glu
       1410                1415                1420
   Leu Pro Ala Lys Asp Asp Pro Asp Ala Pro Leu Gln Pro Val Thr Pro
   1425                1430                1435                1440
   Leu Gln Leu Phe Glu Gly Arg Arg Asn Arg Arg Arg Arg Glu Ala Pro
                   1445                1450                1455
   Lys Val Val Glu Glu Gln Glu Ser Arg Val His Tyr Thr Val Cys Ile
               1460                1465                1470
   Trp Arg Asn Gly Lys Val Gly Leu Ser Gly Met Ala Ile Ala Asp Val
           1475                1480                1485
   Thr Leu Leu Ser Gly Phe His Ala Leu Arg Ala Asp Leu Glu Lys Leu
       1490                1495                1500
   Thr Ser Leu Ser Asp Arg Tyr Val Ser His Phe Glu Thr Glu Gly Pro
   1505                1510                1515                1520
   His Val Leu Leu Tyr Phe Asp Ser Val Pro Thr Ser Arg Glu Cys Val
                   1525                1530                1535
   Gly Phe Glu Ala Val Gln Glu Val Pro Val Gly Leu Val Gln Pro Ala
               1540                1545                1550
   Ser Ala Thr Leu Tyr Asp Tyr Tyr Asn Pro Glu Arg Arg Cys Ser Val
           1555                1560                1565
   Phe Tyr Gly Ala Pro Ser Lys Ser Arg Leu Leu Ala Thr Leu Cys Ser
       1570                1575                1580
   Ala Glu Val Cys Gln Cys Ala Glu Gly Lys Cys Pro Arg Gln Arg Arg
   1585                1590                1595                1600
   Ala Leu Glu Arg Gly Leu Gln Asp Glu Asp Gly Tyr Arg Met Lys Phe
   1605 1610 1615
   Ala Cys Tyr Tyr Pro Arg Val Glu Tyr Gly Phe Gln Val Lys Val Leu
   1620 1625 1630
   Arg Glu Asp Ser Arg Ala Ala Phe Arg Leu Phe Glu Thr Lys Ile Thr
   1635 1640 1645
   Gln Val Leu His Phe Thr Lys Asp Val Lys Ala Ala Ala Asn Gln Met
   1650 1655 1660
   Arg Asn Phe Leu Val Arg Ala Ser Cys Arg Leu Arg Leu Glu Pro Gly
   1665 1670 1675 1680
   Lys Glu Tyr Leu Ile Met Gly Leu Asp Gly Ala Thr Tyr Asp Leu Glu
   1685 1690 1695
   Gly His Pro Gln Tyr Leu Leu Asp Ser Asn Ser Trp Ile Glu Glu Met
   1700 1705 1710
   Pro Ser Glu Arg Leu Cys Arg Ser Thr Arg Gln Arg Ala Ala Cys Ala
   1715 1720 1725
   Gln Leu Asn Asp Phe Leu Gln Glu Tyr Gly Thr Gln Gly Cys Gln Val
   1730 1735 1740
<210> 44
   <211> 597
   <212> PRT
   <213> Homo sapiens
<400> 44
   Met His Pro Pro Lys Thr Pro Ser Gly Ala Leu His Arg Lys Arg Lys
    1               5                  10                  15
   Met Ala Ala Trp Pro Phe Ser Arg Leu Trp Lys Val Ser Asp Pro Ile
               20                  25                  30
   Leu Phe Gln Met Thr Leu Ile Ala Ala Leu Leu Pro Ala Val Leu Gly
           35                  40                  45
   Asn Cys Gly Pro Pro Pro Thr Leu Ser Phe Ala Ala Pro Met Asp Ile
       50                  55                  60
   Thr Leu Thr Glu Thr Arg Phe Lys Thr Gly Thr Thr Leu Lys Tyr Thr
   65                  70                  75                  80
   Cys Leu Pro Gly Tyr Val Arg Ser His Ser Thr Gln Thr Leu Thr Cys
                   85                  90                  95
   Asn Ser Asp Gly Glu Trp Val Tyr Asn Thr Phe Cys Ile Tyr Lys Arg
               100                 105                 110
   Cys Arg His Pro Gly Glu Leu Arg Asn Gly Gln Val Glu Ile Lys Thr
           115                 120                 125
   Asp Leu Ser Phe Gly Ser Gln Ile Glu Phe Ser Cys Ser Glu Gly Phe
       130                 135                 140
   Phe Leu Ile Gly Ser Thr Thr Ser Arg Cys Glu Val Gln Asp Arg Gly
   145                 150                 155                 160
   Val Gly Trp Ser His Pro Leu Pro Gln Cys Glu Ile Val Lys Cys Lys
                   165                 170                 175
   Pro Pro Pro Asp Ile Arg Asn Gly Arg His Ser Gly Glu Glu Asn Phe
               180                 185                 190
   Tyr Ala Tyr Gly Phe Ser Val Thr Tyr Ser Cys Asp Pro Arg Phe Ser
           195                 200                 205
   Leu Leu Gly His Ala Ser Ile Ser Cys Thr Val Glu Asn Glu Thr Ile
       210                 215                 220
   Gly Val Trp Arg Pro Ser Pro Pro Thr Cys Glu Lys Ile Thr Cys Arg
   225                 230                 235                 240
   Lys Pro Asp Val Ser His Gly Glu Met Val Ser Gly Phe Gly Pro Ile
                   245                 250                 255
   Tyr Asn Tyr Lys Asp Thr Ile Val Phe Lys Cys Gln Lys Gly Phe Val
               260                 265                 270
   Leu Arg Gly Ser Ser Val Ile His Cys Asp Ala Asp Ser Lys Trp Asn
           275                 280                 285
   Pro Ser Pro Pro Ala Cys Glu Pro Asn Ser Cys Ile Asn Leu Pro Asp
       290                 295                 300
   Ile Pro His Ala Ser Trp Glu Thr Tyr Pro Arg Pro Thr Lys Glu Asp
   305                 310                 315                 320
   Val Tyr Val Val Gly Thr Val Leu Arg Tyr Arg Cys His Pro Gly Tyr
                   325                 330                 335
   Lys Pro Thr Thr Asp Glu Pro Thr Thr Val Ile Cys Gln Lys Asn Leu
               340                 345                 350
   Arg Trp Thr Pro Tyr Gln Gly Cys Glu Ala Leu Cys Cys Pro Glu Pro
           355                 360                 365
   Lys Leu Asn Asn Gly Glu Ile Thr Gln His Arg Lys Ser Arg Pro Ala
       370                 375                 380
   Asn His Cys Val Tyr Phe Tyr Gly Asp Glu Ile Ser Phe Ser Cys His
   385                 390                 395                 400
   Glu Thr Ser Arg Phe Ser Ala Ile Cys Gln Gly Asp Gly Thr Trp Ser
                   405                 410                 415
   Pro Arg Thr Pro Ser Cys Gly Asp Ile Cys Asn Phe Pro Pro Lys Ile
               420                 425                 430
   Ala His Gly His Tyr Lys Gln Ser Ser Ser Tyr Ser Phe Phe Lys Glu
           435                 440                 445
   Glu Ile Ile Tyr Glu Cys Asp Lys Gly Tyr Ile Leu Val Gly Gln Ala
       450                 455                 460
   Lys Leu Ser Cys Ser Tyr Ser His Trp Ser Ala Pro Ala Pro Gln Cys
   465                 470                 475                 480
   Lys Ala Leu Cys Arg Lys Pro Glu Leu Val Asn Gly Arg Leu Ser Val
                   485                 490                 495
   Asp Lys Asp Gln Tyr Val Glu Pro Glu Asn Val Thr Ile Gln Cys Asp
               500                 505                 510
   Ser Gly Tyr Gly Val Val Gly Pro Gln Ser Ile Thr Cys Ser Gly Asn
           515                 520                 525
   Arg Thr Trp Tyr Pro Glu Val Pro Lys Cys Glu Trp Glu Thr Pro Glu
       530                 535                 540
   Gly Cys Glu Gln Val Leu Thr Gly Lys Arg Leu Met Gln Cys Leu Pro
   545                 550                 555                 560
   Asn Pro Glu Asp Val Lys Met Ala Leu Glu Val Tyr Lys Leu Ser Leu
                   565                 570                 575
   Glu Ile Glu Gln Leu Glu Leu Gln Arg Asp Ser Ala Arg Gln Ser Thr
               580                 585                 590
   Leu Asp Lys Glu Leu
           595
<210> 45
   <211> 584
   <212> PRT
   <213> Homo sapiens
<400> 45
   Met Phe Ala Val Val Phe Phe Ile Leu Ser Leu Met Thr Cys Gln Pro
    1               5                  10                  15
   Gly Val Thr Ala Gln Glu Lys Val Asn Gln Arg Val Arg Arg Ala Ala
               20                  25                  30
   Thr Pro Ala Ala Val Thr Cys Gln Leu Ser Asn Trp Ser Glu Trp Thr
           35                  40                  45
   Asp Cys Phe Pro Cys Gln Asp Lys Lys Tyr Arg His Arg Ser Leu Leu
       50                  55                  60
   Gln Pro Asn Lys Phe Gly Gly Thr Ile Cys Ser Gly Asp Ile Trp Asp
   65                  70                  75                  80
   Gln Ala Ser Cys Ser Ser Ser Thr Thr Cys Val Arg Gln Ala Gln Cys
                   85                  90                  95
   Gly Gln Asp Phe Gln Cys Lys Glu Thr Gly Arg Cys Leu Lys Arg His
               100                 105                 110
   Leu Val Cys Asn Gly Asp Gln Asp Cys Leu Asp Gly Ser Asp Glu Asp
           115                 120                 125
   Asp Cys Glu Asp Val Arg Ala Ile Asp Glu Asp Cys Ser Gln Tyr Glu
       130                 135                 140
   Pro Ile Pro Gly Ser Gln Lys Ala Ala Leu Gly Tyr Asn Ile Leu Thr
   145                 150                 155                 160
   Gln Glu Asp Ala Gln Ser Val Tyr Asp Ala Ser Tyr Tyr Gly Gly Gln
                   165                 170                 175
   Cys Glu Thr Val Tyr Asn Gly Glu Trp Arg Glu Leu Arg Tyr Asp Ser
               180                 185                 190
   Thr Cys Glu Arg Leu Tyr Tyr Gly Asp Asp Glu Lys Tyr Phe Arg Lys
           195                 200                 205
   Pro Tyr Asn Phe Leu Lys Tyr His Phe Glu Ala Leu Ala Asp Thr Gly
       210                 215                 220
   Ile Ser Ser Glu Phe Tyr Asp Asn Ala Asn Asp Leu Leu Ser Lys Val
   225                 230                 235                 240
   Lys Lys Asp Lys Ser Asp Ser Phe Gly Val Thr Ile Gly Ile Gly Pro
                   245                 250                 255
   Ala Gly Ser Pro Leu Leu Val Gly Val Gly Val Ser His Ser Gln Asp
               260                 265                 270
   Thr Ser Phe Leu Asn Glu Leu Asn Lys Tyr Asn Glu Lys Lys Phe Ile
           275                 280                 285
   Phe Thr Arg Ile Phe Thr Lys Val Gln Thr Ala His Phe Lys Met Arg
       290                 295                 300
   Lys Asp Asp Ile Met Leu Asp Glu Gly Met Leu Gln Ser Leu Met Glu
   305                 310                 315                 320
   Leu Pro Asp Gln Tyr Asn Tyr Gly Met Tyr Ala Lys Phe Ile Asn Asp
                   325                 330                 335
   Tyr Gly Thr His Tyr Ile Thr Ser Gly Ser Met Gly Gly Ile Tyr Glu
               340                 345                 350
   Tyr Ile Leu Val Ile Asp Lys Ala Lys Met Glu Ser Leu Gly Ile Thr
           355                 360                 365
   Ser Arg Asp Ile Thr Thr Cys Phe Gly Gly Ser Leu Gly Ile Gln Tyr
       370                 375                 380
   Glu Asp Lys Ile Asn Val Gly Gly Gly Leu Ser Gly Asp His Cys Lys
   385                 390                 395                 400
   Lys Phe Gly Gly Gly Lys Thr Glu Arg Ala Arg Lys Ala Met Ala Val
                   405                 410                 415
   Glu Asp Ile Ile Ser Arg Val Arg Gly Gly Ser Ser Gly Trp Ser Gly
               420                 425                 430
   Gly Leu Ala Gln Asn Arg Ser Thr Ile Thr Tyr Arg Ser Trp Gly Arg
           435                 440                 445
   Ser Leu Lys Tyr Asn Pro Val Val Ile Asp Phe Glu Met Gln Pro Ile
       450                 455                 460
   His Glu Val Leu Arg His Thr Ser Leu Gly Pro Leu Glu Ala Lys Arg
   465                 470                 475                 480
   Gln Asn Leu Arg Arg Ala Leu Asp Gln Tyr Leu Met Glu Phe Asn Ala
                   485                 490                 495
   Cys Arg Cys Gly Pro Cys Phe Asn Asn Gly Val Pro Ile Leu Glu Gly
               500                 505                 510
   Thr Ser Cys Arg Cys Gln Cys Arg Leu Gly Ser Leu Gly Ala Ala Cys
           515                 520                 525
   Glu Gln Thr Gln Thr Glu Gly Ala Lys Ala Asp Gly Ser Trp Ser Cys
       530                 535                 540
   Trp Ser Ser Trp Ser Val Cys Arg Ala Gly Ile Gln Glu Arg Arg Arg
   545                 550                 555                 560
   Glu Cys Asp Asn Pro Ala Pro Gln Asn Gly Gly Ala Ser Cys Pro Gly
                   565                 570                 575
   Arg Lys Val Gln Thr Gln Ala Cys
               580
<210> 46
   <211> 347
   <212> PRT
   <213> Homo sapiens
<400> 46
   Met Ala Leu Leu Phe Ser Leu Ile Leu Ala Ile Cys Thr Arg Pro Gly
    1              5                   10                  15
   Phe Leu Ala Ser Pro Ser Gly Val Arg Leu Val Gly Gly Leu His Arg
               20                  25                  30
   Cys Glu Gly Arg Val Glu Val Glu Gln Lys Gly Gln Trp Gly Thr Val
           35                  40                  45
   Cys Asp Asp Gly Trp Asp Ile Lys Asp Val Ala Val Leu Cys Arg Glu
       50                  55                  60
   Leu Gly Cys Gly Ala Ala Ser Gly Thr Pro Ser Gly Ile Leu Tyr Glu
   65                  70                  75                  80
   Pro Pro Ala Glu Lys Glu Gln Lys Val Leu Ile Gln Ser Val Ser Cys
                   85                  90                  95
   Thr Gly Thr Glu Asp Thr Leu Ala Gln Cys Glu Gln Glu Glu Val Tyr
               100                 105                 110
   Asp Cys Ser His Asp Glu Asp Ala Gly Ala Ser Cys Glu Asn Pro Glu
           115                 120                 125
   Ser Ser Phe Ser Pro Val Pro Glu Gly Val Arg Leu Ala Asp Gly Pro
       130                 135                 140
   Gly His Cys Lys Gly Arg Val Glu Val Lys His Gln Asn Gln Trp Tyr
   145                 150                 155                 160
   Thr Val Cys Gln Thr Gly Trp Ser Leu Arg Ala Ala Lys Val Val Cys
                   165                 170                 175
   Arg Gln Leu Gly Cys Gly Arg Ala Val Leu Thr Gln Lys Arg Cys Asn
               180                 185                 190
   Lys His Ala Tyr Gly Arg Lys Pro Ile Trp Leu Ser Gln Met Ser Cys
           195                 200                 205
   Ser Gly Arg Glu Ala Thr Leu Gln Asp Cys Pro Ser Gly Pro Trp Gly
       210                 215                 220
   Lys Asn Thr Cys Asn His Asp Glu Asp Thr Trp Val Glu Cys Glu Asp
   225                 230                 235                 240
   Pro Phe Asp Leu Arg Leu Val Gly Gly Asp Asn Leu Cys Ser Gly Arg
                   245                 250                 255
   Leu Glu Val Leu His Lys Gly Val Trp Gly Ser Val Cys Asp Asp Asn
               260                 265                 270
   Trp Gly Glu Lys Glu Asp Gln Val Val Cys Lys Gln Leu Gly Cys Gly
           275                 280                 285
   Lys Ser Leu Ser Pro Ser Phe Arg Asp Arg Lys Cys Tyr Gly Pro Gly
       290                 295                 300
   Val Gly Arg Ile Trp Leu Asp Asn Val Arg Cys Ser Gly Glu Glu Gln
   305                 310                 315                 320
   Ser Leu Glu Gln Cys Gln His Arg Phe Trp Gly Phe His Asp Cys Thr
                   325                 330                 335
   His Gln Glu Asp Val Ala Val Ile Cys Ser Gly
               340                 345
<210> 47
   <211> 1065
   <212> PRT
   <213> Homo sapiens
<400> 47
   Met Lys Ile Leu Ile Leu Gly Ile Phe Leu Phe Leu Cys Ser Thr Pro
    1               5                  10                  15
   Ala Trp Ala Lys Glu Lys His Tyr Tyr Ile Gly Ile Ile Glu Thr Thr
               20                  25                  30
   Trp Asp Tyr Ala Ser Asp His Gly Glu Lys Lys Leu Ile Ser Val Asp
           35                  40                  45
   Thr Glu His Ser Asn Ile Tyr Leu Gln Asn Gly Pro Asp Arg Ile Gly
       50                  55                  60
   Arg Leu Tyr Lys Lys Ala Leu Tyr Leu Gln Tyr Thr Asp Glu Thr Phe
   65                  70                  75                  80
   Arg Thr Thr Ile Glu Lys Pro Val Trp Leu Gly Phe Leu Gly Pro Ile
                   85                  90                  95
   Ile Lys Ala Glu Thr Gly Asp Lys Val Tyr Val His Leu Lys Asn Leu
               100                 105                 110
   Ala Ser Arg Pro Tyr Thr Phe His Ser His Gly Ile Thr Tyr Tyr Lys
           115                 120                 125
   Glu His Glu Gly Ala Ile Tyr Pro Asp Asn Thr Thr Asp Phe Gln Arg
       130                 135                 140
   Ala Asp Asp Lys Val Tyr Pro Gly Glu Gln Tyr Thr Tyr Met Leu Leu
   145                 150                 155                 160
   Ala Thr Glu Glu Gln Ser Pro Gly Glu Gly Asp Gly Asn Cys Val Thr
                   165                 170                 175
   Arg Ile Tyr His Ser His Ile Asp Ala Pro Lys Asp Ile Ala Ser Gly
               180                 185                 190
   Leu Ile Gly Pro Leu Ile Ile Cys Lys Lys Asp Ser Leu Asp Lys Glu
           195                 200                 205
   Lys Glu Lys His Ile Asp Arg Glu Phe Val Val Met Phe Ser Val Val
       210                 215                 220
   Asp Glu Asn Phe Ser Trp Tyr Leu Glu Asp Asn Ile Lys Thr Tyr Cys
   225                 230                 235                 240
   Ser Glu Pro Glu Lys Val Asp Lys Asp Asn Glu Asp Phe Gln Glu Ser
                   245                 250                 255
   Asn Arg Met Tyr Ser Val Asn Gly Tyr Thr Phe Gly Ser Leu Pro Gly
               260                 265                 270
   Leu Ser Met Cys Ala Glu Asp Arg Val Lys Trp Tyr Leu Phe Gly Met
           275                 280                 285
   Gly Asn Glu Val Asp Val His Ala Ala Phe Phe His Gly Gln Ala Leu
       290                 295                 300
   Thr Asn Lys Asn Tyr Arg Ile Asp Thr Ile Asn Leu Phe Pro Ala Thr
   305                 310                 315                 320
   Leu Phe Asp Ala Tyr Met Val Ala Gln Asn Pro Gly Glu Trp Met Leu
                   325                 330                 335
   Ser Cys Gln Asn Leu Asn His Leu Lys Ala Gly Leu Gln Ala Phe Phe
               340                 345                 350
   Gln Val Gln Glu Cys Asn Lys Ser Ser Ser Lys Asp Asn Ile Arg Gly
           355                 360                 365
   Lys His Val Arg His Tyr Tyr Ile Ala Ala Glu Glu Ile Ile Trp Asn
       370                 375                 380
   Tyr Ala Pro Ser Gly Ile Asp Ile Phe Thr Lys Glu Asn Leu Thr Ala
   385                 390                 395                 400
   Pro Gly Ser Asp Ser Ala Val Phe Phe Glu Gln Gly Thr Thr Arg Ile
                   405                 410                 415
   Gly Gly Ser Tyr Lys Lys Leu Val Tyr Arg Glu Tyr Thr Asp Ala Ser
               420                 425                 430
   Phe Thr Asn Arg Lys Glu Arg Gly Pro Glu Glu Glu His Leu Gly Ile
           435                 440                 445
   Leu Gly Pro Val Ile Trp Ala Glu Val Gly Asp Thr Ile Arg Val Thr
       450                 455                 460
   Phe His Asn Lys Gly Ala Tyr Pro Leu Ser Ile Glu Pro Ile Gly Val
   465                 470                 475                 480
   Arg Phe Asn Lys Asn Asn Glu Gly Thr Tyr Tyr Ser Pro Asn Tyr Asn
                   485                 490                 495
   Pro Gln Ser Arg Ser Val Pro Pro Ser Ala Ser His Val Ala Pro Thr
               500                 505                 510
   Glu Thr Phe Thr Tyr Glu Trp Thr Val Pro Lys Glu Val Gly Pro Thr
           515                 520                 525
   Asn Ala Asp Pro Val Cys Leu Ala Lys Met Tyr Tyr Ser Ala Val Asp
       530                 535                 540
   Pro Thr Lys Asp Ile Phe Thr Gly Leu Ile Gly Pro Met Lys Ile Cys
   545                 550                 555                 560
   Lys Lys Gly Ser Leu His Ala Asn Gly Arg Gln Lys Asp Val Asp Lys
                   565                 570                 575
   Glu Phe Tyr Leu Phe Pro Thr Val Phe Asp Glu Asn Glu Ser Leu Leu
               580                 585                 590
   Leu Glu Asp Asn Ile Arg Met Phe Thr Thr Ala Pro Asp Gln Val Asp
           595                 600                 605
   Lys Glu Asp Glu Asp Phe Gln Glu Ser Asn Lys Met His Ser Met Asn
       610                 615                 620
   Gly Phe Met Tyr Gly Asn Gln Pro Gly Leu Thr Met Cys Lys Gly Asp
   625                 630                 635                 640
   Ser Val Val Trp Tyr Leu Phe Ser Ala Gly Asn Glu Ala Asp Val His
                   645                 650                 655
   Gly Ile Tyr Phe Ser Gly Asn Thr Tyr Leu Trp Arg Gly Glu Arg Arg
               660                 665                 670
   Asp Thr Ala Asn Leu Phe Pro Gln Thr Ser Leu Thr Leu His Met Trp
           675                 680                 685
   Pro Asp Thr Glu Gly Thr Phe Asn Val Glu Cys Leu Thr Thr Asp His
       690                 695                 700
   Tyr Thr Gly Gly Met Lys Gln Lys Tyr Thr Val Asn Gln Cys Arg Arg
   705                 710                 715                 720
   Gln Ser Glu Asp Ser Thr Phe Tyr Leu Gly Glu Arg Thr Tyr Tyr Ile
                   725                 730                 735
   Ala Ala Val Glu Val Glu Trp Asp Tyr Ser Pro Gln Arg Glu Trp Glu
               740                 745                 750
   Lys Glu Leu His His Leu Gln Glu Gln Asn Val Ser Asn Ala Phe Leu
           755                 760                 765
   Asp Lys Gly Glu Phe Tyr Ile Gly Ser Lys Tyr Lys Lys Val Val Tyr
       770                 775                 780
   Arg Gln Tyr Thr Asp Ser Thr Phe Arg Val Pro Val Glu Arg Lys Ala
   785                 790                 795                 800
   Glu Glu Glu His Leu Gly Ile Leu Gly Pro Gln Leu His Ala Asp Val
                   805                 810                 815
   Gly Asp Lys Val Lys Ile Ile Phe Lys Asn Met Ala Thr Arg Pro Tyr
               820                 825                 830
   Ser Ile His Ala His Gly Val Gln Thr Glu Ser Ser Thr Val Thr Pro
           835                 840                 845
   Thr Leu Pro Gly Glu Thr Leu Thr Tyr Val Trp Lys Ile Pro Glu Arg
       850                 855                 860
   Ser Gly Ala Gly Thr Glu Asp Ser Ala Cys Ile Pro Trp Ala Tyr Tyr
   865                 870                 875                 880
   Ser Thr Val Asp Gln Val Lys Asp Leu Tyr Ser Gly Leu Ile Gly Pro
                   885                 890                 895
   Leu Ile Val Cys Arg Arg Pro Tyr Leu Lys Val Phe Asn Pro Arg Arg
               900                 905                 910
   Lys Leu Glu Phe Ala Leu Leu Phe Leu Val Phe Asp Glu Asn Glu Ser
           915                 920                 925
   Trp Tyr Leu Asp Asp Asn Ile Lys Thr Tyr Ser Asp His Pro Glu Lys
       930                 935                 940
   Val Asn Lys Asp Asp Glu Glu Phe Ile Glu Ser Asn Lys Met His Ala
   945                 950                 955                 960
   Ile Asn Gly Arg Met Phe Gly Asn Leu Gln Gly Leu Thr Met His Val
                   965                 970                 975
   Gly Asp Glu Val Asn Trp Tyr Leu Met Gly Met Gly Asn Glu Ile Asp
               980                 985                 990
   Leu His Thr Val His Phe His Gly His Ser Phe Gln Tyr Lys His Arg
           995                 1000                1005
   Gly Val Tyr Ser Ser Asp Val Phe Asp Ile Phe Pro Gly Thr Tyr Gln
       1010                1015                1020
   Thr Leu Glu Met Phe Pro Arg Thr Pro Gly Ile Trp Leu Leu His Cys
   1025                1030                1035                1040
   His Val Thr Asp His Ile His Ala Gly Met Glu Thr Thr Tyr Thr Val
                   1045                1050                1055
   Leu Gln Asn Glu Asp Thr Lys Ser Gly
               1060                1065
<210> 48
   <211> 458
   <212> PRT
   <213> Homo sapiens
<400> 48
   Met Ser Asp Leu Leu Ser Val Phe Leu His Leu Leu Leu Leu Phe Lys
    1               5                  10                  15
   Leu Val Ala Pro Val Thr Phe Arg His His Arg Tyr Asp Asp Leu Val
               20                  25                  30
   Arg Thr Leu Tyr Lys Val Gln Asn Glu Cys Pro Gly Ile Thr Arg Val
           35                  40                  45
   Tyr Ser Ile Gly Arg Ser Val Glu Gly Arg His Leu Tyr Val Leu Glu
       50                  55                  60
   Phe Ser Asp His Pro Gly Ile His Glu Pro Leu Glu Pro Glu Val Lys
   65                  70                  75                  80
   Tyr Val Gly Asn Met His Gly Asn Glu Ala Leu Gly Arg Glu Leu Met
                   85                  90                  95
   Leu Gln Leu Ser Glu Phe Leu Cys Glu Glu Phe Arg Asn Arg Asn Gln
               100                 105                 110
   Arg Ile Val Gln Leu Ile Gln Asp Thr Arg Ile His Ile Leu Pro Ser
           115                 120                 125
   Met Asn Pro Asp Gly Tyr Glu Val Ala Ala Ala Gln Gly Pro Asn Lys
       130                 135                 140
   Pro Gly Tyr Leu Val Gly Arg Asn Asn Ala Asn Gly Val Asp Leu Asn
   145                 150                 155                 160
   Arg Asn Phe Pro Asp Leu Asn Thr Tyr Ile Tyr Tyr Asn Glu Lys Tyr
                   165                 170                 175
   Gly Gly Pro Asn His His Leu Pro Leu Pro Asp Asn Trp Lys Ser Gln
               180                 185                 190
   Val Glu Pro Glu Thr Arg Ala Val Ile Arg Trp Met His Ser Phe Asn
           195                 200                 205
   Phe Val Leu Ser Ala Asn Leu His Gly Gly Ala Val Val Ala Asn Tyr
       210                 215                 220
   Pro Tyr Asp Lys Ser Phe Glu His Arg Val Arg Gly Val Arg Arg Thr
   225                 230                 235                 240
   Ala Ser Thr Pro Thr Pro Asp Asp Lys Leu Phe Gln Lys Leu Ala Lys
                   245                 250                 255
   Val Tyr Ser Tyr Ala His Gly Trp Met Phe Gln Gly Trp Asn Cys Gly
               260                 265                 270
   Asp Tyr Phe Pro Asp Gly Ile Thr Asn Gly Ala Ser Trp Tyr Ser Leu
           275                 280                 285
   Ser Lys Gly Met Gln Asp Phe Asn Tyr Leu His Thr Asn Cys Phe Glu
       290                 295                 300
   Ile Thr Leu Glu Leu Ser Cys Asp Lys Phe Pro Pro Glu Glu Glu Leu
   305                 310                 315                 320
   Gln Arg Glu Trp Leu Gly Asn Arg Glu Ala Leu Ile Gln Phe Leu Glu
                   325                 330                 335
   Gln Val His Gln Gly Ile Lys Gly Met Val Leu Asp Glu Asn Tyr Asn
               340                 345                 350
   Asn Leu Ala Asn Ala Val Ile Ser Val Ser Gly Ile Asn His Asp Val
           355                 360                 365
   Thr Ser Gly Asp His Gly Asp Tyr Phe Arg Leu Leu Leu Pro Gly Ile
       370                 375                 380
   Tyr Thr Val Ser Ala Thr Ala Pro Gly Tyr Asp Pro Glu Thr Val Thr
   385                 390                 395                 400
   Val Thr Val Gly Pro Ala Glu Pro Thr Leu Val Asn Phe His Leu Lys
                   405                 410                 415
   Arg Ser Ile Pro Gln Val Ser Pro Val Arg Arg Ala Pro Ser Arg Arg
               420                 425                 430
   His Gly Val Arg Ala Lys Val Gln Pro Gln Ala Arg Lys Lys Glu Met
             435                 440                 445
   Glu Met Arg Gln Leu Gln Arg Gly Pro Ala
       450                 455
<210> 49
   <211> 545
   <212> PRT
   <213> Homo sapiens
<400> 49
   Met Leu Pro Gly Ala Trp Leu Leu Trp Thr Ser Leu Leu Leu Leu Ala
   1               5                   10                  15
   Arg Pro Ala Gln Pro Cys Pro Met Gly Cys Asp Cys Phe Val Gln Glu
               20                  25                  30
   Val Phe Cys Ser Asp Glu Glu Leu Ala Thr Val Pro Leu Asp Ile Pro
           35                  40                  45
   Pro Tyr Thr Lys Asn Ile Ile Phe Val Glu Thr Ser Phe Thr Thr Leu
       50                  55                  60
   Glu Thr Arg Ala Phe Gly Ser Asn Pro Asn Leu Thr Lys Val Val Phe
   65                  70                  75                  80
   Leu Asn Thr Gln Leu Cys Gln Phe Arg Pro Asp Ala Phe Gly Gly Leu
                   85                  90                  95
   Pro Arg Leu Glu Asp Leu Glu Val Thr Gly Ser Ser Phe Leu Asn Leu
               100                 105                 110
   Ser Thr Asn Ile Phe Ser Asn Leu Thr Ser Leu Gly Lys Leu Thr Leu
           115                 120                 125
   Asn Phe Asn Met Leu Glu Ala Leu Pro Glu Gly Leu Phe Gln His Leu
       130                 135                 140
   Ala Ala Leu Glu Ser Leu His Leu Gln Gly Asn Gln Leu Gln Ala Leu
   145                 150                 155                 160
   Pro Arg Arg Leu Phe Gln Pro Leu Thr His Leu Lys Thr Leu Asn Leu
                   165                 170                 175
   Ala Gln Asn Leu Leu Ala Gln Leu Pro Glu Glu Leu Phe His Pro Leu
               180                 185                 190
   Thr Ser Leu Gln Thr Leu Lys Leu Ser Asn Asn Ala Leu Ser Gly Leu
           195                 200                 205
   Pro Gln Gly Val Phe Gly Lys Leu Gly Ser Leu Gln Glu Leu Phe Leu
       210                 215                 220
   Asp Ser Asn Asn Ile Ser Glu Leu Pro Pro Gln Val Phe Ser Gln Leu
   225                 230                 235                 240
   Phe Cys Leu Glu Arg Leu Trp Leu Gln Arg Asn Ala Ile Thr His Leu
                   245                 250                 255
   Pro Leu Ser Ile Phe Ala Ser Leu Gly Asn Leu Thr Phe Leu Ser Leu
               260                 265                 270
   Gln Trp Asn Met Leu Arg Val Leu Pro Ala Gly Leu Phe Ala His Thr
           275                 280                 285
   Pro Cys Leu Val Gly Leu Ser Leu Thr His Asn Gln Leu Glu Thr Val
       290                 295                 300
   Ala Glu Gly Thr Phe Ala His Leu Ser Asn Leu Arg Ser Leu Met Leu
   305                 310                 315                 320
   Ser Tyr Asn Ala Ile Thr His Leu Pro Ala Gly Ile Phe Arg Asp Leu
                   325                 330                 335
   Glu Glu Leu Val Lys Leu Tyr Leu Gly Ser Asn Asn Leu Thr Ala Leu
               340                 345                 350
   His Pro Ala Leu Phe Gln Asn Leu Ser Lys Leu Glu Leu Leu Ser Leu
           355                 360                 365
   Ser Lys Asn Gln Leu Thr Thr Leu Pro Glu Gly Ile Phe Asp Thr Asn
       370                 375                 380
   Tyr Asn Leu Phe Asn Leu Ala Leu His Gly Asn Pro Trp Gln Cys Asp
   385                 390                 395                 400
   Cys His Leu Ala Tyr Leu Phe Asn Trp Leu Gln Gln Tyr Thr Asp Arg
                   405                 410                 415
   Leu Leu Asn Ile Gln Thr Tyr Cys Ala Gly Pro Ala Tyr Leu Lys Gly
                   420                 425                 430
   Gln Val Val Pro Ala Leu Asn Glu Lys Gln Leu Val Cys Pro Val Thr
               435                 440                 445
   Arg Asp His Leu Gly Phe Gln Val Thr Trp Pro Asp Glu Ser Lys Ala
       450                 455                 460
   Gly Gly Ser Trp Asp Leu Ala Val Gln Glu Arg Ala Ala Arg Ser Gln
   465                 470                 475                 480
   Cys Thr Tyr Ser Asn Pro Glu Gly Thr Val Val Leu Ala Cys Asp Gln
                       485                 490                 495
   Ala Gln Cys Arg Trp Leu Asn Val Gln Leu Ser Pro Gln Gln Gly Ser
                   500                 505                 510
   Leu Gly Leu Gln Tyr Asn Ala Ser Gln Glu Trp Asp Leu Arg Ser Ser
               515                 520                 525
   Cys Gly Ser Leu Arg Leu Thr Val Ser Ile Glu Ala Arg Ala Ala Gly
       530                 535                 540
   Pro
   545
<210> 50
   <211> 622
   <212> PRT
   <213> Homo sapiens
<400> 50
<210> 51
   <211> 721
   <212> PRT
   <213> Homo sapiens
<400> 51
   Met Glu Arg Ala Ala Pro Ser Arg Arg Val Pro Leu Pro Leu Leu Leu
    1               5                  10                  15
   Leu Gly Gly Leu Ala Leu Leu Ala Ala Gly Val Asp Ala Asp Val Leu
               20                  25                  30
   Leu Glu Ala Cys Cys Ala Asp Gly His Arg Met Ala Thr His Gln Lys
           35                  40                  45
   Asp Cys Ser Leu Pro Tyr Ala Thr Glu Ser Lys Glu Cys Arg Ala Val
       50                  55                  60
   Gly Leu Ala Ser Leu Cys Gln Asp Leu Asn Gly Ala Trp Ala Val Trp
   65                  70                  75                  80
   Lys Val Gly Arg Ala Ser Gln Ala Glu Gly Thr Ala Ser Ala Arg Ala
                   85                  90                  95
   Gln Arg Arg Gly Met Val Gln Glu Gln Cys Cys His Ser Gln Leu Glu
               100                 105                 110
   Glu Leu His Cys Ala Thr Gly Ile Ser Leu Ala Asn Glu Gln Asp Arg
               115                 120                 125
   Cys Ala Thr Pro His Gly Asp Asn Ala Ser Leu Glu Ala Thr Phe Val
       130                 135                 140
   Lys Arg Cys Cys His Cys Cys Leu Leu Gly Arg Ala Ala Gln Ala Gln
   145                 150                 155                 160
   Gly Gln Ser Cys Glu Tyr Ser Leu Met Val Gly Tyr Gln Cys Gly Gln
                   165                 170                 175
   Val Phe Gln Ala Cys Cys Val Lys Ser Gln Glu Thr Gly Asp Leu Asp
               180                 185                 190
   Val Gly Gly Leu Gln Glu Thr Asp Lys Ile Ile Glu Val Glu Glu Glu
           195                 200                 205
   Gln Glu Asp Pro Tyr Leu Asn Asp Arg Cys Arg Gly Gly Gly Pro Cys
       210                 215                 220
   Lys Gln Gln Cys Arg Asp Thr Gly Asp Glu Val Val Cys Ser Cys Phe
   225                 230                 235                 240
   Val Gly Tyr Gln Leu Leu Ser Asp Gly Val Ser Cys Glu Asp Val Asn
                   245                 250                 255
   Glu Cys Ile Thr Gly Ser His Ser Cys Arg Leu Gly Glu Ser Cys Ile
               260                 265                 270
   Asn Thr Val Gly Ser Phe Arg Cys Gln Arg Asp Ser Ser Cys Gly Thr
           275                 280                 285
   Gly Tyr Glu Leu Thr Glu Asp Asn Ser Cys Lys Asp Ile Asp Glu Cys
       290                 295                 300
   Glu Ser Gly Ile His Asn Cys Leu Pro Asp Phe Ile Cys Gln Asn Thr
   305                 310                 315                 320
   Leu Gly Ser Phe Arg Cys Arg Pro Lys Leu Gln Cys Lys Ser Gly Phe
                   325                 330                 335
   Ile Gln Asp Ala Leu Gly Asn Cys Ile Asp Ile Asn Glu Cys Leu Ser
               340                 345                 350
   Ile Ser Ala Pro Cys Pro Ile Gly His Thr Cys Ile Asn Thr Glu Gly
           355                 360                 365
   Ser Tyr Thr Cys Gln Lys Asn Val Pro Asn Cys Gly Arg Gly Tyr His
       370                 375                 380
   Leu Asn Glu Glu Gly Thr Arg Cys Val Asp Val Asp Glu Cys Ala Pro
   385                 390                 395                 400
   Pro Ala Glu Pro Cys Gly Lys Gly His Arg Cys Val Asn Ser Pro Gly
                   405                 410                 415
   Ser Phe Arg Cys Glu Cys Lys Thr Gly Tyr Tyr Phe Asp Gly Ile Ser
               420                 425                 430
   Arg Met Cys Val Asp Val Asn Glu Cys Gln Arg Tyr Pro Gly Arg Leu
           435                 440                 445
   Cys Gly His Lys Cys Glu Asn Thr Leu Gly Ser Tyr Leu Cys Ser Cys
       450                 455                 460
   Ser Val Gly Phe Arg Leu Ser Val Asp Gly Arg Ser Cys Glu Asp Ile
   465                 470                 475                 480
   Asn Glu Cys Ser Ser Ser Pro Cys Ser Gln Glu Cys Ala Asn Val Tyr
                   485                 490                 495
   Gly Ser Tyr Gln Cys Tyr Cys Arg Arg Gly Tyr Gln Leu Ser Asp Val
               500                 505                 510
   Asp Gly Val Thr Cys Glu Asp Ile Asp Glu Cys Ala Leu Pro Thr Gly
           515                 520                 525
   Gly His Ile Cys Ser Tyr Arg Cys Ile Asn Ile Pro Gly Ser Phe Gln
       530                 535                 540
   Cys Ser Cys Pro Ser Ser Gly Tyr Arg Leu Ala Pro Asn Gly Arg Asn
   545                 550                 555                 560
   Cys Gln Asp Ile Asp Glu Cys Val Thr Gly Ile His Asn Cys Ser Ile
                   565                 570                 575
   Asn Glu Thr Cys Phe Asn Ile Gln Gly Gly Phe Arg Cys Leu Ala Phe
               580                 585                 590
   Glu Cys Pro Glu Asn Tyr Arg Arg Ser Ala Ala Thr Arg Cys Glu Arg
           595                 600                 605
   Leu Pro Cys His Glu Asn Arg Glu Cys Ser Lys Leu Pro Leu Arg Ile
       610                 615                 620
   Thr Tyr Tyr His Leu Ser Phe Pro Thr Asn Ile Gln Ala Pro Ala Val
   625                 630                 635                 640
   Val Phe Arg Met Gly Pro Ser Ser Ala Val Pro Gly Asp Ser Met Gln
                   645                 650                 655
   Leu Ala Ile Thr Gly Gly Asn Glu Glu Gly Phe Phe Thr Thr Arg Lys
               660                 665                 670
   Val Ser Pro His Ser Gly Val Val Ala Leu Thr Lys Pro Val Pro Glu
           675                 680                 685
   Pro Arg Asp Leu Leu Leu Thr Val Lys Met Asp Leu Ser Arg His Gly
       690                 695                 700
   Thr Val Ser Ser Phe Val Ala Lys Leu Phe Ile Phe Val Ser Ala Glu
   705                 710                 715                 720
   Leu
<210> 52
   <211> 2386
   <212> PRT
   <213> Homo sapiens
<400> 52
   Met Leu Arg Gly Pro Gly Pro Gly Leu Leu Leu Leu Ala Val Gln Cys
    1               5                  10                  15
   Leu Gly Thr Ala Val Pro Ser Thr Gly Ala Ser Lys Ser Lys Arg Gln
               20                  25                  30
   Ala Gln Gln Met Val Gln Pro Gln Ser Pro Val Ala Val Ser Gln Ser
           35                  40                  45
   Lys Pro Gly Cys Tyr Asp Asn Gly Lys His Tyr Gln Ile Asn Gln Gln
       50                  55                  60
   Trp Glu Arg Thr Tyr Leu Gly Asn Ala Leu Val Cys Thr Cys Tyr Gly
   65                  70                  75                  80
   Gly Ser Arg Gly Phe Asn Cys Glu Ser Lys Pro Glu Ala Glu Glu Thr
                   85                  90                  95
   Cys Phe Asp Lys Tyr Thr Gly Asn Thr Tyr Arg Val Gly Asp Thr Tyr
               100                 105                 110
   Glu Arg Pro Lys Asp Ser Met Ile Trp Asp Cys Thr Cys Ile Gly Ala
           115                 120                 125
   Gly Arg Gly Arg Ile Ser Cys Thr Ile Ala Asn Arg Cys His Glu Gly
       130                 135                 140
   Gly Gln Ser Tyr Lys Ile Gly Asp Thr Trp Arg Arg Pro His Glu Thr
   145                 150                 155                 160
   Gly Gly Tyr Met Leu Glu Cys Val Cys Leu Gly Asn Gly Lys Gly Glu
                   165                 170                 175
   Trp Thr Cys Lys Pro Ile Ala Glu Lys Cys Phe Asp His Ala Ala Gly
               180                 185                 190
   Thr Ser Tyr Val Val Gly Glu Thr Trp Glu Lys Pro Tyr Gln Gly Trp
           195                 200                 205
   Met Met Val Asp Cys Thr Cys Leu Gly Glu Gly Ser Gly Arg Ile Thr
       210                 215                 220
   Cys Thr Ser Arg Asn Arg Cys Asn Asp Gln Asp Thr Arg Thr Ser Tyr
   225                 230                 235                 240
   Arg Ile Gly Asp Thr Trp Ser Lys Lys Asp Asn Arg Gly Asn Leu Leu
                   245                 250                 255
   Gln Cys Ile Cys Thr Gly Asn Gly Arg Gly Glu Trp Lys Cys Glu Arg
               260                 265                 270
   His Thr Ser Val Gln Thr Thr Ser Ser Gly Ser Gly Pro Phe Thr Asp
           275                 280                 285
   Val Arg Ala Ala Val Tyr Gln Pro Gln Pro His Pro Gln Pro Pro Pro
       290                 295                 300
   Tyr Gly His Cys Val Thr Asp Ser Gly Val Val Tyr Ser Val Gly Met
   305                 310                 315                 320
   Gln Trp Leu Lys Thr Gln Gly Asn Lys Gln Met Leu Cys Thr Cys Leu
                   325                 330                 335
   Gly Asn Gly Val Ser Cys Gln Glu Thr Ala Val Thr Gln Thr Tyr Gly
               340                 345                 350
   Gly Asn Ser Asn Gly Glu Pro Cys Val Leu Pro Phe Thr Tyr Asn Gly
           355                 360                 365
   Arg Thr Phe Tyr Ser Cys Thr Thr Glu Gly Arg Gln Asp Gly His Leu
       370                 375                 380
   Trp Cys Ser Thr Thr Ser Asn Tyr Glu Gln Asp Gln Lys Tyr Ser Phe
   385                 390                 395                 400
   Cys Thr Asp His Thr Val Leu Val Gln Thr Arg Gly Gly Asn Ser Asn
                   405                 410                 415
   Gly Ala Leu Cys His Phe Pro Phe Leu Tyr Asn Asn His Asn Tyr Thr
               420                 425                 430
   Asp Cys Thr Ser Glu Gly Arg Arg Asp Asn Met Lys Trp Cys Gly Thr
           435                 440                 445
   Thr Gln Asn Tyr Asp Ala Asp Gln Lys Phe Gly Phe Cys Pro Met Ala
       450                 455                 460
   Ala His Glu Glu Ile Cys Thr Thr Asn Glu Gly Val Met Tyr Arg Ile
   465                 470                 475                 480
   Gly Asp Gln Trp Asp Lys Gln His Asp Met Gly His Met Met Arg Cys
                   485                 490                 495
   Thr Cys Val Gly Asn Gly Arg Gly Glu Trp Thr Cys Ile Ala Tyr Ser
               500                 505                 510
   Gln Leu Arg Asp Gln Cys Ile Val Asp Asp Ile Thr Tyr Asn Val Asn
           515                 520                 525
   Asp Thr Phe His Lys Arg His Glu Glu Gly His Met Leu Asn Cys Thr
       530                 535                 540
   Cys Phe Gly Gln Gly Arg Gly Arg Trp Lys Cys Asp Pro Val Asp Gln
   545                 550                 555                 560
   Cys Gln Asp Ser Glu Thr Gly Thr Phe Tyr Gln Ile Gly Asp Ser Trp
                   565                 570                 575
   Glu Lys Tyr Val His Gly Val Arg Tyr Gln Cys Tyr Cys Tyr Gly Arg
               580                 585                 590
   Gly Ile Gly Glu Trp His Cys Gln Pro Leu Gln Thr Tyr Pro Ser Ser
           595                 600                 605
   Ser Gly Pro Val Glu Val Phe Ile Thr Glu Thr Pro Ser Gln Pro Asn
       610                 615                 620
   Ser His Pro Ile Gln Trp Asn Ala Pro Gln Pro Ser His Ile Ser Lys
   625                 630                 635                 640
   Tyr Ile Leu Arg Trp Arg Pro Lys Asn Ser Val Gly Arg Trp Lys Glu
                   645                 650                 655
   Ala Thr Ile Pro Gly His Leu Asn Ser Tyr Thr Ile Lys Gly Leu Lys
               660                 665                 670
   Pro Gly Val Val Tyr Glu Gly Gln Leu Ile Ser Ile Gln Gln Tyr Gly
           675                 680                 685
   His Gln Glu Val Thr Arg Phe Asp Phe Thr Thr Thr Ser Thr Ser Thr
       690                 695                 700
   Pro Val Thr Ser Asn Thr Val Thr Gly Glu Thr Thr Pro Phe Ser Pro
   705                 710                 715                 720
   Leu Val Ala Thr Ser Glu Ser Val Thr Glu Ile Thr Ala Ser Ser Phe
                   725                 730                 735
   Val Val Ser Trp Val Ser Ala Ser Asp Thr Val Ser Gly Phe Arg Val
               740                 745                 750
   Glu Tyr Glu Leu Ser Glu Glu Gly Asp Glu Pro Gln Tyr Leu Asp Leu
           755                 760                 765
   Pro Ser Thr Ala Thr Ser Val Asn Ile Pro Asp Leu Leu Pro Gly Arg
       770                 775                 780
   Lys Tyr Ile Val Asn Val Tyr Gln Ile Ser Glu Asp Gly Glu Gln Ser
   785                 790                 795                 800
   Leu Ile Leu Ser Thr Ser Gln Thr Thr Ala Pro Asp Ala Pro Pro Asp
                   805                 810                 815
   Thr Thr Val Asp Gln Val Asp Asp Thr Ser Ile Val Val Arg Trp Ser
               820                 825                 830
   Arg Pro Gln Ala Pro Ile Thr Gly Tyr Arg Ile Val Tyr Ser Pro Ser
           835                 840                 845
   Val Glu Gly Ser Ser Thr Glu Leu Asn Leu Pro Glu Thr Ala Asn Ser
       850                 855                 860
   Val Thr Leu Ser Asp Leu Gln Pro Gly Val Gln Tyr Asn Ile Thr Ile
   865                 870                 875                 880
   Tyr Ala Val Glu Glu Asn Gln Glu Ser Thr Pro Val Val Ile Gln Gln
                   885                 890                 895
   Glu Thr Thr Gly Thr Pro Arg Ser Asp Thr Val Pro Ser Pro Arg Asp
               900                 905                 910
   Leu Gln Phe Val Glu Val Thr Asp Val Lys Val Thr Ile Met Trp Thr
           915                 920                 925
   Pro Pro Glu Ser Ala Val Thr Gly Tyr Arg Val Asp Val Ile Pro Val
       930                 935                 940
   Asn Leu Pro Gly Glu His Gly Gln Arg Leu Pro Ile Ser Arg Asn Thr
   945                 950                 955                 960
   Phe Ala Glu Val Thr Gly Leu Ser Pro Gly Val Thr Tyr Tyr Phe Lys
                   965                 970                 975
   Val Phe Ala Val Ser His Gly Arg Glu Ser Lys Pro Leu Thr Ala Gln
               980                 985                 990
   Gln Thr Thr Lys Leu Asp Ala Pro Thr Asn Leu Gln Phe Val Asn Glu
           995                 1000                1005
   Thr Asp Ser Thr Val Leu Val Arg Trp Thr Pro Pro Arg Ala Gln Ile
       1010                1015                1020
   Thr Gly Tyr Arg Leu Thr Val Gly Leu Thr Arg Arg Gly Gln Pro Arg
   1025                1030                1035                1040
   Gln Tyr Asn Val Gly Pro Ser Val Ser Lys Tyr Pro Leu Arg Asn Leu
                   1045                1050                1055
   Gln Pro Ala Ser Glu Tyr Thr Val Ser Leu Val Ala Ile Lys Gly Asn
               1060                1065                1070
   Gln Glu Ser Pro Lys Ala Thr Gly Val Phe Thr Thr Leu Gln Pro Gly
           1075                1080                1085
   Ser Ser Ile Pro Pro Tyr Asn Thr Glu Val Thr Glu Thr Thr Ile Val
       1090                1095                1100
   Ile Thr Trp Thr Pro Ala Pro Arg Ile Gly Phe Lys Leu Gly Val Arg
   1105                1110                1115                1120
   Pro Ser Gln Gly Gly Glu Ala Pro Arg Glu Val Thr Ser Asp Ser Gly
                   1125                1130                1135
   Ser Ile Val Val Ser Gly Leu Thr Pro Gly Val Glu Tyr Val Tyr Thr
               1140                1145                1150
   Ile Gln Val Leu Arg Asp Gly Gln Glu Arg Asp Ala Pro Ile Val Asn
           1155                1160                1165
   Lys Val Val Thr Pro Leu Ser Pro Pro Thr Asn Leu His Leu Glu Ala
       1170                1175                1180
   Asn Pro Asp Thr Gly Val Leu Thr Val Ser Trp Glu Arg Ser Thr Thr
   1185                1190                1195                1200
   Pro Asp Ile Thr Gly Tyr Arg Ile Thr Thr Thr Pro Thr Asn Gly Gln
                   1205                1210                1215
   Gln Gly Asn Ser Leu Glu Glu Val Val His Ala Asp Gln Ser Ser Cys
               1220                1225                1230
   Thr Phe Asp Asn Leu Ser Pro Gly Leu Glu Tyr Asn Val Ser Val Tyr
           1235                1240                1245
   Thr Val Lys Asp Asp Lys Glu Ser Val Pro Ile Ser Asp Thr Ile Ile
       1250                1255                1260
   Pro Ala Val Pro Pro Pro Thr Asp Leu Arg Phe Thr Asn Ile Gly Pro
   1265                1270                1275                1280
   Asp Thr Met Arg Val Thr Trp Ala Pro Pro Pro Ser Ile Asp Leu Thr
                   1285                1290                1295
   Asn Phe Leu Val Arg Tyr Ser Pro Val Lys Asn Glu Glu Asp Val Ala
               1300                1305                1310
   Glu Leu Ser Ile Ser Pro Ser Asp Asn Ala Val Val Leu Thr Asn Leu
           1315                1320                1325
   Leu Pro Gly Thr Glu Tyr Val Val Ser Val Ser Ser Val Tyr Glu Gln
       1330                1335                1340
   His Glu Ser Thr Pro Leu Arg Gly Arg Gln Lys Thr Gly Leu Asp Ser
   1345                1350                1355                1360
   Pro Thr Gly Ile Asp Phe Ser Asp Ile Thr Ala Asn Ser Phe Thr Val
                   1365                1370                1375
   His Trp Ile Ala Pro Arg Ala Thr Ile Thr Gly Tyr Arg Ile Arg His
               1380                1385                1390
   His Pro Glu His Phe Ser Gly Arg Pro Arg Glu Asp Arg Val Pro His
           1395                1400                1405
   Ser Arg Asn Ser Ile Thr Leu Thr Asn Leu Thr Pro Gly Thr Glu Tyr
       1410                1415                1420
   Val Val Ser Ile Val Ala Leu Asn Gly Arg Glu Glu Ser Pro Leu Leu
   1425                1430                1435                1440
   Ile Gly Gln Gln Ser Thr Val Ser Asp Val Pro Arg Asp Leu Glu Val
                   1445                1450                1455
   Val Ala Ala Thr Pro Thr Ser Leu Leu Ile Ser Trp Asp Ala Pro Ala
               1460                1465                1470
   Val Thr Val Arg Tyr Tyr Arg Ile Thr Tyr Gly Glu Thr Gly Gly Asn
           1475                1480                1485
   Ser Pro Val Gln Glu Phe Thr Val Pro Gly Ser Lys Ser Thr Ala Thr
       1490                1495                1500
   Ile Ser Gly Leu Lys Pro Gly Val Asp Tyr Thr Ile Thr Val Tyr Ala
   1505                1510                1515                1520
   Val Thr Gly Arg Gly Asp Ser Pro Ala Ser Ser Lys Pro Ile Ser Ile
                   1525                1530                1535
   Asn Tyr Arg Thr Glu Ile Asp Lys Pro Ser Gln Met Gln Val Thr Asp
               1540                1545                1550
   Val Gln Asp Asn Ser Ile Ser Val Lys Trp Leu Pro Ser Ser Ser Pro
           1555                1560                1565
   Val Thr Gly Tyr Arg Val Thr Thr Thr Pro Lys Asn Gly Pro Gly Pro
       1570                1575                1580
   Thr Lys Thr Lys Thr Ala Gly Pro Asp Gln Thr Glu Met Thr Ile Glu
   1585                1590                1595                1600
   Gly Leu Gln Pro Thr Val Glu Tyr Val Val Ser Val Tyr Ala Gln Asn
                   1605                1610                1615
   Pro Ser Gly Glu Ser Gln Pro Leu Val Gln Thr Ala Val Thr Asn Ile
               1620                1625                1630
   Asp Arg Pro Lys Gly Leu Ala Phe Thr Asp Val Asp Val Asp Ser Ile
           1635                1640                1645
   Lys Ile Ala Trp Glu Ser Pro Gln Gly Gln Val Ser Arg Tyr Arg Val
       1650                1655                1660
   Thr Tyr Ser Ser Pro Glu Asp Gly Ile His Glu Leu Phe Pro Ala Pro
   1665                1670                1675                1680
   Asp Gly Glu Glu Asp Thr Ala Glu Leu Gln Gly Leu Arg Pro Gly Ser
                   1685                1690                1695
   Glu Tyr Thr Val Ser Val Val Ala Leu His Asp Asp Met Glu Ser Gln
               1700                1705                1710
   Pro Leu Ile Gly Thr Gln Ser Thr Ala Ile Pro Ala Pro Thr Asp Leu
           1715                1720                1725
   Lys Phe Thr Gln Val Thr Pro Thr Ser Leu Ser Ala Gln Trp Thr Pro
       1730                1735                1740
   Pro Asn Val Gln Leu Thr Gly Tyr Arg Val Arg Val Thr Pro Lys Glu
   1745                1750                1755                1760
   Lys Thr Gly Pro Met Lys Glu Ile Asn Leu Ala Pro Asp Ser Ser Ser
                   1765                1770                1775
   Val Val Val Ser Gly Leu Met Val Ala Thr Lys Tyr Glu Val Ser Val
               1780                1785                1790
   Tyr Ala Leu Lys Asp Thr Leu Thr Ser Arg Pro Ala Gln Gly Val Val
           1795                1800                1805
   Thr Thr Leu Glu Asn Val Ser Pro Pro Arg Arg Ala Arg Val Thr Asp
       1810                1815                1820
   Ala Thr Glu Thr Thr Ile Thr Ile Ser Trp Arg Thr Lys Thr Glu Thr
   1825                1830                1835                1840
   Ile Thr Gly Phe Gln Val Asp Ala Val Pro Ala Asn Gly Gln Thr Pro
                   1845                1850                1855
   Ile Gln Arg Thr Ile Lys Pro Asp Val Arg Ser Tyr Thr Ile Thr Gly
               1860                1865                1870
   Leu Gln Pro Gly Thr Asp Tyr Lys Ile Tyr Leu Tyr Thr Leu Asn Asp
           1875                1880                1885
   Asn Ala Arg Ser Ser Pro Val Val Ile Asp Ala Ser Thr Ala Ile Asp
       1890                1895                1900
   Ala Pro Ser Asn Leu Arg Phe Leu Ala Thr Thr Pro Asn Ser Leu Leu
   1905                1910                1915                1920
   Val Ser Trp Gln Pro Pro Arg Ala Arg Ile Thr Gly Tyr Ile Ile Lys
                   1925                1930                1935
   Tyr Glu Lys Pro Gly Ser Pro Pro Arg Glu Val Val Pro Arg Pro Arg
               1940                1945                1950
   Pro Gly Val Thr Glu Ala Thr Ile Thr Gly Leu Glu Pro Gly Thr Glu
           1955                1960                1965
   Tyr Thr Ile Tyr Val Ile Ala Leu Lys Asn Asn Gln Lys Ser Glu Pro
       1970                1975                1980
   Leu Ile Gly Arg Lys Lys Thr Asp Glu Leu Pro Gln Leu Val Thr Leu
   1985                1990                1995                2000
   Pro His Pro Asn Leu His Gly Pro Glu Ile Leu Asp Val Pro Ser Thr
                   2005                2010                2015
   Val Gln Lys Thr Pro Phe Val Thr His Pro Gly Tyr Asp Thr Gly Asn
               2020                2025                2030
   Gly Ile Gln Leu Pro Gly Thr Ser Gly Gln Gln Pro Ser Val Gly Gln
           2035                2040                2045
   Gln Met Ile Phe Glu Glu His Gly Phe Arg Arg Thr Thr Pro Pro Thr
       2050                2055                2060
   Thr Ala Thr Pro Ile Arg His Arg Pro Arg Pro Tyr Pro Pro Asn Val
   2065                2070                2075                2080
   Gly Glu Glu Ile Gln Ile Gly His Ile Pro Arg Glu Asp Val Asp Tyr
                   2085                2090                2095
   His Leu Tyr Pro His Gly Pro Gly Leu Asn Pro Asn Ala Ser Thr Gly
               2100                2105                2110
   Gln Glu Ala Leu Ser Gln Thr Thr Ile Ser Trp Ala Pro Phe Gln Asp
           2115                2120                2125
   Thr Ser Glu Tyr Ile Ile Ser Cys His Pro Val Gly Thr Asp Glu Glu
       2130                2135                2140
   Pro Leu Gln Phe Arg Val Pro Gly Thr Ser Thr Ser Ala Thr Leu Thr
   2145                2150                2155                2160
   Gly Leu Thr Arg Gly Ala Thr Tyr Asn Val Ile Val Glu Ala Leu Lys
                   2165                2170                2175
   Asp Gln Gln Arg His Lys Val Arg Glu Glu Val Val Thr Val Gly Asn
               2180                2185                2190
   Ser Val Asn Glu Gly Leu Asn Gln Pro Thr Asp Asp Ser Cys Phe Asp
           2195                2200                2205
   Pro Tyr Thr Val Ser His Tyr Ala Val Gly Asp Glu Trp Glu Arg Met
       2210                2215                2220
   Ser Glu Ser Gly Phe Lys Leu Leu Cys Gln Cys Leu Gly Phe Gly Ser
   2225                2230                2235                2240
   Gly His Phe Arg Cys Asp Ser Ser Arg Trp Cys His Asp Asn Gly Val
                   2245                2250                2255
   Asn Tyr Lys Ile Gly Glu Lys Trp Asp Arg Gln Gly Glu Asn Gly Gln
               2260                2265                2270
   Met Met Ser Cys Thr Cys Leu Gly Asn Gly Lys Gly Glu Phe Lys Cys
           2275                2280                2285
   Asp Pro His Glu Ala Thr Cys Tyr Asp Asp Gly Lys Thr Tyr His Val
       2290                2295                2300
   Gly Glu Gln Trp Gln Lys Glu Tyr Leu Gly Ala Ile Cys Ser Cys Thr
   2305                2310                2315                2320
   Cys Phe Gly Gly Gln Arg Gly Trp Arg Cys Asp Asn Cys Arg Arg Pro
                   2325                2330                2335
   Gly Gly Glu Pro Ser Pro Glu Gly Thr Thr Gly Gln Ser Tyr Asn Gln
               2340                2345                2350
   Tyr Ser Gln Arg Tyr His Gln Arg Thr Asn Thr Asn Val Asn Cys Pro
           2355                2360                2365
   Ile Glu Cys Phe Met Pro Leu Asp Val Gln Ala Asp Arg Glu Asp Ser
       2370                2375                2380
   Arg Glu
   2385
<210> 53
   <211> 406
   <212> PRT
   <213> Homo sapiens
<400> 53
   Met Ser Ala Leu Gly Ala Val Ile Ala Leu Leu Leu Trp Gly Gln Leu
    1               5                  10                  15
   Phe Ala Val Asp Ser Gly Asn Asp Val Thr Asp Ile Ala Asp Asp Gly
               20                  25                  30
   Cys Pro Lys Pro Pro Glu Ile Ala His Gly Tyr Val Glu His Ser Val
           35                  40                  45
   Arg Tyr Gln Cys Lys Asn Tyr Tyr Lys Leu Arg Thr Glu Gly Asp Gly
       50                  55                  60
   Val Tyr Thr Leu Asn Asp Lys Lys Gln Trp Ile Asn Lys Ala Val Gly
   65                  70                  75                  80
   Asp Lys Leu Pro Glu Cys Glu Ala Asp Asp Gly Cys Pro Lys Pro Pro
                   85                  90                  95
   Glu Ile Ala His Gly Tyr Val Glu His Ser Val Arg Tyr Gln Cys Lys
               100                 105                 110
   Asn Tyr Tyr Lys Leu Arg Thr Glu Gly Asp Gly Val Tyr Thr Leu Asn
           115                 120                 125
   Asn Glu Lys Gln Trp Ile Asn Lys Ala Val Gly Asp Lys Leu Pro Glu
       130                 135                 140
   Cys Glu Ala Val Cys Gly Lys Pro Lys Asn Pro Ala Asn Pro Val Gln
   145                 150                 155                 160
   Arg Ile Leu Gly Gly His Leu Asp Ala Lys Gly Ser Phe Pro Trp Gln
                   165                 170                 175
   Ala Lys Met Val Ser His His Asn Leu Thr Thr Gly Ala Thr Leu Ile
               180                 185                 190
   Asn Glu Gln Trp Leu Leu Thr Thr Ala Lys Asn Leu Phe Leu Asn His
           195                 200                 205
   Ser Glu Asn Ala Thr Ala Lys Asp Ile Ala Pro Thr Leu Thr Leu Tyr
       210                 215                 220
   Val Gly Lys Lys Gln Leu Val Glu Ile Glu Lys Val Val Leu His Pro
   225                 230                 235                 240
   Asn Tyr Ser Gln Val Asp Ile Gly Leu Ile Lys Leu Lys Gln Lys Val
                   245                 250                 255
   Ser Val Asn Glu Arg Val Met Pro Ile Cys Leu Pro Ser Lys Asp Tyr
               260                 265                 270
   Ala Glu Val Gly Arg Val Gly Tyr Val Ser Gly Trp Gly Arg Asn Ala
           275                 280                 285
   Asn Phe Lys Phe Thr Asp His Leu Lys Tyr Val Met Leu Pro Val Ala
       290                 295                 300
   Asp Gln Asp Gln Cys Ile Arg His Tyr Glu Gly Ser Thr Val Pro Glu
   305                 310                 315                 320
   Lys Lys Thr Pro Lys Ser Pro Val Gly Val Gln Pro Ile Leu Asn Glu
                   325                 330                 335
   His Thr Phe Cys Ala Gly Met Ser Lys Tyr Gln Glu Asp Thr Cys Tyr
               340                 345                 350
   Gly Asp Ala Gly Ser Ala Phe Ala Val His Asp Leu Glu Glu Asp Thr
           355                 360                 365
   Trp Tyr Ala Thr Gly Ile Leu Ser Phe Asp Lys Ser Cys Ala Val Ala
       370                 375                 380
   Glu Tyr Gly Val Tyr Val Lys Val Thr Ser Ile Gln Asp Trp Val Gln
   385                 390                 395                 400
   Lys Thr Ile Ala Glu Asn
                   405
<210> 54
   <211> 348
   <212> PRT
   <213> Homo sapiens
<400> 54
   Met Ser Asp Leu Gly Ala Val Ile Ser Leu Leu Leu Trp Gly Arg Gln
    1               5                  10                  15
   Leu Phe Ala Leu Tyr Ser Gly Asn Asp Val Thr Asp Ile Ser Asp Asp
               20                  25                  30
   Arg Phe Pro Lys Pro Pro Glu Ile Ala Asn Gly Tyr Val Glu His Leu
           35                  40                  45
   Phe Arg Tyr Gln Cys Lys Asn Tyr Tyr Arg Leu Arg Thr Glu Gly Asp
       50                  55                  60
   Gly Val Tyr Thr Leu Asn Asp Lys Lys Gln Trp Ile Asn Lys Ala Val
   65                  70                  75                  80
   Gly Asp Lys Leu Pro Glu Cys Glu Ala Val Cys Gly Lys Pro Lys Asn
                   85                  90                  95
   Pro Ala Asn Pro Val Gln Arg Ile Leu Gly Gly His Leu Asp Ala Lys
               100                 105                 110
   Gly Ser Phe Pro Trp Gln Ala Lys Met Val Ser His His Asn Leu Thr
           115                 120                 125
   Thr Gly Ala Thr Leu Ile Asn Glu Gln Trp Leu Leu Thr Thr Ala Lys
       130                 135                 140
   Asn Leu Phe Leu Asn His Ser Glu Asn Ala Thr Ala Lys Asp Ile Ala
   145                 150                 155                 160
   Pro Thr Leu Thr Leu Tyr Val Gly Lys Lys Gln Leu Val Glu Ile Glu
                   165                 170                 175
   Lys Val Val Leu His Pro Asn Tyr His Gln Val Asp Ile Gly Leu Ile
               180                 185                 190
   Lys Leu Lys Gln Lys Val Leu Val Asn Glu Arg Val Met Pro Ile Cys
           195                 200                 205
   Leu Pro Ser Lys Asn Tyr Ala Glu Val Gly Arg Val Gly Tyr Val Ser
       210                 215                 220
   Gly Trp Gly Gln Ser Asp Asn Phe Lys Leu Thr Asp His Leu Lys Tyr
   225                 230                 235                 240
   Val Met Leu Pro Val Ala Asp Gln Tyr Asp Cys Ile Thr His Tyr Glu
                   245                 250                 255
   Gly Ser Thr Cys Pro Lys Trp Lys Ala Pro Lys Ser Pro Val Gly Val
               260                 265                 270
   Gln Pro Ile Leu Asn Glu His Thr Phe Cys Val Gly Met Ser Lys Tyr
           275                 280                 285
   Gln Glu Asp Thr Cys Tyr Gly Asp Ala Gly Ser Ala Phe Ala Val His
       290                 295                 300
   Asp Leu Glu Glu Asp Thr Trp Tyr Ala Ala Gly Ile Leu Ser Phe Asp
   305                 310                 315                 320
   Lys Ser Cys Ala Val Ala Glu Tyr Gly Val Tyr Val Lys Val Thr Ser
                   325                 330                 335
   Ile Gln His Trp Val Gln Lys Thr Ile Ala Glu Asn
               340                 345
<210> 55
   <211> 462
   <212> PRT
   <213> Homo sapiens
<400> 55
   Met Ala Arg Val Leu Gly Ala Pro Val Ala Leu Gly Leu Trp Ser Leu
   1               5                   10                  15
   Cys Trp Ser Leu Ala Ile Ala Thr Pro Leu Pro Pro Thr Ser Ala His
               20                  25                  30
   Gly Asn Val Ala Glu Gly Glu Thr Lys Pro Asp Pro Asp Val Thr Glu
           35                  40                  45
   Arg Cys Ser Asp Gly Trp Ser Phe Asp Ala Thr Thr Leu Asp Asp Asn
       50                  55                  60
   Gly Thr Met Leu Phe Phe Lys Gly Glu Phe Val Trp Lys Ser His Lys
   65                  70                  75                  80
   Trp Asp Arg Glu Leu Ile Ser Glu Arg Trp Lys Asn Phe Pro Ser Pro
                   85                  90                  95
   Val Asp Ala Ala Phe Arg Gln Gly His Asn Ser Val Phe Leu Ile Lys
               100                 105                 110
   Gly Asp Lys Val Trp Val Tyr Pro Pro Glu Lys Lys Glu Lys Gly Tyr
           115                 120                 125
   Pro Lys Leu Leu Gln Asp Glu Phe Pro Gly Ile Pro Ser Pro Leu Asp
       130                 135                 140
   Ala Ala Val Glu Cys His Arg Gly Glu Cys Gln Ala Glu Gly Val Leu
   145                 150                 155                 160
   Phe Phe Gln Gly Asp Arg Glu Trp Phe Trp Asp Leu Ala Thr Gly Thr
                   165                 170                 175
   Met Lys Glu Arg Ser Trp Pro Ala Val Gly Asn Cys Ser Ser Ala Leu
               180                 185                 190
   Arg Trp Leu Gly Arg Tyr Tyr Cys Phe Gln Gly Asn Gln Phe Leu Arg
           195                 200                 205
   Phe Asp Pro Val Arg Gly Glu Val Pro Pro Arg Tyr Pro Arg Asp Val
       210                 215                 220
   Arg Asp Tyr Phe Met Pro Cys Pro Gly Arg Gly His Gly His Arg Asn
   225                 230                 235                 240
   Gly Thr Gly His Gly Asn Ser Thr His His Gly Pro Glu Tyr Met Arg
                   245                 250                 255
   Cys Ser Pro His Leu Val Leu Ser Ala Leu Thr Ser Asp Asn His Gly
               260                 265                 270
   Ala Thr Tyr Ala Phe Ser Gly Thr His Tyr Trp Arg Leu Asp Thr Ser
           275                 280                 285
   Arg Asp Gly Trp His Ser Trp Pro Ile Ala His Gln Trp Pro Gln Gly
       290                 295                 300
   Pro Ser Ala Val Asp Ala Ala Phe Ser Trp Glu Glu Lys Leu Tyr Leu
   305                 310                 315                 320
   Val Gln Gly Thr Gln Val Tyr Val Phe Leu Thr Lys Gly Gly Tyr Thr
                   325                 330                 335
   Leu Val Ser Gly Tyr Pro Lys Arg Leu Glu Lys Glu Val Gly Thr Pro
               340                 345                 350
   His Gly Ile Ile Leu Asp Ser Val Asp Ala Ala Phe Ile Cys Pro Gly
           355                 360                 365
   Ser Ser Arg Leu His Ile Met Ala Gly Arg Arg Leu Trp Trp Leu Asp
       370                 375                 380
   Leu Lys Ser Gly Ala Gln Ala Thr Trp Thr Glu Leu Pro Trp Pro His
   385                 390                 395                 400
   Glu Lys Val Asp Gly Ala Leu Cys Met Glu Lys Ser Leu Gly Pro Asn
                   405                 410                 415
   Ser Cys Ser Ala Asn Gly Pro Gly Leu Tyr Leu Ile His Gly Pro Asn
               420                 425                 430
   Leu Tyr Cys Tyr Ser Asp Val Glu Lys Leu Asn Ala Ala Lys Ala Leu
           435                 440                 445
   Pro Gln Pro Gln Asn Val Thr Ser Leu Leu Gly Cys Thr His
       450                 455                 460
<210> 56
   <211> 416
   <212> PRT
   <213> Homo sapiens
<400> 56
   Pro Gly Lys Gly Leu Glu Trp Val Ser Arg Ile Ser Ser Ser Gly Asp
   1               5                   10                  15
   Thr Val Asp Tyr Ala Asp Ser Val Lys Gly Arg Phe Thr Val Ser Arg
               20                  25                  30
   Asp Thr Ala Lys Asn Ser Leu Ser Leu Gln Met Ser Ser Leu Arg Val
           35                  40                  45
   Glu Asp Thr Ala Val Tyr Tyr Cys Ala Arg Thr Tyr Tyr Gly Met Asp
       50                  55                  60
   Val Trp Gly Gln Gly Thr Thr Val Thr Val Ser Ser Ala Ser Pro Thr
   65                  70                  75                  80
   Ser Pro Lys Val Phe Pro Leu Ser Leu Asp Ser Thr Pro Gln Asp Gly
                   85                  90                  95
   Asn Val Val Val Ala Cys Leu Val Gln Gly Phe Phe Pro Gln Glu Pro
               100                 105                 110
   Leu Ser Val Thr Trp Ser Glu Ser Gly Gln Asn Val Thr Ala Arg Asn
           115                 120                 125
   Phe Pro Pro Ser Gln Asp Ala Ser Gly Asp Leu Tyr Thr Thr Ser Ser
       130                 135                 140
   Gln Leu Thr Leu Pro Ala Thr Gln Cys Pro Asp Gly Lys Ser Val Thr
   145                 150                 155                 160
   Cys His Val Lys His Tyr Thr Asn Pro Ser Gln Asp Val Thr Val Pro
                   165                 170                 175
   Cys Pro Val Pro Pro Pro Pro Pro Cys Cys His Pro Arg Leu Ser Leu
               180                 185                 190
   His Arg Pro Ala Leu Glu Asp Leu Leu Leu Gly Ser Glu Ala Asn Leu
           195                 200                 205
   Thr Cys Thr Leu Thr Gly Leu Arg Asp Ala Ser Gly Ala Thr Phe Thr
       210                 215                 220
   Trp Thr Pro Ser Ser Gly Lys Ser Ala Val Gln Gly Pro Pro Glu Arg
   225                 230                 235                 240
   Asp Leu Cys Gly Cys Tyr Ser Val Ser Ser Val Leu Pro Gly Cys Ala
                   245                 250                 255
   Gln Pro Trp Asn His Gly Glu Thr Phe Thr Cys Thr Ala Ala His Pro
               260                 265                 270
   Glu Leu Lys Thr Pro Leu Thr Ala Asn Ile Thr Lys Ser Gly Asn Thr
           275                 280                 285
   Phe Arg Pro Glu Val His Leu Leu Pro Pro Pro Ser Glu Glu Leu Ala
       290                 295                 300
   Leu Asn Glu Leu Val Thr Leu Thr Cys Leu Ala Arg Gly Phe Ser Pro
   305                 310                 315                 320
   Lys Asp Val Leu Val Arg Trp Leu Gln Gly Ser Gln Glu Leu Pro Arg
                   325                 330                 335
   Glu Lys Tyr Leu Thr Trp Ala Ser Arg Gln Glu Pro Ser Gln Gly Thr
               340                 345                 350
   Thr Thr Phe Ala Val Thr Ser Ile Leu Arg Val Ala Ala Glu Asp Trp
           355                 360                 365
   Lys Lys Gly Asp Thr Phe Ser Cys Met Val Gly His Glu Ala Leu Pro
       370                 375                 380
   Leu Ala Phe Thr Gln Lys Thr Ile Asp Arg Leu Ala Gly Lys Pro Thr
   385                 390                 395                 400
   His Val Asn Val Ser Val Val Met Ala Glu Val Asp Gly Thr Cys Tyr
                   405                 410                 415
<210> 57
   <211> 327
   <212> PRT
   <213> Homo sapiens
<400> 57
   Ala Ser Thr Lys Gly Pro Ser Val Phe Pro Leu Ala Pro Cys Ser Arg
    1               5                  10                  15
   Ser Thr Ser Glu Ser Thr Ala Ala Leu Gly Cys Leu Val Lys Asp Tyr
               20                  25                  30
   Phe Pro Glu Pro Val Thr Val Ser Trp Asn Ser Gly Ala Leu Thr Ser
           35                  40                  45
   Gly Val His Thr Phe Pro Ala Val Leu Gln Ser Ser Gly Leu Tyr Ser
       50                  55                  60
   Leu Ser Ser Val Val Thr Val Pro Ser Ser Ser Leu Gly Thr Lys Thr
   65                  70                  75                  80
   Tyr Thr Cys Asn Val Asp His Lys Pro Ser Asn Thr Lys Val Asp Lys
                   85                  90                  95
   Arg Val Glu Ser Lys Tyr Gly Pro Pro Cys Pro Ser Cys Pro Ala Pro
               100                 105                 110
   Glu Phe Leu Gly Gly Pro Ser Val Phe Leu Phe Pro Pro Lys Pro Lys
           115                 120                 125
   Asp Thr Leu Met Ile Ser Arg Thr Pro Glu Val Thr Cys Val Val Val
       130                 135                 140
   Asp Val Ser Gln Glu Asp Pro Glu Val Gln Phe Asn Trp Tyr Val Asp
   145                 150                 155                 160
   Gly Val Glu Val His Asn Ala Lys Thr Lys Pro Arg Glu Glu Gln Phe
                   165                 170                 175
   Asn Ser Thr Tyr Arg Val Val Ser Val Leu Thr Val Leu His Gln Asp
               180                 185                 190
   Trp Leu Asn Gly Lys Glu Tyr Lys Cys Lys Val Ser Asn Lys Gly Leu
           195                 200                 205
   Pro Ser Ser Ile Glu Lys Thr Ile Ser Lys Ala Lys Gly Gln Pro Arg
       210                 215                 220
   Glu Pro Gln Val Tyr Thr Leu Pro Pro Ser Gln Glu Glu Met Thr Lys
   225                 230                 235                 240
   Asn Gln Val Ser Leu Thr Cys Leu Val Lys Gly Phe Tyr Pro Ser Asp
                   245                 250                 255
   Ile Ala Val Glu Trp Glu Ser Asn Gly Gln Pro Glu Asn Asn Tyr Lys
               260                 265                 270
   Thr Thr Pro Pro Val Leu Asp Ser Asp Gly Ser Phe Phe Leu Tyr Ser
           275                 280                 285
   Arg Leu Thr Val Asp Lys Ser Arg Trp Gln Glu Gly Asn Val Phe Ser
       290                 295                 300
   Cys Ser Val Met His Glu Ala Leu His Asn His Tyr Thr Gln Lys Ser
   305                 310                 315                 320
   Leu Ser Leu Ser Leu Gly Lys
                   325
<210> 58
   <211> 452
   <212> PRT
   <213> Homo sapiens
<400> 58
   Gly Ser Ala Ser Ala Pro Thr Leu Phe Pro Leu Val Ser Cys Glu Asn
    1               5                  10                  15
   Ser Pro Ser Asp Thr Ser Ser Val Ala Val Gly Cys Leu Ala Gln Asp
               20                  25                  30
   Phe Leu Pro Asp Ser Ile Thr Leu Ser Trp Lys Tyr Lys Asn Asn Ser
           35                  40                  45
   Asp Ile Ser Ser Thr Arg Gly Phe Pro Ser Val Leu Arg Gly Gly Lys
       50                  55                  60
   Tyr Ala Ala Thr Ser Gln Val Leu Leu Pro Ser Lys Asp Val Met Gln
   65                  70                  75                  80
   Gly Thr Asp Glu His Val Val Cys Lys Val Gln His Pro Asn Gly Asn
                   85                  90                  95
   Lys Glu Lys Asn Val Pro Leu Pro Val Ile Ala Glu Leu Pro Pro Lys
               100                 105                 110
   Val Ser Val Phe Val Pro Pro Arg Asp Gly Phe Phe Gly Asn Pro Arg
           115                 120                 125
   Lys Ser Lys Leu Ile Cys Gln Ala Thr Gly Phe Ser Pro Arg Gln Ile
       130                 135                 140
   Gln Val Ser Trp Leu Arg Glu Gly Lys Gln Val Gly Ser Gly Val Thr
   145                 150                 155                 160
   Thr Asp Gln Val Gln Ala Glu Ala Lys Glu Ser Gly Pro Thr Thr Tyr
                   165                 170                 175
   Lys Val Thr Ser Thr Leu Thr Ile Lys Glu Ser Asp Trp Leu Gly Gln
               180                 185                 190
   Ser Met Phe Thr Cys Arg Val Asp His Arg Gly Leu Thr Phe Gln Gln
           195                 200                 205
   Asn Ala Ser Ser Met Cys Val Pro Asp Gln Asp Thr Ala Ile Arg Val
       210                 215                 220
   Phe Ala Ile Pro Pro Ser Phe Ala Ser Ile Phe Leu Thr Lys Ser Thr
   225                 230                 235                 240
   Lys Leu Thr Cys Leu Val Thr Asp Leu Thr Thr Tyr Asp Ser Val Thr
                   245                 250                 255
   Ile Ser Trp Thr Arg Gln Asn Gly Glu Ala Val Lys Thr His Thr Asn
               260                 265                 270
   Ile Ser Glu Ser His Pro Asn Ala Thr Phe Ser Ala Val Gly Glu Ala
           275                 280                 285
   Ser Ile Cys Glu Asp Asp Trp Asn Ser Gly Glu Arg Phe Thr Cys Thr
       290                 295                 300
   Val Thr His Thr Asp Leu Pro Ser Pro Leu Lys Gln Thr Ile Ser Arg
   305                 310                 315                 320
   Pro Lys Gly Val Ala Leu His Arg Pro Asp Val Tyr Leu Leu Pro Pro
                   325                 330                 335
   Ala Arg Glu Gln Leu Asn Leu Arg Glu Ser Ala Thr Ile Thr Cys Leu
               340                 345                 350
   Val Thr Gly Phe Ser Pro Ala Asp Val Phe Val Gln Trp Met Gln Arg
           355                 360                 365
   Gly Gln Pro Leu Ser Pro Glu Lys Tyr Val Thr Ser Ala Pro Met Pro
       370                 375                 380
   Glu Pro Gln Ala Pro Gly Arg Tyr Phe Ala His Ser Ile Leu Thr Val
   385                 390                 395                 400
   Ser Glu Glu Glu Trp Asn Thr Gly Glu Thr Tyr Thr Cys Val Ala His
                   405                 410                 415
   Glu Ala Leu Pro Asn Arg Val Thr Glu Arg Thr Val Asp Lys Ser Thr
               420                 425                 430
   Gly Lys Pro Thr Leu Tyr Asn Val Ser Leu Val Met Ser Asp Thr Ala
           435                 440                 445
   Gly Thr Cys Tyr
       450
<210> 59
   <211> 375
   <212> PRT
   <213> Homo sapiens
<400> 59
<210> 60
   <211> 131
   <212> PRT
   <213> Homo sapiens
<400> 60
   Leu Val Gln Leu Val Glu Ser Gly Gly Gly Leu Val Gln Pro Gly Arg
    1               5                  10                  15
   Ser Leu Arg Leu Ser Cys Thr Ala Pro Gly Phe Thr Phe Gly Asp Tyr
               20                  25                  30
   Ala Met Ser Trp Val Arg Gln Ala Pro Gly Lys Gly Leu Glu Trp Val
           35                  40                  45
   Gly Phe Ile Arg Ser Lys Ala Tyr Gly Gly Thr Thr Glu Tyr Ala Ala
       50                  55                  60
   Ser Val Lys Gly Arg Phe Thr Ile Ser Arg Asp Asp Ser Lys Ser Ile
   65                  70                  75                  80
   Ala Tyr Leu Gln Met Asn Ser Leu Lys Thr Glu Asp Thr Ala Val Tyr
                   85                  90                  95
   Tyr Cys Thr Arg Asp Gln Asp Cys Thr Asn Gly Val Cys Tyr Thr Phe
               100                 105                 110
   Gly Val Glu Asn Trp Phe Asp Pro Trp Gly Gln Gly Thr Leu Val Thr
                 115                 120                 125
   Val Ser Ser
       130
<210> 61
   <211> 473
   <212> PRT
   <213> Homo sapiens
<400> 61
<210> 62
   <211> 157
   <212> PRT
   <213> Homo sapiens
<400> 62
   Met Lys Asn His Leu Leu Phe Trp Gly Val Leu Ala Val Phe Ile Lys
    1               5                  10                  15
   Ala Val His Val Lys Gly Ser Arg Asp Ser Ser Ala Ser Ala Ser Arg
               20                  25                  30
   Val Ala Gly Ile Thr Ala Gln Glu Asp Glu Arg Ile Val Leu Val Asp
           35                  40                  45
   Asn Lys Cys Lys Cys Ala Arg Ile Thr Ser Arg Ile Ile Arg Ser Ser
       50                  55                  60
   Glu Asp Pro Asn Glu Asp Ile Val Glu Arg Asn Ile Arg Ile Ile Val
   65                  70                  75                  80
   Pro Leu Asn Asn Arg Glu Asn Ile Ser Asp Pro Thr Ser Pro Leu Arg
                   85                  90                  95
   Thr Arg Phe Val Tyr His Leu Ser Asp Leu Cys Lys Lys Cys Asp Pro
               100                 105                 110
   Thr Glu Val Glu Leu Asp Asn Gln Ile Val Thr Ala Thr Gln Ser Asn
           115                 120                 125
   Ile Cys Asp Glu Asp Ser Ala Thr Glu Thr Cys Tyr Thr Tyr Asp Arg
       130                 135                 140
   Asn Lys Cys Tyr Thr Ala Val Val Pro Leu Val Tyr Gly
   145                 150                 155
<210> 63
   <211> 117
   <212> PRT
   <213> Homo sapiens
<400> 63
   Met Asp Met Arg Val Pro Ala Gln Leu Leu Gly Leu Leu Leu Leu Trp
    1               5                  10                  15
   Leu Pro Gly Ala Arg Cys Asp Ile Gln Leu Thr Gln Ser Pro Ser Phe
               20                  25                  30
   Leu Ser Ala Ser Val Gly Asp Arg Val Thr Ile Thr Cys Arg Ala Ser
           35                  40                  45
   Gln Gly Ile Ser Ser Tyr Leu Ala Trp Tyr Gln Gln Lys Pro Gly Lys
       50                  55                  60
   Ala Pro Lys Leu Leu Ile Tyr Ala Ala Ser Thr Leu Gln Ser Gly Val
   65                  70                  75                  80
   Pro Ser Arg Phe Ser Gly Ser Gly Ser Gly Thr Glu Phe Thr Leu Thr
                   85                  90                  95
   Ile Ser Ser Leu Gln Pro Glu Asp Phe Ala Thr Tyr Tyr Cys Gln Gln
               100                 105                 110
   Leu Asn Ser Tyr Pro
           115
<210> 64
   <211> 106
   <212> PRT
   <213> Homo sapiens
<400> 64
   Gly Gln Pro Lys Ala Ala Pro Ser Val Thr Leu Phe Pro Pro Ser Ser
   1               5                   10                  15
   Glu Glu Leu Gln Ala Asn Lys Ala Thr Leu Val Cys Leu Ile Ser Asp
               20                  25                  30
   Phe Tyr Pro Gly Ala Val Thr Val Ala Trp Lys Ala Asp Ser Ser Pro
           35                  40                  45
   Val Lys Ala Gly Val Glu Thr Thr Thr Pro Ser Lys Gln Ser Asn Asn
       50                  55                  60
   Lys Tyr Ala Ala Ser Ser Tyr Leu Ser Leu Thr Pro Glu Gln Trp Lys
   65                  70                  75                  80
   Ser His Arg Ser Tyr Ser Cys Gln Val Thr His Glu Gly Ser Thr Val
                   85                  90                  95
   Glu Lys Thr Val Ala Pro Thr Glu Cys Ser
               100                 105
<210> 65
   <211> 233
   <212> PRT
   <213> Homo sapiens
<400> 65
<210> 66
   <211> 98
   <212> PRT
   <213> Homo sapiens
<400> 66
   Gln Ala Val Val Thr Gln Glu Pro Ser Leu Thr Val Ser Pro Gly Gly
   1 5 10 15
   Thr Val Thr Leu Thr Cys Gly Ser Ser Thr Gly Ala Val Thr Ser Gly
   20 25 30
   His Tyr Pro Tyr Trp Phe Gln Gln Lys Pro Gly Gln Ala Pro Arg Thr
   35 40 45
   Leu Ile Tyr Asp Thr Ser Asn Lys His Ser Trp Thr Pro Ala Arg Phe
   50 55 60
   Ser Gly Ser Leu Leu Gly Gly Lys Ala Ala Leu Thr Leu Leu Gly Ala
   65 70 75 80
   Gln Pro Glu Asp Glu Ala Glu Tyr Tyr Cys Leu Leu Ser Tyr Ser Gly
   85 90 95
   Ala Arg
<210> 67
   <211> 890
   <212> PRT
   <213> Homo sapiens
<400> 67
   Met Ala Phe Ala Trp Trp Pro Cys Leu Ile Leu Ala Leu Leu Ser Ser
   1 5 10 15
   Leu Ala Ala Ser Gly Phe Pro Arg Ser Pro Phe Arg Leu Leu Gly Lys
   20 25 30
   Arg Ser Leu Pro Glu Gly Val Ala Asn Gly Ile Glu Val Tyr Ser Thr
   35 40 45
   Lys Ile Asn Ser Lys Val Thr Ser Arg Phe Ala His Asn Val Val Thr
   50 55 60
   Met Arg Ala Val Asn Arg Ala Asp Thr Ala Lys Glu Val Ser Phe Asp
   65 70 75 80
   Val Glu Leu Pro Lys Thr Ala Phe Ile Thr Asn Phe Thr Leu Thr Ile
   85 90 95
   Asp Gly Val Thr Tyr Pro Gly Asn Val Lys Glu Lys Glu Val Ala Lys
   100 105 110
   Lys Gln Tyr Glu Lys Ala Val Ser Gln Gly Lys Thr Ala Gly Leu Val
   115 120 125
   Lys Ala Ser Gly Arg Lys Leu Glu Lys Phe Thr Val Ser Val Asn Val
   130 135 140
   Ala Ala Gly Ser Lys Val Thr Phe Glu Leu Thr Tyr Glu Glu Leu Leu
   145 150 155 160
   Lys Arg His Lys Gly Lys Tyr Glu Met Tyr Leu Lys Val Gln Pro Lys
   165 170 175
   Gln Leu Val Lys His Phe Glu Ile Glu Val Asp Ile Phe Glu Pro Gln
   180 185 190
   Gly Ile Ser Met Leu Asp Ala Glu Ala Ser Phe Ile Thr Asn Asp Leu
   195 200 205
   Leu Gly Ser Ala Leu Thr Lys Ser Phe Ser Gly Lys Lys Gly His Val
   210 215 220
   Ser Phe Lys Pro Ser Leu Asp Gln Gln Arg Ser Cys Pro Thr Cys Thr
   225 230 235 240
   Asp Ser Leu Leu Asn Gly Asp Phe Thr Ile Thr Tyr Asp Val Asn Arg
   245 250 255
   Glu Ser Pro Gly Asn Val Gln Ile Val Asn Gly Tyr Phe Val His Phe
   260 265 270
   Phe Ala Pro Gln Gly Leu Pro Val Val Pro Lys Asn Val Ala Phe Val
   275 280 285
   Ile Asp Ile Ser Gly Ser Met Ala Gly Arg Lys Leu Glu Gln Thr Lys
   290 295 300
   Glu Ala Leu Leu Arg Ile Leu Glu Asp Met Gln Glu Glu Asp Tyr Leu
   305                 310                 315                 320
   Asn Phe Ile Leu Phe Ser Gly Asp Val Ser Thr Trp Lys Glu His Leu
                   325                 330                 335
   Val Gln Ala Thr Pro Glu Asn Leu Gln Glu Ala Arg Thr Phe Val Lys
               340                 345                 350
   Ser Met Glu Asp Lys Gly Met Thr Asn Ile Asn Asp Gly Leu Leu Arg
           355                 360                 365
   Gly Ile Ser Met Leu Asn Lys Ala Arg Glu Glu His Arg Ile Pro Glu
       370                 375                 380
   Arg Ser Thr Ser Ile Val Ile Met Leu Thr Asp Gly Asp Ala Asn Val
   385                 390                 395                 400
   Gly Glu Ser Arg Pro Glu Lys Ile Gln Glu Asn Val Arg Asn Ala Ile
                   405                 410                 415
   Gly Gly Lys Phe Pro Leu Tyr Asn Leu Gly Phe Gly Asn Asn Leu Asn
               420                 425                 430
   Tyr Asn Phe Leu Glu Asn Met Ala Leu Glu Asn His Gly Phe Ala Arg
           435                 440                 445
   Arg Ile Tyr Glu Asp Ser Asp Ala Asp Leu Gln Leu Gln Gly Phe Tyr
       450                 455                 460
   Glu Glu Val Ala Asn Pro Leu Leu Thr Gly Val Glu Met Glu Tyr Pro
   465                 470                 475                 480
   Glu Asn Ala Ile Leu Asp Leu Thr Gln Asn Thr Tyr Gln His Phe Tyr
                   485                 490                 495
   Asp Gly Ser Glu Ile Val Val Ala Gly Arg Leu Val Asp Glu Asp Met
               500                 505                 510
   Asn Ser Phe Lys Ala Asp Val Lys Gly His Gly Ala Thr Asn Asp Leu
           515                 520                 525
   Thr Phe Thr Glu Glu Val Asp Met Lys Glu Met Glu Lys Ala Leu Gln
       530                 535                 540
   Glu Arg Asp Tyr Ile Phe Gly Asn Tyr Ile Glu Arg Leu Trp Ala Tyr
   545                 550                 555                 560
   Leu Thr Ile Glu Gln Leu Leu Glu Lys Arg Lys Asn Ala His Gly Glu
                   565                 570                 575
   Glu Lys Glu Asn Leu Thr Ala Arg Ala Leu Asp Leu Ser Leu Lys Tyr
               580                 585                 590
   His Phe Val Thr Pro Leu Thr Ser Met Val Val Thr Lys Pro Glu Asp
           595                 600                 605
   Asn Glu Asp Glu Arg Ala Ile Ala Asp Lys Pro Gly Glu Asp Ala Glu
       610                 615                 620
   Ala Thr Pro Val Ser Pro Ala Met Ser Tyr Leu Thr Ser Tyr Gln Pro
   625                 630                 635                 640
   Pro Gln Asn Pro Tyr Tyr Tyr Val Asp Gly Asp Pro His Phe Ile Ile
                   645                 650                 655
   Gln Ile Pro Glu Lys Asp Asp Ala Leu Cys Phe Asn Ile Asp Glu Ala
               660                 665                 670
   Pro Gly Thr Val Leu Arg Leu Ile Gln Asp Ala Val Thr Gly Leu Thr
           675                 680                 685
   Val Asn Gly Gln Ile Thr Gly Asp Lys Arg Gly Ser Pro Asp Ser Lys
       690                 695                 700
   Thr Arg Lys Thr Tyr Phe Gly Lys Leu Gly Ile Ala Asn Ala Gln Met
   705                 710                 715                 720
   Asp Phe Gln Val Glu Val Thr Thr Glu Lys Ile Thr Leu Trp Asn Arg
                   725                 730                 735
   Ala Val Pro Ser Thr Phe Ser Trp Leu Asp Thr Val Thr Val Thr Gln
               740                 745                 750
   Asp Gly Leu Ser Met Met Ile Asn Arg Lys Asn Met Val Val Ser Phe
           755                 760                 765
   Gly Asp Gly Val Thr Phe Val Val Val Leu His Gln Val Trp Lys Lys
       770                 775                 780
   His Pro Val His Arg Asp Phe Leu Gly Phe Tyr Val Val Asp Ser His
   785                 790                 795                 800
   Arg Met Ser Ala Gln Thr His Gly Leu Leu Gly Gln Phe Phe Gln Pro
                   805                 810                 815
   Phe Asp Phe Lys Val Ser Asp Ile Arg Pro Gly Ser Asp Pro Thr Lys
               820                 825                 830
   Pro Asp Ala Thr Leu Val Val Lys Asn His Gln Leu Ile Val Thr Arg
           835                 840                 845
   Gly Ser Gln Lys Asp Tyr Arg Lys Asp Ala Ser Ile Gly Thr Lys Val
       850                 855                 860
   Val Cys Trp Phe Val His Asn Asn Gly Glu Gly Leu Ile Asp Gly Val
   865                 870                 875                 880
   His Thr Asp Tyr Ile Val Pro Asn Leu Phe
                   885                 890
<210> 68
   <211> 638
   <212> PRT
   <213> Homo sapiens
<400> 68
   Met Ile Leu Phe Lys Gln Ala Thr Tyr Phe Ile Ser Leu Phe Ala Thr
    1               5                  10                  15
   Val Ser Cys Gly Cys Leu Thr Gln Leu Tyr Glu Asn Ala Phe Phe Arg
               20                  25                  30
   Gly Gly Asp Val Ala Ser Met Tyr Thr Pro Asn Ala Gln Tyr Cys Gln
           35                  40                  45
   Met Arg Cys Thr Phe His Pro Arg Cys Leu Leu Phe Ser Phe Leu Pro
       50                  55                  60
   Ala Ser Ser Ile Asn Asp Met Glu Lys Arg Phe Gly Cys Phe Leu Lys
   65                  70                  75                  80
   Asp Ser Val Thr Gly Thr Leu Pro Lys Val His Arg Thr Gly Ala Val
                   85                  90                  95
   Ser Gly His Ser Leu Lys Gln Cys Gly His Gln Ile Ser Ala Cys His
               100                 105                 110
   Arg Asp Ile Tyr Lys Gly Val Asp Met Arg Gly Val Asn Phe Asn Val
           115                 120                 125
   Ser Lys Val Ser Ser Val Glu Glu Cys Gln Lys Arg Cys Thr Asn Asn
       130                 135                 140
   Ile Arg Cys Gln Phe Phe Ser Tyr Ala Thr Gln Thr Phe His Lys Ala
   145                 150                 155                 160
   Glu Tyr Arg Asn Asn Cys Leu Leu Lys Tyr Ser Pro Gly Gly Thr Pro
                   165                 170                 175
   Thr Ala Ile Lys Val Leu Ser Asn Val Glu Ser Gly Phe Ser Leu Lys
               180                 185                 190
   Pro Cys Ala Leu Ser Glu Ile Gly Cys His Met Asn Ile Phe Gln His
           195                 200                 205
   Leu Ala Phe Ser Asp Val Asp Val Ala Arg Val Leu Thr Pro Asp Ala
       210                 215                 220
   Phe Val Cys Arg Thr Ile Cys Thr Tyr His Pro Asn Cys Leu Phe Phe
   225                 230                 235                 240
   Thr Phe Tyr Thr Asn Val Trp Lys Ile Glu Ser Gln Arg Asn Val Cys
                   245                 250                 255
   Leu Leu Lys Thr Ser Glu Ser Gly Thr Pro Ser Ser Ser Thr Pro Gln
               260                 265                 270
   Glu Asn Thr Ile Ser Gly Tyr Ser Leu Leu Thr Cys Lys Arg Thr Leu
           275                 280                 285
   Pro Glu Pro Cys His Ser Lys Ile Tyr Pro Gly Val Asp Phe Gly Gly
       290                 295                 300
   Glu Glu Leu Asn Val Thr Phe Val Lys Gly Val Asn Val Cys Gln Glu
   305                 310                 315                 320
   Thr Cys Thr Lys Met Ile Arg Cys Gln Phe Phe Thr Tyr Ser Leu Leu
                   325                 330                 335
   Pro Glu Asp Cys Lys Glu Glu Lys Cys Lys Cys Phe Leu Arg Leu Ser
               340                 345                 350
   Met Asp Gly Ser Pro Thr Arg Ile Ala Tyr Gly Thr Gln Gly Ser Ser
           355                 360                 365
   Gly Tyr Ser Leu Arg Leu Cys Asn Thr Gly Asp Asn Ser Val Cys Thr
       370                 375                 380
   Thr Lys Thr Ser Thr Arg Ile Val Gly Gly Thr Asn Ser Ser Trp Gly
   385                 390                 395                 400
   Glu Trp Pro Trp Gln Val Ser Leu Gln Val Lys Leu Thr Ala Gln Arg
                   405                 410                 415
   His Leu Cys Gly Gly Ser Leu Ile Gly His Gln Trp Val Leu Thr Ala
               420                 425                 430
   Ala His Cys Phe Asp Gly Leu Pro Leu Gln Asp Val Trp Arg Ile Tyr
           435                 440                 445
   Ser Gly Ile Leu Asn Leu Ser Asp Ile Thr Lys Asp Thr Pro Phe Ser
       450                 455                 460
   Gln Ile Lys Glu Ile Ile Ile His Gln Asn Tyr Lys Val Ser Glu Gly
   465                 470                 475                 480
   Asn His Asp Ile Ala Leu Ile Lys Leu Gln Ala Pro Leu Asn Tyr Thr
                   485                 490                 495
   Glu Phe Gln Lys Pro Ile Cys Leu Pro Ser Lys Gly Asp Thr Ser Thr
               500                 505                 510
   Ile Tyr Thr Asn Cys Trp Val Thr Gly Trp Gly Phe Ser Lys Glu Lys
           515                 520                 525
   Gly Glu Ile Gln Asn Ile Leu Gln Lys Val Asn Ile Pro Leu Val Thr
       530                 535                 540
   Asn Glu Glu Cys Gln Lys Arg Tyr Gln Asp Tyr Lys Ile Thr Gln Arg
   545                 550                 555                 560
   Met Val Cys Ala Gly Tyr Lys Glu Gly Gly Lys Asp Ala Cys Lys Gly
                   565                 570                 575
   Asp Ser Gly Gly Pro Leu Val Cys Lys His Asn Gly Met Trp Arg Leu
               580                 585                 590
   Val Gly Ile Thr Ser Trp Gly Glu Gly Cys Ala Arg Arg Glu Gln Pro
           595                 600                 605
   Gly Val Tyr Thr Lys Val Ala Glu Tyr Met Asp Trp Ile Leu Glu Lys
       610                 615                 620
   Thr Gln Ser Ser Asp Gly Lys Ala Gln Met Gln Ser Pro Ala
   625                 630                 635
<210> 69
   <211> 639
   <212> PRT
   <213> Homo sapiens
<400> 69
<210> 70
   <211> 4548
   <212> PRT
   <213> Homo sapiens
<400> 70
   Met Glu His Lys Glu Val Val Leu Leu Leu Leu Leu Phe Leu Lys Ser
   1               5                   10                  15
   Ala Ala Pro Glu Gln Ser His Val Val Gln Asp Cys Tyr His Gly Asp
               20                  25                  30
   Gly Gln Ser Tyr Arg Gly Thr Tyr Ser Thr Thr Val Thr Gly Arg Thr
           35                  40                  45
   Cys Gln Ala Trp Ser Ser Met Thr Pro His Gln His Asn Arg Thr Thr
       50                  55                  60
   Glu Asn Tyr Pro Asn Ala Gly Leu Ile Met Asn Tyr Cys Arg Asn Pro
   65                  70                  75                  80
   Asp Ala Val Ala Ala Pro Tyr Cys Tyr Thr Arg Asp Pro Gly Val Arg
                   85                  90                  95
   Trp Glu Tyr Cys Asn Leu Thr Gln Cys Ser Asp Ala Glu Gly Thr Ala
               100                 105                 110
   Val Ala Pro Pro Thr Val Thr Pro Val Pro Ser Leu Glu Ala Pro Ser
           115                 120                 125
   Glu Gln Ala Pro Thr Glu Gln Arg Pro Gly Val Gln Glu Cys Tyr His
       130                 135                 140
   Gly Asn Gly Gln Ser Tyr Arg Gly Thr Tyr Ser Thr Thr Val Thr Gly
   145                 150                 155                 160
   Arg Thr Cys Gln Ala Trp Ser Ser Met Thr Pro His Ser His Ser Arg
                   165                 170                 175
   Thr Pro Glu Tyr Tyr Pro Asn Ala Gly Leu Ile Met Asn Tyr Cys Arg
               180                 185                 190
   Asn Pro Asp Ala Val Ala Ala Pro Tyr Cys Tyr Thr Arg Asp Pro Gly
           195                 200                 205
   Val Arg Trp Glu Tyr Cys Asn Leu Thr Gln Cys Ser Asp Ala Glu Gly
       210                 215                 220
   Thr Ala Val Ala Pro Pro Thr Val Thr Pro Val Pro Ser Leu Glu Ala
   225                 230                 235                 240
   Pro Ser Glu Gln Ala Pro Thr Glu Gln Arg Pro Gly Val Gln Glu Cys
                   245                 250                 255
   Tyr His Gly Asn Gly Gln Ser Tyr Arg Gly Thr Tyr Ser Thr Thr Val
               260                 265                 270
   Thr Gly Arg Thr Cys Gln Ala Trp Ser Ser Met Thr Pro His Ser His
           275                 280                 285
   Ser Arg Thr Pro Glu Tyr Tyr Pro Asn Ala Gly Leu Ile Met Asn Tyr
       290                 295                 300
   Cys Arg Asn Pro Asp Ala Val Ala Ala Pro Tyr Cys Tyr Thr Arg Asp
   305                 310                 315                 320
   Pro Gly Val Arg Trp Glu Tyr Cys Asn Leu Thr Gln Cys Ser Asp Ala
                   325                 330                 335
   Glu Gly Thr Ala Val Ala Pro Pro Thr Val Thr Pro Val Pro Ser Leu
               340                 345                 350
   Glu Ala Pro Ser Glu Gln Ala Pro Thr Glu Gln Arg Pro Gly Val Gln
           355                 360                 365
   Glu Cys Tyr His Gly Asn Gly Gln Ser Tyr Arg Gly Thr Tyr Ser Thr
       370                 375                 380
   Thr Val Thr Gly Arg Thr Cys Gln Ala Trp Ser Ser Met Thr Pro His
   385                 390                 395                 400
   Ser His Ser Arg Thr Pro Glu Tyr Tyr Pro Asn Ala Gly Leu Ile Met
                   405                 410                 415
   Asn Tyr Cys Arg Asn Pro Asp Ala Val Ala Ala Pro Tyr Cys Tyr Thr
               420                 425                 430
   Arg Asp Pro Gly Val Arg Trp Glu Tyr Cys Asn Leu Thr Gln Cys Ser
           435                 440                 445
   Asp Ala Glu Gly Thr Ala Val Ala Pro Pro Thr Val Thr Pro Val Pro
       450                 455                 460
   Ser Leu Glu Ala Pro Ser Glu Gln Ala Pro Thr Glu Gln Arg Pro Gly
   465                 470                 475                 480
   Val Gln Glu Cys Tyr His Gly Asn Gly Gln Ser Tyr Arg Gly Thr Tyr
                   485                 490                 495
   Ser Thr Thr Val Thr Gly Arg Thr Cys Gln Ala Trp Ser Ser Met Thr
               500                 505                 510
   Pro His Ser His Ser Arg Thr Pro Glu Tyr Tyr Pro Asn Ala Gly Leu
           515                 520                 525
   Ile Met Asn Tyr Cys Arg Asn Pro Asp Ala Val Ala Ala Pro Tyr Cys
       530                 535                 540
   Tyr Thr Arg Asp Pro Gly Val Arg Trp Glu Tyr Cys Asn Leu Thr Gln
   545                 550                 555                 560
   Cys Ser Asp Ala Glu Gly Thr Ala Val Ala Pro Pro Thr Val Thr Pro
                   565                 570                 575
   Val Pro Ser Leu Glu Ala Pro Ser Glu Gln Ala Pro Thr Glu Gln Arg
               580                 585                 590
   Pro Gly Val Gln Glu Cys Tyr His Gly Asn Gly Gln Ser Tyr Arg Gly
           595                 600                 605
   Thr Tyr Ser Thr Thr Val Thr Gly Arg Thr Cys Gln Ala Trp Ser Ser
       610                 615                 620
   Met Thr Pro His Ser His Ser Arg Thr Pro Glu Tyr Tyr Pro Asn Ala
   625                 630                 635                 640
   Gly Leu Ile Met Asn Tyr Cys Arg Asn Pro Asp Ala Val Ala Ala Pro
                   645                 650                 655
   Tyr Cys Tyr Thr Arg Asp Pro Gly Val Arg Trp Glu Tyr Cys Asn Leu
               660                 665                 670
   Thr Gln Cys Ser Asp Ala Glu Gly Thr Ala Val Ala Pro Pro Thr Val
           675                 680                 685
   Thr Pro Val Pro Ser Leu Glu Ala Pro Ser Glu Gln Ala Pro Thr Glu
       690                 695                 700
   Gln Arg Pro Gly Val Gln Glu Cys Tyr His Gly Asn Gly Gln Ser Tyr
   705                 710                 715                 720
   Arg Gly Thr Tyr Ser Thr Thr Val Thr Gly Arg Thr Cys Gln Ala Trp
                   725                 730                 735
   Ser Ser Met Thr Pro His Ser His Ser Arg Thr Pro Glu Tyr Tyr Pro
               740                 745                 750
   Asn Ala Gly Leu Ile Met Asn Tyr Cys Arg Asn Pro Asp Ala Val Ala
           755                 760                 765
   Ala Pro Tyr Cys Tyr Thr Arg Asp Pro Gly Val Arg Trp Glu Tyr Cys
       770                 775                 780
   Asn Leu Thr Gln Cys Ser Asp Ala Glu Gly Thr Ala Val Ala Pro Pro
   785                 790                 795                 800
   Thr Val Thr Pro Val Pro Ser Leu Glu Ala Pro Ser Glu Gln Ala Pro
                   805                 810                 815
   Thr Glu Gln Arg Pro Gly Val Gln Glu Cys Tyr His Gly Asn Gly Gln
               820                 825                 830
   Ser Tyr Arg Gly Thr Tyr Ser Thr Thr Val Thr Gly Arg Thr Cys Gln
           835                 840                 845
   Ala Trp Ser Ser Met Thr Pro His Ser His Ser Arg Thr Pro Glu Tyr
       850                 855                 860
   Tyr Pro Asn Ala Gly Leu Ile Met Asn Tyr Cys Arg Asn Pro Asp Ala
   865                 870                 875                 880
   Val Ala Ala Pro Tyr Cys Tyr Thr Arg Asp Pro Gly Val Arg Trp Glu
                   885                 890                 895
   Tyr Cys Asn Leu Thr Gln Cys Ser Asp Ala Glu Gly Thr Ala Val Ala
               900                 905                 910
   Pro Pro Thr Val Thr Pro Val Pro Ser Leu Glu Ala Pro Ser Glu Gln
           915                 920                 925
   Ala Pro Thr Glu Gln Arg Pro Gly Val Gln Glu Cys Tyr His Gly Asn
       930                 935                 940
   Gly Gln Ser Tyr Arg Gly Thr Tyr Ser Thr Thr Val Thr Gly Arg Thr
   945                 950                 955                 960
   Cys Gln Ala Trp Ser Ser Met Thr Pro His Ser His Ser Arg Thr Pro
                   965                 970                 975
   Glu Tyr Tyr Pro Asn Ala Gly Leu Ile Met Asn Tyr Cys Arg Asn Pro
               980                 985                 990
   Asp Ala Val Ala Ala Pro Tyr Cys Tyr Thr Arg Asp Pro Gly Val Arg
           995                 1000                1005
   Trp Glu Tyr Cys Asn Leu Thr Gln Cys Ser Asp Ala Glu Gly Thr Ala
       1010                1015                1020
   Val Ala Pro Pro Thr Val Thr Pro Val Pro Ser Leu Glu Ala Pro Ser
   1025                1030                1035                1040
   Glu Gln Ala Pro Thr Glu Gln Arg Pro Gly Val Gln Glu Cys Tyr His
                   1045                1050                1055
   Gly Asn Gly Gln Ser Tyr Arg Gly Thr Tyr Ser Thr Thr Val Thr Gly
               1060                1065                1070
   Arg Thr Cys Gln Ala Trp Ser Ser Met Thr Pro His Ser His Ser Arg
           1075                1080                1085
   Thr Pro Glu Tyr Tyr Pro Asn Ala Gly Leu Ile Met Asn Tyr Cys Arg
       1090                1095                1100
   Asn Pro Asp Ala Val Ala Ala Pro Tyr Cys Tyr Thr Arg Asp Pro Gly
   1105                1110                1115                1120
   Val Arg Trp Glu Tyr Cys Asn Leu Thr Gln Cys Ser Asp Ala Glu Gly
                   1125                1130                1135
   Thr Ala Val Ala Pro Pro Thr Val Thr Pro Val Pro Ser Leu Glu Ala
               1140                1145                1150
   Pro Ser Glu Gln Ala Pro Thr Glu Gln Arg Pro Gly Val Gln Glu Cys
           1155                1160                1165
   Tyr His Gly Asn Gly Gln Ser Tyr Arg Gly Thr Tyr Ser Thr Thr Val
       1170                1175                1180
   Thr Gly Arg Thr Cys Gln Ala Trp Ser Ser Met Thr Pro His Ser His
   1185                1190                1195                1200
   Ser Arg Thr Pro Glu Tyr Tyr Pro Asn Ala Gly Leu Ile Met Asn Tyr
                   1205                1210                1215
   Cys Arg Asn Pro Asp Ala Val Ala Ala Pro Tyr Cys Tyr Thr Arg Asp
               1220                1225                1230
   Pro Gly Val Arg Trp Glu Tyr Cys Asn Leu Thr Gln Cys Ser Asp Ala
           1235                1240                1245
   Glu Gly Thr Ala Val Ala Pro Pro Thr Val Thr Pro Val Pro Ser Leu
       1250                1255                1260
   Glu Ala Pro Ser Glu Gln Ala Pro Thr Glu Gln Arg Pro Gly Val Gln
   1265                1270                1275                1280
   Glu Cys Tyr His Gly Asn Gly Gln Ser Tyr Arg Gly Thr Tyr Ser Thr
                   1285                1290                1295
   Thr Val Thr Gly Arg Thr Cys Gln Ala Trp Ser Ser Met Thr Pro His
               1300                1305                1310
   Ser His Ser Arg Thr Pro Glu Tyr Tyr Pro Asn Ala Gly Leu Ile Met
           1315                1320                1325
   Asn Tyr Cys Arg Asn Pro Asp Ala Val Ala Ala Pro Tyr Cys Tyr Thr
       1330                1335                1340
   Arg Asp Pro Gly Val Arg Trp Glu Tyr Cys Asn Leu Thr Gln Cys Ser
   1345                1350                1355                1360
   Asp Ala Glu Gly Thr Ala Val Ala Pro Pro Thr Val Thr Pro Val Pro
                   1365                1370                1375
   Ser Leu Glu Ala Pro Ser Glu Gln Ala Pro Thr Glu Gln Arg Pro Gly
               1380                1385                1390
   Val Gln Glu Cys Tyr His Gly Asn Gly Gln Ser Tyr Arg Gly Thr Tyr
           1395                1400                1405
   Ser Thr Thr Val Thr Gly Arg Thr Cys Gln Ala Trp Ser Ser Met Thr
       1410                1415                1420
   Pro His Ser His Ser Arg Thr Pro Glu Tyr Tyr Pro Asn Ala Gly Leu
   1425                1430                1435                1440
   Ile Met Asn Tyr Cys Arg Asn Pro Asp Ala Val Ala Ala Pro Tyr Cys
                   1445                1450                1455
   Tyr Thr Arg Asp Pro Gly Val Arg Trp Glu Tyr Cys Asn Leu Thr Gln
               1460                1465                1470
   Cys Ser Asp Ala Glu Gly Thr Ala Val Ala Pro Pro Thr Val Thr Pro
           1475                1480                1485
   Val Pro Ser Leu Glu Ala Pro Ser Glu Gln Ala Pro Thr Glu Gln Arg
       1490                1495                1500
   Pro Gly Val Gln Glu Cys Tyr His Gly Asn Gly Gln Ser Tyr Arg Gly
   1505                1510                1515                1520
   Thr Tyr Ser Thr Thr Val Thr Gly Arg Thr Cys Gln Ala Trp Ser Ser
                   1525                1530                1535
   Met Thr Pro His Ser His Ser Arg Thr Pro Glu Tyr Tyr Pro Asn Ala
               1540                1545                1550
   Gly Leu Ile Met Asn Tyr Cys Arg Asn Pro Asp Ala Val Ala Ala Pro
           1555                1560                1565
   Tyr Cys Tyr Thr Arg Asp Pro Gly Val Arg Trp Glu Tyr Cys Asn Leu
       1570                1575                1580
   Thr Gln Cys Ser Asp Ala Glu Gly Thr Ala Val Ala Pro Pro Thr Val
   1585                1590                1595                1600
   Thr Pro Val Pro Ser Leu Glu Ala Pro Ser Glu Gln Ala Pro Thr Glu
                   1605                1610                1615
   Gln Arg Pro Gly Val Gln Glu Cys Tyr His Gly Asn Gly Gln Ser Tyr
               1620                1625                1630
   Arg Gly Thr Tyr Ser Thr Thr Val Thr Gly Arg Thr Cys Gln Ala Trp
           1635                1640                1645
   Ser Ser Met Thr Pro His Ser His Ser Arg Thr Pro Glu Tyr Tyr Pro
       1650                1655                1660
   Asn Ala Gly Leu Ile Met Asn Tyr Cys Arg Asn Pro Asp Ala Val Ala
   1665                1670                1675                1680
   Ala Pro Tyr Cys Tyr Thr Arg Asp Pro Gly Val Arg Trp Glu Tyr Cys
                   1685                1690                1695
   Asn Leu Thr Gln Cys Ser Asp Ala Glu Gly Thr Ala Val Ala Pro Pro
               1700                1705                1710
   Thr Val Thr Pro Val Pro Ser Leu Glu Ala Pro Ser Glu Gln Ala Pro
           1715                1720                1725
   Thr Glu Gln Arg Pro Gly Val Gln Glu Cys Tyr His Gly Asn Gly Gln
       1730                1735                1740
   Ser Tyr Arg Gly Thr Tyr Ser Thr Thr Val Thr Gly Arg Thr Cys Gln
   1745                1750                1755                1760
   Ala Trp Ser Ser Met Thr Pro His Ser His Ser Arg Thr Pro Glu Tyr
                   1765                1770                1775
   Tyr Pro Asn Ala Gly Leu Ile Met Asn Tyr Cys Arg Asn Pro Asp Ala
               1780                1785                1790
   Val Ala Ala Pro Tyr Cys Tyr Thr Arg Asp Pro Gly Val Arg Trp Glu
           1795                1800                1805
   Tyr Cys Asn Leu Thr Gln Cys Ser Asp Ala Glu Gly Thr Ala Val Ala
       1810                1815                1820
   Pro Pro Thr Val Thr Pro Val Pro Ser Leu Glu Ala Pro Ser Glu Gln
   1825                1830                1835                1840
   Ala Pro Thr Glu Gln Arg Pro Gly Val Gln Glu Cys Tyr His Gly Asn
                   1845                1850                1855
   Gly Gln Ser Tyr Arg Gly Thr Tyr Ser Thr Thr Val Thr Gly Arg Thr
               1860                1865                1870
   Cys Gln Ala Trp Ser Ser Met Thr Pro His Ser His Ser Arg Thr Pro
           1875                1880                1885
   Glu Tyr Tyr Pro Asn Ala Gly Leu Ile Met Asn Tyr Cys Arg Asn Pro
       1890                1895                1900
   Asp Ala Val Ala Ala Pro Tyr Cys Tyr Thr Arg Asp Pro Gly Val Arg
   1905                1910                1915                1920
   Trp Glu Tyr Cys Asn Leu Thr Gln Cys Ser Asp Ala Glu Gly Thr Ala
                   1925                1930                1935
   Val Ala Pro Pro Thr Val Thr Pro Val Pro Ser Leu Glu Ala Pro Ser
               1940                1945                1950
   Glu Gln Ala Pro Thr Glu Gln Arg Pro Gly Val Gln Glu Cys Tyr His
           1955                1960                1965
   Gly Asn Gly Gln Ser Tyr Arg Gly Thr Tyr Ser Thr Thr Val Thr Gly
       1970                1975                1980
   Arg Thr Cys Gln Ala Trp Ser Ser Met Thr Pro His Ser His Ser Arg
   1985                1990                1995                2000
   Thr Pro Glu Tyr Tyr Pro Asn Ala Gly Leu Ile Met Asn Tyr Cys Arg
                   2005                2010                2015
   Asn Pro Asp Ala Val Ala Ala Pro Tyr Cys Tyr Thr Arg Asp Pro Gly
               2020                2025                2030
   Val Arg Trp Glu Tyr Cys Asn Leu Thr Gln Cys Ser Asp Ala Glu Gly
           2035                2040                2045
   Thr Ala Val Ala Pro Pro Thr Val Thr Pro Val Pro Ser Leu Glu Ala
       2050                2055                2060
   Pro Ser Glu Gln Ala Pro Thr Glu Gln Arg Pro Gly Val Gln Glu Cys
   2065                2070                2075                2080
   Tyr His Gly Asn Gly Gln Ser Tyr Arg Gly Thr Tyr Ser Thr Thr Val
                   2085                2090                2095
   Thr Gly Arg Thr Cys Gln Ala Trp Ser Ser Met Thr Pro His Ser His
               2100                2105                2110
   Ser Arg Thr Pro Glu Tyr Tyr Pro Asn Ala Gly Leu Ile Met Asn Tyr
           2115                2120                2125
   Cys Arg Asn Pro Asp Ala Val Ala Ala Pro Tyr Cys Tyr Thr Arg Asp
       2130                2135                2140
   Pro Gly Val Arg Trp Glu Tyr Cys Asn Leu Thr Gln Cys Ser Asp Ala
   2145                2150                2155                2160
   Glu Gly Thr Ala Val Ala Pro Pro Thr Val Thr Pro Val Pro Ser Leu
                   2165                2170                2175
   Glu Ala Pro Ser Glu Gln Ala Pro Thr Glu Gln Arg Pro Gly Val Gln
               2180                2185                2190
   Glu Cys Tyr His Gly Asn Gly Gln Ser Tyr Arg Gly Thr Tyr Ser Thr
           2195                2200                2205
   Thr Val Thr Gly Arg Thr Cys Gln Ala Trp Ser Ser Met Thr Pro His
       2210                2215                2220
   Ser His Ser Arg Thr Pro Glu Tyr Tyr Pro Asn Ala Gly Leu Ile Met
   2225                2230                2235                2240
   Asn Tyr Cys Arg Asn Pro Asp Ala Val Ala Ala Pro Tyr Cys Tyr Thr
                   2245                2250                2255
   Arg Asp Pro Gly Val Arg Trp Glu Tyr Cys Asn Leu Thr Gln Cys Ser
               2260                2265                2270
   Asp Ala Glu Gly Thr Ala Val Ala Pro Pro Thr Val Thr Pro Val Pro
           2275                2280                2285
   Ser Leu Glu Ala Pro Ser Glu Gln Ala Pro Thr Glu Gln Arg Pro Gly
       2290                2295                2300
   Val Gln Glu Cys Tyr His Gly Asn Gly Gln Ser Tyr Arg Gly Thr Tyr
   2305                2310                2315                2320
   Ser Thr Thr Val Thr Gly Arg Thr Cys Gln Ala Trp Ser Ser Met Thr
                   2325                2330                2335
   Pro His Ser His Ser Arg Thr Pro Glu Tyr Tyr Pro Asn Ala Gly Leu
               2340                2345                2350
   Ile Met Asn Tyr Cys Arg Asn Pro Asp Ala Val Ala Ala Pro Tyr Cys
           2355                2360                2365
   Tyr Thr Arg Asp Pro Gly Val Arg Trp Glu Tyr Cys Asn Leu Thr Gln
       2370                2375                2380
   Cys Ser Asp Ala Glu Gly Thr Ala Val Ala Pro Pro Thr Val Thr Pro
   2385                2390                2395                2400
   Val Pro Ser Leu Glu Ala Pro Ser Glu Gln Ala Pro Thr Glu Gln Arg
                   2405                2410                2415
   Pro Gly Val Gln Glu Cys Tyr His Gly Asn Gly Gln Ser Tyr Arg Gly
               2420                2425                2430
   Thr Tyr Ser Thr Thr Val Thr Gly Arg Thr Cys Gln Ala Trp Ser Ser
           2435                2440                2445
   Met Thr Pro His Ser His Ser Arg Thr Pro Glu Tyr Tyr Pro Asn Ala
       2450                2455                2460
   Gly Leu Ile Met Asn Tyr Cys Arg Asn Pro Asp Ala Val Ala Ala Pro
   2465                2470                2475                2480
   Tyr Cys Tyr Thr Arg Asp Pro Gly Val Arg Trp Glu Tyr Cys Asn Leu
   2485 2490 2495
   Thr Gln Cys Ser Asp Ala Glu Gly Thr Ala Val Ala Pro Pro Thr Val
   2500 2505 2510
   Thr Pro Val Pro Ser Leu Glu Ala Pro Ser Glu Gln Ala Pro Thr Glu
   2515 2520 2525
   Gln Arg Pro Gly Val Gln Glu Cys Tyr His Gly Asn Gly Gln Ser Tyr
   2530 2535 2540
   Arg Gly Thr Tyr Ser Thr Thr Val Thr Gly Arg Thr Cys Gln Ala Trp
   2545 2550 2555 2560
   Ser Ser Met Thr Pro His Ser His Ser Arg Thr Pro Glu Tyr Tyr Pro
   2565 2570 2575
   Asn Ala Gly Leu Ile Met Asn Tyr Cys Arg Asn Pro Asp Ala Val Ala
   2580 2585 2590
   Ala Pro Tyr Cys Tyr Thr Arg Asp Pro Gly Val Arg Trp Glu Tyr Cys
   2595 2600 2605
   Asn Leu Thr Gln Cys Ser Asp Ala Glu Gly Thr Ala Val Ala Pro Pro
   2610 2615 2620
   Thr Val Thr Pro Val Pro Ser Leu Glu Ala Pro Ser Glu Gln Ala Pro
   2625 2630 2635 2640
   Thr Glu Gln Arg Pro Gly Val Gln Glu Cys Tyr His Gly Asn Gly Gln
   2645 2650 2655
   Ser Tyr Arg Gly Thr Tyr Ser Thr Thr Val Thr Gly Arg Thr Cys Gln
   2660 2665 2670
   Ala Trp Ser Ser Met Thr Pro His Ser His Ser Arg Thr Pro Glu Tyr
   2675 2680 2685
   Tyr Pro Asn Ala Gly Leu Ile Met Asn Tyr Cys Arg Asn Pro Asp Ala
   2690 2695 2700
   Val Ala Ala Pro Tyr Cys Tyr Thr Arg Asp Pro Gly Val Arg Trp Glu
   2705 2710 2715 2720
   Tyr Cys Asn Leu Thr Gln Cys Ser Asp Ala Glu Gly Thr Ala Val Ala
   2725 2730 2735
   Pro Pro Thr Val Thr Pro Val Pro Ser Leu Glu Ala Pro Ser Glu Gln
   2740 2745 2750
   Ala Pro Thr Glu Gln Arg Pro Gly Val Gln Glu Cys Tyr His Gly Asn
   2755 2760 2765
   Gly Gln Ser Tyr Arg Gly Thr Tyr Ser Thr Thr Val Thr Gly Arg Thr
   2770 2775 2780
   Cys Gln Ala Trp Ser Ser Met Thr Pro His Ser His Ser Arg Thr Pro
   2785 2790 2795 2800
   Glu Tyr Tyr Pro Asn Ala Gly Leu Ile Met Asn Tyr Cys Arg Asn Pro
   2805 2810 2815
   Asp Ala Val Ala Ala Pro Tyr Cys Tyr Thr Arg Asp Pro Gly Val Arg
   2820 2825 2830
   Trp Glu Tyr Cys Asn Leu Thr Gln Cys Ser Asp Ala Glu Gly Thr Ala
   2835 2840 2845
   Val Ala Pro Pro Thr Val Thr Pro Val Pro Ser Leu Glu Ala Pro Ser
   2850 2855 2860
   Glu Gln Ala Pro Thr Glu Gln Arg Pro Gly Val Gln Glu Cys Tyr His
   2865 2870 2875 2880
   Gly Asn Gly Gln Ser Tyr Arg Gly Thr Tyr Ser Thr Thr Val Thr Gly
   2885 2890 2895
   Arg Thr Cys Gln Ala Trp Ser Ser Met Thr Pro His Ser His Ser Arg
   2900 2905 2910
   Thr Pro Glu Tyr Tyr Pro Asn Ala Gly Leu Ile Met Asn Tyr Cys Arg
   2915 2920 2925
   Asn Pro Asp Ala Val Ala Ala Pro Tyr Cys Tyr Thr Arg Asp Pro Gly
   2930 2935 2940
   Val Arg Trp Glu Tyr Cys Asn Leu Thr Gln Cys Ser Asp Ala Glu Gly
   2945 2950 2955 2960
   Thr Ala Val Ala Pro Pro Thr Val Thr Pro Val Pro Ser Leu Glu Ala
   2965 2970 2975
   Pro Ser Glu Gln Ala Pro Thr Glu Gln Arg Pro Gly Val Gln Glu Cys
                   2980                2985                2990
   Tyr His Gly Asn Gly Gln Ser Tyr Arg Gly Thr Tyr Ser Thr Thr Val
               2995                3000                3005
   Thr Gly Arg Thr Cys Gln Ala Trp Ser Ser Met Thr Pro His Ser His
           3010                3015                3020
   Ser Arg Thr Pro Glu Tyr Tyr Pro Asn Ala Gly Leu Ile Met Asn Tyr
   3025                3030                3035                3040
   Cys Arg Asn Pro Asp Ala Val Ala Ala Pro Tyr Cys Tyr Thr Arg Asp
                       3045                3050                3055
   Pro Gly Val Arg Trp Glu Tyr Cys Asn Leu Thr Gln Cys Ser Asp Ala
                   3060                3065                3070
   Glu Gly Thr Ala Val Ala Pro Pro Thr Val Thr Pro Val Pro Ser Leu
               3075                3080                3085
   Glu Ala Pro Ser Glu Gln Ala Pro Thr Glu Gln Arg Pro Gly Val Gln
           3090                3095                3100
   Glu Cys Tyr His Gly Asn Gly Gln Ser Tyr Arg Gly Thr Tyr Ser Thr
   3105                3110                3115                3120
   Thr Val Thr Gly Arg Thr Cys Gln Ala Trp Ser Ser Met Thr Pro His
                       3125                3130                3135
   Ser His Ser Arg Thr Pro Glu Tyr Tyr Pro Asn Ala Gly Leu Ile Met
                   3140                3145                3150
   Asn Tyr Cys Arg Asn Pro Asp Ala Val Ala Ala Pro Tyr Cys Tyr Thr
               3155                3160                3165
   Arg Asp Pro Gly Val Arg Trp Glu Tyr Cys Asn Leu Thr Gln Cys Ser
           3170                3175                3180
   Asp Ala Glu Gly Thr Ala Val Ala Pro Pro Thr Val Thr Pro Val Pro
   3185                3190                3195                3200
   Ser Leu Glu Ala Pro Ser Glu Gln Ala Pro Thr Glu Gln Arg Pro Gly
                       3205                3210                3215
   Val Gln Glu Cys Tyr His Gly Asn Gly Gln Ser Tyr Arg Gly Thr Tyr
                   3220                3225                3230
   Ser Thr Thr Val Thr Gly Arg Thr Cys Gln Ala Trp Ser Ser Met Thr
               3235                3240                3245
   Pro His Ser His Ser Arg Thr Pro Glu Tyr Tyr Pro Asn Ala Gly Leu
           3250                3255                3260
   Ile Met Asn Tyr Cys Arg Asn Pro Asp Ala Val Ala Ala Pro Tyr Cys
   3265                3270                3275                3280
   Tyr Thr Arg Asp Pro Gly Val Arg Trp Glu Tyr Cys Asn Leu Thr Gln
                       3285                3290                3295
   Cys Ser Asp Ala Glu Gly Thr Ala Val Ala Pro Pro Thr Val Thr Pro
                   3300                3305                3310
   Val Pro Ser Leu Glu Ala Pro Ser Glu Gln Ala Pro Thr Glu Gln Arg
               3315                3320                3325
   Pro Gly Val Gln Glu Cys Tyr His Gly Asn Gly Gln Ser Tyr Arg Gly
           3330                3335                3340
   Thr Tyr Ser Thr Thr Val Thr Gly Arg Thr Cys Gln Ala Trp Ser Ser
   3345                3350                3355                3360
   Met Thr Pro His Ser His Ser Arg Thr Pro Glu Tyr Tyr Pro Asn Ala
                       3365                3370                3375
   Gly Leu Ile Met Asn Tyr Cys Arg Asn Pro Asp Pro Val Ala Ala Pro
                   3380                3385                3390
   Tyr Cys Tyr Thr Arg Asp Pro Ser Val Arg Trp Glu Tyr Cys Asn Leu
               3395                3400                3405
   Thr Gln Cys Ser Asp Ala Glu Gly Thr Ala Val Ala Pro Pro Thr Ile
           3410                3415                3420
   Thr Pro Ile Pro Ser Leu Glu Ala Pro Ser Glu Gln Ala Pro Thr Glu
   3425                3430                3435                3440
   Gln Arg Pro Gly Val Gln Glu Cys Tyr His Gly Asn Gly Gln Ser Tyr
                       3445                3450                3455
   Gln Gly Thr Tyr Phe Ile Thr Val Thr Gly Arg Thr Cys Gln Ala Trp
                   3460                3465                3470
   Ser Ser Met Thr Pro His Ser His Ser Arg Thr Pro Ala Tyr Tyr Pro
               3475                3480                3485
   Asn Ala Gly Leu Ile Lys Asn Tyr Cys Arg Asn Pro Asp Pro Val Ala
       3490                3495                3500
   Ala Pro Trp Cys Tyr Thr Thr Asp Pro Ser Val Arg Trp Glu Tyr Cys
   3505                3510                3515                3520
   Asn Leu Thr Arg Cys Ser Asp Ala Glu Trp Thr Ala Phe Val Pro Pro
                   3525                3530                3535
   Asn Val Ile Leu Ala Pro Ser Leu Glu Ala Phe Phe Glu Gln Ala Leu
               3540                3545                3550
   Thr Glu Glu Thr Pro Gly Val Gln Asp Cys Tyr Tyr His Tyr Gly Gln
           3555                3560                3565
   Ser Tyr Arg Gly Thr Tyr Ser Thr Thr Val Thr Gly Arg Thr Cys Gln
       3570                3575                3580
   Ala Trp Ser Ser Met Thr Pro His Gln His Ser Arg Thr Pro Glu Asn
   3585                3590                3595                3600
   Tyr Pro Asn Ala Gly Leu Thr Arg Asn Tyr Cys Arg Asn Pro Asp Ala
                   3605                3610                3615
   Glu Ile Arg Pro Trp Cys Tyr Thr Met Asp Pro Ser Val Arg Trp Glu
               3620                3625                3630
   Tyr Cys Asn Leu Thr Gln Cys Leu Val Thr Glu Ser Ser Val Leu Ala
           3635                3640                3645
   Thr Leu Thr Val Val Pro Asp Pro Ser Thr Glu Ala Ser Ser Glu Glu
       3650                3655                3660
   Ala Pro Thr Glu Gln Ser Pro Gly Val Gln Asp Cys Tyr His Gly Asp
   3665                3670                3675                3680
   Gly Gln Ser Tyr Arg Gly Ser Phe Ser Thr Thr Val Thr Gly Arg Thr
                   3685                3690                3695
   Cys Gln Ser Trp Ser Ser Met Thr Pro His Trp His Gln Arg Thr Thr
               3700                3705                3710
   Glu Tyr Tyr Pro Asn Gly Gly Leu Thr Arg Asn Tyr Cys Arg Asn Pro
           3715                3720                3725
   Asp Ala Glu Ile Ser Pro Trp Cys Tyr Thr Met Asp Pro Asn Val Arg
       3730                3735                3740
   Trp Glu Tyr Cys Asn Leu Thr Gln Cys Pro Val Thr Glu Ser Ser Val
   3745                3750                3755                3760
   Leu Ala Thr Ser Thr Ala Val Ser Glu Gln Ala Pro Thr Glu Gln Ser
                   3765                3770                3775
   Pro Thr Val Gln Asp Cys Tyr His Gly Asp Gly Gln Ser Tyr Arg Gly
               3780                3785                3790
   Ser Phe Ser Thr Thr Val Thr Gly Arg Thr Cys Gln Ser Trp Ser Ser
           3795                3800                3805
   Met Thr Pro His Trp His Gln Arg Thr Thr Glu Tyr Tyr Pro Asn Gly
       3810                3815                3820
   Gly Leu Thr Arg Asn Tyr Cys Arg Asn Pro Asp Ala Glu Ile Arg Pro
   3825                3830                3835                3840
   Trp Cys Tyr Thr Met Asp Pro Ser Val Arg Trp Glu Tyr Cys Asn Leu
                   3845                3850                3855
   Thr Gln Cys Pro Val Met Glu Ser Thr Leu Leu Thr Thr Pro Thr Val
               3860                3865                3870
   Val Pro Val Pro Ser Thr Glu Leu Pro Ser Glu Glu Ala Pro Thr Glu
           3875                3880                3885
   Asn Ser Thr Gly Val Gln Asp Cys Tyr Arg Gly Asp Gly Gln Ser Tyr
       3890                3895                3900
   Arg Gly Thr Leu Ser Thr Thr Ile Thr Gly Arg Thr Cys Gln Ser Trp
   3905                3910                3915                3920
   Ser Ser Met Thr Pro His Trp His Arg Arg Ile Pro Leu Tyr Tyr Pro
                   3925                3930                3935
   Asn Ala Gly Leu Thr Arg Asn Tyr Cys Arg Asn Pro Asp Ala Glu Ile
               3940                3945                3950
   Arg Pro Trp Cys Tyr Thr Met Asp Pro Ser Val Arg Trp Glu Tyr Cys
           3955                3960                3965
   Asn Leu Thr Arg Cys Pro Val Thr Glu Ser Ser Val Leu Thr Thr Pro
       3970                3975                3980
   Thr Val Ala Pro Val Pro Ser Thr Glu Ala Pro Ser Glu Gln Ala Pro
   3985                3990                3995                4000
   Pro Glu Lys Ser Pro Val Val Gln Asp Cys Tyr His Gly Asp Gly Arg
                   4005                4010                4015
   Ser Tyr Arg Gly Ile Ser Ser Thr Thr Val Thr Gly Arg Thr Cys Gln
               4020                4025                4030
   Ser Trp Ser Ser Met Ile Pro His Trp His Gln Arg Thr Pro Glu Asn
           4035                4040                4045
   Tyr Pro Asn Ala Gly Leu Thr Glu Asn Tyr Cys Arg Asn Pro Asp Ser
       4050                4055                4060
   Gly Lys Gln Pro Trp Cys Tyr Thr Thr Asp Pro Cys Val Arg Trp Glu
   4065                4070                4075                4080
   Tyr Cys Asn Leu Thr Gln Cys Ser Glu Thr Glu Ser Gly Val Leu Glu
                   4085                4090                4095
   Thr Pro Thr Val Val Pro Val Pro Ser Met Glu Ala His Ser Glu Ala
               4100                4105                4110
   Ala Pro Thr Glu Gln Thr Pro Val Val Arg Gln Cys Tyr His Gly Asn
           4115                4120                4125
   Gly Gln Ser Tyr Arg Gly Thr Phe Ser Thr Thr Val Thr Gly Arg Thr
       4130                4135                4140
   Cys Gln Ser Trp Ser Ser Met Thr Pro His Arg His Gln Arg Thr Pro
   4145                4150                4155                4160
   Glu Asn Tyr Pro Asn Asp Gly Leu Thr Met Asn Tyr Cys Arg Asn Pro
                   4165                4170                4175
   Asp Ala Asp Thr Gly Pro Trp Cys Phe Thr Met Asp Pro Ser Ile Arg
               4180                4185                4190
   Trp Glu Tyr Cys Asn Leu Thr Arg Cys Ser Asp Thr Glu Gly Thr Val
           4195                4200                4205
   Val Ala Pro Pro Thr Val Ile Gln Val Pro Ser Leu Gly Pro Pro Ser
       4210                4215                4220
   Glu Gln Asp Cys Met Phe Gly Asn Gly Lys Gly Tyr Arg Gly Lys Lys
   4225                4230                4235                4240
   Ala Thr Thr Val Thr Gly Thr Pro Cys Gln Glu Trp Ala Ala Gln Glu
                   4245                4250                4255
   Pro His Arg His Ser Thr Phe Ile Pro Gly Thr Asn Lys Trp Ala Gly
               4260                4265                4270
   Leu Glu Lys Asn Tyr Cys Arg Asn Pro Asp Gly Asp Ile Asn Gly Pro
           4275                4280                4285
   Trp Cys Tyr Thr Met Asn Pro Arg Lys Leu Phe Asp Tyr Cys Asp Ile
       4290                4295                4300
   Pro Leu Cys Ala Ser Ser Ser Phe Asp Cys Gly Lys Pro Gln Val Glu
   4305                4310                4315                4320
   Pro Lys Lys Cys Pro Gly Ser Ile Val Gly Gly Cys Val Ala His Pro
                   4325                4330                4335
   His Ser Trp Pro Trp Gln Val Ser Leu Arg Thr Arg Phe Gly Lys His
               4340                4345                4350
   Phe Cys Gly Gly Thr Leu Ile Ser Pro Glu Trp Val Leu Thr Ala Ala
           4355                4360                4365
   His Cys Leu Lys Lys Ser Ser Arg Pro Ser Ser Tyr Lys Val Ile Leu
       4370                4375                4380
   Gly Ala His Gln Glu Val Asn Leu Glu Ser His Val Gln Glu Ile Glu
   4385                4390                4395                4400
   Val Ser Arg Leu Phe Leu Glu Pro Thr Gln Ala Asp Ile Ala Leu Leu
                   4405                4410                4415
   Lys Leu Ser Arg Pro Ala Val Ile Thr Asp Lys Val Met Pro Ala Cys
               4420                4425                4430
   Leu Pro Ser Pro Asp Tyr Met Val Thr Ala Arg Thr Glu Cys Tyr Ile
           4435                4440                4445
   Thr Gly Trp Gly Glu Thr Gln Gly Thr Phe Gly Thr Gly Leu Leu Lys
       4450                4455                4460
   Glu Ala Gln Leu Leu Val Ile Glu Asn Glu Val Cys Asn His Tyr Lys
   4465                4470                4475                4480
   Tyr Ile Cys Ala Glu His Leu Ala Arg Gly Thr Asp Ser Cys Gln Gly
                   4485                4490                4495
   Asp Ser Gly Gly Pro Leu Val Cys Phe Glu Lys Asp Lys Tyr Ile Leu
   4500 4505 4510
   Gln Gly Val Thr Ser Trp Gly Leu Gly Cys Ala Arg Pro Asn Lys Pro
   4515 4520 4525
   Gly Val Tyr Ala Arg Val Ser Arg Phe Val Thr Trp Ile Glu Gly Met
   4530 4535 4540
   Met Arg Asn Asn
   4545
<210> 71
   <211> 133
   <212> PRT
   <213> Homo sapiens
<400> 71
   Leu Glu Ser Gly Gly Gly Val Val Gln Pro Gly Lys Ser Leu Arg Leu
   1 5 10 15
   Ser Cys Ala Ala Ser Gly Phe Thr Phe Arg Asn Tyr Gly Met His Trp
   20 25 30
   Val Arg Gln Ala Pro Gly Lys Gly Leu Glu Trp Val Ala Gly Ile Ser
   35 40 45
   Ser Asp Gly Arg Lys Lys Lys Tyr Val Asp Ser Val Lys Gly Arg Phe
   50 55 60
   Phe Ile Ser Arg Asp Asn Ser Lys Asn Thr Leu Tyr Leu Gln Leu Asn
   65 70 75 80
   Ser Leu Arg Ala Glu Asp Thr Ala Val Tyr Tyr Cys Ala Lys Asp Glu
   85 90 95
   Phe Ser Ser Thr Arg Lys Asn Phe Leu Thr Gly Gln Ser Lys Thr Phe
   100 105 110
   Ala Ala Tyr Tyr Gly Met Asp Val Trp Gly Gln Gly Thr Thr Val Thr
   115 120 125
   Val Ser Ser Ala Ser
   130
<210> 72
   <211> 201
   <212> PRT
   <213> Homo sapiens
<400> 72
   Met Ala Leu Ser Trp Val Leu Thr Val Leu Ser Leu Leu Pro Leu Leu
    1              5                   10                  15
   Glu Ala Gln Ile Pro Leu Cys Ala Asn Leu Val Pro Val Pro Ile Thr
               20                  25                  30
   Asn Ala Thr Leu Asp Gln Ile Thr Gly Lys Trp Phe Tyr Ile Ala Ser
           35                  40                  45
   Ala Phe Arg Asn Glu Glu Tyr Asn Lys Ser Val Gln Glu Ile Gln Ala
       50                  55                  60
   Thr Phe Phe Tyr Phe Thr Pro Asn Lys Thr Glu Asp Thr Ile Phe Leu
   65                  70                  75                  80
   Arg Glu Tyr Gln Thr Arg Gln Asp Gln Cys Ile Tyr Asn Thr Thr Tyr
                   85                  90                  95
   Leu Asn Val Gln Arg Glu Asn Gly Thr Ile Ser Arg Tyr Val Gly Gly
               100                 105                 110
   Gln Glu His Phe Ala His Leu Leu Ile Leu Arg Asp Thr Lys Thr Tyr
           115                 120                 125
   Met Leu Ala Phe Asp Val Asn Asp Glu Lys Asn Trp Gly Leu Ser Val
       130                 135                 140
   Tyr Ala Asp Lys Pro Glu Thr Thr Lys Glu Gln Leu Gly Glu Phe Tyr
   145                 150                 155                 160
   Glu Ala Leu Asp Cys Leu Arg Ile Pro Lys Ser Asp Val Val Tyr Thr
                   165                 170                 175
   Asp Trp Lys Lys Asp Lys Cys Glu Pro Leu Glu Lys Gln His Glu Lys
               180                 185                 190
   Glu Arg Lys Gln Glu Glu Gly Glu Ser
           195                 200
<210> 73
   <211> 201
   <212> PRT
   <213> Homo sapiens
<400> 73
   Met Ala Leu Ser Trp Val Leu Thr Val Leu Ser Leu Leu Pro Leu Leu
   1               5                   10                  15
   Glu Ala Gln Ile Pro Leu Cys Ala Asn Leu Val Pro Val Pro Ile Thr
               20                  25                  30
   Asn Ala Thr Leu Asp Arg Ile Thr Gly Lys Trp Phe Tyr Ile Ala Ser
           35                  40                  45
   Ala Phe Arg Asn Glu Glu Tyr Asn Lys Ser Val Gln Glu Ile Gln Ala
       50                  55                  60
   Thr Phe Phe Tyr Phe Thr Pro Asn Lys Thr Glu Asp Thr Ile Phe Leu
   65                  70                  75                  80
   Arg Glu Tyr Gln Thr Arg Gln Asn Gln Cys Phe Tyr Asn Ser Ser Tyr
                   85                  90                  95
   Leu Asn Val Gln Arg Glu Asn Gly Thr Val Ser Arg Tyr Glu Gly Gly
               100                 105                 110
   Arg Glu His Val Ala His Leu Leu Phe Leu Arg Asp Thr Lys Thr Leu
           115                 120                 125
   Met Phe Gly Ser Tyr Leu Asp Asp Glu Lys Asn Trp Gly Leu Ser Phe
       130                 135                 140
   Tyr Ala Asp Lys Pro Glu Thr Thr Lys Glu Gln Leu Gly Glu Phe Tyr
   145                 150                 155                 160
   Glu Ala Leu Asp Cys Leu Cys Ile Pro Arg Ser Asp Val Met Tyr Thr
                   165                 170                 175
   Asp Trp Lys Lys Asp Lys Cys Glu Pro Leu Glu Lys Gln His Glu Lys
               180                 185                 190
   Glu Arg Lys Gln Glu Glu Gly Glu Ser
           195                 200
<210> 74
   <211> 101
   <212> PRT
   <213> Homo sapiens
<400> 74
   Met Ser Ser Ala Ala Gly Phe Cys Ala Ser Arg Pro Gly Leu Leu Phe
   1               5                   10                  15
   Leu Gly Leu Leu Leu Leu Pro Leu Val Val Ala Phe Ala Ser Ala Glu
               20                  25                  30
   Ala Glu Glu Asp Gly Asp Leu Gln Cys Leu Cys Val Lys Thr Thr Ser
           35                  40                  45
   Gln Val Arg Pro Arg His Ile Thr Ser Leu Glu Val Ile Lys Ala Gly
       50                  55                  60
   Pro His Cys Pro Thr Ala Gln Leu Ile Ala Thr Leu Lys Asn Gly Arg
   65                  70                  75                  80
   Lys Ile Cys Leu Asp Leu Gln Ala Pro Leu Tyr Lys Lys Ile Ile Lys
                   85                  90                  95
   Lys Leu Leu Glu Ser
               100
<210> 75
   <211> 1482
   <212> PRT
   <213> Homo sapiens
<400> 75
   Met Arg Lys Asp Arg Leu Leu His Leu Cys Leu Val Leu Leu Leu Ile
   1               5                   10                  15
   Leu Leu Ser Ala Ser Asp Ser Asn Ser Thr Glu Pro Gln Tyr Met Val
               20                  25                  30
   Leu Val Pro Ser Leu Leu His Thr Glu Ala Pro Lys Lys Gly Cys Val
           35                  40                  45
   Leu Leu Ser His Leu Asn Glu Thr Val Thr Val Ser Ala Ser Leu Glu
       50                  55                  60
   Ser Gly Arg Glu Asn Arg Ser Leu Phe Thr Asp Leu Val Ala Glu Lys
   65                  70                  75                  80
   Asp Leu Phe His Cys Val Ser Phe Thr Leu Pro Arg Ile Ser Ala Ser
                   85                  90                  95
   Ser Glu Val Ala Phe Leu Ser Ile Gln Ile Lys Gly Pro Thr Gln Asp
               100                 105                 110
   Phe Arg Lys Arg Asn Thr Val Leu Val Leu Asn Thr Gln Ser Leu Val
           115                 120                 125
   Phe Val Gln Thr Asp Lys Pro Met Tyr Lys Pro Gly Gln Thr Val Arg
       130                 135                 140
   Phe Arg Val Val Ser Val Asp Glu Asn Phe Arg Pro Arg Asn Glu Leu
   145                 150                 155                 160
   Ile Pro Leu Ile Tyr Leu Glu Asn Pro Arg Arg Asn Arg Ile Ala Gln
                   165                 170                 175
   Trp Gln Ser Leu Lys Leu Glu Ala Gly Ile Asn Gln Leu Ser Phe Pro
               180                 185                 190
   Leu Ser Ser Glu Pro Ile Gln Gly Ser Tyr Arg Val Val Val Gln Thr
           195                 200                 205
   Glu Ser Gly Gly Arg Ile Gln His Pro Phe Thr Val Glu Glu Phe Val
       210                 215                 220
   Leu Pro Lys Phe Glu Val Lys Val Gln Val Pro Lys Ile Ile Ser Ile
   225                 230                 235                 240
   Met Asp Glu Lys Val Asn Ile Thr Val Cys Gly Glu Tyr Thr Tyr Gly
                   245                 250                 255
   Lys Pro Val Pro Gly Leu Ala Thr Val Ser Leu Cys Arg Lys Leu Ser
               260                 265                 270
   Arg Val Leu Asn Cys Asp Lys Gln Glu Val Cys Glu Glu Phe Ser Gln
           275                 280                 285
   Gln Leu Asn Ser Asn Gly Cys Ile Thr Gln Gln Val His Thr Lys Met
       290                 295                 300
   Leu Gln Ile Thr Asn Thr Gly Phe Glu Met Lys Leu Arg Val Glu Ala
   305                 310                 315                 320
   Arg Ile Arg Glu Glu Gly Thr Asp Leu Glu Val Thr Ala Asn Arg Ile
                   325                 330                 335
   Ser Glu Ile Thr Asn Ile Val Ser Lys Leu Lys Phe Val Lys Val Asp
               340                 345                 350
   Ser His Phe Arg Gln Gly Ile Pro Phe Phe Ala Gln Val Leu Leu Val
           355                 360                 365
   Asp Gly Lys Gly Val Pro Ile Pro Asn Lys Leu Phe Phe Ile Ser Val
       370                 375                 380
   Asn Asp Ala Asn Tyr Tyr Ser Asn Ala Thr Thr Asn Glu Gln Gly Leu
   385                 390                 395                 400
   Ala Gln Phe Ser Ile Asn Thr Thr Ser Ile Ser Val Asn Lys Leu Phe
                   405                 410                 415
   Val Arg Val Phe Thr Val His Pro Asn Leu Cys Phe His Tyr Ser Trp
               420                 425                 430
   Val Ala Glu Asp His Gln Gly Ala Gln His Thr Ala Asn Arg Val Phe
           435                 440                 445
   Ser Leu Ser Gly Ser Tyr Ile His Leu Glu Pro Val Ala Gly Thr Leu
       450                 455                 460
   Pro Cys Gly His Thr Glu Thr Ile Thr Ala His Tyr Thr Leu Asn Arg
   465                 470                 475                 480
   Gln Ala Met Gly Glu Leu Ser Glu Leu Ser Phe His Tyr Leu Ile Met
                   485                 490                 495
   Ala Lys Gly Val Ile Val Arg Ser Gly Thr His Thr Leu Pro Val Glu
               500                 505                 510
   Ser Gly Asp Met Lys Gly Ser Phe Ala Leu Ser Phe Pro Val Glu Ser
           515                 520                 525
   Asp Val Ala Pro Ile Ala Arg Met Phe Ile Phe Ala Ile Leu Pro Asp
       530                 535                 540
   Gly Glu Val Val Gly Asp Ser Glu Lys Phe Glu Ile Glu Asn Cys Leu
   545                 550                 555                 560
   Ala Asn Lys Val Asp Leu Ser Phe Ser Pro Ala Gln Ser Pro Pro Ala
                   565                 570                 575
   Ser His Ala His Leu Gln Val Ala Ala Ala Pro Gln Ser Leu Cys Ala
               580                 585                 590
   Leu Arg Ala Val Asp Gln Ser Val Leu Leu Met Lys Pro Glu Ala Glu
           595                 600                 605
   Leu Ser Val Ser Ser Val Tyr Asn Leu Leu Thr Val Lys Asp Leu Thr
       610                 615                 620
   Asn Phe Pro Asp Asn Val Asp Gln Gln Glu Glu Glu Gln Gly His Cys
   625                 630                 635                 640
   Pro Arg Pro Phe Phe Ile His Asn Gly Ala Ile Tyr Val Pro Leu Ser
                   645                 650                 655
   Ser Asn Glu Ala Asp Ile Tyr Ser Phe Leu Lys Gly Met Gly Leu Lys
               660                 665                 670
   Val Phe Thr Asn Ser Lys Ile Arg Lys Pro Lys Ser Cys Ser Val Ile
           675                 680                 685
   Pro Ser Val Ser Ala Gly Ala Val Gly Gln Gly Tyr Tyr Gly Ala Gly
       690                 695                 700
   Leu Gly Val Val Glu Arg Pro Tyr Val Pro Gln Leu Gly Thr Tyr Asn
   705                 710                 715                 720
   Val Ile Pro Leu Asn Asn Glu Gln Ser Ser Gly Pro Val Pro Glu Thr
                   725                 730                 735
   Val Arg Ser Tyr Phe Pro Glu Thr Trp Ile Trp Glu Leu Val Ala Val
               740                 745                 750
   Asn Ser Ser Gly Val Ala Glu Val Gly Val Thr Val Pro Asp Thr Ile
           755                 760                 765
   Thr Glu Trp Lys Ala Gly Ala Phe Cys Leu Ser Glu Asp Ala Gly Leu
       770                 775                 780
   Gly Ile Ser Ser Thr Ala Ser Leu Arg Ala Phe Gln Pro Phe Phe Val
   785                 790                 795                 800
   Glu Leu Thr Met Pro Tyr Ser Val Ile Arg Gly Glu Val Phe Thr Leu
                   805                 810                 815
   Lys Ala Thr Val Leu Asn Tyr Leu Pro Lys Cys Ile Arg Val Ser Val
               820                 825                 830
   Gln Leu Lys Ala Ser Pro Ala Phe Leu Ala Ser Gln Asn Thr Lys Gly
           835                 840                 845
   Glu Glu Ser Tyr Cys Ile Cys Gly Asn Glu Arg Gln Thr Leu Ser Trp
       850                 855                 860
   Thr Val Thr Pro Lys Thr Leu Gly Asn Val Asn Phe Ser Val Ser Ala
   865                 870                 875                 880
   Glu Ala Met Gln Ser Leu Glu Leu Cys Gly Asn Glu Val Val Glu Val
                   885                 890                 895
   Pro Glu Ile Lys Arg Lys Asp Thr Val Ile Lys Thr Leu Leu Val Glu
               900                 905                 910
   Ala Glu Gly Ile Glu Gln Glu Lys Thr Phe Ser Ser Met Thr Cys Ala
           915                 920                 925
   Ser Gly Ala Asn Val Ser Glu Gln Leu Ser Leu Lys Leu Pro Ser Asn
       930                 935                 940
   Val Val Lys Glu Ser Ala Arg Ala Ser Phe Ser Val Leu Gly Asp Ile
   945                 950                 955                 960
   Leu Gly Ser Ala Met Gln Asn Ile Gln Asn Leu Leu Gln Met Pro Tyr
                   965                 970                 975
   Gly Cys Gly Glu Gln Asn Met Val Leu Phe Ala Pro Asn Ile Tyr Val
               980                 985                 990
   Leu Asn Tyr Leu Asn Glu Thr Gln Gln Leu Thr Gln Glu Ile Lys Ala
           995                 1000                1005
   Lys Ala Val Gly Tyr Leu Ile Thr Gly Tyr Gln Arg Gln Leu Asn Tyr
       1010                1015                1020
   Lys His Gln Asp Gly Ser Tyr Ser Thr Phe Gly Glu Arg Tyr Gly Arg
   1025                1030                1035                1040
   Asn Gln Gly Asn Thr Trp Leu Thr Ala Phe Val Leu Lys Thr Phe Ala
                   1045                1050                1055
   Gln Ala Arg Ser Tyr Ile Phe Ile Asp Glu Ala His Ile Thr Gln Ser
               1060                1065                1070
   Leu Thr Trp Leu Ser Gln Met Gln Lys Asp Asn Gly Cys Phe Arg Ser
           1075                1080                1085
   Ser Gly Ser Leu Leu Asn Asn Ala Ile Lys Gly Gly Val Glu Asp Glu
       1090                1095                1100
   Ala Thr Leu Ser Ala Tyr Val Thr Ile Ala Leu Leu Glu Ile Pro Leu
   1105                1110                1115                1120
   Pro Val Thr Asn Pro Ile Val Arg Asn Ala Leu Phe Cys Leu Glu Ser
                   1125                1130                1135
   Ala Trp Asn Val Ala Lys Glu Gly Thr His Gly Ser His Val Tyr Thr
               1140                1145                1150
   Lys Ala Leu Leu Ala Tyr Ala Phe Ser Leu Leu Gly Lys Gln Asn Gln
           1155                1160                1165
   Asn Arg Glu Ile Leu Asn Ser Leu Asp Lys Glu Ala Val Lys Glu Asp
       1170                1175                1180
   Asn Leu Val His Trp Glu Arg Pro Gln Arg Pro Lys Ala Pro Val Gly
   1185                1190                1195                1200
   His Leu Tyr Gln Thr Gln Ala Pro Ser Ala Glu Val Glu Met Thr Ser
                   1205                1210                1215
   Tyr Val Leu Leu Ala Tyr Leu Thr Ala Gln Pro Ala Pro Thr Ser Gly
               1220                1225                1230
   Asp Leu Thr Ser Ala Thr Asn Ile Val Lys Trp Ile Met Lys Gln Gln
           1235                1240                1245
   Asn Ala Gln Gly Gly Phe Ser Ser Thr Gln Asp Thr Val Val Ala Leu
       1250                1255                1260
   His Ala Leu Ser Arg Tyr Gly Ala Ala Thr Phe Thr Arg Thr Glu Lys
   1265                1270                1275                1280
   Thr Ala Gln Val Thr Val Gln Asp Ser Gln Thr Phe Ser Thr Asn Phe
                   1285                1290                1295
   Gln Val Asp Asn Asn Asn Leu Leu Leu Leu Gln Gln Ile Ser Leu Pro
               1300                1305                1310
   Glu Leu Pro Gly Glu Tyr Val Ile Thr Val Thr Gly Glu Arg Cys Val
           1315                1320                1325
   Tyr Leu Gln Thr Ser Met Lys Tyr Asn Ile Leu Pro Glu Lys Glu Asp
       1330                1335                1340
   Ser Pro Phe Ala Leu Lys Val Gln Thr Val Pro Gln Thr Cys Asp Gly
   1345                1350                1355                1360
   His Lys Ala His Thr Ser Phe Gln Ile Ser Leu Thr Ile Ser Tyr Thr
                   1365                1370                1375
   Gly Asn Arg Pro Ala Ser Asn Met Val Ile Val Asp Val Lys Met Val
               1380                1385                1390
   Ser Gly Phe Ile Pro Leu Lys Pro Thr Val Lys Met Leu Glu Arg Ser
           1395                1400                1405
   Ser Ser Val Ser Arg Thr Glu Val Ser Asn Asn His Val Leu Ile Tyr
       1410                1415                1420
   Val Glu Gln Val Thr Asn Gln Thr Leu Ser Phe Ser Phe Met Val Leu
   1425                1430                1435                1440
   Gln Asp Ile Pro Val Gly Asp Leu Lys Pro Ala Ile Val Lys Val Tyr
                   1445                1450                1455
   Asp Tyr Tyr Glu Thr Asp Glu Ser Val Val Ala Glu Tyr Ile Ala Pro
                   1460                1465                1470
   Cys Ser Thr Asp Thr Glu His Gly Asn Val
           1475                1480
<210> 76
   <211> 134
   <212> PRT
   <213> Homo sapiens
<400> 76
   Met Glu Thr Pro Ala Gln Leu Leu Phe Leu Leu Leu Val Trp Leu Pro
    1               5                  10                  15
   Asp Met Ser Glu Glu Ile Ile Leu Thr Gln Ser Pro Ala Thr Leu Ser
               20                  25                  30
   Val Ser Pro Gly Glu Arg Ala Thr Leu Ser Cys Arg Ala Ser Gln Ser
           35                  40                  45
   Val Ser Thr Asn Leu Ala Trp Tyr Gln Gln Lys Pro Gly Gln Ala Pro
       50                  55                  60
   Arg Leu Leu Ile Tyr Gly Ala Ser Ile Arg Ala Thr Gly Ile Ser Ala
   65                  70                  75                  80
   Arg Phe Ser Gly Ser Gly Ser Gly Thr Glu Phe Thr Leu Asn Ile Thr
                   85                  90                  95
   Ser Leu Gln Ser Gly Asp Leu Ala Leu Tyr Phe Cys Gln Gln Tyr Gly
               100                 105                 110
   Asp Trp Pro Pro Tyr Thr Phe Gly Gln Gly Thr Lys Leu Glu Ile Lys
           115                 120                 125
   Arg Thr Val Ala Ala Pro
       130
<210> 77
   <211> 93
   <212> PRT
   <213> Homo sapiens
<400> 77
   Met Leu Thr Glu Leu Glu Lys Ala Leu Asn Ser Ile Ile Asp Val Tyr
    1               5                  10                  15
   His Lys Tyr Ser Leu Ile Lys Gly Asn Phe His Ala Val Tyr Arg Asp
               20                  25                  30
   Asp Leu Lys Lys Leu Leu Glu Thr Glu Cys Pro Gln Tyr Ile Arg Lys
           35                  40                  45
   Lys Gly Ala Asp Val Trp Phe Lys Glu Leu Asp Ile Asn Thr Asp Gly
       50                  55                  60
   Ala Val Asn Phe Gln Glu Phe Leu Ile Leu Val Ile Lys Met Gly Val
   65                  70                  75                  80
   Ala Ala His Lys Lys Ser His Glu Glu Ser His Lys Glu
                   85                  90
<210> 78
   <211> 238
   <212> PRT
   <213> Homo sapiens
<400> 78
   Glu Val Gln Leu Leu Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Ala Met Ser Trp Val Arg Gln Ala Pro Gly Lys Gly Leu Glu Trp Val
           35                  40                  45
   Ser Ala Ile Ser Gly Ser Gly Gly Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Lys Ser Phe Ser Phe Phe Asp Tyr Trp Gly Gln Gly Thr Leu Val
               100                 105                 110
   Thr Val Ser Ser Gly Asp Gly Ser Ser Gly Gly Ser Gly Gly Ala Ser
           115                 120                 125
   Thr Gly Glu Ile Val Leu Thr Gln Ser Pro Gly Thr Leu Ser Leu Ser
       130                 135                 140
   Pro Gly Glu Arg Ala Thr Leu Ser Cys Arg Ala Ser Gln Ser Val Ser
   145                 150                 155                 160
   Ser Ser Tyr Leu Ala Trp Tyr Gln Gln Lys Pro Gly Gln Ala Pro Arg
                   165                 170                 175
   Leu Leu Ile Tyr Gly Ala Ser Ser Arg Ala Thr Gly Ile Pro Asp Arg
               180                 185                 190
   Phe Ser Gly Ser Gly Ser Gly Thr Asp Phe Thr Leu Thr Ile Ser Arg
           195                 200                 205
   Leu Glu Pro Glu Asp Phe Ala Val Tyr Tyr Cys Gln Gln Gly Gly Trp
       210                 215                 220
   Leu Pro Tyr Thr Phe Gly Gln Gly Thr Lys Val Glu Ile Lys
   225                 230                 235
<210> 79
   <211> 464
   <212> PRT
   <213> Homo sapiens
<400> 79
<210> 80
   <211> 491
   <212> PRT
   <213> Homo sapiens
<400> 80
   Met Ala Leu Leu Trp Gly Leu Leu Val Leu Ser Trp Ser Cys Leu Gln
   1               5                   10                  15
   Gly Pro Cys Ser Val Phe Ser Pro Val Ser Ala Met Glu Pro Leu Gly
               20                  25                  30
   Arg Gln Leu Thr Ser Gly Pro Asn Gln Glu Gln Val Ser Pro Leu Thr
           35                  40                  45
   Leu Leu Lys Leu Gly Asn Gln Glu Pro Gly Gly Gln Thr Ala Leu Lys
       50                  55                  60
   Ser Pro Pro Gly Val Cys Ser Arg Asp Pro Thr Pro Glu Gln Thr His
   65                  70                  75                  80
   Arg Leu Ala Arg Ala Met Met Ala Phe Thr Ala Asp Leu Phe Ser Leu
                   85                  90                  95
   Val Ala Gln Thr Ser Thr Cys Pro Asn Leu Ile Leu Ser Pro Leu Ser
               100                 105                 110
   Val Ala Leu Ala Leu Ser His Leu Ala Leu Gly Ala Gln Asn His Thr
           115                 120                 125
   Leu Gln Arg Leu Gln Gln Val Leu His Ala Gly Ser Gly Pro Cys Leu
       130                 135                 140
   Pro His Leu Leu Ser Arg Leu Cys Gln Asp Leu Gly Pro Gly Ala Phe
   145                 150                 155                 160
   Arg Leu Ala Ala Arg Met Tyr Leu Gln Lys Gly Phe Pro Ile Lys Glu
                   165                 170                 175
   Asp Phe Leu Glu Gln Ser Glu Gln Leu Phe Gly Ala Lys Pro Val Ser
               180                 185                 190
   Leu Thr Gly Lys Gln Glu Asp Asp Leu Ala Asn Ile Asn Gln Trp Val
           195                 200                 205
   Lys Glu Ala Thr Glu Gly Lys Ile Gln Glu Phe Leu Ser Gly Leu Pro
       210                 215                 220
   Glu Asp Thr Val Leu Leu Leu Leu Asn Ala Ile His Phe Gln Gly Phe
   225                 230                 235                 240
   Trp Arg Asn Lys Phe Asp Pro Ser Leu Thr Gln Arg Asp Ser Phe His
                   245                 250                 255
   Leu Asp Glu Gln Phe Thr Val Pro Val Glu Met Met Gln Ala Arg Thr
               260                 265                 270
   Tyr Pro Leu Arg Trp Phe Leu Leu Glu Gln Pro Glu Ile Gln Val Ala
           275                 280                 285
   His Phe Pro Phe Lys Asn Asn Met Ser Phe Val Val Leu Val Pro Thr
       290                 295                 300
   His Phe Glu Trp Asn Val Ser Gln Val Leu Ala Asn Leu Ser Trp Asp
   305                 310                 315                 320
   Thr Leu His Pro Pro Leu Val Trp Glu Arg Pro Thr Lys Val Arg Leu
                   325                 330                 335
   Pro Lys Leu Tyr Leu Lys His Gln Met Asp Leu Val Ala Thr Leu Ser
               340                 345                 350
   Gln Leu Gly Leu Gln Glu Leu Phe Gln Ala Pro Asp Leu Arg Gly Ile
           355                 360                 365
   Ser Glu Gln Ser Leu Val Val Ser Gly Val Gln His Gln Ser Thr Leu
       370                 375                 380
   Glu Leu Ser Glu Val Gly Val Glu Ala Ala Ala Ala Thr Ser Ile Ala
   385                 390                 395                 400
   Met Ser Arg Met Ser Leu Ser Ser Phe Ser Val Asn Arg Pro Phe Leu
                   405                 410                 415
   Phe Phe Ile Phe Glu Asp Thr Thr Gly Leu Pro Leu Phe Val Gly Ser
               420                 425                 430
   Val Arg Asn Pro Asn Pro Ser Ala Pro Arg Glu Leu Lys Glu Gln Gln
           435                 440                 445
   Asp Ser Pro Gly Asn Lys Asp Phe Leu Gln Ser Leu Lys Gly Phe Pro
       450                 455                 460
   Arg Gly Asp Lys Leu Phe Gly Pro Asp Leu Lys Leu Val Pro Pro Met
   465                 470                 475                 480
   Glu Glu Asp Tyr Pro Gln Phe Gly Ser Pro Lys
                   485                 490
<210> 81
   <211> 505
   <212> PRT
   <213> Homo sapiens
<400> 81
   Met Ala Ser Arg Leu Thr Leu Leu Thr Leu Leu Leu Leu Leu Leu Ala
    1               5                  10                  15
   Gly Phe Leu Glu Pro Gln Asp Arg Ala Ser Ser Asn Pro Asn Ala Thr
               20                  25                  30
   Ser Ser Ser Ser Gln Asp Pro Glu Ser Leu Gln Asp Arg Gly Glu Gly
           35                  40                  45
   Lys Val Ala Thr Thr Val Ile Ser Lys Met Leu Phe Val Glu Pro Ile
       50                  55                  60
   Leu Glu Val Ser Ser Leu Pro Thr Thr Asn Ser Thr Thr Asn Ser Ala
   65                  70                  75                  80
   Thr Lys Ile Thr Ala Asn Thr Thr Asp Glu Pro Thr Thr Gln Pro Thr
                   85                  90                  95
   Thr Glu Pro Thr Thr Gln Pro Thr Ile Gln Pro Thr Gln Pro Thr Thr
               100                 105                 110
   Gln Leu Pro Thr Asp Ser Pro Thr Gln Pro Thr Thr Gly Ser Phe Cys
           115                 120                 125
   Pro Gly Pro Val Thr Leu Cys Ser Asp Leu Glu Ser His Ser Thr Glu
       130                 135                 140
   Ala Val Leu Gly Asp Ala Leu Val Asp Phe Ser Leu Lys Leu Tyr His
   145                 150                 155                 160
   Ala Phe Ser Ala Met Lys Lys Val Glu Thr Asn Met Ala Phe Ser Pro
                   165                 170                 175
   Phe Ser Ile Ala Ser Leu Leu Thr Gln Val Leu Leu Gly Ala Gly Glu
               180                 185                 190
   Asn Thr Lys Thr Asn Leu Glu Ser Ile Leu Ser Tyr Pro Lys Asp Phe
           195                 200                 205
   Thr Cys Val His Gln Ala Leu Lys Gly Phe Thr Thr Lys Gly Val Thr
       210                 215                 220
   Ser Val Ser Gln Ile Phe His Ser Pro Asp Leu Ala Ile Arg Asp Thr
   225                 230                 235                 240
   Phe Val Asn Ala Ser Arg Thr Leu Tyr Ser Ser Ser Pro Arg Val Leu
                   245                 250                 255
   Ser Asn Asn Ser Asp Ala Asn Leu Glu Leu Ile Asn Thr Trp Val Ala
               260                 265                 270
   Lys Asn Thr Asn Asn Lys Ile Ser Arg Leu Leu Asp Ser Leu Pro Ser
           275                 280                 285
   Asp Thr Arg Leu Val Leu Leu Asn Ala Ile Tyr Leu Ser Ala Lys Trp
       290                 295                 300
   Lys Thr Thr Phe Asp Pro Lys Lys Thr Arg Met Glu Pro Phe His Phe
   305                 310                 315                 320
   Lys Asn Ser Val Ile Lys Val Pro Met Met Asn Ser Lys Lys Tyr Pro
                   325                 330                 335
   Val Ala His Phe Ile Asp Gln Thr Leu Lys Ala Lys Val Gly Gln Leu
               340                 345                 350
   Gln Leu Ser His Asn Leu Ser Leu Val Ile Leu Val Pro Gln Asn Leu
           355                 360                 365
   Lys His Arg Leu Glu Asp Met Glu Gln Ala Leu Ser Pro Ser Val Phe
       370                 375                 380
   Lys Ala Ile Met Glu Lys Leu Glu Met Ser Lys Phe Gln Pro Thr Leu
   385                 390                 395                 400
   Leu Thr Leu Pro Arg Ile Lys Val Thr Thr Ser Gln Asp Met Leu Ser
                   405                 410                 415
   Ile Met Glu Lys Leu Glu Phe Phe Asp Phe Ser Tyr Asp Leu Asn Leu
               420                 425                 430
   Cys Gly Leu Thr Glu Asp Pro Asp Leu Gln Val Ser Ala Met Gln His
           435                 440                 445
   Gln Thr Val Leu Glu Leu Thr Glu Thr Gly Val Glu Ala Ala Ala Ala
       450                 455                 460
   Ser Ala Ile Ser Val Ala Arg Thr Leu Leu Val Phe Glu Val Gln Gln
   465                 470                 475                 480
   Pro Phe Leu Phe Val Leu Trp Asp Gln Gln His Lys Phe Pro Val Phe
                   485                 490                 495
   Met Gly Arg Val Tyr Asp Pro Arg Ala
               500                 505
<210> 82
   <211> 698
   <212> PRT
   <213> Homo sapiens
<400> 82
   Met Arg Leu Ala Val Gly Ala Leu Leu Val Cys Ala Val Leu Gly Leu
    1               5                  10                  15
   Cys Leu Ala Val Pro Asp Lys Thr Val Arg Trp Cys Ala Val Ser Glu
               20                  25                  30
   His Glu Ala Thr Lys Cys Gln Ser Phe Arg Asp His Met Lys Ser Val
           35                  40                  45
   Ile Pro Ser Asp Gly Pro Ser Val Ala Cys Val Lys Lys Ala Ser Tyr
       50                  55                  60
   Leu Asp Cys Ile Arg Ala Ile Ala Ala Asn Glu Ala Asp Ala Val Thr
   65                  70                  75                  80
   Leu Asp Ala Gly Leu Val Tyr Asp Ala Tyr Leu Ala Pro Asn Asn Leu
                   85                  90                  95
   Lys Pro Val Val Ala Glu Phe Tyr Gly Ser Lys Glu Asp Pro Gln Thr
               100                 105                 110
   Phe Tyr Tyr Ala Val Ala Val Val Lys Lys Asp Ser Gly Phe Gln Met
           115                 120                 125
   Asn Gln Leu Arg Gly Lys Lys Ser Cys His Thr Gly Leu Gly Arg Ser
       130                 135                 140
   Ala Gly Trp Asn Ile Pro Ile Gly Leu Leu Tyr Cys Asp Leu Pro Glu
   145                 150                 155                 160
   Pro Arg Lys Pro Leu Glu Lys Ala Val Ala Asn Phe Phe Ser Gly Ser
                   165                 170                 175
   Cys Ala Pro Cys Ala Asp Gly Thr Asp Phe Pro Gln Leu Cys Gln Leu
               180                 185                 190
   Cys Pro Gly Cys Gly Cys Ser Thr Leu Asn Gln Tyr Phe Gly Tyr Ser
           195                 200                 205
   Gly Ala Phe Lys Cys Leu Lys Asp Gly Ala Gly Asp Val Ala Phe Val
       210                 215                 220
   Lys His Ser Thr Ile Phe Glu Asn Leu Ala Asn Lys Ala Asp Arg Asp
   225                 230                 235                 240
   Gln Tyr Glu Leu Leu Cys Leu Asp Asn Thr Arg Lys Pro Val Asp Glu
                   245                 250                 255
   Tyr Lys Asp Cys His Leu Ala Gln Val Pro Ser His Thr Val Val Ala
               260                 265                 270
   Arg Ser Met Gly Gly Lys Glu Asp Leu Ile Trp Glu Leu Leu Asn Gln
           275                 280                 285
   Ala Gln Glu His Phe Gly Lys Asp Lys Ser Lys Glu Phe Gln Leu Phe
       290                 295                 300
   Ser Ser Pro His Gly Lys Asp Leu Leu Phe Lys Asp Ser Ala His Gly
   305                 310                 315                 320
   Phe Leu Lys Val Pro Pro Arg Met Asp Ala Lys Met Tyr Leu Gly Tyr
                   325                 330                 335
   Glu Tyr Val Thr Ala Ile Arg Asn Leu Arg Glu Gly Thr Cys Pro Glu
               340                 345                 350
   Ala Pro Thr Asp Glu Cys Lys Pro Val Lys Trp Cys Ala Leu Ser His
           355                 360                 365
   His Glu Arg Leu Lys Cys Asp Glu Trp Ser Val Asn Ser Val Gly Lys
       370                 375                 380
   Ile Glu Cys Val Ser Ala Glu Thr Thr Glu Asp Cys Ile Ala Lys Ile
   385                 390                 395                 400
   Met Asn Gly Glu Ala Asp Ala Met Ser Leu Asp Gly Gly Phe Val Tyr
                   405                 410                 415
   Ile Ala Gly Lys Cys Gly Leu Val Pro Val Leu Ala Glu Asn Tyr Asn
               420                 425                 430
   Lys Ser Asp Asn Cys Glu Asp Thr Pro Glu Ala Gly Tyr Phe Ala Ile
           435                 440                 445
   Ala Val Val Lys Lys Ser Ala Ser Asp Leu Thr Trp Asp Asn Leu Lys
       450                 455                 460
   Gly Lys Lys Ser Cys His Thr Ala Val Gly Arg Thr Ala Gly Trp Asn
   465                 470                 475                 480
   Ile Pro Met Gly Leu Leu Tyr Asn Lys Ile Asn His Cys Arg Phe Asp
                   485                 490                 495
   Glu Phe Phe Ser Glu Gly Cys Ala Pro Gly Ser Lys Lys Asp Ser Ser
               500                 505                 510
   Leu Cys Lys Leu Cys Met Gly Ser Gly Leu Asn Leu Cys Glu Pro Asn
           515                 520                 525
   Asn Lys Glu Gly Tyr Tyr Gly Tyr Thr Gly Ala Phe Arg Cys Leu Val
       530                 535                 540
   Glu Lys Gly Asp Val Ala Phe Val Lys His Gln Thr Val Pro Gln Asn
   545                 550                 555                 560
   Thr Gly Gly Lys Asn Pro Asp Pro Trp Ala Lys Asn Leu Asn Glu Lys
                   565                 570                 575
   Asp Tyr Glu Leu Leu Cys Leu Asp Gly Thr Arg Lys Pro Val Glu Glu
               580                 585                 590
   Tyr Ala Asn Cys His Leu Ala Arg Ala Pro Asn His Ala Val Val Thr
           595                 600                 605
   Arg Lys Asp Lys Glu Ala Cys Val His Lys Ile Leu Arg Gln Gln Gln
       610                 615                 620
   His Leu Phe Gly Ser Asn Val Thr Asp Cys Ser Gly Asn Phe Cys Leu
   625                 630                 635                 640
   Phe Arg Ser Glu Thr Lys Asp Leu Leu Phe Arg Asp Asp Thr Val Cys
                   645                 650                 655
   Leu Ala Lys Leu His Asp Arg Asn Thr Tyr Glu Lys Tyr Leu Gly Glu
               660                 665                 670
   Glu Tyr Val Lys Ala Val Gly Asn Leu Arg Lys Cys Ser Thr Ser Ser
           675                 680                 685
   Leu Leu Glu Ala Cys Thr Phe Arg Arg Pro
       690                 695
<210> 83
   <211> 1170
   <212> PRT
   <213> Homo sapiens
<400> 83
<210> 84
   <211> 147
   <212> PRT
   <213> Homo sapiens
<400> 84
<210> 85
   <211> 478
   <212> PRT
   <213> Homo sapiens
<400> 85
   Met Ala Pro Leu Arg Pro Leu Leu Ile Leu Ala Leu Leu Ala Trp Val
   1 5 10 15
   Ala Leu Ala Asp Gln Glu Ser Cys Lys Gly Arg Cys Thr Glu Gly Phe
   20 25 30
   Asn Val Asp Lys Lys Cys Gln Cys Asp Glu Leu Cys Ser Tyr Tyr Gln
   35 40 45
   Ser Cys Cys Thr Asp Tyr Thr Ala Glu Cys Lys Pro Gln Val Thr Arg
   50 55 60
   Gly Asp Val Phe Thr Met Pro Glu Asp Glu Tyr Thr Val Tyr Asp Asp
   65 70 75 80
   Gly Glu Glu Lys Asn Asn Ala Thr Val His Glu Gln Val Gly Gly Pro
   85 90 95
   Ser Leu Thr Ser Asp Leu Gln Ala Gln Ser Lys Gly Asn Pro Glu Gln
   100 105 110
   Thr Pro Val Leu Lys Pro Glu Glu Glu Ala Pro Ala Pro Glu Val Gly
   115 120 125
   Ala Ser Lys Pro Glu Gly Ile Asp Ser Arg Pro Glu Thr Leu His Pro
   130 135 140
   Gly Arg Pro Gln Pro Pro Ala Glu Glu Glu Leu Cys Ser Gly Lys Pro
   145 150 155 160
   Phe Asp Ala Phe Thr Asp Leu Lys Asn Gly Ser Leu Phe Ala Phe Arg
   165 170 175
   Gly Gln Tyr Cys Tyr Glu Leu Asp Glu Lys Ala Val Arg Pro Gly Tyr
   180 185 190
   Pro Lys Leu Ile Arg Asp Val Trp Gly Ile Glu Gly Pro Ile Asp Ala
   195 200 205
   Ala Phe Thr Arg Ile Asn Cys Gln Gly Lys Thr Tyr Leu Phe Lys Gly
   210 215 220
   Ser Gln Tyr Trp Arg Phe Glu Asp Gly Val Leu Asp Pro Asp Tyr Pro
   225 230 235 240
   Arg Asn Ile Ser Asp Gly Phe Asp Gly Ile Pro Asp Asn Val Asp Ala
   245 250 255
   Ala Leu Ala Leu Pro Ala His Ser Tyr Ser Gly Arg Glu Arg Val Tyr
   260 265 270
   Phe Phe Lys Gly Lys Gln Tyr Trp Glu Tyr Gln Phe Gln His Gln Pro
   275 280 285
   Ser Gln Glu Glu Cys Glu Gly Ser Ser Leu Ser Ala Val Phe Glu His
   290 295 300
   Phe Ala Met Met Gln Arg Asp Ser Trp Glu Asp Ile Phe Glu Leu Leu
   305 310 315 320
   Phe Trp Gly Arg Thr Ser Ala Gly Thr Arg Gln Pro Gln Phe Ile Ser
   325 330 335
   Arg Asp Trp His Gly Val Pro Gly Gln Val Asp Ala Ala Met Ala Gly
   340 345 350
   Arg Ile Tyr Ile Ser Gly Met Ala Pro Arg Pro Ser Leu Ala Lys Lys
   355 360 365
   Gln Arg Phe Arg His Arg Asn Arg Lys Gly Tyr Arg Ser Gln Arg Gly
                   370                 375                 380
   His Ser Arg Gly Arg Asn Gln Asn Ser Arg Arg Pro Ser Arg Ala Thr
   385                 390                 395                 400
   Trp Leu Ser Leu Phe Ser Ser Glu Glu Ser Asn Leu Gly Ala Asn Asn
                   405                 410                 415
   Tyr Asp Asp Tyr Arg Met Asp Trp Leu Val Pro Ala Thr Cys Glu Pro
               420                 425                 430
   Ile Gln Ser Val Phe Phe Phe Ser Gly Asp Lys Tyr Tyr Arg Val Asn
           435                 440                 445
   Leu Arg Thr Arg Arg Val Asp Thr Val Asp Pro Pro Tyr Pro Arg Ser
       450                 455                 460
   Ile Ala Gln Tyr Trp Leu Gly Cys Pro Ala Pro Gly His Leu
   465                 470                 475
<210> 86
   <211> 2813
   <212> PRT
   <213> Homo sapiens
<400> 86
   Met Ile Pro Ala Arg Phe Ala Gly Val Leu Leu Ala Leu Ala Leu Ile
    1               5                  10                  15
   Leu Pro Gly Thr Leu Cys Ala Glu Gly Thr Arg Gly Arg Ser Ser Thr
               20                  25                  30
   Ala Arg Cys Ser Leu Phe Gly Ser Asp Phe Val Asn Thr Phe Asp Gly
           35                  40                  45
   Ser Met Tyr Ser Phe Ala Gly Tyr Cys Ser Tyr Leu Leu Ala Gly Gly
       50                  55                  60
   Cys Gln Lys Arg Ser Phe Ser Ile Ile Gly Asp Phe Gln Asn Gly Lys
   65                  70                  75                  80
   Arg Val Ser Leu Ser Val Tyr Leu Gly Glu Phe Phe Asp Ile His Leu
                   85                  90                  95
   Phe Val Asn Gly Thr Val Thr Gln Gly Asp Gln Arg Val Ser Met Pro
               100                 105                 110
   Tyr Ala Ser Lys Gly Leu Tyr Leu Glu Thr Glu Ala Gly Tyr Tyr Lys
           115                 120                 125
   Leu Ser Gly Glu Ala Tyr Gly Phe Val Ala Arg Ile Asp Gly Ser Gly
       130                 135                 140
   Asn Phe Gln Val Leu Leu Ser Asp Arg Tyr Phe Asn Lys Thr Cys Gly
   145                 150                 155                 160
   Leu Cys Gly Asn Phe Asn Ile Phe Ala Glu Asp Asp Phe Met Thr Gln
                   165                 170                 175
   Glu Gly Thr Leu Thr Ser Asp Pro Tyr Asp Phe Ala Asn Ser Trp Ala
               180                 185                 190
   Leu Ser Ser Gly Glu Gln Trp Cys Glu Arg Ala Ser Pro Pro Ser Ser
           195                 200                 205
   Ser Cys Asn Ile Ser Ser Gly Glu Met Gln Lys Gly Leu Trp Glu Gln
       210                 215                 220
   Cys Gln Leu Leu Lys Ser Thr Ser Val Phe Ala Arg Cys His Pro Leu
   225                 230                 235                 240
   Val Asp Pro Glu Pro Phe Val Ala Leu Cys Glu Lys Thr Leu Cys Glu
                   245                 250                 255
   Cys Ala Gly Gly Leu Glu Cys Ala Cys Pro Ala Leu Leu Glu Tyr Ala
               260                 265                 270
   Arg Thr Cys Ala Gln Glu Gly Met Val Leu Tyr Gly Trp Thr Asp His
           275                 280                 285
   Ser Ala Cys Ser Pro Val Cys Pro Ala Gly Met Glu Tyr Arg Gln Cys
       290                 295                 300
   Val Ser Pro Cys Ala Arg Thr Cys Gln Ser Leu His Ile Asn Glu Met
   305                 310                 315                 320
   Cys Gln Glu Arg Cys Val Asp Gly Cys Ser Cys Pro Glu Gly Gln Leu
                   325                 330                 335
   Leu Asp Glu Gly Leu Cys Val Glu Ser Thr Glu Cys Pro Cys Val His
               340                 345                 350
   Ser Gly Lys Arg Tyr Pro Pro Gly Thr Ser Leu Ser Arg Asp Cys Asn
           355                 360                 365
   Thr Cys Ile Cys Arg Asn Ser Gln Trp Ile Cys Ser Asn Glu Glu Cys
       370                 375                 380
   Pro Gly Glu Cys Leu Val Thr Gly Gln Ser His Phe Lys Ser Phe Asp
   385                 390                 395                 400
   Asn Arg Tyr Phe Thr Phe Ser Gly Ile Cys Gln Tyr Leu Leu Ala Arg
                   405                 410                 415
   Asp Cys Gln Asp His Ser Phe Ser Ile Val Ile Glu Thr Val Gln Cys
               420                 425                 430
   Ala Asp Asp Arg Asp Ala Val Cys Thr Arg Ser Val Thr Val Arg Leu
           435                 440                 445
   Pro Gly Leu His Asn Ser Leu Val Lys Leu Lys His Gly Ala Gly Val
       450                 455                 460
   Ala Met Asp Gly Gln Asp Val Gln Leu Pro Leu Leu Lys Gly Asp Leu
   465                 470                 475                 480
   Arg Ile Gln His Thr Val Thr Ala Ser Val Arg Leu Ser Tyr Gly Glu
                   485                 490                 495
   Asp Leu Gln Met Asp Trp Asp Gly Arg Gly Arg Leu Leu Val Lys Leu
               500                 505                 510
   Ser Pro Val Tyr Ala Gly Lys Thr Cys Gly Leu Cys Gly Asn Tyr Asn
           515                 520                 525
   Gly Asn Gln Gly Asp Asp Phe Leu Thr Pro Ser Gly Leu Ala Glu Pro
       530                 535                 540
   Arg Val Glu Asp Phe Gly Asn Ala Trp Lys Leu His Gly Asp Cys Gln
   545                 550                 555                 560
   Asp Leu Gln Lys Gln His Ser Asp Pro Cys Ala Leu Asn Pro Arg Met
                   565                 570                 575
   Thr Arg Phe Ser Glu Glu Ala Cys Ala Val Leu Thr Ser Pro Thr Phe
               580                 585                 590
   Glu Ala Cys His Arg Ala Val Ser Pro Leu Pro Tyr Leu Arg Asn Cys
           595                 600                 605
   Arg Tyr Asp Val Cys Ser Cys Ser Asp Gly Arg Glu Cys Leu Cys Gly
       610                 615                 620
   Ala Leu Ala Ser Tyr Ala Ala Ala Cys Ala Gly Arg Gly Val Arg Val
   625                 630                 635                 640
   Ala Trp Arg Glu Pro Gly Arg Cys Glu Leu Asn Cys Pro Lys Gly Gln
                   645                 650                 655
   Val Tyr Leu Gln Cys Gly Thr Pro Cys Asn Leu Thr Cys Arg Ser Leu
               660                 665                 670
   Ser Tyr Pro Asp Glu Glu Cys Asn Glu Ala Cys Leu Glu Gly Cys Phe
           675                 680                 685
   Cys Pro Pro Gly Leu Tyr Met Asp Glu Arg Gly Asp Cys Val Pro Lys
       690                 695                 700
   Ala Gln Cys Pro Cys Tyr Tyr Asp Gly Glu Ile Phe Gln Pro Glu Asp
   705                 710                 715                 720
   Ile Phe Ser Asp His His Thr Met Cys Tyr Cys Glu Asp Gly Phe Met
                   725                 730                 735
   His Cys Thr Met Ser Gly Val Pro Gly Ser Leu Leu Pro Asp Ala Val
               740                 745                 750
   Leu Ser Ser Pro Leu Ser His Arg Ser Lys Arg Ser Leu Ser Cys Arg
           755                 760                 765
   Pro Pro Met Val Lys Leu Val Cys Pro Ala Asp Asn Leu Arg Ala Glu
       770                 775                 780
   Gly Leu Glu Cys Thr Lys Thr Cys Gln Asn Tyr Asp Leu Glu Cys Met
   785                 790                 795                 800
   Ser Met Gly Cys Val Ser Gly Cys Leu Cys Pro Pro Gly Met Val Arg
                   805                 810                 815
   His Glu Asn Arg Cys Val Ala Leu Glu Arg Cys Pro Cys Phe His Gln
               820                 825                 830
   Gly Lys Glu Tyr Ala Pro Gly Glu Thr Val Lys Ile Gly Cys Asn Thr
           835                 840                 845
   Cys Val Cys Gln Asp Arg Lys Trp Asn Cys Thr Asp His Val Cys Asp
       850                 855                 860
   Ala Thr Cys Ser Thr Ile Gly Met Ala His Tyr Leu Thr Phe Asp Gly
   865                 870                 875                 880
   Leu Lys Tyr Leu Phe Pro Gly Glu Cys Gln Tyr Val Leu Val Gln Asp
                   885                 890                 895
   Tyr Cys Gly Ser Asn Pro Gly Thr Phe Arg Ile Leu Val Gly Asn Lys
               900                 905                 910
   Gly Cys Ser His Pro Ser Val Lys Cys Lys Lys Arg Val Thr Ile Leu
           915                 920                 925
   Val Glu Gly Gly Glu Ile Glu Leu Phe Asp Gly Glu Val Asn Val Lys
       930                 935                 940
   Arg Pro Met Lys Asp Glu Thr His Phe Glu Val Val Glu Ser Gly Arg
   945                 950                 955                 960
   Tyr Ile Ile Leu Leu Leu Gly Lys Ala Leu Ser Val Val Trp Asp Arg
                   965                 970                 975
   His Leu Ser Ile Ser Val Val Leu Lys Gln Thr Tyr Gln Glu Lys Val
               980                 985                 990
   Cys Gly Leu Cys Gly Asn Phe Asp Gly Ile Gln Asn Asn Asp Leu Thr
           995                 1000                1005
   Ser Ser Asn Leu Gln Val Glu Glu Asp Pro Val Asp Phe Gly Asn Ser
       1010                1015                1020
   Trp Lys Val Ser Ser Gln Cys Ala Asp Thr Arg Lys Val Pro Leu Asp
   1025                1030                1035                1040
   Ser Ser Pro Ala Thr Cys His Asn Asn Ile Met Lys Gln Thr Met Val
                   1045                1050                1055
   Asp Ser Ser Cys Arg Ile Leu Thr Ser Asp Val Phe Gln Asp Cys Asn
               1060                1065                1070
   Lys Leu Val Asp Pro Glu Pro Tyr Leu Asp Val Cys Ile Tyr Asp Thr
           1075                1080                1085
   Cys Ser Cys Glu Ser Ile Gly Asp Cys Ala Cys Phe Cys Asp Thr Ile
       1090                1095                1100
   Ala Ala Tyr Ala His Val Cys Ala Gln His Gly Lys Val Val Thr Trp
   1105                1110                1115                1120
   Arg Thr Ala Thr Leu Cys Pro Gln Ser Cys Glu Glu Arg Asn Leu Arg
                   1125                1130                1135
   Glu Asn Gly Tyr Glu Cys Glu Trp Arg Tyr Asn Ser Cys Ala Pro Ala
               1140                1145                1150
   Cys Gln Val Thr Cys Gln His Pro Glu Pro Leu Ala Cys Pro Val Gln
           1155                1160                1165
   Cys Val Glu Gly Cys His Ala His Cys Pro Pro Gly Lys Ile Leu Asp
       1170                1175                1180
   Glu Leu Leu Gln Thr Cys Val Asp Pro Glu Asp Cys Pro Val Cys Glu
   1185                1190                1195                1200
   Val Ala Gly Arg Arg Phe Ala Ser Gly Lys Lys Val Thr Leu Asn Pro
                   1205                1210                1215
   Ser Asp Pro Glu His Cys Gln Ile Cys His Cys Asp Val Val Asn Leu
               1220                1225                1230
   Thr Cys Glu Ala Cys Gln Glu Pro Gly Gly Leu Val Val Pro Pro Thr
           1235                1240                1245
   Asp Ala Pro Val Ser Pro Thr Thr Leu Tyr Val Glu Asp Ile Ser Glu
       1250                1255                1260
   Pro Pro Leu His Asp Phe Tyr Cys Ser Arg Leu Leu Asp Leu Val Phe
   1265                1270                1275                1280
   Leu Leu Asp Gly Ser Ser Arg Leu Ser Glu Ala Glu Phe Glu Val Leu
                   1285                1290                1295
   Lys Ala Phe Val Val Asp Met Met Glu Arg Leu Arg Ile Ser Gln Lys
               1300                1305                1310
   Trp Val Arg Val Ala Val Val Glu Tyr His Asp Gly Ser His Ala Tyr
           1315                1320                1325
   Ile Gly Leu Lys Asp Arg Lys Arg Pro Ser Glu Leu Arg Arg Ile Ala
       1330                1335                1340
   Ser Gln Val Lys Tyr Ala Gly Ser Gln Val Ala Ser Thr Ser Glu Val
   1345 1350 1355 1360
   Leu Lys Tyr Thr Leu Phe Gln Ile Phe Ser Lys Ile Asp Arg Pro Glu
   1365 1370 1375
   Ala Ser Arg Ile Thr Leu Leu Leu Met Ala Ser Gln Glu Pro Gln Arg
   1380 1385 1390
   Met Ser Arg Asn Phe Val Arg Tyr Val Gln Gly Leu Lys Lys Lys Lys
   1395 1400 1405
   Val Ile Val Ile Pro Val Gly Ile Gly Pro His Ala Asn Leu Lys Gln
   1410 1415 1420
   Ile Arg Leu Ile Glu Lys Gln Ala Pro Glu Asn Lys Ala Phe Val Leu
   1425 1430 1435 1440
   Ser Ser Val Asp Glu Leu Glu Gln Gln Arg Asp Glu Ile Val Ser Tyr
   1445 1450 1455
   Leu Cys Asp Leu Ala Pro Glu Ala Pro Pro Pro Thr Leu Pro Pro Asp
   1460 1465 1470
   Met Ala Gln Val Thr Val Gly Pro Gly Leu Leu Gly Val Ser Thr Leu
   1475 1480 1485
   Gly Pro Lys Arg Asn Ser Met Val Leu Asp Val Ala Phe Val Leu Glu
   1490 1495 1500
   Gly Ser Asp Lys Ile Gly Glu Ala Asp Phe Asn Arg Ser Lys Glu Phe
   1505 1510 1515 1520
   Met Glu Glu Val Ile Gln Arg Met Asp Val Gly Gln Asp Ser Ile His
   1525 1530 1535
   Val Thr Val Leu Gln Tyr Ser Tyr Met Val Thr Val Glu Tyr Pro Phe
   1540 1545 1550
   Ser Glu Ala Gln Ser Lys Gly Asp Ile Leu Gln Arg Val Arg Glu Ile
   1555 1560 1565
   Arg Tyr Gln Gly Gly Asn Arg Thr Asn Thr Gly Leu Ala Leu Arg Tyr
   1570 1575 1580
   Leu Ser Asp His Ser Phe Leu Val Ser Gln Gly Asp Arg Glu Gln Ala
   1585 1590 1595 1600
   Pro Asn Leu Val Tyr Met Val Thr Gly Asn Pro Ala Ser Asp Glu Ile
   1605 1610 1615
   Lys Arg Leu Pro Gly Asp Ile Gln Val Val Pro Ile Gly Val Gly Pro
   1620 1625 1630
   Asn Ala Asn Val Gln Glu Leu Glu Arg Ile Gly Trp Pro Asn Ala Pro
   1635 1640 1645
   Ile Leu Ile Gln Asp Phe Glu Thr Leu Pro Arg Glu Ala Pro Asp Leu
   1650 1655 1660
   Val Leu Gln Arg Cys Cys Ser Gly Glu Gly Leu Gln Ile Pro Thr Leu
   1665 1670 1675 1680
   Ser Pro Ala Pro Asp Cys Ser Gln Pro Leu Asp Val Ile Leu Leu Leu
   1685 1690 1695
   Asp Gly Ser Ser Ser Phe Pro Ala Ser Tyr Phe Asp Glu Met Lys Ser
   1700 1705 1710
   Phe Ala Lys Ala Phe Ile Ser Lys Ala Asn Ile Gly Pro Arg Leu Thr
   1715 1720 1725
   Gln Val Ser Val Leu Gln Tyr Gly Ser Ile Thr Thr Ile Asp Val Pro
   1730 1735 1740
   Trp Asn Val Val Pro Glu Lys Ala His Leu Leu Ser Leu Val Asp Val
   1745 1750 1755 1760
   Met Gln Arg Glu Gly Gly Pro Ser Gln Ile Gly Asp Ala Leu Gly Phe
   1765 1770 1775
   Ala Val Arg Tyr Leu Thr Ser Glu Met His Gly Ala Arg Pro Gly Ala
   1780 1785 1790
   Ser Lys Ala Val Val Ile Leu Val Thr Asp Val Ser Val Asp Ser Val
   1795 1800 1805
   Asp Ala Ala Ala Asp Ala Ala Arg Ser Asn Arg Val Thr Val Phe Pro
   1810 1815 1820
   Ile Gly Ile Gly Asp Arg Tyr Asp Ala Ala Gln Leu Arg Ile Leu Ala
   1825 1830 1835 1840
   Gly Pro Ala Gly Asp Ser Asn Val Val Lys Leu Gln Arg Ile Glu Asp
                   1845                1850                1855
   Leu Pro Thr Met Val Thr Leu Gly Asn Ser Phe Leu His Lys Leu Cys
               1860                1865                1870
   Ser Gly Phe Val Arg Ile Cys Met Asp Glu Asp Gly Asn Glu Lys Arg
           1875                1880                1885
   Pro Gly Asp Val Trp Thr Leu Pro Asp Gln Cys His Thr Val Thr Cys
       1890                1895                1900
   Gln Pro Asp Gly Gln Thr Leu Leu Lys Ser His Arg Val Asn Cys Asp
   1905                1910                1915                1920
   Arg Gly Leu Arg Pro Ser Cys Pro Asn Ser Gln Ser Pro Val Lys Val
                   1925                1930                1935
   Glu Glu Thr Cys Gly Cys Arg Trp Thr Cys Pro Cys Val Cys Thr Gly
               1940                1945                1950
   Ser Ser Thr Arg His Ile Val Thr Phe Asp Gly Gln Asn Phe Lys Leu
           1955                1960                1965
   Thr Gly Ser Cys Ser Tyr Val Leu Phe Gln Asn Lys Glu Gln Asp Leu
       1970                1975                1980
   Glu Val Ile Leu His Asn Gly Ala Cys Ser Pro Gly Ala Arg Gln Gly
   1985                1990                1995                2000
   Cys Met Lys Ser Ile Glu Val Lys His Ser Ala Leu Ser Val Glu Leu
                   2005                2010                2015
   His Ser Asp Met Glu Val Thr Val Asn Gly Arg Leu Val Ser Val Pro
               2020                2025                2030
   Tyr Val Gly Gly Asn Met Glu Val Asn Val Tyr Gly Ala Ile Met His
           2035                2040                2045
   Glu Val Arg Phe Asn His Leu Gly His Ile Phe Thr Phe Thr Pro Gln
       2050                2055                2060
   Asn Asn Glu Phe Gln Leu Gln Leu Ser Pro Lys Thr Phe Ala Ser Lys
   2065                2070                2075                2080
   Thr Tyr Gly Leu Cys Gly Ile Cys Asp Glu Asn Gly Ala Asn Asp Phe
                   2085                2090                2095
   Met Leu Arg Asp Gly Thr Val Thr Thr Asp Trp Lys Thr Leu Val Gln
               2100                2105                2110
   Glu Trp Thr Val Gln Arg Pro Gly Gln Thr Cys Gln Pro Ile Leu Glu
           2115                2120                2125
   Glu Gln Cys Leu Val Pro Asp Ser Ser His Cys Gln Val Leu Leu Leu
       2130                2135                2140
   Pro Leu Phe Ala Glu Cys His Lys Val Leu Ala Pro Ala Thr Phe Tyr
   2145                2150                2155                2160
   Ala Ile Cys Gln Gln Asp Ser Cys His Gln Glu Gln Val Cys Glu Val
                   2165                2170                2175
   Ile Ala Ser Tyr Ala His Leu Cys Arg Thr Asn Gly Val Cys Val Asp
               2180                2185                2190
   Trp Arg Thr Pro Asp Phe Cys Ala Met Ser Cys Pro Pro Ser Leu Val
           2195                2200                2205
   Tyr Asn His Cys Glu His Gly Cys Pro Arg His Cys Asp Gly Asn Val
       2210                2215                2220
   Ser Ser Cys Gly Asp His Pro Ser Glu Gly Cys Phe Cys Pro Pro Asp
   2225                2230                2235                2240
   Lys Val Met Leu Glu Gly Ser Cys Val Pro Glu Glu Ala Cys Thr Gln
                   2245                2250                2255
   Cys Ile Gly Glu Asp Gly Val Gln His Gln Phe Leu Glu Ala Trp Val
               2260                2265                2270
   Pro Asp His Gln Pro Cys Gln Ile Cys Thr Cys Leu Ser Gly Arg Lys
           2275                2280                2285
   Val Asn Cys Thr Thr Gln Pro Cys Pro Thr Ala Lys Ala Pro Thr Cys
       2290                2295                2300
   Gly Leu Cys Glu Val Ala Arg Leu Arg Gln Asn Ala Asp Gln Cys Cys
   2305                2310                2315                2320
   Pro Glu Tyr Glu Cys Val Cys Asp Pro Val Ser Cys Asp Leu Pro Pro
                   2325                2330                2335
   Val Pro His Cys Glu Arg Gly Leu Gln Pro Thr Leu Thr Asn Pro Gly
               2340                2345                2350
   Glu Cys Arg Pro Asn Phe Thr Cys Ala Cys Arg Lys Glu Glu Cys Lys
           2355                2360                2365
   Arg Val Ser Pro Pro Ser Cys Pro Pro His Arg Leu Pro Thr Leu Arg
       2370                2375                2380
   Lys Thr Gln Cys Cys Asp Glu Tyr Glu Cys Ala Cys Asn Cys Val Asn
   2385                2390                2395                2400
   Ser Thr Val Ser Cys Pro Leu Gly Tyr Leu Ala Ser Thr Ala Thr Asn
                   2405                2410                2415
   Asp Cys Gly Cys Thr Thr Thr Thr Cys Leu Pro Asp Lys Val Cys Val
               2420                2425                2430
   His Arg Ser Thr Ile Tyr Pro Val Gly Gln Phe Trp Glu Glu Gly Cys
           2435                2440                2445
   Asp Val Cys Thr Cys Thr Asp Met Glu Asp Ala Val Met Gly Leu Arg
       2450                2455                2460
   Val Ala Gln Cys Ser Gln Lys Pro Cys Glu Asp Ser Cys Arg Ser Gly
   2465                2470                2475                2480
   Phe Thr Tyr Val Leu His Glu Gly Glu Cys Cys Gly Arg Cys Leu Pro
                   2485                2490                2495
   Ser Ala Cys Glu Val Val Thr Gly Ser Pro Arg Gly Asp Ser Gln Ser
               2500                2505                2510
   Ser Trp Lys Ser Val Gly Ser Gln Trp Ala Ser Pro Glu Asn Pro Cys
           2515                2520                2525
   Leu Ile Asn Glu Cys Val Arg Val Lys Glu Glu Val Phe Ile Gln Gln
       2530                2535                2540
   Arg Asn Val Ser Cys Pro Gln Leu Glu Val Pro Val Cys Pro Ser Gly
   2545                2550                2555                2560
   Phe Gln Leu Ser Cys Lys Thr Ser Ala Cys Cys Pro Ser Cys Arg Cys
                   2565                2570                2575
   Glu Arg Met Glu Ala Cys Met Leu Asn Gly Thr Val Ile Gly Pro Gly
               2580                2585                2590
   Lys Thr Val Met Ile Asp Val Cys Thr Thr Cys Arg Cys Met Val Gln
           2595                2600                2605
   Val Gly Val Ile Ser Gly Phe Lys Leu Glu Cys Arg Lys Thr Thr Cys
       2610                2615                2620
   Asn Pro Cys Pro Leu Gly Tyr Lys Glu Glu Asn Asn Thr Gly Glu Cys
   2625                2630                2635                2640
   Cys Gly Arg Cys Leu Pro Thr Ala Cys Thr Ile Gln Leu Arg Gly Gly
                   2645                2650                2655
   Gln Ile Met Thr Leu Lys Arg Asp Glu Thr Leu Gln Asp Gly Cys Asp
               2660                2665                2670
   Thr His Phe Cys Lys Val Asn Glu Arg Gly Glu Tyr Phe Trp Glu Lys
           2675                2680                2685
   Arg Val Thr Gly Cys Pro Pro Phe Asp Glu His Lys Cys Leu Ala Glu
       2690                2695                2700
   Gly Gly Lys Ile Met Lys Ile Pro Gly Thr Cys Cys Asp Thr Cys Glu
   2705                2710                2715                2720
   Glu Pro Glu Cys Asn Asp Ile Thr Ala Arg Leu Gln Tyr Val Lys Val
                   2725                2730                2735
   Gly Ser Cys Lys Ser Glu Val Glu Val Asp Ile His Tyr Cys Gln Gly
               2740                2745                2750
   Lys Cys Ala Ser Lys Ala Met Tyr Ser Ile Asp Ile Asn Asp Val Gln
           2755                2760                2765
   Asp Gln Cys Ser Cys Cys Ser Pro Thr Arg Thr Glu Pro Met Gln Val
       2770                2775                2780
   Ala Leu His Cys Thr Asn Gly Ser Val Val Tyr His Glu Val Leu Asn
   2785                2790                2795                2800
   Ala Met Glu Cys Lys Cys Ser Pro Arg Lys Cys Ser Lys
                   2805                2810
<210> 87
   <211> 234
   <212> PRT
   <213> Homo sapiens
<400> 87
<210> 88
   <211> 177
   <212> PRT
   <213> Homo sapiens
<400> 88
   Met Gly Cys Cys Gly Cys Ser Arg Gly Cys Gly Ser Gly Cys Gly Gly
    1               5                  10                  15
   Cys Gly Ser Ser Cys Gly Gly Cys Gly Ser Gly Cys Gly Gly Cys Gly
               20                  25                  30
   Ser Gly Arg Gly Gly Cys Gly Ser Gly Cys Gly Gly Cys Ser Ser Ser
           35                  40                  45
   Cys Gly Gly Cys Gly Ser Arg Cys Tyr Val Pro Val Cys Cys Cys Lys
       50                  55                  60
   Pro Val Cys Ser Trp Val Pro Ala Cys Ser Cys Thr Ser Cys Gly Ser
   65                  70                  75                  80
   Cys Gly Gly Ser Lys Gly Gly Cys Gly Ser Cys Gly Gly Ser Lys Gly
                   85                  90                  95
   Gly Cys Gly Ser Cys Gly Gly Ser Lys Gly Gly Cys Gly Ser Cys Gly
               100                 105                 110
   Cys Ser Gln Ser Ser Cys Cys Lys Pro Cys Cys Cys Ser Ser Gly Cys
           115                 120                 125
   Gly Ser Ser Cys Cys Gln Ser Ser Cys Cys Lys Pro Cys Cys Cys Gln
       130                 135                 140
   Ser Ser Cys Cys Val Pro Val Cys Cys Gln Ser Ser Cys Cys Lys Pro
   145                 150                 155                 160
   Cys Cys Cys Gln Ser Asn Cys Cys Val Pro Val Cys Cys Gln Cys Lys
                   165                 170                 175
   Ile
<210> 89
   <211> 222
   <212> PRT
   <213> Homo sapiens
<400> 89
   Met Lys Leu Pro Leu Leu Leu Ala Leu Leu Phe Gly Ala Val Ser Ala
   1               5                   10                  15
   Leu His Leu Arg Ser Glu Thr Ser Thr Phe Glu Thr Pro Leu Gly Ala
               20                  25                  30
   Lys Thr Leu Pro Glu Asp Glu Glu Thr Pro Glu Gln Glu Met Glu Glu
           35                  40                  45
   Thr Pro Cys Arg Glu Leu Glu Glu Glu Glu Glu Trp Gly Ser Gly Ser
       50                  55                  60
   Glu Asp Ala Ser Lys Lys Asp Gly Ala Val Glu Ser Ile Ser Val Pro
   65                  70                  75                  80
   Asp Met Val Asp Lys Asn Leu Thr Cys Pro Glu Glu Glu Asp Thr Val
                   85                  90                  95
   Lys Val Val Gly Ile Pro Gly Cys Gln Thr Cys Arg Tyr Leu Leu Val
               100                 105                 110
   Arg Ser Leu Gln Thr Phe Ser Gln Ala Trp Phe Thr Cys Arg Arg Cys
           115                 120                 125
   Tyr Arg Gly Asn Leu Val Ser Ile His Asn Phe Asn Ile Asn Tyr Arg
       130                 135                 140
   Ile Gln Cys Ser Val Ser Ala Leu Asn Gln Gly Gln Val Trp Ile Gly
   145                 150                 155                 160
   Gly Arg Ile Thr Gly Ser Gly Arg Cys Arg Arg Phe Gln Trp Val Asp
                   165                 170                 175
   Gly Ser Arg Trp Asn Phe Ala Tyr Trp Ala Ala His Gln Pro Trp Ser
               180                 185                 190
   Arg Gly Gly His Cys Val Ala Leu Cys Thr Arg Gly Gly His Trp Arg
           195                 200                 205
   Arg Ala His Cys Leu Arg Arg Leu Pro Phe Ile Cys Ser Tyr
       210                 215                 220
<210> 90
   <211> 188
   <212> PRT
   <213> Homo sapiens
<400> 90
   Met Phe His Gln Ile Trp Ala Ala Leu Leu Tyr Phe Tyr Gly Ile Ile
   1               5                   10                  15
   Leu Asn Ser Ile Tyr Gln Cys Pro Glu His Ser Gln Leu Thr Thr Leu
               20                  25                  30
   Gly Val Asp Gly Lys Glu Phe Pro Glu Val His Leu Gly Gln Trp Tyr
           35                  40                  45
   Phe Ile Ala Gly Ala Ala Pro Thr Lys Glu Glu Leu Ala Thr Phe Asp
       50                  55                  60
   Pro Val Asp Asn Ile Val Phe Asn Met Ala Ala Gly Ser Ala Pro Met
   65                  70                  75                  80
   Gln Leu His Leu Arg Ala Thr Ile Arg Met Lys Asp Gly Leu Cys Val
                   85                  90                  95
   Pro Arg Lys Trp Ile Tyr His Leu Thr Glu Gly Ser Thr Asp Leu Arg
               100                 105                 110
   Thr Glu Gly Arg Pro Asp Met Lys Thr Glu Leu Phe Ser Ser Ser Cys
           115                 120                 125
   Pro Gly Gly Ile Met Leu Asn Glu Thr Gly Gln Gly Tyr Gln Arg Phe
       130                 135                 140
   Leu Leu Tyr Asn Arg Ser Pro His Pro Pro Glu Lys Cys Val Glu Glu
   145                 150                 155                 160
   Phe Lys Ser Leu Thr Ser Cys Leu Asp Ser Lys Ala Phe Leu Leu Thr
                   165                 170                 175
   Pro Arg Asn Gln Glu Ala Cys Glu Leu Ser Asn Asn
               180                 185
<210> 91
   <211> 1676
   <212> PRT
   <213> Homo sapiens
<400> 91
   Met Gly Leu Leu Gly Ile Leu Cys Phe Leu Ile Phe Leu Gly Lys Thr
    1               5                  10                  15
   Trp Gly Gln Glu Gln Thr Tyr Val Ile Ser Ala Pro Lys Ile Phe Arg
               20                  25                  30
   Val Gly Ala Ser Glu Asn Ile Val Ile Gln Val Tyr Gly Tyr Thr Glu
           35                  40                  45
   Ala Phe Asp Ala Thr Ile Ser Ile Lys Ser Tyr Pro Asp Lys Lys Phe
       50                  55                  60
   Ser Tyr Ser Ser Gly His Val His Leu Ser Ser Glu Asn Lys Phe Gln
   65                  70                  75                  80
   Asn Ser Ala Ile Leu Thr Ile Gln Pro Lys Gln Leu Pro Gly Gly Gln
                   85                  90                  95
   Asn Pro Val Ser Tyr Val Tyr Leu Glu Val Val Ser Lys His Phe Ser
               100                 105                 110
   Lys Ser Lys Arg Met Pro Ile Thr Tyr Asp Asn Gly Phe Leu Phe Ile
           115                 120                 125
   His Thr Asp Lys Pro Val Tyr Thr Pro Asp Gln Ser Val Lys Val Arg
       130                 135                 140
   Val Tyr Ser Leu Asn Asp Asp Leu Lys Pro Ala Lys Arg Glu Thr Val
   145                 150                 155                 160
   Leu Thr Phe Ile Asp Pro Glu Gly Ser Glu Val Asp Met Val Glu Glu
                   165                 170                 175
   Ile Asp His Ile Gly Ile Ile Ser Phe Pro Asp Phe Lys Ile Pro Ser
               180                 185                 190
   Asn Pro Arg Tyr Gly Met Trp Thr Ile Lys Ala Lys Tyr Lys Glu Asp
           195                 200                 205
   Phe Ser Thr Thr Gly Thr Ala Tyr Phe Glu Val Lys Glu Tyr Val Leu
       210                 215                 220
   Pro His Phe Ser Val Ser Ile Glu Pro Glu Tyr Asn Phe Ile Gly Tyr
   225                 230                 235                 240
   Lys Asn Phe Lys Asn Phe Glu Ile Thr Ile Lys Ala Arg Tyr Phe Tyr
                   245                 250                 255
   Asn Lys Val Val Thr Glu Ala Asp Val Tyr Ile Thr Phe Gly Ile Arg
               260                 265                 270
   Glu Asp Leu Lys Asp Asp Gln Lys Glu Met Met Gln Thr Ala Met Gln
           275                 280                 285
   Asn Thr Met Leu Ile Asn Gly Ile Ala Gln Val Thr Phe Asp Ser Glu
       290                 295                 300
   Thr Ala Val Lys Glu Leu Ser Tyr Tyr Ser Leu Glu Asp Leu Asn Asn
   305                 310                 315                 320
   Lys Tyr Leu Tyr Ile Ala Val Thr Val Ile Glu Ser Thr Gly Gly Phe
                   325                 330                 335
   Ser Glu Glu Ala Glu Ile Pro Gly Ile Lys Tyr Val Leu Ser Pro Tyr
               340                 345                 350
   Lys Leu Asn Leu Val Ala Thr Pro Leu Phe Leu Lys Pro Gly Ile Pro
           355                 360                 365
   Tyr Pro Ile Lys Val Gln Val Lys Asp Ser Leu Asp Gln Leu Val Gly
       370                 375                 380
   Gly Val Pro Val Thr Leu Asn Ala Gln Thr Ile Asp Val Asn Gln Glu
   385                 390                 395                 400
   Thr Ser Asp Leu Asp Pro Ser Lys Ser Val Thr Arg Val Asp Asp Gly
                   405                 410                 415
   Val Ala Ser Phe Val Leu Asn Leu Pro Ser Gly Val Thr Val Leu Glu
               420                 425                 430
   Phe Asn Val Lys Thr Asp Ala Pro Asp Leu Pro Glu Glu Asn Gln Ala
           435                 440                 445
   Arg Glu Gly Tyr Arg Ala Ile Ala Tyr Ser Ser Leu Ser Gln Ser Tyr
       450                 455                 460
   Leu Tyr Ile Asp Trp Thr Asp Asn His Lys Ala Leu Leu Val Gly Glu
   465                 470                 475                 480
   His Leu Asn Ile Ile Val Thr Pro Lys Ser Pro Tyr Ile Asp Lys Ile
                   485                 490                 495
   Thr His Tyr Asn Tyr Leu Ile Leu Ser Lys Gly Lys Ile Ile His Phe
               500                 505                 510
   Gly Thr Arg Glu Lys Phe Ser Asp Ala Ser Tyr Gln Ser Ile Asn Ile
           515                 520                 525
   Pro Val Thr Gln Asn Met Val Pro Ser Ser Arg Leu Leu Val Tyr Tyr
       530                 535                 540
   Ile Val Thr Gly Glu Gln Thr Ala Glu Leu Val Ser Asp Ser Val Trp
   545                 550                 555                 560
   Leu Asn Ile Glu Glu Lys Cys Gly Asn Gln Leu Gln Val His Leu Ser
                   565                 570                 575
   Pro Asp Ala Asp Ala Tyr Ser Pro Gly Gln Thr Val Ser Leu Asn Met
               580                 585                 590
   Ala Thr Gly Met Asp Ser Trp Val Ala Leu Ala Ala Val Asp Ser Ala
           595                 600                 605
   Val Tyr Gly Val Gln Arg Gly Ala Lys Lys Pro Leu Glu Arg Val Phe
       610                 615                 620
   Gln Phe Leu Glu Lys Ser Asp Leu Gly Cys Gly Ala Gly Gly Gly Leu
   625                 630                 635                 640
   Asn Asn Ala Asn Val Phe His Leu Ala Gly Leu Thr Phe Leu Thr Asn
                   645                 650                 655
   Ala Asn Ala Asp Asp Ser Gln Glu Asn Asp Glu Pro Cys Lys Glu Ile
               660                 665                 670
   Leu Arg Pro Arg Arg Thr Leu Gln Lys Lys Ile Glu Glu Ile Ala Ala
           675                 680                 685
   Lys Tyr Lys His Ser Val Val Lys Lys Cys Cys Tyr Asp Gly Ala Cys
       690                 695                 700
   Val Asn Asn Asp Glu Thr Cys Glu Gln Arg Ala Ala Arg Ile Ser Leu
   705                 710                 715                 720
   Gly Pro Arg Cys Ile Lys Ala Phe Thr Glu Cys Cys Val Val Ala Ser
                   725                 730                 735
   Gln Leu Arg Ala Asn Ile Ser His Lys Asp Met Gln Leu Gly Arg Leu
               740                 745                 750
   His Met Lys Thr Leu Leu Pro Val Ser Lys Pro Glu Ile Arg Ser Tyr
           755                 760                 765
   Phe Pro Glu Ser Trp Leu Trp Glu Val His Leu Val Pro Arg Arg Lys
       770                 775                 780
   Gln Leu Gln Phe Ala Leu Pro Asp Ser Leu Thr Thr Trp Glu Ile Gln
   785                 790                 795                 800
   Gly Val Gly Ile Ser Asn Thr Gly Ile Cys Val Ala Asp Thr Val Lys
                   805                 810                 815
   Ala Lys Val Phe Lys Asp Val Phe Leu Glu Met Asn Ile Pro Tyr Ser
               820                 825                 830
   Val Val Arg Gly Glu Gln Ile Gln Leu Lys Gly Thr Val Tyr Asn Tyr
           835                 840                 845
   Arg Thr Ser Gly Met Gln Phe Cys Val Lys Met Ser Ala Val Glu Gly
       850                 855                 860
   Ile Cys Thr Ser Glu Ser Pro Val Ile Asp His Gln Gly Thr Lys Ser
   865                 870                 875                 880
   Ser Lys Cys Val Arg Gln Lys Val Glu Gly Ser Ser Ser His Leu Val
                   885                 890                 895
   Thr Phe Thr Val Leu Pro Leu Glu Ile Gly Leu His Asn Ile Asn Phe
               900                 905                 910
   Ser Leu Glu Thr Trp Phe Gly Lys Glu Ile Leu Val Lys Thr Leu Arg
           915                 920                 925
   Val Val Pro Glu Gly Val Lys Arg Glu Ser Tyr Ser Gly Val Thr Leu
       930                 935                 940
   Asp Pro Arg Gly Ile Tyr Gly Thr Ile Ser Arg Arg Lys Glu Phe Pro
   945                 950                 955                 960
   Tyr Arg Ile Pro Leu Asp Leu Val Pro Lys Thr Glu Ile Lys Arg Ile
                   965                 970                 975
   Leu Ser Val Lys Gly Leu Leu Val Gly Glu Ile Leu Ser Ala Val Leu
               980                 985                 990
   Ser Gln Glu Gly Ile Asn Ile Leu Thr His Leu Pro Lys Gly Ser Ala
           995                 1000                1005
   Glu Ala Glu Leu Met Ser Val Val Pro Val Phe Tyr Val Phe His Tyr
       1010                1015                1020
   Leu Glu Thr Gly Asn His Trp Asn Ile Phe His Ser Asp Pro Leu Ile
   1025                1030                1035                1040
   Glu Lys Gln Lys Leu Lys Lys Lys Leu Lys Glu Gly Met Leu Ser Ile
                   1045                1050                1055
   Met Ser Tyr Arg Asn Ala Asp Tyr Ser Tyr Ser Val Trp Lys Gly Gly
               1060                1065                1070
   Ser Ala Ser Thr Trp Leu Thr Ala Phe Ala Leu Arg Val Leu Gly Gln
           1075                1080                1085
   Val Asn Lys Tyr Val Glu Gln Asn Gln Asn Ser Ile Cys Asn Ser Leu
       1090                1095                1100
   Leu Trp Leu Val Glu Asn Tyr Gln Leu Asp Asn Gly Ser Phe Lys Glu
   1105                1110                1115                1120
   Asn Ser Gln Tyr Gln Pro Ile Lys Leu Gln Gly Thr Leu Pro Val Glu
                   1125                1130                1135
   Ala Arg Glu Asn Ser Leu Tyr Leu Thr Ala Phe Thr Val Ile Gly Ile
               1140                1145                1150
   Arg Lys Ala Phe Asp Ile Cys Pro Leu Val Lys Ile Asp Thr Ala Leu
           1155                1160                1165
   Ile Lys Ala Asp Asn Phe Leu Leu Glu Asn Thr Leu Pro Ala Gln Ser
       1170                1175                1180
   Thr Phe Thr Leu Ala Ile Ser Ala Tyr Ala Leu Ser Leu Gly Asp Lys
   1185                1190                1195                1200
   Thr His Pro Gln Phe Arg Ser Ile Val Ser Ala Leu Lys Arg Glu Ala
                   1205                1210                1215
   Leu Val Lys Gly Asn Pro Pro Ile Tyr Arg Phe Trp Lys Asp Asn Leu
               1220                1225                1230
   Gln His Lys Asp Ser Ser Val Pro Asn Thr Gly Thr Ala Arg Met Val
           1235                1240                1245
   Glu Thr Thr Ala Tyr Ala Leu Leu Thr Ser Leu Asn Leu Lys Asp Ile
       1250                1255                1260
   Asn Tyr Val Asn Pro Val Ile Lys Trp Leu Ser Glu Glu Gln Arg Tyr
   1265                1270                1275                1280
   Gly Gly Gly Phe Tyr Ser Thr Gln Asp Thr Ile Asn Ala Ile Glu Gly
                   1285                1290                1295
   Leu Thr Glu Tyr Ser Leu Leu Val Lys Gln Leu Arg Leu Ser Met Asp
               1300                1305                1310
   Ile Asp Val Ser Tyr Lys His Lys Gly Ala Leu His Asn Tyr Lys Met
           1315                1320                1325
   Thr Asp Lys Asn Phe Leu Gly Arg Pro Val Glu Val Leu Leu Asn Asp
       1330                1335                1340
   Asp Leu Ile Val Ser Thr Gly Phe Gly Ser Gly Leu Ala Thr Val His
   1345                1350                1355                1360
   Val Thr Thr Val Val His Lys Thr Ser Thr Ser Glu Glu Val Cys Ser
                   1365                1370                1375
   Phe Tyr Leu Lys Ile Asp Thr Gln Asp Ile Glu Ala Ser His Tyr Arg
               1380                1385                1390
   Gly Tyr Gly Asn Ser Asp Tyr Lys Arg Ile Val Ala Cys Ala Ser Tyr
           1395                1400                1405
   Lys Pro Ser Arg Glu Glu Ser Ser Ser Gly Ser Ser His Ala Val Met
       1410                1415                1420
   Asp Ile Ser Leu Pro Thr Gly Ile Ser Ala Asn Glu Glu Asp Leu Lys
   1425                1430                1435                1440
   Ala Leu Val Glu Gly Val Asp Gln Leu Phe Thr Asp Tyr Gln Ile Lys
                   1445                1450                1455
   Asp Gly His Val Ile Leu Gln Leu Asn Ser Ile Pro Ser Ser Asp Phe
               1460                1465                1470
   Leu Cys Val Arg Phe Arg Ile Phe Glu Leu Phe Glu Val Gly Phe Leu
           1475                1480                1485
   Ser Pro Ala Thr Phe Thr Val Tyr Glu Tyr His Arg Pro Asp Lys Gln
       1490                1495                1500
   Cys Thr Met Phe Tyr Ser Thr Ser Asn Ile Lys Ile Gln Lys Val Cys
   1505                1510                1515                1520
   Glu Gly Ala Ala Cys Lys Cys Val Glu Ala Asp Cys Gly Gln Met Gln
                   1525                1530                1535
   Glu Glu Leu Asp Leu Thr Ile Ser Ala Glu Thr Arg Lys Gln Thr Ala
               1540                1545                1550
   Cys Lys Pro Glu Ile Ala Tyr Ala Tyr Lys Val Ser Ile Thr Ser Ile
           1555                1560                1565
   Thr Val Glu Asn Val Phe Val Lys Tyr Lys Ala Thr Leu Leu Asp Ile
       1570                1575                1580
   Tyr Lys Thr Gly Glu Ala Val Ala Glu Lys Asp Ser Glu Ile Thr Phe
   1585                1590                1595                1600
   Ile Lys Lys Val Thr Cys Thr Asn Ala Glu Leu Val Lys Gly Arg Gln
                   1605                1610                1615
   Tyr Leu Ile Met Gly Lys Glu Ala Leu Gln Ile Lys Tyr Asn Phe Ser
               1620                1625                1630
   Phe Arg Tyr Ile Tyr Pro Leu Asp Ser Leu Thr Trp Ile Glu Tyr Trp
           1635                1640                1645
   Pro Arg Asp Thr Thr Cys Ser Ser Cys Gln Ala Phe Leu Ala Asn Leu
       1650                1655                1660
   Asp Glu Phe Ala Glu Asp Ile Phe Leu Asn Gly Cys
   1665                1670                1675
<210> 92
   <211> 110
   <212> PRT
   <213> Homo sapiens
<400> 92
   Gln Ser Val Leu Thr Gln Pro Pro Ser Val Ser Ala Ala Pro Gly Gln
   1               5                   10                  15
   Lys Val Thr Ile Ser Cys Ser Gly Ser Ser Ser Asn Ile Gly Lys Asn
               20                  25                  30
   Tyr Val Ser Trp Tyr Gln Gln Leu Pro Gly Ala Ala Pro Lys Leu Leu
           35                  40                  45
   Ile Tyr Glu Ser Asp Lys Arg Pro Ser Gly Ile Pro Asp Arg Phe Ser
       50                  55                  60
   Gly Ser Lys Ser Gly Thr Ser Ala Thr Leu Gly Ile Thr Gly Leu Gln
   65                  70                  75                  80
   Thr Gly Asp Glu Ala Ala Tyr Tyr Cys Gly Thr Trp Asp His Ser Leu
                   85                  90                  95
   Asn Ala Gly Val Phe Gly Gly Gly Thr Thr Leu Thr Val Leu
               100                 105                 110
<210> 93
   <211> 764
   <212> PRT
   <213> Homo sapiens
<400> 93
<210> 94
   <211> 544
   <212> PRT
   <213> Homo sapiens
<400> 94
<210> 95
   <211> 782
   <212> PRT
   <213> Homo sapiens
<400> 95
<210> 96
   <211> 108
   <212> PRT
   <213> Homo sapiens
<400> 96
   Glu Ile Val Met Thr Gln Ser Pro Ala Thr Leu Ser Val Ser Pro Gly
    1               5                  10                  15
   Glu Arg Ala Thr Leu Ser Cys Arg Ala Ser Gln Ser Val Ser Ser Asn
               20                  25                  30
   Leu Ala Trp Tyr Gln Gln Lys Pro Gly Gln Ala Pro Arg Leu Leu Ile
           35                  40                  45
   Tyr Cys Ala Ser Thr Arg Ala Thr Gly Ile Pro Ala Arg Phe Ser Gly
       50                  55                  60
   Ser Gly Ser Gly Thr Glu Phe Thr Leu Thr Ile Ser Ser Leu Gln Phe
   65                  70                  75                  80
   Glu Asp Phe Ala Val Tyr Tyr Cys Gln His Tyr Asn Asn Trp Pro Phe
                   85                  90                  95
   Thr Phe Gly Pro Gly Thr Lys Val Asp Ile Lys Arg
               100                 105
<210> 97
   <211> 235
   <212> PRT
   <213> Homo sapiens
<400> 97
<210> 98
   <211> 234
   <212> PRT
   <213> Homo sapiens
<400> 98
<210> 99
   <211> 644
   <212> PRT
   <213> Homo sapiens
<400> 99
   Met Lys Leu Ile Thr Ile Leu Phe Leu Cys Ser Arg Leu Leu Leu Ser
   1               5                   10                  15
   Leu Thr Gln Glu Ser Gln Ser Glu Glu Ile Asp Cys Asn Asp Lys Asp
               20                  25                  30
   Leu Phe Lys Ala Val Asp Ala Ala Leu Lys Lys Tyr Asn Ser Gln Asn
           35                  40                  45
   Gln Ser Asn Asn Gln Phe Val Leu Tyr Arg Ile Thr Glu Ala Thr Lys
       50                  55                  60
   Thr Val Gly Ser Asp Thr Phe Tyr Ser Phe Lys Tyr Glu Ile Lys Glu
   65                  70                  75                  80
   Gly Asp Cys Pro Val Gln Ser Gly Lys Thr Trp Gln Asp Cys Glu Tyr
                   85                  90                  95
   Lys Asp Ala Ala Lys Ala Ala Thr Gly Glu Cys Thr Ala Thr Val Gly
               100                 105                 110
   Lys Arg Ser Ser Thr Lys Phe Ser Val Ala Thr Gln Thr Cys Gln Ile
           115                 120                 125
   Thr Pro Ala Glu Gly Pro Val Val Thr Ala Gln Tyr Asp Cys Leu Gly
       130                 135                 140
   Cys Val His Pro Ile Ser Thr Gln Ser Pro Asp Leu Glu Pro Ile Leu
   145                 150                 155                 160
   Arg His Gly Ile Gln Tyr Phe Asn Asn Asn Thr Gln His Ser Ser Leu
                   165                 170                 175
   Phe Met Leu Asn Glu Val Lys Arg Ala Gln Arg Gln Val Val Ala Gly
               180                 185                 190
   Leu Asn Phe Arg Ile Thr Tyr Ser Ile Val Gln Thr Asn Cys Ser Lys
           195                 200                 205
   Glu Asn Phe Leu Phe Leu Thr Pro Asp Cys Lys Ser Leu Trp Asn Gly
       210                 215                 220
   Asp Thr Gly Glu Cys Thr Asp Asn Ala Tyr Ile Asp Ile Gln Leu Arg
   225                 230                 235                 240
   Ile Ala Ser Phe Ser Gln Asn Cys Asp Ile Tyr Pro Gly Lys Asp Phe
                   245                 250                 255
   Val Gln Pro Pro Thr Lys Ile Cys Val Gly Cys Pro Arg Asp Ile Pro
               260                 265                 270
   Thr Asn Ser Pro Glu Leu Glu Glu Thr Leu Thr His Thr Ile Thr Lys
           275                 280                 285
   Leu Asn Ala Glu Asn Asn Ala Thr Phe Tyr Phe Lys Ile Asp Asn Val
       290                 295                 300
   Lys Lys Ala Arg Val Gln Val Val Ala Gly Lys Lys Tyr Phe Ile Asp
   305                 310                 315                 320
   Phe Val Ala Arg Glu Thr Thr Cys Ser Lys Glu Ser Asn Glu Glu Leu
                   325                 330                 335
   Thr Glu Ser Cys Glu Thr Lys Lys Leu Gly Gln Ser Leu Asp Cys Asn
               340                 345                 350
   Ala Glu Val Tyr Val Val Pro Trp Glu Lys Lys Ile Tyr Pro Thr Val
           355                 360                 365
   Asn Cys Gln Pro Leu Gly Met Ile Ser Leu Met Lys Arg Pro Pro Gly
       370                 375                 380
   Phe Ser Pro Phe Arg Ser Ser Arg Ile Gly Glu Ile Lys Glu Glu Thr
   385                 390                 395                 400
   Thr Val Ser Pro Pro His Thr Ser Met Ala Pro Ala Gln Asp Glu Glu
                   405                 410                 415
   Arg Asp Ser Gly Lys Glu Gln Gly His Thr Arg Arg His Asp Trp Gly
               420                 425                 430
   His Glu Lys Gln Arg Lys His Asn Leu Gly His Gly His Lys His Glu
   435 440 445
   Arg Asp Gln Gly His Gly His Gln Arg Gly His Gly Leu Gly His Gly
   450 455 460
   His Glu Gln Gln His Gly Leu Gly His Gly His Lys Phe Lys Leu Asp
   465 470 475 480
   Asp Asp Leu Glu His Gln Gly Gly His Val Leu Asp His Gly His Lys
   485 490 495
   His Lys His Gly His Gly His Gly Lys His Lys Asn Lys Gly Lys Lys
   500 505 510
   Asn Gly Lys His Asn Gly Trp Lys Thr Glu His Leu Ala Ser Ser Ser
   515 520 525
   Glu Asp Ser Thr Thr Pro Ser Ala Gln Thr Gln Glu Lys Thr Glu Gly
   530 535 540
   Pro Thr Pro Ile Pro Ser Leu Ala Lys Pro Gly Val Thr Val Thr Phe
   545 550 555 560
   Ser Asp Phe Gln Asp Ser Asp Leu Ile Ala Thr Met Met Pro Pro Ile
   565 570 575
   Ser Pro Ala Pro Ile Gln Ser Asp Asp Asp Trp Ile Pro Asp Ile Gln
   580 585 590
   Ile Asp Pro Asn Gly Leu Ser Phe Asn Pro Ile Ser Asp Phe Pro Asp
   595 600 605
   Thr Thr Ser Pro Lys Cys Pro Gly Arg Pro Trp Lys Ser Val Ser Glu
   610 615 620
   Ile Asn Pro Thr Thr Gln Met Lys Glu Ser Tyr Tyr Phe Asp Leu Thr
   625 630 635 640
   Asp Gly Leu Ser
<210> 100
   <211> 623
   <212> PRT
   <213> Homo sapiens
<400> 100

## Description

### FIELD

The present disclosure relates to biomarkers of preterm birth, biomarkers of term birth, and methods of use thereof. In particular, the present disclosure provides methods of determining whether a pregnant woman is at an increased risk for premature delivery. The present disclosure further provides methods for decreasing a pregnant woman's risk for premature delivery.

### BACKGROUND

In humans, preterm birth (PTB) is defined as delivery of a pregnancy before 37 weeks of gestation (Honest et al., Health Technology Assessment, 13:1-3, 2009). PTB is associated with significant morbidity and mortality. Premature babies born before 37 weeks, if they survive, have a high incidence of visual and hearing defects, lung disease, developmental delays, and cerebral palsy (Ables and Chauhan, J Family Practice, 54:245-252, 2005).

Preterm deliveries are categorized as elective (about 25% of all premature births) or spontaneous (about 75% of all premature births). Elective preterm deliveries occur as a result of maternal or fetal complications such as hypertension and diabetes (Honest et al., supra, 2009). Spontaneous preterm deliveries occur as a result of preterm labor or preterm premature rupture of membranes (Ables and Chauhan, supra, 2005).

Relatively little progress has been made in determining whether a pregnant woman is at risk for PTB. The lack of tools for identifying risk early in pregnancy coupled with an inability to pinpoint underlying etiology prevents clinicians from proactively detecting and managing the at-risk pregnancy hampering their efforts to improve pregnancy outcomes. In fact, the rate of preterm birth has steadily increased and reflects approximately 15 million of the 187 million births worldwide. In the United States, the preterm birth rate approaches 12.7% resulting in over 500,000 premature infants born each year. Moreover, during the first year of life, between 10 and 20% of premature infants die (March of Dimes, Born Too Soon, 2012; and Society for Maternal Fetal Medicine, High Risk Pregnancy Care, Research, and Education for Over 35 Years, 2010).

A pregnancy ending prematurely is multifactorial in its etiology and remains difficult to predict. For a pregnancy to progress uneventfully, an exquisite balance between numerous physiological systems must be maintained - including the dynamic immunological status of host and fetus at the placental interface. Subtle changes in this balance may be reflected in the identification of a unique set of blood-derived biomarkers found only in pregnancies that end prematurely. The utility and accuracy of novel biomarkers to predict spontaneous preterm birth remains elusive (Conde-Agudelo et al., BJOG, 118:1042-1054, 2011). Further compounding the problem is the complexity of harvesting such markers from biological fluids, the lack of stability of such markers and classic signal to noise issues incumbent to all biomarker studies.

Since the inability to accurately diagnose preterm labor or to determine a pregnant woman's risk of PTB has potentially catastrophic consequences, there is a need in the art for diagnostic and prognostic methods with improved specificity and sensitivity. This need is particularly great considering that timely administration of therapeutic agents may prevent preterm labor or otherwise prolong pregnancy.

WO 2009/031721 A1 discloses that hemopexin precursor is measured for evaluating whether a pregnant woman is at risk of recurrent spontaneous abortion.

WO 2008/063928 A1 describes antibodies as therapeutic compounds against preterm birth.

US 2012/021442 A1 discloses progesterone as a therapeutic against preterm birth.

WO 2008/098734 A1 describes an antibody specific for haemoglobin and/or heme for the prophylaxis of preeclampsia.

Tita et al., American Journal of Obstetrics and Gynaecology, Mosby, St Louis, MO, US, vol. 200, no. 3, 1 March 2009, pages 219-224, describes progesterone as a therapeutic for the prevention of preterm birth.

Pereira et al, Journal of Proteome Research, vol. 6, no. 4, 1 January 2007, pages 1269-1276, discloses the identification of differently expressed proteins such as serotransferrin precursor in preterm birth versus control samples.

### BRIEF SUMMARY

The present disclosure relates to biomarkers of preterm birth, biomarkers of term birth, and methods of use thereof. In particular, the present disclosure provides methods of determining whether a pregnant woman is at an increased risk for premature delivery. The present disclosure further provides methods for decreasing a pregnant woman's risk for premature delivery.

In particular, the present disclosure provides methods for assessing the risk of preterm birth for a pregnant subject, the method comprising: (a) detecting a level of one or more proteins in a sample from the pregnant subject, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, CIS, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; and (b) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; or determining that the pregnant subject is at not at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is below a threshold level, and/or when the level of one or more of proteins of the term birth group consisting of APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is above a threshold level.

The present disclosure provides methods for assessing the risk of preterm birth for a pregnant subject, comprising: (a) preparing a microparticle-enriched fraction from a sample from the pregnant subject; (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, CIS, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; and (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; or determining that the pregnant subject is at not at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is below a threshold level, and/or when the level of one or more of proteins of the term birth group consisting of APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is above a threshold level.

Additionally, the present disclosure provides methods of decreasing the risk of preterm birth for a pregnant subject, comprising: (a) preparing a microparticle-enriched fraction from a sample from the pregnant subject; (b) detecting a level of one or more proteins in the fraction, wherein the one or more proteins are selected from the group consisting of A1BG, AFM, AHSG, ALB, AMBP, APCS, APOA1, APOD, APOH, APOL1, APOM, ATRN, AZGP1, C1QC, C1R, CIS, C3, C4A, C4B, C4BPA, C5, C8A, CD5L, CFB, CFH, CP, CPN1, CPN2, CYP2U1, F12, GSN, HP, HPR, HPX, IGHA1, IGHA2, IGHG1, IGHG4, IGHM, IGHM1, IGHM2, IGHV4-31, IGHVa, IGHVb, IGJ, IGKC, IGKV, IGKV1-9, IGKV3-20, IGKV3D-15, IGLC2, IGLL1, IGLV3-19, IGLV7-43, IGLVa, IGLVb, IGLVc, IGLVd, ITIH1, ITIH2, ITIH3, KNG1, KRT1, KRT10, KRT2, KRT6A, KRT9, KRTAP5-2, LGALS3BP, LPA, PF4, PPBP, PRG2, PZP, PZPs, SAA2-SAA4, SERPINF2, SERPING1, TF, VTN, and VWF; (c) determining that the pregnant subject is at an increased risk of preterm birth when the level of one or more proteins of the preterm birth group consisting of A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF is above a threshold level, and/or when the level of one or more of proteins of the term birth group consisting of APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs is below a threshold level; and (d) administering a therapeutic agent to the subject in an amount effective to decrease the risk of preterm birth.

Preferably, the methods of the preceding paragraphs further comprise: (b2) detecting a level of one or more further proteins in the fraction from the pregnant subject, wherein the one or more further proteins are selected from the group consisting of A2M, APOB, C9, F2, FBLN1, FN1, FN1s, GC, ITIH4, KLKB1, ORM1, ORM2, S100A8, S100A9, SERPINA1, SERPINC1, THBS1, and TTR; and (c2) determining that the pregnant subject is at an increased risk of preterm birth when the level of one or more further proteins of the further preterm birth group consisting of A2M, C9, FBLN1, FN1, FN1s, GC, ITIH4, KLKB1, ORM1, ORM2, S100A8, S100A9, SERPINA1, SERPINC1, and TTR, is above a threshold level, and/or when the level of one or more of further proteins of the further term birth group consisting of APOB, C9, and THBS1 is below a threshold level in the sample. Preferably, the detecting step comprises detecting the level of one or more proteins of the preterm group subset consisting of alpha-1B-glycoprotein (A1BG), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), and serotransferrin (TF), and/or detecting the level of one or more proteins of the term group subset consisting of complement component 1r (C1r), complement component 3 (C3), complement component 4B (C4B), complement factor H (CFH), Ig heavy chain alpha 2 (IGHA2), and Ig kappa variable 1-9 (IGKV1-9). Preferably, the detecting step comprises detecting the level of all twelve of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF). Preferably, the detecting step comprises detecting the level of all six of complement component 1r (C1r), complement component 3 (C3), complement component 4B (C4B), complement factor H (CFH), Ig heavy chain alpha 2 (IGHA2), and Ig kappa variable1-9 (IGKV1-9). Preferably, the methods comprise a further step after (c) of providing a risk score by enumerating the preterm birth markers above the threshold that were detected and the term birth markers below the threshold that were detected. Preferably, the detecting step comprises detecting the level of at least 2, 3, 4, or 5 of the proteins in the fraction, or detecting the level of at least 2, 3, 4, 5, 6, 7, 8, 9, 10, 11, 12, 13, 14, 15, 16, 17, 18, 19 or 20 of the proteins in the fraction. Preferably, the detecting step comprises detecting the level of at least 5, at least 10, at least 15, at least 20, at least 25, at least 30, at least 35, at least 40, at least 45, at least 50, at least 55, at least 60, at least 65, at least 70, at least 75, or at least 80 out of the (99) proteins in the fraction. Preferably, the pregnant subject is a primigravida woman. Preferably, the pregnant subject is a multigravida woman. Preferably, the sample is taken from the pregnant subject during the second trimester. Preferably, the sample is taken from the pregnant subject within 15 to 17 weeks of gestation. Preferably, the sample is blood, saliva, sweat, nasal secretions, tears, urine, amniotic fluid, or cervicovaginal fluid. Preferably, the sample is a blood sample, preferably is serum or plasma. Preferably, the preparing step does not comprise the use of exoquik or the like. Preferably, the preparing step comprises size-exclusion chromatography with an agarose (e.g., SEPHAROSE) solid phase and an aqueous liquid phase. Preferably, the aqueous phase is water, alternatively, the aqueous phase is a buffer. Preferably, the detecting step comprises measuring binding of an antibody specific to each of the one or more proteins. Preferably, the detecting step comprises a technique selected from the group consisting of enzyme-linked immunosorbent assay (ELISA), western blot and antibody microarray. Preferably, the detecting step comprises liquid chromatography/mass spectrometry (LC/MS). Preferably, the therapeutic agent is selected from the group consisting of a hormone, a steroid, intravenous immunoglobulin, and a tumor-necrosis factor-alpha antagonist. Preferably, the therapeutic agent is progesterone. Preferably, the therapeutic agent comprises a cervical cerclage.

In a first aspect of the invention, there is provided a method for assessing risk of preterm birth for a pregnant subject, the method comprising:
(a) preparing a microparticle-enriched fraction from a sample from the pregnant subject, wherein the preparing step comprises_using size-exclusion chromatography with an agarose solid phase and an aqueous liquid phase;
(b) detecting a level of two or more proteins in the fraction, wherein the two or more proteins are
   one or more proteins of the preterm group subset consisting of hemopexin (HPX), alpha-1B-glycoprotein (A1BG), serum albumin (ALB), apolipoprotein D (APOD), apolipoprotein L1 (APOL1), zinc-alpha-2-glycoprotein (AZGP1), Ig heavy chain mu 1 (IGHM1), IgM heavy chain mu 2 (IGHM2), and serotransferrin (TF), wherein the protein or at least one of the proteins is HPX, and
   one or more proteins of the term group subset consisting of complement component 1r (C1r), complement component 3 (C3), complement component 4B (C4B), complement factor H (CFH), Ig heavy chain alpha 2 (IGHA2), and Ig kappa variable1-9 (IGKV1-9); and
(c) determining that the pregnant subject is at an increased risk of preterm birth when the level of one or more proteins of the preterm birth group consisting of A1BG, ALB, APOD, APOL1, AZGP1, HPX, IGHM1, IGHM2, and TF is above a threshold level, and/or when the level of one or more of proteins of the term birth group consisting of C1R, C3, C4B, CFH, IGHA2, and IGKV1-9 is below a threshold level; or
   determining that the pregnant subject is not at an increased risk of preterm birth when the level of one or more proteins of the preterm birth group consisting of A1BG, ALB, APOD, APOL1, AZGP1, HPX, IGHM1, IGHM2and TF, is below a threshold level, and/or when the level of one or more of proteins of the term birth group consisting of C1R, C3, CFH, IGHA2, IGKV1-9, is above a threshold level.

Preferred embodiments of the invention in any of its various aspects are as described below or as defined in the sub-claims.

### DESCRIPTION OF THE DRAWINGS

**FIG. 1** provides a Western blot analysis of SEC peak 1 material derived from the serum of pregnant women. Six individual patient samples were analyzed. Antigens detected include: FN1, fibronectin; HSP, heat shock protein 70; and CD9, a tetraspanin cell surface protein commonly associated with extracellular vesicles.
**FIG. 2** provides a flow chart of an exemplary method for assessing risk of preterm birth.

### DETAILED DESCRIPTION

Current methods indicate that placental-derived microparticles freely circulate in high titer in maternal blood and reflect the status of the syncytiotrophoblast maternal-fetal interface. Subtle homeostatic changes are reflected in a unique set of dysregulated, microparticle-associated proteins when pregnancy complications occur. As described in more detail in the example section, frozen maternal serum samples (n=48) obtained between 15-17 weeks gestation were analyzed from asymptomatic women subsequently having live births (e.g., excluded women having multiple births, still births, systemic disease, or delivering fetuses with known anomalies). Microparticles were isolated from blood samples using gel filtration, with samples blinded to outcome. Proteins were extracted and analyzed using an open proteomic LC-MS differential analysis between term (>37wks) and preterm cohorts (<34wks). Among 213 proteins identified, a unique pattern was observed in serum microparticles isolated from asymptomatic patients that subsequently delivered preterm. Using an ANOVA assessment and rigorous reproducibility, accuracy, and confidence criteria, 18 proteins were characterized from 99 statistically significant proteins identified across two study phases differentiating the cohorts, with functional analysis implicating inflammatory and cell injury pathways. Thus, the present disclosure provides a library of statistically-valued, microparticle-associated biomarkers that are useful for the early identification of women at risk for preterm birth.

### Definitions

In order to facilitate an understanding of the disclosure, selected terms used in the application will be discussed below.

The term "microparticle" refers to an extracellular microvesicle or lipid raft protein aggregate having a hydrodynamic diameter of from about 50 to about 5000 nm. As such the term microparticle encompasses exosomes (about 50 to about 100 nm), microvesicles (about 100 to about 300 nm), ectosomes (about 50 to about 1000 nm), apoptotic bodies (about 50 to about 5000 nm) and lipid protein aggregates of the same dimensions.

The term "microparticle-associated protein" refers to a protein or fragment thereof (e.g., polypeptide) that is detectable in a microparticle-enriched sample from a mammalian (e.g., human) subject. As such the term "microparticle-associated protein" is not restricted to proteins or fragments thereof that are physically associated with microparticles at the time of detection.

The term "about" as used herein in reference to a value refers to 90 to 110% of that value. For instance a diameter of about 1000 nm is a diameter within the range of 900 nm to 1100 nm.

The phrase "increased risk of preterm birth" as used herein indicates that a pregnant subject has a greater likelihood of having a spontaneous preterm birth (before 38 weeks gestation) when one or more preterm birth markers are detected and/or when one or more term birth markers are not detected. Numerically an increased risk is associated with a hazard ratio of over 1.0, preferably over 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, or 3.0 for preterm birth.

### Protein Biomarkers

The present disclosure relates to tools for assessing and decreasing risk of preterm birth. The methods of the present disclosure include a step of detecting the expression level of a microparticle-associated protein in a biological sample from a pregnant test subject, where the protein is selected from Table I.

**Table I. Microparticle-Associated Proteins Differentially Expressed in Preterm Birth**

| **Protein Name** | **Symbol** | **IPI** | **UniProtKB** |
|---|---|---|---|
| Alpha-2-Macroglobulin | A2M | IPI01010737.1 | P01023 |
| Apolipoprotein B | APOB | IPI00022229.2 | P04114 |
| Apolipoprotein H | APOH | IPI00298828.3 | P02749 |
| Complement C1r | C1R | IPI00956148.2 | Q53HU9 |
| Complement C1s | C1S | IPI00017696.1 | P09871 |
| Complement C3 | C3 | IPI00783987.2 | P01024 |
| Complement Factor H | CFH | IPI00029739.5 | P08603 |
| Immunoglobulin Heavy Chain Mu | IGHM | IPI00418153.1 | Q6N030 |
| Immunoglobulin Heavy Chain Gamma 1 | IGPHG 1 | IPI00384938.1 | Q7Z351 |
| Keratin 1 | KRT1 | IPI00220327.4 | P04264 |
| Keratin 10 | KRT10 | IPI00009865.4 | P13645 |
| Lectin galactoside-binding soluble 3-BP | LGALS3BP | IPI00023673.1 | Q08380 |
| S100 Calcium-Binding Protein A9 | S100A9 | IPI00027462.1 | P06702 |
| Alpha-1-Antitrypsin | SERPINA1 | IPI00553177.1 | P01009 |
| Alpha-1B-Glycoprotein | A1BG | IPI00022895.8 | P04217 |
| Albumin | ALB | IPI00745872.2 | P02768 |
| Apolipoprotein L1 | APOL1 | IPI00186903.4 | 014791 |
| Complement C4B (Chido Blood Group) | C4B | IPI00418163.3 | Q6U2E9 |
| Complement C9 | C9 | IPI00022395.1 | P02748 |
| Coagulation Factor XII | F12 | IPI00019581.2 | P00748 |
| Fibronectin 1 short | FN1s | IPI00922213.2 | B4DTK1 |
| Group-specific component (vitamin D-BP) | GC | IPI00968027.1 | P02774 |
| Immunoglobulin Heavy Chain Alpha 1 | IGHA1 | IPI00449920.1 | P01876 |
| Immunoglobulin Heavy Chain Mu 2 | IGHM2 | IPI00896380.1 | P01871 -1 |
| Immunoglobulin Kappa Constant | IGKC | IPI00979250.1 | P01834 |
| Immunoglobulin Lambda-Like 1 | IGLL1 | IPI00013438.1 | P15814 |
| Inter-alpha-trypsin inhibitor H1 | ITIH1 | IPI00292530.1 | P19827 |
| Inter-alpha-trypsin inhibitor H2 | ITIH2 | IPI00305461.4 | P19823 |
| Inter-alpha trypsin inhibitor H4 | ITIH4 | IPI00944960.1 | B2RMS9 |
| Keratin 6A | KRT6A | IPI00300725.7 | P02538 |
| Pro-platelet basic protein | PPBP | IPI00022445.1 | P02775 |
| Pregnancy zone protein short | PZPLs | IPI0092211 7.1 | B7Z7M2 |
| Serum amyloid A SAA2-SAA4 | SAA2-SAA4 | IPI00975939.1 | * |
| Afamin | AFM | IPI00019943.1 | P43652 |
| Alpha-2-HS-glycoprotein | AHSG | IPI00022431.2 | B7Z8Q2 |
| Alpha-1-microglobulin/bikunin precursor | AMBP | IPI00022426.1 | P02760 |
| Amyloid P component, serum | APCS | IPI00022391.1 | P02743 |
| Apolipoprotein A1 | APOA1 | IPI00021841.1 | P02647 |
| Apolipoprotein D | APOD | IPI00924574.1 | C9JF17 |
| Attractin | ATRN | IPI00027235.1 | O75882 |
| Zinc-alpha-2-glycoprotein | AZGP1 | IPI00 166729.4 | P25311 |
| Complement C1q C | C1QC | IPI000223 94.2 | P02747 |
| Complement C4A (Rodgers Blood Group) | C4A | IPI00843913.3 | B0V2C8 |
| Complement C4 binding protein alpha chain | C4BPA | IPI00021727.1 | P04003 |
| Complement C8 alpha chain | C8A | IPI00011252.1 | P07357 |
| CD5 antigen-like | CD5L | IPI00025204.1 | 043866 |
| Ceruloplasmin | CP | IPI00017601.1 | P00450 |
| Carboxypeptidase N, polypeptide 1 | CPN1 | IPI00010295.1 | P15169 |
| Carboxypeptidase N, polypeptide 2 | CPN2 | IPI00479116.2 | P22792 |
| Coagulation factor II (prothrombin) | F2 | IPI00019568.1 | P00734 |
| Fibulin 1 | FBLN1 | IPI00889740.1 | B1AHL2 |
| Fibronectin 1 | FN1 | IPI00022418.2 | P02751 |
| Haptoglobin | HP | IPI00641737.2 | P00738 |
| Haptoglobin-related protein | HPR | IPI00607707.2 | P00739 |
| Hemopexin | HPX | IPI00022488.1 | P02790 |
| Immunoglobulin Heavy Chain Alpha 2 | IGHA2 | IPI00940245.1 | Q9NPP6 |
| Immunoglobulin Heavy Chain Gamma 4 | IGHG4 | IPI00930442.1 | P01861 |
| Immunoglobulin Heavy Chain Mu 1 | IGHM1 | IPI00479708.6 | P01871 -1 |
| Immunoglobulin Heavy Chain Variable | IGHV | IPI00854743.1 | Q0ZCH6 |
| Immunoglobulin Heavy Chain Variable4-31 | IGHV4-31 | IPI00930124.1 | Q6MZV7 |
| Immunoglobulin J Chain | IGJ | IPI00964840.2 | D6RHJ6 |
| Immunoglobulin Kappa Variable 1-9 | IGKVI-9 | IPI00829751.3 | A2JA19 |
| Immunoglobulin Lambda Constant 2 | IGLC2 | IPI00154742.6 | P0CG05 |
| Immunoglobulin Lambda Variable3-19 | IGLV3-19 | IPI00829640.1 | Q6GMW4 |
| Immunoglobulin Lambda Variable 7-43 | IGLV7-43 | IPI00553092.2 | Q5NV83 |
| Inter-alpha-trypsin inhibitor H3 | ITIH3 | PI:IPI00028413.8 | Q06033 |
| Kallikrein B 1 | KLKB1 | IPI00654888.4 | P03952 |
| Keratin 2 | KRT2 | IPI00021304.1 | P35908 |
| Lipoprotein A | LPA | IPI00029168.1 | P08519 |
| Immunoglobulin Heavy Chain Variable b | IGHVb | IPI00828191.3 | A2NKM7 |
| Alpha-1-acid glycoprotein 1 | ORM1 | IPI00022429.3 | P02763 |
| Alpha-1-acid glycoprotein 2 | ORM2 | IPI00020091.1 | P19652 |
| Platelet Factor 4 | PF4 | IPI00022446.1 | P02776 |
| Pregnancy Zone Protein | PZP | IPI00025426.3 | P20742 |
| Immunoglobulin Kappa Variable | IGKV | IPI00816794.1 | A0N7J6 |
| S100 Calcium Binding Protein A8 | S100A8 | IPI00007047.1 | P05109 |
| Immunoglobulin Kappa Variable3-20 | IGKV3-20 | IPI00916434.1 | A2KBC3 |
| Antithrombin-III | SERPINC1 | IPI00032179.3 | P01008 |
| Alpha-2-antiplasmin | SERPINF2 | IPI00879231.1 | P08697 |
| Plasma protease C1 inhibitor | SERPING1 | IPI00877698.2 | B4E1F0 |
| Transferrin | TF | IPI00022463.2 | P02787 |
| Thrombospondin-1 | THBS1 | IPI00296099.6 | P07996 |
| Transthyretin | TTR | IPI00940791.2 | P02766 |
| Vitronectin | VTN | IPI00298971.1 | P04004 |
| von Willebrand factor | VWF | IPI00023014.3 | P04275 |
| Immunoglobulin Lambda Variable a | IGLVa | IPI00973531.1 | Q7Z2U7 |
| Keratin-associated protein 5-2 | KRTAP5-2 | IPI00555784.1 | Q701N4 |
| Proteoglycan 2 | PRG2 | IPI0084 7535.1 | P13727 |
| Apolipoprotein M | APOM | IPI00030739.1 | 095445 |
| Complement C5 | C5 | IPI00032291.2 | P01031 |
| Immunoglobulin Lambda Variable b | IGLVb | IPI00890733.1 | B1N7B9 |
| Complement Factor B | CFB | IPI00019591.2 | Q53F89 |
| Cytochrome P450 2U1 | CYP2U1 | IPI00783946.1 | Q7Z449 |
| Gelsolin | GSN | IPI00026314.1 | P06396 |
| Immunoglobulin Kappa Variable3D-15 | IGKV3D-15 | IPI01026045.1 | Q9UL83 |
| Immunoglobulin Lambda Variable c | IGLVc | IPI00827875.1 | A2NUT2 |
| Immunoglobulin Lambda Variable d | IGLVd | IPI00719373.3 | Q6NS95 |
| Kininogen-1 | KNG1 | IPI00215894.1 | P01042 |
| Keratin 9 | KRT9 | IPI000 19359.4 | P35527 |

### 1. ALPHA-2-MACROGLOBULIN (A2M)

A2M is a secreted protein. The mature protein extends from residues 24-1474, after cleavage of the signal peptide extending from 1-23. A2M is a proteinase inhibitor. It possesses a domain termed the "bait region," which contains specific cleavage sites for all four classes of proteinases. When a proteinase cleaves the bait region, a conformational change is induced in A2M thereby trapping the proteinase. The amino acid sequence of full length A2M is set forth as SEQ ID NO:1 (P01023).

### 2. APOLIPOPROTEIN B-100 (APOB)

APOB is a secreted protein and a major protein constituent of chylomicrons, LDL, and VLDL. The mature protein extends from residues 28-4563, after cleavage of the signal peptide extending from 1-27. APOB contains a vitellogenin protein domain. The amino acid sequence of full length APOB is set forth as SEQ ID NO:2 (P04114).

### 3. APOLIPOPROTEIN H (APOH)

APOH, also known as beta-2-glycoprotein, is a secreted protein. The mature protein extends from residues 20-345, after cleavage of the signal peptide extending from 1-19. APOH contains four CCP/SCR domains. The amino acid sequence of full length APOH is set forth as SEQ ID NO:3 (P02749).

### 4. COMPLEMENT COMPONENT 1, R SUBCOMPONENT (C1R)

C1R is a secreted protein belonging to the peptidase S1 family. The mature protein extends from residues 18-705, after cleavage of the signal peptide extending from 1-17. C1R is further cleaved into two peptides: complement C1r subcomponent light chain (residues 18-462) and heavy chain (residues 464-705). The amino acid sequence of full length C1R is set forth as SEQ ID NO:4 (Q53HU9).

### 5. COMPLEMENT COMPONENT 1, S SUBCOMPONENT (CIS)

CIS is a secreted protein. The mature protein extends from residues 16-688, after cleavage of the signal peptide extending from 1-15. CIS is a serine protease that shows sequence similarity to the peptidase S1 family of proteins. The amino acid sequence of full length C1S is set forth as SEQ ID NO:5 (P09871).

### 6. COMPLEMENT COMPONENT 3 (C3)

C3 is secreted protein. The mature protein extends from 23-1663, after cleavage of the signal peptide extending from 1-22. The mature protein may be further processed into ten fragments. C3 contains both NTR and anaphylatoxin-like domains. The amino acid sequence of full length C3 is set forth as SEQ ID NO:6 (P01024).

### 7. COMPLEMENT FACTOR H (CFH)

CFH is a secreted protein. The mature protein extends from residues 19-1231, after cleavage of the signal peptide extending from 1-18. CFH contains CCP/SCR domains and functions as a cofactor in the inactivation of C3and C5 convertase in the alternate complement pathway. The amino acid sequence of full length CFH is set forth as SEQ ID NO:7 (P08603).

### 8. IMMUNOGLOBULIN HEAVY CHAIN MU (IGHM)

IGHM is an immunoglobulin heavy chain sequence. The amino acid sequence (518 residues) of IGHM is set forth as SEQ ID NO:8 (Q6N030).

### 9. IMMUNOGLOBULIN HEAVY CHAIN GAMMA 1 (IGHG1)

IGHG1 is an immunoglobulin heavy chain sequence. The amino acid sequence (482 residues) of IGHG1 is set forth as SEQ ID NO:9 (Q7Z351).

### 10. KERATIN 1 (KRT1)

KRT1 shows sequence similarity to members of the intermediate filament family of proteins. The amino acid sequence (644 residues) of KRT1 is set forth as SEQ ID NO:10 (P04264).

### 11. KERATIN 10 (KRT10)

KRT10 shows sequence similarity to members of the intermediate filament family of proteins. The amino acid sequence (584 residues) of KRT10 is set forth as SEQ ID NO:11 (P13645).

### 12. LECTIN, GALACTOSIDE-BINDING, SOLUBLE, 3-BINDING PROTEIN (LGALS3BP)

LGALS3BP is secreted protein found ubiquitously in body fluids. The mature protein extends from residues 19-585, after cleavage of the signal peptide extending from 1-18. LGALS3BP contains one of each, BACK, BTB, and SRCR protein domains. The amino acid sequence of full length LGALS3BP is set forth as SEQ ID NO:12 (Q08380).

### 13. S100 CALCIUM-BINDING PROTEIN A9 (S100A9)

S100A9, also known as calgranulin B, extends from residues 1-114. The amino acid sequence of S100A9 is set forth as SEQ ID NO:13 (P06702).

### 14. ALPHA-1-ANTITRYPSIN (SERPINA1)

SERPINA1 is a secreted protein. The mature protein extends from residues 25-418, after cleavage of the signal peptide extending from 1-24. SERPINA1 is an inhibitor of serine proteases, and belongs to the serpin family of proteins. The amino acid sequence of full length SERPINA1 is set forth as SEQ ID NO:14 (P01009).

### 15. ALPHA-1-B-GLYCOPROTEIN (A1BG)

A1BG is a secreted protein. The mature protein extends from residues 22-495, after cleavage of the signal peptide extending from 1-21. A1BG shows sequence similarity to the variable regions of some immunoglobulin supergene family members. The amino acid sequence of full length A1BG is set forth as SEQ ID NO:15 (P04217).

### 16. ALBUMIN (ALB)

ALB is a secreted protein. The mature protein extends from residues 25-609, after cleavage of the signal peptide extending from 1-18 and the propeptide sequence. The amino acid sequence of full length ALB is set forth as SEQ ID NO:16 (P02768).

### 17. APOLIPOPROTEIN L1 (APOL1)

APOL1 is a secreted protein, mainly associated with large high-density lipoprotein particles. The mature protein extends from residues 28-398, after cleavage of the signal peptide extending from 1-27. The amino acid sequence of full length APOL1 is set forth as SEQ ID NO: 17 (014791).

### 18. COMPLEMENT COMPONENT 4B (C4B)

C4B is a secreted protein, also known as basic C4 and Chido blood group. The amino acid sequence (1744 residues) of full length C4B is set forth as SEQ ID NO: 18 (Q6U2E9).

### 19. COMPLEMENT COMPONENT 9 (C9)

C9 is a secreted protein. The mature protein extends from residues 22-559, after cleavage of the signal peptide extending from 1-21. The mature protein may be further processed into two fragments, C9a, C9b. C9 shows sequence similarity to the complement C6/C7/C8/C9 family of proteins. The amino acid sequence of full length C9 is set forth as SEQ ID NO: 19 (P02748).

### 20. COAGULATION FACTOR XII (F12)

F12 is a secreted protein. The mature protein extends from residues 20-615, after cleavage of the signal peptide extending from 1-19. This mature protein may further be cleaved into four peptides, beta-factor XIIa part 1 and part 2; and coagulation factor XIIa light and heavy chains. F12 shows sequence similarity to the peptidase S1 family of proteins. The amino acid sequence of full length F12 is set forth as SEQ ID NO:20 (P00748).

### 21. FIBRONECTIN 1 short (FN1s)

FN1s is a short isoform of FN1. The amino acid sequence of FN1s is set forth as SEQ ID NO:21 (B4DTK1).

### 22. VITAMIN D-BINDING PROTEIN (GC)

GC is a secreted protein. The mature protein extends from residues17-474, after cleavage of the signal peptide extending from 1-16. GC shows sequence similarity to the ALB/AFP/VDB family of proteins. The amino acid sequence of full length GC is set forth as SEQ ID NO:22 (P02774).

### 23. IMMUNOGLOBULIN HEAVY CHAIN ALPHA 1 (IGHA1)

IGHA1 is an immunoglobulin heavy chain sequence. The amino acid sequence (353 residues) of IGHA1 is set forth as SEQ ID NO:23 (P01876).

### 24. IMMUNOGLOBULIN HEAVY CHAIN MU 2 (IGHM2)

IGHM is an immunoglobulin heavy chain protein having two isoforms, secreted (isoform 1) and single-pass type I membrane (isoform 2). The amino acid sequence of isoform 2, which corresponds to the amino acid sequence (473 residues) of IGHM2 is set forth as SEQ ID NO:24 (P01871-2).

### 25. IMMUNOGLOBULIN KAPPA CONSTANT (IGKC)

IGKC is an immunoglobulin light chain sequence. The amino acid sequence (106 residues) of IGKC is set forth as SEQ ID NO:25 (P01834).

### 26. IMMUNOGLOBULIN LAMBDA-LIKE 1 (IGLL1)

IGLL1 is secreted protein. The mature protein extends from residues 38-213, after cleavage of the signal peptide from 1-17. The amino acid sequence of full length IGLL1 is set forth as SEQ ID NO:26 (P15814).

### 27. INTER-ALPHA-TRYPSIN INHIBITOR H1 (ITIH1)

ITIH1 is a secreted protein. The mature protein extends from residues 35-672, after cleavage of the signal peptide extending from 1-27, and the propeptides extending from 28-34, and 673-911. The amino acid sequence of full length ITIH1 is set forth as SEQ ID NO:27 (P19827).

### 28. INTER-ALPHA-TRYPSIN INHIBITOR H2 (ITIH2)

ITIH2 is a secreted protein. The mature protein extends from residues 55-702, after cleavage of the signal peptide extending from 1-18, and the propeptides extending from 19-54, and 703-946. The amino acid sequence of full length ITIH2 is set forth as SEQ ID NO:28 (P19823).

### 29. INTER-ALPHA-TRYPSIN INHIBITOR H4 (ITIH4)

ITIH4 is a secreted protein. The mature protein extends from residues 29-930, after cleavage of the signal peptide extending from 1-28. This mature protein may further be cleaved into two smaller chains by removal of the propeptide extending from 662-668. The amino acid sequence of full length ITIH4 is set forth as SEQ ID NO:29 (B2RMS9).

### 30. KERATIN 6A (KRT6A)

KRT6A is a cytoskeleton protein. The mature protein extends from residues 2-564, after cleavage of the initiator methionine at position 1. KRT6A shows sequence similarity to the intermediate filament family of proteins. The amino acid sequence of KRT6A is set forth as SEQ ID NO:30 (P02538).

### 31. PRO-PLATELET BASIC PROTEIN (PPBP)

PPBP is a secreted protein. The mature protein extends from residues 35-128, after cleavage of the signal peptide extending from 1-34. PPBP shows sequence similarity to the intercrine alpha family of proteins. The amino acid sequence of full length PPBP is set forth as SEQ ID NO:31 (P02775).

### 32. PREGNANCY ZONE PROTEIN s (PZPs)

PZPs is a short form of PZP. The amino acid sequence of PZPs is set forth as SEQ ID NO:32 (B7Z7M2).

### 33. SERUM AMYLOID A2 PRECURSOR (SAA2-SAA4)

SAA2-SAA4 is a secreted serum amyloid protein precursor. The protein extends from residues 19-208, after cleavage of the signal peptide extending from 1-18. The amino acid sequence of full length SAA2-SAA4 is set forth as SEQ ID NO:33 (GenBank Accession No. NP_001186673).

### 34. AFAMIN (AFM)

AFM is a secreted protein. The mature protein extends from residues 22-599, after cleavage of the signal peptide from 1-21. AFM shows sequence similarity to the ALB/AFP/VDB family of proteins. The amino acid sequence of full length AFM is set forth as SEQ ID NO:34 (P43652).

### 35. ALPHA-2-HS-GLYCOPROTEIN (AHSG)

AHSG is a protein predicted to residue in the extracellular space. AHSG shows sequence similarity to the CY superfamily of proteins. The amino acid sequence (433 residues) of AHSG is set forth as SEQ ID NO:35 (B7Z8Q2).

### 36. ALPHA-1-MICROGLOBULIN/BIKUNIN PRECURSOR (AMBP)

AMBP is a secreted protein. The mature protein extends from residues 20-344, after cleavage of signal peptide from 1-19. The mature protein may be further processed into three peptides, alpha-1-microglobulin, inter-alpha-trypsin inhibitor light chain, and trypstatin. AMBP shows sequence similarity to the calycin superfamily, lipocalin family of proteins. The amino acid sequence of full length AMBP is set forth as SEQ ID NO:36 (P02760).

### 37. AMYLOID P COMPONENT, SERUM (APCS)

APCS is a secreted protein. The mature protein extends from residues 20-223, after cleavage of the signal peptide extending from 1-19. APCS belongs to the pentaxin family of proteins involved in immune response. It can interact with DNA and histones, and may also scavenge nuclear material from damaged circulating cells, as well as function as a calcium dependent lectin. The amino acid sequence of full length APCS is set forth as SEQ ID NO:37 (P02743).

### 38. APOLIPOPROTEIN A1 (APOA1)

APOA1 is a secreted protein. The mature protein extends from residues 25-267, after cleavage of signal peptide from 1-18 and a propeptide. APOA1 shows sequence similarity to the apolipoprotein A1/A4/E family of proteins. The amino acid sequence of full length APOA1 is set forth as SEQ ID NO:38 (P02647).

### 39. APOLIPOPROTEIN D (APOD)

APOD is a secreted protein. The mature protein extends from residues 21-189, after cleavage of the signal peptide extending from 1-20. APOD shows sequence similarity to other members of the alpha-2 microglobulin protein superfamily. The amino acid sequence of APOD is set forth as SEQ ID NO:39 (C9JF17).

### 40. ATTRACTIN (ATRN)

ATRN is a membrane protein (isoform 1); however, isoforms 2 and 3, produced by alternative splicing, are secreted. The mature protein (isoform 1) extends from residues 84-1429, after cleavage of the signal peptide from 1-28, and the propeptide from 29-83. The amino acid sequence of full length ATRN is set forth as SEQ ID NO:40 (O75882).

### 41. ZINC-ALPHA-2-GLYCOPROTEIN 1 (AZGP1)

AZGP1 is a secreted protein. The mature protein extends from residues 21-298, after cleavage of the signal peptide extending from 1-20. AZGPlbelongs to the MHC class I family of proteins, and contains an Ig-like C1-type (immunoglobulin-like) domain. The amino acid sequence of full length AZGP1 is set forth as SEQ ID NO:41 (P25311).

### 42. COMPLEMENT COMPONENT 1, Q SUBCOMPONENT, C CHAIN (C1QC)

C1QC is a secreted protein. The mature protein extends from residues 29-245, after cleavage of the signal peptide extending from 1-28. C1QC contains C1q and collagen-like protein domains. The amino acid sequence of full length C1QC is set forth as SEQ ID NO:42 (P02747).

### 43. COMPLEMENT COMPONENT 4A (C4A)

C4A is a secreted protein, also known as acidic C4 and Rodgers blood group. The amino acid sequence (1744 residues) of full length C4A is set forth as SEQ ID NO:43 (B0V2C8).

### 44. COMPLEMENT COMPONENT 4 BINDING PROTEIN ALPHA (C4BPA)

C4BPA is a secreted protein. The mature protein extends from residues 49-597, after cleavage of the signal peptide extending from 1-48. C4BPA contains CCP/SCR domains and controls the classical pathway of complement activation. It also interacts with anticoagulant protein S and serum amyloid P component. The amino acid sequence of full length C4BPA is set forth as SEQ ID NO:44 (P04003).

### 45. COMPLEMENT COMPONENT 8 ALPHA CHAIN (C8A)

C8A is a secreted protein that is part of the membrane attack complex (MAC). The mature protein extends from residues 31-584, after cleavage of the signal peptide extending from 1-20, and the propeptide from 21-30. C8A shows sequence similarity to the complement C6/C7/C8/C9 family of proteins. The amino acid sequence of full length C8A is set forth as SEQ ID NO:45 (P07357).

### 46. CD5 ANTIGEN-LIKE (CD5L)

CD5L is a secreted protein. The mature protein extends from residues 20-347, after cleavage of the signal peptide from 1-19. CD5L contains three SRCR protein domains. The amino acid sequence of full length CD5L is set forth as SEQ ID NO:46 (O43866).

### 47. CERULOPLASMIN (CP)

CP, also known as ferroxidase, is a secreted protein. The mature protein extends from residues 20-1065, after cleavage of the signal peptide extending from 1-19. CP shows sequence similarity to the multicopper oxidase family of proteins. The amino acid sequence of full length CP is set forth as SEQ ID NO:47 (P00450).

### 48. CARBOXYPEPTIDASE N, POLYPEPTIDE 1 (CPN1)

CPN1, also known as anaphylatoxin inactivator, is a secreted protein. The mature protein extends from residues 21-458, after cleavage of the signal peptide extending from 1-20. CPN1 shows sequence similarity to the peptidase M14 family of proteins. The amino acid sequence of full length CPN is set forth as SEQ ID NO:48 (P15169).

### 49. CARBOXYPEPTIDASE N, POLYPEPTIDE 2 (CPN2)

CPN2 is a secreted protein. The mature protein extends from residues 22-545, after cleavage of the signal peptide extending from 1-21. CPN2 contains LLR, LLRCT, and LLRNT protein domains. The amino acid sequence of full length CPN2 is set forth as SEQ ID NO:49 (P22792).

### 50. COAGULATION FACTOR II (F2)

F2, also known as prothrombin, is a secreted protein. The mature protein extends from residues 44-622, after cleavage of the signal peptide extending from 1-24, and the propeptide from 25-43. The mature protein may be further processed into four peptides, activation peptide fragment 1 and 2; and thrombin light and heavy chain. F2 shows sequence similarity to the peptidase S1 family of proteins. The amino acid sequence of full length F2 is set forth as SEQ ID NO:50 (P00734).

### 51. FIBULIN 1 (FBLN1)

FBLN1 is an extracellular matrix protein than contains an EGF-like domain. The mature protein extends from residues 30-703, after cleavage of the signal peptide from 1-29. The amino acid sequence of full length FBLN1 is set forth as SEQ ID NO:51 (B1AHL2).

### 52. FIBRONECTIN (FN1)

FN1 is a secreted protein. The mature protein extends from residues 32-2386, after cleavage of the signal peptide extending from 1-31. The mature protein may be cleaved into four peptides, anastellin, ugl-Y1, ugl-Y2, and ugl-Y3. FN1 contains fibronectin type-I, type-II, and type-III protein domains. There are over a dozen isoforms produced by alternative splicing. The amino acid sequence of FN1 is set forth as SEQ ID NO:52 (P02751).

### 53. HAPTOGLOBIN (HP)

HP is a secreted protein. The mature protein extends from residues 19-406, after cleavage of the signal peptide from 1-18. The mature protein may be further cleaved into two peptides, haptoglobin alpha and haptoglobin beta. HP shows sequence similarity to the peptidase S1 family of proteins. The amino acid sequence of full length HP is set forth as SEQ ID NO:53 (P00738).

### 54. HAPTOGLOBIN-RELATED PROTEIN (HPR)

HPR is a secreted protein. The mature protein extends from residues 20-348, after cleavage of the signal peptide from 1-19. HPR shows sequence similarity to the peptidase S1 family of proteins. The amino acid sequence of full length HPR is set forth as SEQ ID NO:54 (P00739).

### 55. HEMOPEXIN (HPX)

HPX is a secreted protein. The mature protein extends from residues 24-462, after cleavage of the signal peptide extending from 1-23. HPX belongs to a family of proteins involved in transporting heme to the liver for iron recovery. The amino acid sequence of full length HPX is set forth as SEQ ID NO:55 (P02790).

### 56. IMMUNOGLOBULIN HEAVY CHAIN ALPHA 2 (IGHA2)

IGHA2 is an immunoglobulin heavy chain sequence. The amino acid sequence (416 residues) of IGHA2 is set forth as SEQ ID NO: 56 (Q9NPP6).

### 57. IMMUNOGLOBULIN HEAVY CHAIN GAMMA 4 (IGHG4)

IGHG4 is an immunoglobulin heavy chain sequence. The amino acid sequence (327 residues) of IGHG4 is set forth as SEQ ID NO:57 (P01861).

### 58. IMMUNOGLOBULIN HEAVY CHAIN MU 1 (IGHM1)

IGHM is an immunoglobulin heavy chain protein having two isoforms, secreted (isoform 1) and single-pass type I membrane (isoform 2). The amino acid sequence (452 residues) of isoform 1 is set forth as SEQ ID NO:58 (P01871-1).
The amino acid sequence (375 residues) of IGHM1 is set forth as SEQ ID NO:59.

### 59. IMMUNGLOBULIN HEAVY CHAIN VARIABLE REGION a (IGHVa)

IGHVa is an immunoglobulin heavy chain variable region sequence that extends from residues 1-131. The amino acid sequence of IGHVa is set forth as SEQ ID NO:60 (Q0ZCH6).

### 60. IMMUNOGLOBULIN HEAVY CHAIN VARIABLE REGION 4-31 (IGHV4-31)

IGHV4-31 is an immunoglobulin heavy chain sequence. The amino acid sequence (473 residues) of IGHV4-31 is set forth as SEQ ID NO:61 (Q6MZV7).

### 61. IMMUNOGLOBULIN J CHAIN (IGJ)

IGJ is a linker protein that links immunoglobulin monomers (IgM to pentamers, IgA to dimers) and binds these immunoglobulins polymers to the secretory component. The mature protein extends from residues 23-157, after cleavage of the signal peptide extending from 1-22. The amino acid sequence of full length IGJ is set forth as SEQ ID NO:62 (D6RHJ6).

### 62. IMMUNOGLOBULIN KAPPA VARIABLE 1-9 (IGKV1-9)

IGKV1-9 is an immunoglobulin light chain sequence. The amino acid sequence (117 residues) of IGLV is set forth as SEQ ID NO:63.

### 63. IMMUNOGLOBULIN LAMBDA CONSTANT 2 (IGLC2)

IGLC2 is an immunoglobulin light chain sequence. The amino acid sequence of IGLC2 (106 residues) is set forth as SEQ ID NO:64 (P0CG05).

### 64. IMMUNOGLOBULIN LAMBDA CHAIN VARIABLE 3-19 (IGLV3-19)

IGLV3-19 is an immunoglobulin light chain sequence. The amino acid sequence (233 residues) of IGLV3-19 is set forth as SEQ ID NO:65 (Q6GMW4).

### 65. IMMUNOGLOBULIN LAMBDA CHAIN VARIABLE 7-46 (IGLV7-46)

IGLV7-46 is an immunoglobulin light chain sequence. The amino acid sequence (98 residues) of IGLV7-46 is set forth as SEQ ID NO:66 (Q5NV83).

### 66. INTER-ALPHA-TRYPSIN INHIBITOR HEAVY CHAIN H3 (ITIH3)

ITIH3 is a secreted protein. The mature protein extends from residues 35-651, after cleavage of the signal peptide extending from 1-20, and the propeptides from 21-34, and 652-890. The amino acid sequence of full length ITIH3 is set forth as SEQ ID NO:67 (Q06033).

### 67. KALLIKREIN B1 (KLKB1)

KLKB1 is a secreted protein. The mature protein extends from residues 20-638, after cleavage of the signal peptide extending from 1-19. The mature protein may further be cleaved into two peptides, plasma kallikrein heavy chain and light chain. KLKB1 shows sequence similarity to the peptidase S1 family of proteins. The amino acid sequence of full length KLKB1 is set forth as SEQ ID NO:68 (P03952).

### 68. KERATIN (KRT2)

KRT2 is a cytoskeletal protein. KRT2 shows sequence similarity to the intermediate filament family of proteins. The amino acid sequence (639 residues) of KRT2 is set forth as SEQ ID NO:69 (P35908).

### 69. LIPOPROTEIN A (LPA)

LPA is the main constituent of lipoprotein(a). The mature protein extends from residues 20-4548, after cleavage of the signal peptide extending from 1-19. LPA shows sequence similarity to the peptidase S1 family, plasminogen subfamily of proteins. The amino acid sequence of full length LPA is set forth as SEQ ID NO:70 (P08519).

### 70. IMMUNOGLOBULIN HEAVY CHAIN VARIABLE b (IGHVb)

IGHVb (fragment of the NANUC-2 antibody) is an immunoglobulin heavy chain sequence. The amino acid sequence (133 residues) of IGHVb is set forth as SEQ ID NO:71 (A2NKM7).

### 71. OROSOMUCOID-1 (ORM1)

ORM1, also known as alpha-1-acid glycoprotein 1, is a secreted protein. The mature protein extends from residues 19-201, after cleavage of the signal peptide extending from 1-18. ORM1 shows sequence similarity to the calycin superfamily, lipocalin family of proteins. The amino acid sequence of full length ORM1 is set forth as SEQ ID NO:72 (P02763).

### 72. OROSOMUCOID-2 ALPHA-1-ACID GLYCOPROTEIN 2 (ORM2)

ORM2, also known as alpha-1-acid glycoprotein 2, is a secreted protein. The mature protein extends from residues 19-201, after cleavage of the signal peptide extending from 1-18. ORM2 shows sequence similarity to the calycin superfamily, lipocalin family of proteins. The amino acid sequence of full length ORM2 is set forth as SEQ ID NO:73 (P19652).

### 73. PLATELET FACTOR 4 (PF4)

PF4 is a secreted protein that is released during platelet aggregation, and neutralizes the anticoagulant effect of heparin. Mature PF4 protein extends from residues 32-101, after cleavage of the signal peptide extending from 1-31. A shorter form of PF4 extends from 48-101, and is a more potent inhibitor than the longer form. The amino acid sequence of full length PF4 is set forth as SEQ ID NO:74 (P02776).

### 74. PREGNANCY ZONE PROTEIN (PZP)

PZP is a secreted protein. The mature protein extends from residues 26-1482, after cleavage of the signal peptide extending from 1-25. PZP shows sequence similarity to the protease inhibitor 139 family of proteins. The amino acid sequence of full length PZP is set forth as SEQ ID NO:75 (P20742).

### 75. IMMUNGLOBULIN KAPPA CHAIN VARIABLE (IGKV)

IGKV (fragment of the REV25-2 antibody) is an immunoglobulin light chain sequence. The amino acid sequence (134 residues) of IGKV is set forth as SEQ ID NO:76 (A0N7J6).

### 76. S100 CALCIUM BINDING PROTEIN A8 (S100A8)

S100A8, also known as calgranulin A, extends from residues 1-93. The amino acid sequence of S100A8 is set forth as SEQ ID NO:77 (P05109).

### 77. IMMUNOGLOBULIN KAPPA VARIABLE 3-20 (IGKV3-20)

IGKV3-20 (scFV) is an immunoglobulin light chain sequence. The amino acid sequence (238 residues) of IGKV3-20 is set forth as SEQ ID NO:78 (A2KBC3).

### 78. ANTITHROMBIN-III (SERPINC1)

SERPINC1 is a secreted protein. The mature protein extends from residues 33-464, after cleavage of the signal peptide extending from 1-32. SERPINC1 is an inhibitor of serine proteases, and belongs to the serpin family of proteins. It is involved in regulating the blood coagulation cascade. The amino acid sequence of full length SERPINC1 is set forth as SEQ ID NO:79 (P01008).

### 79. ALPHA-2-ANTIPLASMIN (SERPINF2)

SERPINF2 is a secreted protein. The mature protein extends from residues 40-491, after cleavage of the signal peptide extending from 1-27 and propeptide from 28-39. The amino acid sequence of full length SERPINF2 is set forth as SEQ ID NO:80 (P08697).

### 80. PLASMA PROTEASE C1 INHIBITOR (SERPING1)

SERPING1 is a protease inhibitor extending from residues 1-505. The amino acid sequence of SERPING1 is set forth as SEQ ID NO:81 (B4E1F0).

### 81. TRANSFERRIN (TF)

TF is a secreted protein. The mature protein extends from residues 20-698, after cleavage of the signal peptide extending from 1-19. The amino acid sequence of full length TF is set forth as SEQ ID NO:82 (P02787).

### 82. THROMBOSPONDIN-1 (THBS1)

THBS1 is a glycoprotein that mediates cell-to-cell and cell-to-matrix interactions. The mature protein extends from residues 19-1170, after cleavage of the signal peptide extending from 1-18. The amino acid sequence of full length THBS1 is set forth as SEQ ID NO:83 (P07996).

### 83. TRANSTHYRETIN (TTR)

TTR is a thyroid hormone-binding protein. The mature protein extends from residues 21-147, after cleavage of the signal peptide extending from 1-20. The amino acid sequence of full length TTR is set forth as SEQ ID NO:84 (P02766).

### 84. VITRONECTIN (VTN)

VTN is a secreted protein. The mature protein extends from 20-478, after cleavage of the signal peptide extending from 1-19. The mature protein may further be cleaved into three peptides, vitronectin V65 subunit, vitronectin V10 subunit, and somatomedin-B. The amino acid sequence of full length VTN is set forth as SEQ ID NO:85 (P04004).

### 85. VON WILLEBRAND FACTOR (VWF)

VWF is a secreted protein. The mature protein extends from 23-2813, after cleavage of the signal peptide extending from 1-22. This mature protein may further be cleaved into two peptides von Willebrand antigen 2, and von Willenbrand factor. The amino acid sequence of full length VWF is set forth as SEQ ID NO:86 (P04275).

### 86. IMMUNOGLOBULIN LAMBDA CHAIN VARIABLE a (IGLVa)

IGLVa is an immunoglobulin light chain sequence extending from residues 1-234. The amino acid sequence of IGLVa is set forth as SEQ ID NO:87 (Q7Z2U7).

### 87. KERATIN-ASSOCIATED PROTEIN 5-2 (KRTAP5-2)

KRTAP5-2 interacts with hair keratins, and extends from residues 1-177. The amino acid sequence of KRTAP5-2 is set forth as SEQ ID NO:88 (Q701N4).

### 88. PROTEOGLYCAN 2 (PRG2)

PRG2 is protein that comes in a proform that is secreted, and a processed form that is found in the matrix of the eosinophil's large specific granule. The mature protein extends from residues 106-222, after cleavage of the signal peptide extending from 1-16, and a propeptide from 17-105. PRG2 contains a C-type lectin domain. The amino acid sequence of full length PRG2 is set forth as SEQ ID NO:89 (P13727).

### 89. APOLIPOPROTEIN M (APOM)

APOM is a secreted protein extending from residues 1-188. APOM binds sphingosine-1-phosphate, myristic acid, palmitic acid and stearic acid, retinol, all-trans-retinoic acid and 9-cis-retinoic acid. APOM shows sequence similarity to the calycin superfamily, lipocalin family of proteins. The amino acid sequence of full length APOM is set forth as SEQ ID NO:90 (O95445).

### 90. COMPLEMENT COMPONENT 5 (C5)

C5 is a secreted protein. A signal peptide extends from 1-18, and a propeptide extends from 674-677. C5 may further be cleaved into four peptides: C5 beta chain, C5 alpha chain, C5a anaphylatoxin, and C5 alpha' chain. The amino acid sequence (1676 residues) of full length C5 is set forth as SEQ ID NO:91 (P01031).

### 91. IMMUNOGLOBULIN LAMBDA CHAIN VARIABLE b

IGLVb (fragment of a cryocrystalglobulin 2 or CC2 antibody) is an immunoglobulin light chain sequence. The amino acid sequence (110 residues) of IGLVb is set forth as SEQ ID NO:92 (B1N7B9).

### 92. COMPLEMENT FACTOR B (CFB)

CFB shows sequence similarity to the peptidase S1 family of proteins. The amino acid sequence (764 residues) of CFB is set forth as SEQ ID NO:93 (Q53F89).

### 93. CYTOCHROME P450 2U1 (CYP2U1)

CYP2U1 is a multi-pass membrane protein extending from residues 1-544. CYP2U1 catalyzes the hydroxylation of arachidonic acid, docosahexaenoic acid and other long chain fatty acids. The amino acid sequence of full length CYP2U1 is set forth as SEQ ID NO:94 (Q7Z449).

### 94. GELSOLIN (GSN)

GSN, also known as actin-depolymerizing factor, is a calcium-regulated, actin-modulating protein that binds to the plus ends of actin monomers or filaments, preventing monomer exchange. GSN exists in two isoforms: isoform 1 is secreted, and isoform 2 is cytoplasmic. The mature isoform 1 protein extends from residues 28-782, after cleavage of the signal peptide extending from 1-27. The mature isoform 2 protein extends from residues 52-782.GSN shows sequence similarity to the villin family of proteins. The amino acid sequence of full length GSN is set forth as SEQ ID NO:95 (P06396).

### 95. IMMUNOGLOBULIN KAPPA VARIABLE 3D-15 (IGKV3D-15)

IGKV3D-15 is an immunoglobulin light chain sequence. The amino acid sequence (108 residues) of IGKV3D-15 is set forth as SEQ ID NO:96 (Q9UL83).

### 96. IMMUNOGLOBULIN LAMBDA CHAIN VARIABLE c (IGLVc)

IGLVc is an immunoglobulin light chain sequence. The amino acid sequence (235 residues) of IGLVc is set forth as SEQ ID NO:97 (A2NUT2).

### 97. IMMUNOGLOBULIN LAMBDA CHAIN VARIABLE d (IGLVd)

IGLVd is an immunoglobulin light chain sequence. The amino acid sequence (234 residues) of IGLVd is set forth as SEQ ID NO:98 (Q6NS95).

### 98. KININOGEN-1 (KNG1)

KNG1 is a secreted protein. The mature protein extends from residues 19-434, after cleavage of the signal peptide extending from 1-18. The mature protein may further be cleaved into six peptides, T-kinin, bradykinin, lysyl-bradykinin, low molecular weight growth-promoting factor, and kininogen-1 heavy and light chains. The amino acid sequence of full length KNG1 is set forth as SEQ ID NO:99 (P01042).

### 99. KERATIN 9 (KRT9)

KRT9 shows sequence similarity to the intermediate filament family of proteins. The amino acid sequence (623 residues) of KRT9 is set forth as SEQ ID NO:100 (P35527).

Microparticle-associated proteins of the present disclosure are not limited to the exemplary amino acid sequences shown above or listed in the sequence database entries of Table I. Rather the protein biomarkers of the present disclosure also include variants of the proteins listed in Table I. "Protein" as used herein refers to a polypeptide or fragment thereof.

"Variants" include proteins having one to several substitution(s), deletion(s), and/or addition(s) of an amino acid in comparison to a reference sequence. Conservative substitution tables providing functionally similar amino acids are well-known in the art. The following eight groups contain amino acids that are conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M).

"Fragments" include polypeptides that are shorter in length than the full length or mature protein of interest. If the length of a protein is x amino acids, a fragment is x-1 amino acids of that protein. The fragment may be shorter than this (e.g., x-2, x-3, x-4, ...), and is preferably 100 amino acids or less (e.g., 90, 80, 70, 60, 50, 40, 30, 20 or 10 amino acids or less). The fragment may be as short as 4 amino acids, but is preferably longer (e.g., 5, 6, 7, 8, 9, 10, 12, 15, 20, 25, 30, 35, 40, 50, 60, 70, 80, 90, or 100 amino acids).

The terms "identical" or percent "identity," in the context of two or more polypeptide sequences, refer to two or more sequences or subsequences that are the same. Two sequences are "substantially identical" if two sequences have a specified percentage of amino acid residues that are the same (e.g., 75%, 80%, 85%, 90%, 95%, 96%, 97%, 98% or 99% identity over a specified region, or, when not specified, over the entire sequence), when compared and aligned for maximum correspondence over a comparison window, or designated region as measured using known sequence comparison algorithms or by manual alignment and visual inspection. Preferably, the identity exists over a region that is at least about 10 amino acids in length, or more preferably over a region that is 20, 50, 100, 200, or more amino acids in length.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters. When comparing two sequences for identity, the sequences do not necessarily have to be contiguous, but any gap would carry with it a penalty that would reduce the overall percent identity. For blastn, the default parameters are Gap opening penalty=5 and Gap extension penalty=2. For blastp, the default parameters are Gap opening penalty=11 and Gap extension penalty=1.

Two examples of algorithms that are suitable for determining percent sequence identity and sequence similarity are the BLAST and BLAST 2.0 algorithms (Altschul et al., Nuc. Acids Res. 25:3389-3402, 1977; and Altschul et al., J. Mol. Biol. 215:403-410, 1990). Software for performing BLAST analyses is publicly available through the National Center for Biotechnology Information. This algorithm involves first identifying high-scoring sequence pairs (HSPs) by identifying short words of length W in the query sequence, which either match or satisfy some positive-valued threshold score T when aligned with a word of the same length in a database sequence. T is referred to as the neighborhood word score threshold. These initial neighborhood word hits act as seeds for initiating searches to find longer HSPs containing them. The word hits are extended in both directions along each sequence for as far as the cumulative alignment score can be increased. Cumulative scores are calculated using, for amino acid sequences, a scoring matrix. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T, and X determine the sensitivity and speed of the alignment. For amino acid sequences, the BLASTP program uses as defaults a word length of 3, and expectation (E) of 10, and the BLOSUM62 scoring matrix (Henikoff and Henikoff, Proc. Natl. Acad. Sci. USA 89:109-15, 1989) alignments (B) of 50, expectation (E) of 10, M=5, N=-4, and a comparison of both strands.

The BLAST algorithm also performs a statistical analysis of the similarity between two sequences (Karlin and Altschul, Proc. Natl. Acad. Sci. USA 90:5873-5787, 1993). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, more preferably less than about 0.01, and most preferably less than about 0.001.

The present disclosure provides tools for detecting the expression level of a microparticle-associated protein. "Detecting the expression level" includes detecting the presence (e.g., level above a threshold or detectable level) or detecting the absence (e.g., level below a threshold or undetectable level) of a microparticle-associated protein in a sample from a pregnant subject.

During development of the present disclosure numerous microparticle-associated proteins were determined to be elevated in samples from subjects having preterm births (as compared to samples from subjects have term births), and are therefore termed "preterm birth biomarkers." The preterm birth biomarkers identified as described in Example 1 include the following: A1BG, A2M, AFM, AHSG, ALB, AMBP, APOD, APOL1, APOM, ATRN, AZGP1, C8A, C9, CD5L, CFB, CPN2, F12, FBLN1, FN1, FN1s, GC, GSN, HPR, HPX, IGHM1, IGHM2, IGJ, IGLVb, ITIH1, ITIH2, ITIH3, ITIH4, KLKB1, KNG1, KRT1, KRT10, KRT2, KRT6A, LGALS3BP, ORM1, ORM2, PRG2, S100A8, S100A9, SAA2-SAA4, SERPINA1, SERPINC1, SERPINF2, SERPING1, TF, TTR, VTN, and VWF. Preferably, the methods comprise detecting one or more preterm birth biomarkers selected from the group consisting of: A1BG, AFM, AHSG, ALB, AMBP, APOD, APOL1, APOM, ATRN, AZGP1, C8A, CD5L, CFB, CPN2, F12, GSN, HPR, HPX, IGHM1, IGHM2, IGJ, IGLVb, ITIH1, ITIH2, ITIH3, KNG1, KRT1, KRT10, KRT2, KRT6A, LGALS3BP, PRG2, SAA2-SAA4, SERPINF2, SERPING1, TF, VTN, and VWF. Preferably, the methods further comprise detecting one or more preterm birth biomarkers selected from the group consisting of A2M, C9, FBLN1, FN1, FN1s, GC, ITIH4, KLKB1, ORM1, ORM2, S100A8, S100A9, SERPINA1, SERPINC1, and TTR. Preferably, the methods comprise detecting from four to twelve (e.g.,4, 5, 6, 7, 8, 9, 10, 11 or 12)of the preterm birth biomarkers selected from the group consisting of A1BG, A2M, ALB, APOD, APOL1, AZGP1, HPX, IGHM1, IGHM2, SERPINA1, SERPINC1 and TF.

Additionally during development of the present disclosure numerous microparticle-associated proteins were determined to be reduced in samples from subjects having preterm births (as compared to samples from subjects have term births), and are therefore termed "term birth biomarkers." The term birth biomarkers identified as described in Example 1 include the following: APCS, APOA1, APOB, APOH, C1QC, C1R, CIS, C3, C4A, C4B, C4BPA, C5, C9, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, PZPs, and THBS1. Preferably, the methods comprise detecting one or more term birth biomarkers selected from the group consisting of: APCS, APOA1, APOH, C1QC, C1R, CIS, C3, C4A, C4B, C4BPA, C5, CFH, CP, CPN1, CYP2U1, F2, HP, IGHA1, IGHA2, IGHG1, IGHG4, IGHM, IGHV4-31, IGHVa, IGHVb, IGKC, IGKV, IGKV1-9, IGKV3-20, IGKV3D-15, IGLC2, IGLL1, IGLV3-19, IGLV7-43, IGLVa, IGLVc, IGLVd, KRT9, KRTAP5-2, LPA, PF4, PPBP, PZP, and PZPs. Preferably, the methods further comprise detecting one or more term birth biomarkers selected from the group consisting of APOB, C9 and THBS1. Preferably, the methods comprise detecting from one to six (e.g., 1, 2, 3, 4, 5 or 6) of the term birth biomarkers selected from the group consisting of C1R, C3, C4B, CFH, IGHA2, and IGKVI-9.

### Pregnant Subjects

Preferably, the subject is a pregnant woman. Preferably, the pregnant woman is in the first trimester (e.g., weeks 1-12 of gestation), second trimester (e.g., weeks 13-28 of gestation) or third trimester of pregnancy (e.g., weeks 29-37 of gestation). Preferably, the pregnant woman is in early pregnancy (e.g., from 8, 9, 10, 11, 12, 13, 14, 15, 16, 17, 18, 19 or 20, but earlier than 21 weeks of gestation; from 20, 19, 18, 17, 16, 15, 14, 13, 12, 11, 10 or 9, but later than 8 weeks of gestation). Preferably, the pregnant woman is in mid-pregnancy (e.g., from 21, 22, 23, 24, 25, 26, 27, 28, 29 or 30, but earlier than 31 weeks of gestation; from 30, 29, 28, 27, 26, 25, 24, 23, 22 or 21, but later than 20 weeks of gestation). Preferably, the pregnant woman is in late pregnancy (e.g., from 31, 32, 33, 34, 35, 36 or 37, but earlier than 38 weeks of gestation; from 37, 36, 35, 34, 33, 32 or 31, but later than 30 weeks of gestation). The stage of pregnancy can be calculated from the first day of the last normal menstrual period of the pregnant subject.

Preferably, the pregnant human subject is primagravida. Preferably, the pregnant subject multigravida. Preferably, the pregnant subject may have had at least one prior spontaneous preterm birth (e.g., birth prior to week 38 of gestation). Preferably, the pregnant human subject is asymnptomatic. Preferably, the subject may have a risk factor of PTB such as a history of pre-gestational hypertension, diabetes mellitus, kidney disease, known thrombophilias and/or other significant preexisting medical condition (e.g., short cervical length).

### Samples

A sample for use in the methods of the present disclosure is a biological sample obtained from a pregnant subject prior to 38 weeks of gestation. Preferably, the sample is collected during a stage of pregnancy described in the preceding section. Preferably, the sample is a blood, saliva, tears, sweat, nasal secretions, urine, amniotic fluid or cervicovaginal fluid sample. Preferably, the sample is a blood sample, preferably serum or plasma. Preferably, the sample has been stored frozen (e.g., -20°C or -80°C).

### Methods For Assessing Risk of Preterm Birth

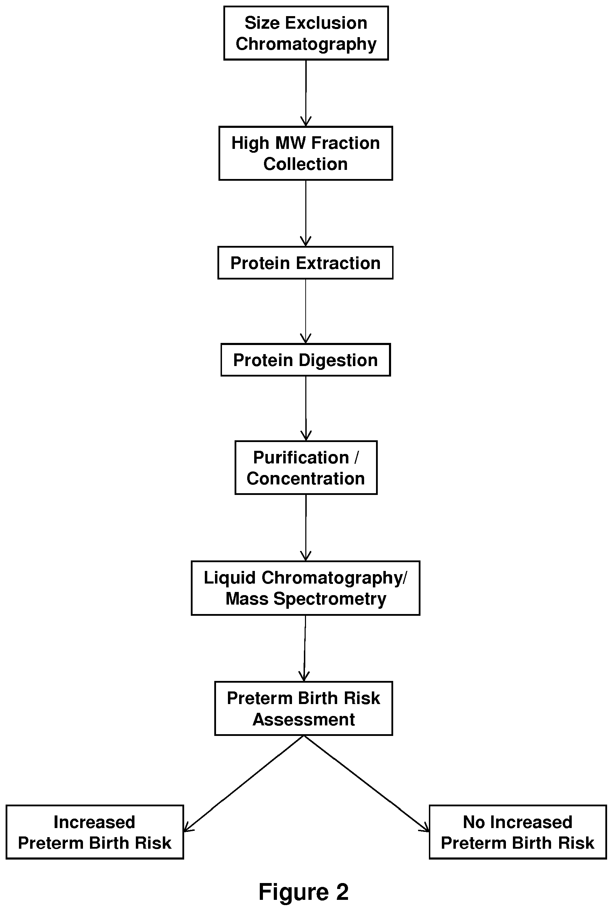

Preferably, detecting the expression level (e.g., including detecting the presence) of one or both of preterm birth biomarkers and term birth biomarkers is done using a liquid chromatography / mass spectrometry (LC/MS)- based proteomic analysis such as that depicted in the flow chart of Figure 2. Preferably, the method involves subjecting a sample to size exclusion chromatography and collecting the high molecular weight fraction to obtain a microparticle-enriched sample. The microparticle-enriched sample is then extracted before digestion with a proteolytic enzyme such as trypsin to obtain a digested sample comprising a plurality of peptides. The digested sample is then subjected to a peptide purification / concentration step before liquid chromatography and mass spectrometry to obtain a proteomic profile of the sample. Preferably, the purification / concentration step comprises reverse phase chromatography (e.g., ZIPTIP pipette tip with 0.2 µL C18 resin, from Millipore Corporation, Billerica, MA). The proteomic profile is analyzed to determine the presence of one or both of preterm birth biomarkers and term birth biomarkers. Preferably, preparation of the proteomic profile is done using one or both of the Rosetta ELUCIDATOR data management and analysis system (Ceiba, Boston, MA) and the Mascot search engine (Matrix Systems, Boston MA), which uses mass spectrometry data to identify proteins from primary sequence databases (Pappin et al., Curr Biol, 3:327-332, 1993; Perkins et al., Electrophoresis, 20:35551-2567, 1999; and Koenig et al., J. Proteome Res, 7:3708-3717, 2008).

Preferably, detecting the expression level (e.g., including detecting the presence) of one or both of preterm birth biomarkers and term birth biomarkers is done using an antibody-based method. Suitable antibody-based methods include but are not limited to enzyme linked immunosorbent assay (ELISA), Western blot, and antibody microarray.

Preferably, the methods include calculating a risk score of preterm birth before gestational week 34 based upon the detection of the expression level of 1 to 18 microparticle-associated proteins of the smallplex consisting of all twelve of alpha-1B-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF); and all six of (C4B), complement factor H (CFH), Ig heavy chain alpha 2 (IGHA2), and Ig kappa variable1-9 (IGKV1-9). The risk score is calculated based on a quintile scale shown in Table II.

**Table II. Quintile Scale for Risk Score Calculation for Smallplex**

| **Preterm Birth Biomarkers above threshold and Term Birth Biomarkers below threshold (# out of 18 total)** | **Score** |
|---|---|
| 0 | 0 (low risk) |
| 1-2 | 1 |
| 3-5 | 2 |
| 6-8 | 3 |
| 9-10 | 4 |
| 11-18 | 5 (high risk) |

### Methods For Reducing Risk of Preterm Birth

Cervical cerclage and progesterone supplementation have been shown to be effective in preventing preterm birth (Committee on Practice Bulletins, Obstetrics & Gynecology, 120:964-973, 2012).

Currently progesterone supplementation for the prevention of recurrent spontaneous preterm birth is offered to: women with a singleton pregnancy and a prior spontaneous preterm birth; and women with no history of spontaneous preterm birth who have an incidentally detected very short cervix (<15 mm). The present disclosure provides tools to identify additional pregnant subjects that may benefit from progesterone supplementation. These subjects include the following: pregnant women who are primigravidas without a history of risk and without an incidentally detected very short cervix; and pregnant women who are multigravidas but who did not previously have a spontaneous preterm birth.

Pregnant subjects determined to be at increased risk for preterm birth are recommended to receive or are administered progesterone until 36 weeks of gestation (e.g., upon identification or between 16 weeks, 0 days and 20 weeks, 6 days gestation until 36 weeks gestation). Preferably, progesterone supplementation comprises 250 mg weekly intramuscular injections. Preferably, the weekly progesterone supplementation comprises administration of MAKENA hydroxyprogesterone caproate injection (Ther-Rx Corporation, St. Louis, MO). Preferably, progesterone supplementation comprises vaginal progesterone in doses between 50 and 300 mg daily, between 75 and 200 mg daily or between 90 and 110 mg daily.

Preferably, in women with a singleton pregnancy determined to be at increased risk for preterm birth and who have had a documented prior spontaneous preterm birth at less than 34 weeks of gestation and short cervical length (less than 25 mm) before 24 weeks of gestation, are recommended to receive or are given a cervical cerclage (also known as tracheloplasty or cervical stitch). Preferably, the cervical cerclage is a McDonald cerclage, alternatively it is a Shirodkar cerclage or an abdominal cerclage.

### Kits

Preferably, a kit of reagents capable of one or both of preterm birth biomarkers and term birth biomarkers in a sample is provided. Reagents capable of detecting protein biomarkers include but are not limited to antibodies. Antibodies capable of detecting protein biomarkers are also typically directly or indirectly linked to a molecule such as a fluorophore or an enzyme, which can catalyze a detectable reaction to indicate the binding of the reagents to their respective targets.

Preferably, the kits further comprise sample processing materials comprising a high molecular gel filtration composition (e.g., agarose such as SEPHAROSE) in a low volume (e.g., 1ml) vertical column for rapid preparation of a microparticle-enriched sample from plasma. For instance, the microparticle-enriched sample can be prepared at the point of care before freezing and shipping to an analytical laboratory for further processing.

Preferably, the kits further comprise instructions for assessing risk of preterm birth. As used herein, the term "instructions" refers to directions for using the reagents contained in the kit for detecting the presence (including determining the expression level) of a protein(s) of interest in a sample from a subject. The proteins of interest may comprise one or both of preterm birth biomarkers and term birth biomarkers. Preferably, the instructions further comprise the statement of intended use required by the U.S. Food and Drug Administration (FDA) in labeling in vitro diagnostic products. The FDA classifies in vitro diagnostics as medical devices and required that they be approved through the 510(k) procedure. Information required in an application under 510(k) includes: 1) The in vitro diagnostic product name, including the trade or proprietary name, the common or usual name, and the classification name of the device; 2) The intended use of the product; 3) The establishment registration number, if applicable, of the owner or operator submitting the 510(k) submission; the class in which the in vitro diagnostic product was placed under section 513 of the FD&C Act, if known, its appropriate panel, or, if the owner or operator determines that the device has not been classified under such section, a statement of that determination and the basis for the determination that the in vitro diagnostic product is not so classified; 4) Proposed labels, labeling and advertisements sufficient to describe the in vitro diagnostic product, its intended use, and directions for use, including photographs or engineering drawings, where applicable; 5) A statement indicating that the device is similar to and/or different from other in vitro diagnostic
products of comparable type in commercial distribution in the U.S., accompanied by data to support the statement; 6) A 510(k) summary of the safety and effectiveness data upon which the substantial equivalence determination is based; or a statement that the 510(k) safety and effectiveness information supporting the FDA finding of substantial equivalence will be made available to any person within 30 days of a written request; 7) A statement that the submitter believes, to the best of their knowledge, that all data and information submitted in the premarket notification are truthful and accurate and that no material fact has been omitted; and 8) Any additional information regarding the in vitro diagnostic product requested that is necessary for the FDA to make a substantial equivalency determination.

### EXAMPLES

Abbreviations: BSA (bovine serum albumin); LC (liquid chromatography); MS (mass spectrometry); P1 (first pregnancy); P2 (second pregnancy); PTB (preterm birth); PT/T (preterm/term ratio); QC (quality control); TIC (total ion current)

### EXAMPLE 1

### Serum Microparticle-Associated Biomarkers Predictive of Risk of Preterm Birth

This example details the proteomic differentiation of maternal serum microparticles obtained from term and preterm patients early in the second trimester. The methods described herein exploit the fact that placental-derived microparticles freely circulate in high titer in maternal blood and reflect the status of the maternal-fetal interface.

### Materials and Methods

*Patient Population.* This study represents a case-controlled analysis of proteomic biomarkers collected from frozen serum samples obtained from early stage pregnant women, from July to November 2009 and released in December 2011, as part of a government initiative under informed consent by Biomnis, France. Non-fasting serum samples were collected for genetic screening and stored in a bio-repository from which a cohort of 168 serum samples were secured and evaluated for study inclusion. Inclusion criteria included normal healthy asymptomatic pregnant women with the absence of a history of pre-gestational hypertension, diabetes mellitus, kidney disease, known thrombophilias or any other significant preexisting chronic medical disease. Medical annotation confirmed gravida conditions (first or second pregnancy), no multiple gestations, live birth, absence of congenital abnormalities and non-in vitro fertilization and non-intracytoplasmic sperm injection pregnancy. Medical annotation from the repository included maternal age, weight, height, and reported tobacco use. Sample data included confirmation of blood draw at weeks 15-17 gestation, infant's birth date and calculation of gestational duration. Outcome status included baby's sex and weight, gestational age (full term birth defined as ≥ 37 weeks gestation), preterm birth (preterm defined as non-iatrogenic spontaneous preterm birth between 23 weeks 0/7 days and 34 weeks 0/7 days gestation).

*Sample Handling.* All samples were frozen -20°C if transit was scheduled for 4 hours or greater from the time of the initial blood draw or refrigerated if transit to the central lab was scheduled to occur less than 4 hours after serum blood draw. After being blinded by identifying the sample number, all serum samples were stored at -80°C (Biomnis, Lyon France). Samples were transported from the repository to the analytical labs (Kannapolis, NC) frozen on dry ice in ∼ 1-2 mL aliquots for the purpose of isolating serum microvesicles and characterizing the proteomic content of serum fractions. Once thawed on the bench at room temperature (∼22°C), microparticles were isolated from 1 mL serum samples by gel filtration methods (Gercel-Taylor et al., Anal Biochem, 428:44-53, 2012). All serum samples were processed and LC/MS analysis was conducted in a double-blinded manner until the end of the study.

*Microparticle Isolation.* Microparticles were isolated from healthy AB serum for quality control and calibration purposes (Sigma Aldrich, St. Louis, MO), and in a blinded manner from term and preterm patient serum samples (n=48). Serum fractions and microparticles were isolated using a 2% such as SEPHAROSE size-exclusion chromatography column (Bio-Rad Econo column 2.5 cm ID x 10 cm with 100 mL of 40% v/v such as SEPHAROSE slurry in ddH20). Samples were processed using a GE AKTA Purifier 10 system (GE Healthcare, Piscataway, NJ) with a Frac 950 fraction collector at room temperature using a flow rate of 2mL/min and a 30 second collection periodicity to obtain 1 mL elution samples.

Serum fractions yielded two partially resolved peaks (peaks 1 and 2) with retention volumes of ∼10 mL and ∼35 mL when monitored by A280. Peak 1 elution samples (< 15 minutes) consisted of high molecular weight fractions of analytical interest that could not permeate the size exclusion column (extracellular vesicles and protein aggregates). The first 7 x 1 mL fractions associated with peak 1 absorbance at A280 were collected, pooled, separated into 7 x 1-mL aliquots for subsequent processing and stored at -80°C. Each pooled fraction was analyzed for total protein and exosomal content quantified and a split aliquot was processed by LC/MS after an ultrafiltration procedure.

The majority of the remaining elution volume and conductivity was associated with the latter peak (∼ 40mL) as might be expected for ions, charged amino acids/peptides and other low molecular weight species (Gercel-Taylor et al., Anal Biochem, 428:44-53, 2012). This parameter was used to demark the beginning of the contaminating second peak, which is devoid of microparticles.

*Microparticle Concentration and Analysis.* A 1 mL aliquot was thawed at room temperature, and subjected to centrifugation at 4,000 x g for approximately 10 min at room temperature using a 1M Da cut off Vivaspin ultrafiltration spin column (Sartorius, Stedim North America Inc. NY) to achieve a 3-fold reduction in sample volume. The spin column was inverted and centrifuged for 5 min at 4,000 x g to collect the retentate harboring the desired microparticles. Total protein concentration in the exosomal preparation was determined using a Thermo Scientific BCA Protein Assay after concentration.

Microvesicular particles (microparticles) were quantified using the Nanoparticle Tracking Analysis (Dragovic et al., Nanomedicine, 7:780-788, 2011) method (NanoSight, LM 10 system, software: NanoSight NTA version 2.2 NanoSight Ltd. Wiltshire, UK). This stochastic single particle detection method is based on light scattering from individual particles using the Stokes-Einstein relationship to measure the average (time-averaged, field of view) number of particles and their predicted hydrodynamic diameter.

The instrument was calibrated and validated with standard beads from 50 - 400 nm in diameter (NanoSight, Wiltshire, UK). One hundred nm beads were run each day to calibrate the system. These notionally 100 nm diameter beads (n >30) yielded experimental diameters of ∼ 97 (±2) nm in aqueous, low viscosity (∼1 cP) solutions at room temperature. All calibrations and patient-derived microvesicles were analyzed in triplicate. Samples were blinded as to cohort of origin and were processed contemporaneously.

*Protein Extraction and Purification.* Proteins were solubilized from the concentrated peak 1 fractions for LC/MS analysis using a modified TRIzol® method (Invitrogen Inc. Carlsbad, CA). Extraction samples were prepared in duplicate and split prior to protein precipitation to form a total of four protein pellets per sample. Initial sample extraction aliquots consisted of 240 µl of cold 50 mM ammonium bicarbonate (stored at 4°C) and 60 µl of concentrated microparticle sample. A 1.0 mL aliquot of TRIzol® Reagent was added and the sample tube was vortexed and incubated at room temperature for 5 minutes. A total of 200 µl of chloroform was added and the sample tube was inverted several times and incubated at room temperature for three minutes. The sample was centrifuged at 12,000 x g for 15 minutes at 4°C on a Microfuge 22R centrifuge. The top aqueous phase was removed and 300 µl of pure ethanol was added to the sample and centrifuged at 2,000 x g at for five minutes at 4°C. The Phenolethanol solution was split into two aliquots and 750 µl of isopropyl alcohol was added. The samples were centrifuged at 12,000 x g for 10 minutes at 4°C to pellet the proteins. Each protein pellet was washed in triplicate with 0.3M guanidine HCL followed by a wash with pure ethanol. Protein pellets were suspended in a solution of 50 µl of ammonium bicarbonate (50 mM) and 150 µl of 0.1% RapiGest surfactant (Waters Corporation, Milford, MA)..

The four pellets from sample extraction were combined to provide sufficient material for digestion. A volume of each sample corresponding to 35 µg of protein (based on the protein quantitation results) was used. The sample volumes were made equal by adding 50 mM ammonium bicarbonate (AmBic) to a volume of 110 µL. Disulfide bonds were reduced by adding 200 mM dithiothreitol to each sample and heating the tubes to 80°C for 15 minutes. Preparations were alkylated with 400 mM iodoacetamide (IA) by placing the tubes in the dark for 30 minutes at room temperature. Preparations were digested by adding 1.4 µg trypsin (Promega Corporation, Madison, WI) to each tube and incubating overnight at 37°C. An internal standard, alcohol dehydrogenase digest from yeast (Waters Corporation, Milford, MA), was added to a final concentration of 50 fmol/µg protein. The samples were then concentrated and purified prior to LC/MS analysis using a Millipore ZipTip with 0.2 µL C18 resin (Millipore Corporation, Billerica, MA) eluted with 60µl of a 20% formic acid/80% acetonitrile solution. Samples were dried on a centrifugal vacuum evaporator and reconstituted in 50 µl 0.1% formic acid in water.

*LC*/*MS Quality Control and Chromatography.* Multiple standards were prepared to evaluate method and system performance throughout the study including the use of bovine serum albumin (BSA), which was subjected to reduction/alkylation and digestion in parallel with sample digestion. In addition, a standard *E. coli* digest (Waters) and BSA digest (Michrom) were used to verify instrument performance before and after sample analysis. A 300 fmol injection of the BSA digest was identified by MASCOT with >200 peptide fragments covering greater that 70% of the protein. In addition, a 1 µg injection of an *E. coli* digest resulted in the detection of >250 proteins.

A pooled microparticle QC standard was prepared by mixing 5 µL of each of the 24 microparticle samples and used during analysis of the study sample set. Prior to sample analysis, a minimum of two injections and LC/MS/MS analyses of the pooled microparticle QC standard was performed. Data consistency and system performance were ensured by utilizing an acceptable %RSD of less than 20%. The %RSD of the sample total ion chromatogram was 5.2% and the number of proteins identified had a %RSD of 3.3%

*Analytical Methods.* Analytical sample were analyzed on a Thermo Scientific LTQ Orbitrap XL mass spectrometry system (LTQ Orbitrap XL) coupled to a Waters nanoACQUITY UPLC system (nanoACQUITY UPLC) using standard sample parameters.

Study samples injections were sequenced in a blinded manner and bracketed by a pair of pooled microparticle QC standard injections after every eight sample injections. Data from all study samples were acquired using Data Dependent™ scans (Nth order double play) on the LTQ Orbitrap XL. Database searches were performed in Elucidator (Rosetta Biosoftware) using MASCOT (Matrix Sciences, London, UK).

*Data Analysis.* The pooled microparticle QC and the study samples were evaluated to confirm data quality. Mass spectrometry total ion current (TIC) outputs were assessed for signal quality and changes in signal intensity. Results were also monitored for signal trends, such as a consistent increase or decrease in TIC maximum values, and MASCOT search results were used to monitor the quality of the MS data. No significant outliers were identified during this preliminary evaluation.

Raw MS data files for the pooled microparticle QCs and study samples, collected on the Thermo Orbitrap XL system, were processed in Elucidator. MS data were grouped in Elucidator based on sample groups, identified as group A (term), group B (preterm) and QC. Sample groups were used to assist in data alignment, feature identification, and for QC assessment and group comparisons. Data were processed from retention time 20-180 min.

A label-free differential expression LC/MS/MS method was used to qualitatively compare protein expression levels between two sample groups. The samples were analyzed and the results were processed using the Rosetta Elucidator software package (version 3.3.0.1.SP3_CRE52.21, Rosetta Biosoftware). Data processing in Elucidator was performed in multiple steps that included mass correction and mass signal alignment of detected signals, retention time alignment of detected signals, and feature selection, with features representing mass signals detected within the study set. Mass signals were annotated using the Peptide Tellers function in the Elucidator with the corresponding peptide and protein information based on the database search results using a 1% false discovery rate cut off. Data processing of the Thermo Orbitrap data resulted in detection of ∼200 proteins. QC review of the aligned data indicated the mass signals were correctly aligned in the Elucidator software. For retention time alignment, mathematical adjustments were performed to time align the same detected signals across all samples. QC assessment of this retention time shift indicated that all adjustments had a maximum shift of <1.5 min.

The % CVs across the pooled QC samples and the study samples, grouped into groups A and B, were evaluated to confirm data quality. Data demonstrate that the % CVs for all of the isotope groups monitored across the samples were smaller for the replicate pooled QC injections compared to the A and B sample groups.

Thermo Orbitrap data files were searched using the MASCOT search engine against the IPI (Human, v3.87) database released September 27, 2011 appended with yeast ADH (EMBL-European Bioinformatics Institute, Hinxton, Cambridge, UK). The aligned mass features were annotated with these database search results using the results from the system Peptide Tellers and a predicted error rate of 1%.

MS data were summarized to the feature level, normalized to the mean of the features, and an ANOVA test was performed to compare the expression results between sample groups A and B. Candidate differentially expressed features were determined based on a p<0.05, 0.01 and 0.005. Differentially expressed features were summarized by protein based on the results of the database search.

Data from the studies (n=48 patients representing ∼500,000 peptide fragments), medical annotation and raw intensity and peptide scores were compiled into an automated database for subsequent interrogation. The mean intensity scores were transformed as described by the vendor (Rosetta Inpharmatics Seattle, WA) and blindly analyzed by an independent third party (MF, BIoIT Solutions, Silver Springs, MD).

Significant differences at the peptide and protein levels were characterized in both the primigravida (P1 and second pregnancy (P2) populations. Biological relevance was defined when statistical significance was observed in both P1 and P2 cohorts (p<0.05). In addition, definition of biomarker relevance required that the differential significance defined by the Elucidator system be reconfirmed by blinded third party, that directional ratios in preterm and term cohorts be observed in both P1 and P2 groups, and that the significance was observed in two or more peptide fragments with an average PT/T ratio >1.0. For all determinations the protein teller criteria was set at > 0.9 and false positive protein identification < 1%.

### Results

The demographics of the asymptomatic term and preterm populations (n= 48) presenting between weeks 15 and 17 of gestation are summarized in Table 1-1. No significant differences were noted amongst these groups.

**Table 1-1. Patient Demographics**

| **Parameter** | **Term Birth** | **Preterm Birth** | **p Value** |
|---|---|---|---|
| Sample size | 24 | 24 | |
| Primigravida (P1) | 12 | 12 | NS |
| 2nd pregnancy (P2) | 12 | 12 | NS |
| Maternal age (yrs) | 28.9 +/- 1.75 | 27.75 +/- 3.8 | NS |
| Maternal body mass index | 23.9 +/- 3.57 | 28.4 +/- 4.0 | NS |
| Prior preterm delivery | 0 | 0 | NS |
| Multiparity | 0 | 0 | NS |
| Tobacco use | 1 | 4 | NS |
| Gestational age at delivery (wks) | > 37 | < 34 | |
| Birth weight | 3305 +/- 228 | 2415 +/- 330 | <0.01 |

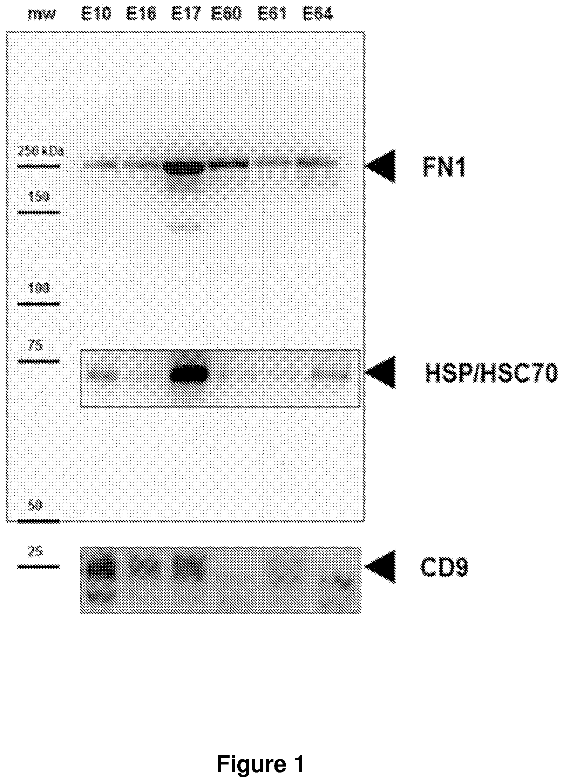

The extracellular vesicles isolated from serum samples eluted by gel filtration methods were characteristic of circulating serum vesicles reported by others (Gercel-Taylor et al., Anal Biochem, 428:44-53, 2012), and expressed canonical proteins indicative of suck particles (FIG. 1)

Serum microparticles isolated from the two groups were not significantly different across any of the phenotypic parameters evaluated at weeks 15-17 gestation including particle density (4.4 - 6.1 x x10¹¹ per ml patient serum), mean particle size (67-109 nm), and protein yield (488 -560 µg/ml sera). The serum vesicles that were isolated from the high molecular (peak 1) fractions, however, differed markedly in protein signature between the preterm and term cohorts.

A total of ∼500,000 peptide fragments were identified in the open proteomic assessment of the 48 patient samples and QC specimens. Study 1 (primigravida PI) identified mass features of 216 proteins with significant differential signal patterns between preterm and term cohorts, which correlated to 85 proteins with putative differentially expressed mass signals based on a cutoff of p< 0.05. Study 2 (second pregnancies P2) identified mass features of 176 proteins with differential signal patterns between preterm and term cohorts using an ANOVA, which correlated to 54 proteins with differentially expressed mass signals based on a cutoff of p< 0.05. Within the proteins observed in the second pregnancies cohort (P2), 32 were observed to be present and statistically significant in the primagravida cohort (P1).

An independent statistical interrogation of the label free Elucidator data were reanalyzed by an independent third party blinded to outcome. These data revealed a total of 99 proteins that were statistically different in expression. From these 99, a total of 18 proteins were selected for validation in future prospective trials. Twelve of the 18 prioritized biomarkers are highly associated with preterm outcome (Table 1-2), while six of the 18 are associated with term outcome (Table 1-3).

**Table 1-2. Microparticle Preterm Birth Biomarkers**

| **Symbol** | **Protein Name** | **p Value** |
|---|---|---|
| A1BG | Alpha-1B-glycoprotein | 0.01 |
| A2M | Alpha-2-macroglobulin | 0.005 |
| ALB | Albumin | 0.01 |
| APOD | Apolipoprotein D | 0.05 |
| APOL1 | Apoliprotein L1 | 0.01 |
| AZGP1 | Zinc-alpha-2-glycoprotein | 0.05 |
| HPX | Hemopexin | 0.05 |
| IGHM1 | IgM Heavy Chain 1 (secreted) | 0.05 |
| IGHM2 | IgM Heavy Chain 2 (membrane-bound) | 0.01 |
| SERPINA1 | Alpha-1-anti-trypsin | 0.005 |
| SERPINC 1 | Anti-thrombin | 0.05 |
| TF | Transferrin | 0.05 |

**Table 1-3. Microparticle Term Birth Biomarkers**

| **Symbol** | **Protein Name** | **p Value** |
|---|---|---|
| C1R | Complement C1r | 0.005 |
| C3 | Complement C3 | 0.005 |
| C4B | Complement C4B | 0.01 |
| CFH | Complement Factor H | 0.005 |
| IGHA2 | IgA Heavy Chain | 0.05 |
| IGKVI-9 | Ig Kappa Variable 1-9 | 0.05 |

The multiplex analysis allowed for a differential characterization of proteins and patients into hierarchical relationships of cohort relevance. Table 1-4 summarizes the accuracy and false positive rates derived from the data. The multiplexed characterization of accuracy based upon cluster analysis revealed a high level of precision in accurately defining patients at risk as early as week 15 for unexpected preterm delivery prior to week 34. The associated probability for the described events has a sensitivity range from 85.4-97.9 % (41-47 accurate detection in 48 cases) depending upon the probability of error level set for the window of discrimination. The false positive rates ranged from 3-7 out of 48 samples evaluated, again depending upon the probability of error level chosen.

**Table 1-4. Predictive Value of Term and Spontaneous Preterm Birth Markers (N=48)**

| Probability Factors | Accurate Out of Expected | | Accurate Out of Actual | |
|---|---|---|---|---|
| | Term | SPTB | Term | SPTB |
| 0.05 | 20/24 | 21/24 | 20/23 | 21/25 |
| 0.01 | 22/24 | 22/24 | 22/24 | 22/24 |
| 0.005 | 23/24 | 23/24 | 24/25 | 23/23 |

Tables 1-5 and 1-6 summarize the functional analysis of the markers identified in both the term and SPTB cohorts. Using Ingenuity Systems Pathway Analysis Software (IPA, Redwood, CA, USA), a detailed comparison of the molecular pathway analysis of the microvesicle proteins expressed in each group revealed that the biomarkers in the SPTB population were associated with five major pathways and different markedly from the pattern in the term population when evaluated at weeks 15-17 of gestation. The focus molecules detected in the SPTB microparticles largely reflect immune, inflammatory and injury pathways. In contrast, the focus molecules in the term microparticles detected early in the second trimester were associated with cell-to-cell signaling.

**Table 1-5. Ingenuity Pathway Analysis in SPTB Microparticles**

| **ID** | **Molecules in Network** | **Score** | **Focus Molecule** | **Top Functions** |
|---|---|---|---|---|
| 1 | A1BG, AHSG, AMBP, APOD, C9, C4B (includes others), Complement component 1, Cytokeratin, elastase, F12, FBLN1, GC, HDL, HPR, HPX, lti, ITIH1, ITIH2, ITIH3, ITIH4, Kallikrein, KLKB1, KRT1, KRT2, KRT10, KRT6A, NFkB (complex), SERPINC1, SERPINF2, SERPING1, Tcf 1/3/4, trypsin, TTR, VTN | 67 | 26 | Antigen Presentation, Humoral Immune Response, Inflammatory Response |
| 2 | A2M, AFM, ALB, Ap1, collagen, Collagen type IV, Collagen(s), Creb, ERK1/2, Fibrinogen, FN1, GPIIB-IIIA, Growth hormone, Iga, Ige, IgG1, IgG, IGHM*, IGJ, IGLL1/IGLL5, IL1, Immunoglobulin, Insulin, Integrin alpha V beta 3, Laminin, LDH, LDL, LGALS3BP, LRP, ORM1/ORM2, S100A8, SERPINA1, TF, TGF beta, vWF | 29 | 13 | Organismal Injury and Abnormalities, Respiratory Disease, Tissue Morphology |
| 3 | Akt, Alpha catenin, AZGP1, CCL15, CD3, CD27, CHCHD2, chemokine, CPN2, HTRA1, IGLL1/IGLL5, IL17R, Integrin, Interferon alpha, Jnk, Mapk, MMP11, Mmp, Neurotropin, P38 MAPK, PAX3, PDESA, PI3K (complex), PIK3C2B, Pkc(s), PRSS1/PRSS3, Ptk, S100, S100A8, S100A9, S100B, SLC3A2, TFEC, TNFRSF4, Vegf | 9 | 5 | Cell-to-Cell Signaling and Interaction, Inflammatory Response, Cardiovascular Disease |
| 4 | APP, C2, C5, C6, C7, C8, C9, C4B (includes others), C5-C6-C7-C8-C9, C8A, C8B, C8G, CD59 (includes EG: 25407), Cd59a, CD5L, CFB, chondroitin sulfate B, CHRNA7, chymotrypsin, Complement compound 1, CPB2, CPN1, GPR77, HABP2, hemoglobin, IL1B, membrane attack, methylamine, PPP1R15A, PRDX2, S100B, Serine Protease, SMPD2, SYVN1, TNFAIP2 | 8 | 5 | Antigen Presentation, Humoral Immune Response, Inflammatory Response |
| 5 | ADCYAP1 (includes EG: 11516), ASIP, ATRN, COPSS | 2 | 1 | Cell-to-Cell Signaling and Interaction, Cellular Assembly and Organization, Cellular Growth and Proliferation |

**Table 1-6. Ingenuity Pathway Analysis in Term Microparticles**

| **ID** | **Molecules in Network** | **Score** | **Focus Molecule** | **Top Functions** |
|---|---|---|---|---|
| 1 | APCS, APOA1, APOB, APOH, C3, Clq, C1QC, C1R, CIS, C4B (includes others), C4BP, C4BPA, CFH, Collagen(s), Complement component 1, CP, Fibrin, Fibrinogen, HDL, IgG1, IgG, IgG2a, IGHG4, IGHM, IGKC, Igm, IL1, Immunoglobulin, LDL, LDL-cholesterol, LPA, NFkB (complex), PF4, Pro-inflammatory Cytokine, SYK/ZAP | 44 | 17 | Cell-To-Cell Signaling and Interaction, Tissue Development, Antigen Presentation |
| 2 | Akt, BAG4, C3, Cd55/Daf2, chemokine, CR1L, CXCL16, CXCR7, ERK, ERK1/2, F2, GALNT2, GEM, HABP2, IGHM, IL12 (family), immune, jnk, Mac1, MGAT3, MS4A1, P38 MAPK, PDGF BB, PI3K (complex), Pkc(s), PLA2G6, plasminogen activator, PPBP, PZP, Rac, SERPINF2, SLAMF1, TGFBI (includes EG: 21803), THBS1, Vegf | 12 | 6 | Inflammatory Response, Cellular Movement, Hematological System Development and Function |
| 3 | CD40, CDK2API, ELANE, FCAR, IGHA1, IGHA2, IL6, IL8, ITGAM (includes EG: 16409), ITGB2, MBL2, Mucin, NFKBIA, PI3K (complex), RELA, TCF3, TGF beta, TLR4, TNF | 4 | 2 | Cell- To-Cell Signaling and Interaction, Antigen Presentation, Inflammatory Response |

The use of extracellular vesicles circulating in high titer in blood as windows to understand disease processes has recently gained considerable attention in the field of molecular biology (Olver and Vidal, Subcell Biochem, 43:99-131, 2007; Simpson et al., Proteomics, 8:4083-99, 2008; Thery, F1000 Biol Rep, 3:15, 2011; and Pant et al., Biochem Pharmacol, 83:1484-94, 2012). The present study demonstrates the clinical utility of using circulating extracellular vesicles as enrichment factors to identify protein biomarkers indicative of risk for preterm birth. A key advantage in utilizing a microparticle-enriched fraction is that it contains protease-resistant microvesicles of endocytic origin (e.g., exosomes), which are thought to capture relevant biomarkers of disease processes (Simpson et al., supra 2008). In addition to containing nucleic acid and protein markers reflective of the tissue of origin, these shed particles have been reported to play an immunomodulatory role in normal pregnancy (Taylor et al., J Immunol, 176:1534-1542, 2006; Sabapatha et al., Am J Reprod Immunol, 56:345-55, 2006; and Atay et al., Am J Reprod Immunol, 65:65-77, 2011).

The present disclosure is an in-depth, open proteomic evaluation of microvesicular biomarkers harvested from maternal serum confirming the use of multiplexed proteins as early warning signals of preterm birth. It is of interest to note that the methods used in this example do not employ expensive protein extraction and affinity purification schemes such as affinity columns for the removal of the high abundant proteins (Tang et al., J Proteome Res, 10:4005-4017, 2011). Thus these methods allow for the rapid and cost effective isolation microparticles from a biological sample from a patient. In addition to the intrinsic benefit of studying microparticle proteomics, the fractionation process allows for detection of the sub proteome proteins outside the typical high abundant species. It is of interest to note that six (e.g., A1BG, APOD, APOL1, AZGP1, HPX and SERPINC1) of the prioritized twelve biomarkers associated with preterm birth are outside of the classical 20 species associated with high abundance proteins that define 97% of the proteome. Preferably, highly abundant plasma proteins such as albumin, IgG, IgA, IgM, IgD, transferrin, fibrinogen, alpha2-macroglobulin, alphal-antitrypsin, haptoglobin, alphal-acid glycoprotein, ceruloplasmin, APOA1, APOA2, APOB, C1q, C3, C4, plasminogen and prealbumin are not depleted from the blood sample.

The protein biomarkers characterized as discrete entities in this study have been previously described in circulating exosomes (www.exocarta.com) but have never been defined as unique or multiplexed markers associated with preterm birth. Several protein biomarkers identified in the present study have been previously defined as potential serum biomarkers in preterm birth. Previous interest in using α1 antitrypsin (Stella et al., Am J Obstet Gynecol, 201:387, 2009), anti-thrombin (Esplin et al., Am J Obstet Gynecol, 204:391, 2011) and alpha-2-macroglobulin in recurrent pregnancy loss (Saunders et al., Am J Reprod Immunol, 68:438-49, 2012) as circulating biomarker has been reported. The present study represents the first demonstration of differential expression of these and other proteins as markers for preterm risk as early as weeks 15 to 17 of gestation in an asymptomatic population.

The present disclosure confirms the utility of a rapid isolation from sera (30 minutes versus classical 30 hour extractions) of circulating microparticles. Unique protein biomarkers were evaluation using standard LC/MS procedures amenable to a routine CLIA facility. The intended use of such an assay is to be utilized as a non-invasive test to identify pregnant women at risk for premature birth as early as 15 weeks gestation. This would represent a first in human test utilizing a standard blood sample taken between weeks 15 and 17 of gestation. The 1 ml maternal serum sample is sent to a central lab for LC/MS processing and risk results provided in 2-4 days with a targeted risk profile for the later onset of preterm birth. Importantly, this would permit the health care provider and the pregnant subject to take steps to reduce her risk of preterm birth.

## Claims

1. A method for assessing risk of preterm birth for a pregnant subject, the method comprising:
(a) preparing a microparticle-enriched fraction from a sample from the pregnant subject, wherein the preparing step comprises_using size-exclusion chromatography with an agarose solid phase and an aqueous liquid phase;
(b) detecting a level of two or more proteins in the fraction, wherein the two or more proteins are
one or more proteins of the preterm group subset consisting of hemopexin (HPX), alpha-1B-glycoprotein (A1BG), serum albumin (ALB), apolipoprotein D (APOD), apolipoprotein L1 (APOL1), zinc-alpha-2-glycoprotein (AZGP1), Ig heavy chain mu 1 (IGHM1), IgM heavy chain mu 2 (IGHM2), and serotransferrin (TF), wherein the protein or at least one of the proteins is HPX, and
one or more proteins of the term group subset consisting of complement component 1r (Clr), complement component 3 (C3), complement component 4B (C4B), complement factor H (CFH), Ig heavy chain alpha 2 (IGHA2), and Ig kappa variablel-9 (IGKV1-9); and
(c) determining that the pregnant subject is at an increased risk of preterm birth when the level of one or more proteins of the preterm birth group consisting of A1BG, ALB, APOD, APOL1, AZGP1, HPX, IGHM1, IGHM2, and TF is above a threshold level, and/or when the level of one or more of proteins of the term birth group consisting of C1R, C3, C4B, CFH, IGHA2, and IGKV1-9 is below a threshold level; or
determining that the pregnant subject is not at an increased risk of preterm birth when the level of one or more proteins of the preterm birth group consisting of A1BG, ALB, APOD, APOL1, AZGP1, HPX, IGHM1, IGHM2 and TF, is below a threshold level, and/or when the level of one or more of proteins of the term birth group consisting of C1R, C3, C4B, CFH, IGHA2, IGKV1-9, is above a threshold level.

2. The method of claim 1, further comprising:
(b2) detecting a level of one or more further proteins in the fraction from the pregnant subject, wherein the one or more further proteins are selected from the group consisting of A2M, APOB, C9, F2, FBLN1, FN1, FNls, GC, ITIH4, KLKB1, ORM1, ORM2, S100A8, S100A9, SERPINA1, SERPINC1, THBS1, and TTR; and
(c2) determining that the pregnant subject is at an increased risk of preterm birth when the level of one or more further proteins of the further preterm birth group consisting of A2M, C9, FBLN1, FN1, FN1s, GC, ITIH4, KLKB1, ORM1, ORM2, S100A8, S100A9, SERPINA1, SERPINC1, and TTR, is above a threshold level, and/or when the level of one or more of further proteins of the further term birth group consisting of APOB, C9, and THBS1 is below a threshold level in the sample.

3. The method of claim 1 or claim 2, wherein the detecting step comprises detecting the level of all twelve of alpha-IB-glycoprotein (A1BG), alpha-2-macroglobulin (A2M), serum albumin (ALB), apolipoprotein D (APOD), apolipoprotein L1 (APOL1), zinc-alpha-2-glycoprotein (AZGP1), hemopexin (HPX), Ig heavy chain mu 1 (IGHM1), IgM heavy chain mu 2 (IGHM2), alpha-1-antitrypsin (SERPINA1), antithrombin-III (SERPINC1), and serotransferrin (TF).

4. The method of any of the preceding claims, wherein the detecting step comprises detecting the level of all six of complement component 1r (Clr), complement component 3 (C3), complement component 4B (C4B), complement factor H (CFH), Ig heavy chain alpha 2 (IGHA2), and Ig kappa variablel-9 (IGKV1-9).

5. The method of any of the preceding claims, comprising a further step after (c) of providing a risk score by enumerating the preterm birth markers above the threshold that were detected and the term birth markers below the threshold that were detected.

6. The method of any of the preceding claims, wherein the pregnant subject is a primigravida woman or a multigravida woman.

7. The method of any of the preceding claims, wherein the sample is taken from the pregnant subject during the second trimester.

8. The method of any of the preceding claims, wherein the sample is taken from the pregnant subject within 15 to 17 weeks of gestation.

9. The method of any of the preceding claims, wherein the sample is serum or plasma.

10. The method of any of the preceding claims, wherein the preparing step further comprises using ultrafiltration.

11. The method of any of the preceding claims, wherein the size exclusion chromatography comprises an agarose solid phase and an aqueous liquid phase, and optionally wherein the aqueous liquid phase is water.

12. The method of any of the preceding claims, wherein the detecting step comprises measuring binding of an antibody specific to each of the one or more proteins, and optionally wherein the detecting step comprises a technique selected from the group consisting of enzyme-linked immunosorbent assay (ELISA), western blot and antibody microarray.

13. The method of any of the preceding claims, wherein the detecting step comprises liquid chromatography/mass spectrometry (LC/MS).

## Patentansprüche

1. Verfahren zur Beurteilung des Risikos einer Frühgeburt für eine Schwangere, wobei das Verfahren Folgendes umfasst:
(a) Herstellung einer mit Mikropartikeln angereicherten Fraktion aus einer Probe von der Schwangeren, wobei der Herstellungsschritt die Verwendung der Größenausschlusschromatographie mit einer Agarose-Festphase und einer wässrigen Flüssigphase umfasst;
(b) Nachweis eines Spiegels von zwei oder mehr Proteinen in der Fraktion, wobei die zwei oder mehr Proteine
ein oder mehr Protein(e) von der Untergruppe einer Frühgeburtengruppe sind, bestehend aus Hämopexin (HPX), Alpha-1B-Glykoprotein (AlBG), Serumalbumin (ALB), Apolipoprotein D (APOD), Apolipoprotein L1 (APOL1), Zink-Alpha-2-Glykoprotein (AZGP1), der Ig-Schwerkette my 1 (IGHM1), der IgM-Schwerkette my 2 (IGHM2) und Serotransferrin (TF), wobei das Protein oder mindestens eines der Proteine HPX ist, und
ein oder mehr Protein(e) von der Untergruppe einer termingerechten Geburtengruppe, bestehend aus der Komplementkomponente lr (Clr), der Komplementkomponente 3 (C3), der Komplementkomponente 4B (C4B), dem Komplementfaktor H (CFH), der Ig-Schwerkette alpha 2 (IGHA2) und der Ig-kappa-Variablen 1-9 (IGKV1-9); und
(c) Bestimmung, dass bei der Schwangeren ein erhöhtes Risiko für eine Frühgeburt vorliegt, wenn der Spiegel von einem oder mehr Protein(en) von der Frühgeburtengruppe, bestehend aus A1BG, ALB, APOD, APOL1, AZGP1, HPX, IGHM1, IGHM2 und TF, über einem Grenzwert liegt, und/oder wenn der Spiegel von einem oder mehr Protein(en) von der termingerechten Geburtengruppe, bestehend aus C1R, C3, C4B, CFH, IGHA2 und IGKV1-9, unter einem Grenzwert liegt; oder
Bestimmung, dass bei der Schwangeren kein erhöhtes Risiko für eine Frühgeburt vorliegt, wenn der Spiegel von einem oder mehr Protein(en) von der Frühgeburtengruppe, bestehend aus A1BG, ALB, APOD, APOL1, AZGP1, HPX, IGHM1, IGHM2 und TF, unter einem Grenzwert liegt, und/oder wenn der Spiegel von einem oder mehr Protein(en) von der termingerechten Geburtengruppe, bestehend aus C1R, C3, C4B, CFH, IGHA2, IGKV1-9, über einem Grenzwert liegt.

2. Verfahren nach Anspruch 1, weiter umfassend:
(b2) Nachweis eines Spiegels von einem oder mehr weiteren Protein(en) in der Fraktion von der Schwangeren, wobei das eine oder mehr weitere Protein(e) aus der Gruppe ausgewählt ist/sind, bestehend aus A2M, APOB, C9, F2, FBLN1, FN1, FNls, GC, ITIH4, KLKB1, ORM1, ORM2, S100A8, S100A9, SERPINA1, SERPINC1, THBS1 und TTR; und
(c2) Bestimmung, dass die Schwangere ein erhöhtes Risiko für eine Frühgeburt aufweist, wenn der Spiegel von einem oder mehr weiteren Protein(en) von der weiteren Frühgeburtengruppe, bestehend aus A2M, C9, FBLN1, FN1, FNls, GC, ITIH4, KLKB1, ORM1, ORM2, S100A8, S100A9, SERPINA1, SERPINC1 und TTR, über einem Grenzwert liegt, und/oder wenn der Spiegel von einem oder mehr weiteren Protein(en) der weiteren termingerechten Geburtengruppe, bestehend aus APOB, C9 und THBS1, unter einem Grenzwert in der Probe liegt.

3. Verfahren nach Anspruch 1 oder Anspruch 2, wobei der Nachweisschritt den Nachweis des Spiegels von allen zwölf von Folgendem umfasst: Alpha-1B-Glykoprotein (A1BG), Alpha-2-Makroglobulin (A2M), Serumalbumin (ALB), Apolipoprotein D (APOD), Apolipoprotein L1 (APOL1), Zink-Alpha-2-Glykoprotein (AZGP1), Hämopexin (HPX), Ig-Schwerketten my 1 (IGHM1), IgM-Schwerketten my 2 (IGHM2), Alpha-1-Antitrypsin (SERPINA1), Antithrombin III (SERPINC1) und Serotransferrin (TF).

4. Verfahren nach einem der vorangehenden Ansprüche, wobei der Nachweisschritt den Nachweis des Spiegels von allen sechs von Folgendem umfasst: der Komplementkomponente lr (Clr), der Komplementkomponente 3 (C3), der Komplementkomponente 4B (C4B), dem Komplementfaktor H (CFH), der Ig-Schwerkette-Alpha-2 (IGHA2) und der Ig-kappa-Variablen 1-9 (IGKV1-9).

5. Verfahren nach einem der vorangehenden Ansprüche, umfassend einen weiteren Schritt nach (c) zur Bereitstellung eines Risikoscores mittels Aufzählen der Frühgeburtenmarker über dem Grenzwert und der Frühgeburtenmarker unter dem Grenzwert, die nachgewiesen wurden.

6. Verfahren nach einem der vorangehenden Ansprüche, wobei die Schwangere eine erstgebärende Frau oder eine mehrgebärende Frau ist.

7. Verfahren nach einem der vorangehenden Ansprüche, wobei der Schwangeren die Probe während des zweiten Trimenons entnommen wird.

8. Verfahren nach einem der vorangehenden Ansprüche, wobei der Schwangeren die Probe innerhalb der 15. bis 17. Schwangerschaftswoche entnommen wird.

9. Verfahren nach einem der vorangehenden Ansprüche, wobei die Probe Serum oder Plasma ist.

10. Verfahren nach einem der vorangehenden Ansprüche, wobei der Herstellungsschritt weiter die Verwendung von Ultrafiltration umfasst.

11. Verfahren nach einem der vorangehenden Ansprüche, wobei die Größenausschlusschromatographie eine Agarose-Festphase und eine wässrige Flüssigphase umfasst, und gegebenenfalls wobei die wässrige Flüssigphase Wasser ist.

12. Verfahren nach einem der vorangehenden Ansprüche, wobei der Nachweisschritt das Messen der Bindung eines Antikörpers, spezifisch an jedes von dem einen oder mehr Protein(en) umfasst, und gegebenenfalls wobei der Nachweisschritt ein Verfahren umfasst, das aus der Gruppe ausgewählt ist, bestehend aus dem Enzyme-linked Immunosorbent Assay (ELISA), dem Western Blot und dem Antikörper-Mikroarray.

13. Verfahren nach einem der vorangehenden Ansprüche, wobei der Nachweisschritt die Flüssigkeitschromatographie/Massenspektrometrie (LC/MS) umfasst.

## Revendications

1. Méthode d'évaluation du risque de naissance prématurée chez un sujet enceinte, la méthode comprenant les étapes qui consistent à :
(a) préparer une fraction enrichie en microparticules à partir d'un échantillon obtenu chez le sujet enceinte, l'étape de préparation comprenant l'utilisation d'une chromatographie d'exclusion stérique avec une phase solide à base d'agarose et une phase liquide aqueuse ;
(b) détecter un taux de deux ou plusieurs protéines dans la fraction, les deux ou plusieurs protéines étant :
une ou plusieurs protéines d'un sous-ensemble du groupe « naissance prématurée » consistant en les suivantes : hémopexine (HPX), alpha-lB-glycoprotéine (AlBG), albumine sérique (ALB), apolipoprotéine D (APOD), apolipoprotéine L1 (APOL1), zinc-alpha-2-glycoprotéine (AZGP1), région mu 1 de la chaîne lourde des Ig (IGHM1), région mu 2 de la chaîne lourde des IgM (IGHM2) et sérotransferrine (TF), où la protéine ou au moins une des protéines est l'HPX et
une ou plusieurs protéines d'un sous-ensemble du groupe « naissance à terme » consistant en les suivantes : composant 1R du complément (C1R), composant 3 du complément (C3), composant 4B du complément (C4B), facteur H du complément (CFH), région alpha 2 de la chaîne lourde des Ig (IGHA2) et région variable kappa 1-9 des Ig (IGKV1-9) ; et
(c) établir que le sujet enceinte est à risque accru de naissance prématurée quand le taux de une ou plusieurs protéines du groupe « naissance prématurée » consistant en A1BG, ALB, APOD, APOL1, AZGP1, HPX, IGHM1, IGHM2 et TF est en dessus d'un niveau seuil et/ou quand le taux de une ou plusieurs protéines du groupe « naissance à terme » consistant en C1R, C3, C4B, CFH, IGHA2 et IGKV1-9 est en dessous d'un niveau seuil ; ou
établir que le sujet enceinte n'est pas à risque accru de naissance prématurée quand le taux de une ou plusieurs protéines du groupe « naissance prématurée » consistant en A1BG, ALB, APOD, APOL1, AZGP1, HPX, IGHM1, IGHM2 et TF est en dessous d'un niveau seuil et/ou quand le taux de une ou plusieurs protéines du groupe « naissance à terme » consistant en C1R, C3, C4B, CFH, IGHA2 et IGKV1-9 est en dessus d'un niveau seuil.

2. Méthode de la revendication 1 comprenant en outre les étapes consistant à :
(b2) détecter un taux de une ou plusieurs protéines supplémentaires dans la fraction obtenue à partir du sujet enceinte, les une ou plusieurs protéines supplémentaires étant sélectionnées dans le groupe consistant en les suivantes : A2M, APOB, C9, F2, FBLN1, FN1, FN1s, GC, ITIH4, KLKB1, ORM1, ORM2, S100A8, S100A9, SERPINA1, SERPINC1, THBS1 et TTR ; et
(c2) établir que le sujet enceinte est à risque accru de naissance prématurée quand le taux de une ou plusieurs protéines supplémentaires du second groupe « naissance prématurée » consistant en A2M, C9, FBLN1, FN1, FN1s, GC, ITIH4, KLKB1, ORM1, ORM2, S100A8, S100A9, SERPINA1, SERPINC1 et TTR est en dessus d'un niveau seuil et/ou quand le taux de une ou plusieurs protéines supplémentaires du second groupe « naissance à terme » consistant en APOB, C9 et THBS1 est en dessous d'un niveau seuil dans l'échantillon.

3. Méthode de la revendication 1 ou de la revendication 2, où l'étape de détection comprend la détection des taux de l'ensemble des douze marqueurs suivants : alpha-lB-glycoprotéine (AlBG), alpha-2-macroglobuline (A2M), albumine sérique (ALB), apolipoprotéine D (APOD), apolipoprotéine L1 (APOL1), zinc-alpha-2-glycoprotéine (AZGP1), hémopexine (HPX), région mu 1 de la chaîne lourde des Ig (IGHM1), région mu 2 de la chaîne lourde des IgM (IGHM2), alpha-1-antitrypsine (SERPINA1), antithrombine-III (SERPINC1) et sérotransferrine (TF).

4. Méthode de l'une quelconque des revendications précédentes, où l'étape de détection comprend la détection des taux de l'ensemble des six marqueurs suivants : composant 1R du complément (C1R), composant 3 du complément (C3), composant 4B du complément (C4B), facteur H du complément (CFH), région alpha 2 de la chaîne lourde des Ig (IGHA2) et région variable kappa 1-9 des Ig (IGKV1-9).

5. Méthode de l'une quelconque des revendications précédentes comprenant, après (c), une étape supplémentaire consistant à fournir un score du risque en énumérant les marqueurs de naissance prématurée dont le taux est en dessus du seuil qui sont détectés et les marqueurs de naissance à terme dont le taux est en dessous du seuil qui sont détectés.

6. Méthode de l'une quelconque des revendications précédentes, où le sujet enceinte est une femme primipare ou une femme multipare.

7. Méthode de l'une quelconque des revendications précédentes, où l'échantillon est obtenu chez le sujet enceinte pendant le deuxième trimestre.

8. Méthode de l'une quelconque des revendications précédentes, où l'échantillon est obtenu chez le sujet enceinte aux semaines 15 à 17 de la grossesse.

9. Méthode de l'une quelconque des revendications précédentes, où l'échantillon est le sérum ou le plasma.

10. Méthode de l'une quelconque des revendications précédentes, où l'étape de préparation comprend en outre l'utilisation d'une ultrafiltration.

11. Méthode de l'une quelconque des revendications précédentes, où la chromatographie d'exclusion stérique comprend une phase solide à base d'agarose et une phase liquide aqueuse, la phase liquide aqueuse étant éventuellement l'eau.

12. Méthode de l'une quelconque des revendications précédentes, où l'étape de détection comprend une mesure de la fixation d'un anticorps spécifique de chacune des une ou plusieurs protéines, et éventuellement où l'étape de détection comprend une technique sélectionnée dans le groupe consistant en un dosage d'immunosorption liée à enzyme (méthode ELISA), un Western blot et une puce à anticorps.

13. Méthode de l'une quelconque des revendications précédentes, où l'étape de détection comprend une chromatographie liquide/spectrométrie de masse (CL/SM).
